# EUROPEAN PATENT APPLICATION

(11) **EP 2 042 500 A1**
(43) Date of publication of application: **01.04.2009**
(21) Application number: 07075832.1
(22) Date of filing: 21.09.2007
(51) Int. Cl.: C07D 471/04, A61K 31/437, A61P 9/00, A61P 35/00, A61P 17/06

(54) **N-[4-(1-H-pyrazolo[3,4-c]pyridin-4-yl)phenyl]-N'-[(hetero)aryl]-urea compounds, pharmaceutical compositions comprising them, their preparation and their use as Tie2-kinase inhibitors**

(71) Applicant: Bayer Schering Pharma Aktiengesellschaft, 13353 Berlin (DE)
(72) Inventor: Hartung, Ingo, 13469 Berlin (DE); Kettschau, Georg, 13187 Berlin (DE); Schumann, Ingrid, 14052 Berlin (DE); Thierauch, Karl-Heinz, 14169 Berlin (DE); Boemer, Ulf, 16548 Glienicke/ Nordbahn (DE)

(57) **Abstract**

The invention relates to 3-H-pyrazolopyridines according to the general formula (I): 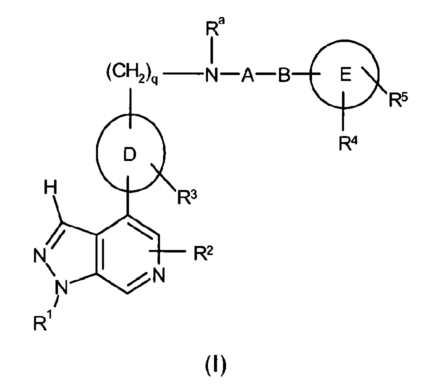 in which A, B, D, E, R^{a}, R¹, R², R³, R⁴, R⁵ and q are as defined in the claims, and salts, N-oxides, solvates and prodrugs thereof, to pharmaceutical compositions comprising said 3-H-pyrazolopyridine compounds, to methods of preparing said 3-H-pyrazolopyridines, to intermediate compounds useful in said methods, to uses of said intermediate compounds in the preparation of said 3-H-pyrazolopyridines, as well as to uses of said 3-H-pyrazolopyridines for manufacturing a pharmaceutical composition for the treatment of diseases of dysregulated vascular growth or of diseases which are accompanied with dysregulated vascular growth, wherein the compounds effectively interfere with Tie2 signalling.

## Description

The present invention relates to 3-H-pyrazolopyridine compounds of general formula (I) and salts, N-oxides, solvates and prodrugs thereof, to pharmaceutical compositions comprising said 3-H-pyrazolopyridine compounds, to methods of preparing said 3-H-pyrazolopyridines, to intermediate compounds useful in said methods, to uses of said intermediate compounds in the preparation of said 3-H-pyrazolopyridines, as well as to uses of said 3-H-pyrazolopyridines.

### SCIENTIFIC BACKGROUND

Dysregulated vascular growth plays a critical role in a variety of inflammatory diseases, in particular psoriasis, delayed type hypersensitivity, contact dermatitis, asthma, multiple sclerosis, restenosis, rheumatoid arthritis and inflammatory bowl disease. Aberrant vascular growth is also involved in neovascular ocular diseases such as age-related macular degeneration and diabetic retinopathy. Additionally, sustained vascular growth is accepted as one hallmark of cancer development (Hanahan, D.; Weinberg, R. A. Cell 2000, 100, 57). While tumors initially grow either as an avascular mass or by co-opting existing host vessels, growth beyond a few mm³ in size is depending on the induction of vessel neogrowth in order to sufficiently provide the tumor with oxygen and nutrients. Induction of angiogenesis is a prerequisite that the tumor surpasses a certain size (the so called angiogenic switch). An intricate signalling interaction network between cancer cells and the tumor microenvironment triggers the induction of vessel growth from existing vasculature. The dependence of tumors on neovascularization has led to a new treatment paradigm in cancer therapy (Ferrara et al. Nature 2005, 438, 967; Carmeliet Nature 2005, 438, 932). Blocking tumor neovascularization by small molecule or antibody-mediated inhibition of relevant signal transduction pathways holds a great promise for extending currently available therapy options.

The development of the cardiovascular system involves two basic stages. In the initial vasculogenesis stage, which only occurs during embryonal development, angioblasts differentiate into endothelial cells which subsequently form a primitive vessel network. The subsequent stage, termed angiogenesis, involves the remodeling of the initial vasculature and sprouting of new vessels (Risau, W. Nature 1997, 386, 671; Jain, R. K. Not. Med. 2003, 9, 685). Physiologically, angiogenesis occurs in wound healing, muscle growth, the female cycle and in the above mentioned disease states.

It has been found that receptor tyrosine kinases of the vascular endothelial growth factor (VEGF) family and the Tie (tyrosine kinase with immunoglobulin and epidermal growth factor homology domain) receptor tyrosine kinases are essential for both developmental and disease-associated angiogenesis (Ferrara et al Nat. Med. 2003, 9, 669; Dumont et al. Genes Dev. 1994, 8, 1897; Sato et al. Nature 1995, 376, 70).

In adults the Tie2 receptor tyrosine kinase is selectively expressed on endothelial cells (EC) of the adult vasculature (Schlaeger et al. Proc. Nat. Acad. Sci. USA 1997, 94, 3058). Immunohistochemical analysis demonstrated the expression of Tie2 in adult rat tissues undergoing angiogenesis. During ovarian folliculogenesis, Tie2 is expressed in neovessels of the developing corpus luteum. Four endogeneous ligands - angiopoietins 1 to 4 - have been identified for the type 1 transmembrane Tie2 (also named Tek) receptor, while no ligands have been identified so far for the Tie1 receptor. Binding of the extracellular Tie2 domain to the C-terminal fibrinogen-like domains of the various angiopoietins leads to significantly different cellular effects. In addition, heterodimerizations between Tie1 and Tie2 receptors have been postulated to influence ligand binding.

Binding of Ang1 to Tie2 expressed on EC induces receptor cross-phosphorylation and kinase activation thus triggering various intracellular signalling pathways. The intracellular C-terminal tail of the Tie2 protein plays a crucial role in Tie2 signalling (Shewchuk et al. Structure 2000, 8, 1105). Upon ligand binding, a conformational change is induced which removes the C-tait out of its inhibitory conformation thus allowing kinase activation by cross-phoshorylation of various Tyr residues in the C-tail, which subsequently function as docking sites for phosphotyrosine-binding (PTB) site possessing down-stream mediators. Cellular effects initiated by Ang1 activation of Tie2 include inhibition of EC apoptosis, stimulation of EC migration and blood vessel reorganization, suppression of inflammatory gene expression and suppression of vascular permeability (Brindle et al. Circ. Res. 2006, 98, 1014). In contrast to VEGF-VEGFR signalling in EC, Ang1 activation of Tie2 does not stimulate EC proliferation in the majority of published assay settings.

The anti-apoptotic effect of Tie2 signalling was shown to be mediated mainly by the PI3K-Akt signalling axis which is activated by binding of the regulatory p85 subunit of PI3K to Y1102 in the Tie2 C-tail (DeBusk et al. Exp. Cell. Res. 2004, 298, 167; Papapetropoulos et al. J. Biol. Chem. 2000, 275, 9102; Kim et al. Circ. Res. 2000, 86, 24). In contrast, the chemotactic response downstream of the activated Tie2 receptor requires crosstalk between PI3K and the adaptor protein Dok-R. Membrane localization of Dok-R via binding of its pleckstrin homology (PH) domain to PI3K and simultaneous binding to Y1108 in the Tie2 C-tail via its PTB domain leads to Dok-R phoshorylation and downstream signalling via Nck and Pak-1 (Jones et al. Mol. Cell Biol. 2003, 23, 2658; Master et al. EMBO J. 2001, 20, 5919). PI3K-mediated recruitment of the adaptor protein ShcA to Y1102 of the Tie2 C-tail is also believed to induce cellular sprouting and motility effects involving activation of endothelial nitric oxide synthase (eNOS), focal adhesion kinase (FAK) and the GTPases RhoA and Rac1. Other downstream mediators of Tie2 signalling include the adaptor protein Grb2, which mediates Erk1/2 stimulation, and the SHP-2 phosphatase.

In conclusion, basal activation of the Tie2 pathway by Ang1 is believed to maintain quiescence and integrity of the endothelium of the adult vasculature by providing a cell survival signal for ECs and by maintaining the integrity of the EC lining of blood vessels (Peters et al. Recent Prog. Horm. Res. 2004, 59, 51).

In contrast to Ang1, Ang2 is not able to activate Tie2 on EC unless Ang2 is present in high concentration or for prolonged periods. However, Ang2 functions as a Tie2 agonist in non-endothelial cells transfected with Tie2. The structural basis for this context-dependence of the Ang2-Tie2 interaction is to date not understood.

In endothelial cells, however, Ang2 functions as Tie2 antagonist and thus blocks the agonistic activity of Ang1 (Maisonpierre et al. Science 1997, 277, 55). Ang2 binding to Tie2 prevents Ang1-mediated Tie2 activation which leads to vessel destabilization and results in vessel regression in the absence of pro-angiogenic stimuli such as VEGF. While Ang1 is widely expressed by periendothelial cells in quiescent vasculature such as pericytes or smooth muscle cells, Ang2 expression occurs in areas of ongoing angiogenesis. Ang2 can be stored in Weibel-Palade bodies in the cytoplasm of EC allowing for a quick vascular response upon stimulation.

Ang1 and Ang2 are expressed in the corpus luteum, with Ang2 localizing to the leading edge of proliferating vessels and Ang1 localizing diffusively behind the leading edge. Ang2 expression is *inter alia* initiated by hypoxia (Pichiule et al. J. Biol. Chem. 2004, 279, 12171). Ang2 is upregulated in the tumor vasculature and represents one of the earliest tumor markers. In the hypoxic tumor tissue, Ang2 expression induces vessel permeability and - in the presence of e.g. pro-angiogenic VEGF - triggers angiogenesis. After VEGF mediated EC proliferation and vessel sprouting maturation of the newly formed vessels again necessitates Tie2 activation by Ang1. Therefore, a subtle balancing of Tie2 activity plays a pivotal role in the early as well as late stages of neovascularization. These observations render the Tie2 RTK an attractive target for anti-angiogenesis therapy in diseases caused by or associated with dysregulated vascular growth. However, it remains to be shown if targeting the Tie2 pathway alone will be sufficient to achieve efficacious blockade of neovascularization. In certain diseases or disease subtypes it might be necessary or more efficacious to block several angiogenesis-relevant signalling pathways simultaneously. In certain oncological diseases it might be beneficial to block angiogenesis-relevant signalling pathways and, in addition, certain kinases, the activity of which plays a critical role for cancer cell proliferation and/or invasion of surrounding tissues by cancer cells. For example, activity of Ret kinase has been shown to play a critical role in certain forms of e.g. thyroid cancer. Simultaneous inhibition of angiogenesis-relevant signalling pathways, for example of Tie2 signalling, and Ret kinase activity may therefore be more efficacious for the treatment of these forms of e.g. thyroid cancer than inhibition of angiogenesis alone. The tyrosine kinase TrkB is often overexpressed in human cancers and it has been implied that TrkB signalling promotes tumor formation and metastasis (see for example Desmet, C. J.; Peeper, D. S. Cell. Mol. Life Sci. 2006, 63, 755). Simultaneous inhibition of angiogenesis-relevant signalling pathways, for example of Tie2 signalling, and TrkB kinase activity may therefore be more efficacious for treating tumors, which are characterized by increased TrkB activity, than inhibition of angiogenesis alone.

Various theories have been discussed to explain the differential effects of Ang1 and Ang2 on Tie2 downstream signalling events. Binding of Ang1 and Ang2 in a structurally different manner to the Tie2 ectodomain could induce ligand-specific conformational changes of the intracellular kinase domain explaining different cellular effects. Mutational studies however point toward similar binding sites of Ang1 and Ang2. In contrast, various publications have focussed on different oligomerization states of Ang1 vs. Ang2 as basis for different receptor multimerization states upon ligand binding. Only Ang1 present in its tetramer or higher-order structure initiates Tie2 activation in EC while Ang2 was reported to exist as a homodimer in its native state (Kim et al. J. Biol. Chem. 2005, 280, 20126; Davis et al. Nat. Struc. Biol. 2003, 10, 38; Barton et al. Structure 2005, 13, 825). Finally, specific interactions of Ang1 or Ang2 with additional cell-specific co-receptors could be responsible for the different cellular effects of Ang1 vs. Ang2 binding to Tie2. Interaction of Ang1 with integrin α5β1 has been reported to be essential for certain cellular effects (Carlson et al. J. Biol. Chem. 2001, 276, 26516; Dallabrida et al. Circ. Res. 2005, 96, e8). Integrin α5β1 associates constitutively with Tie2 and increases the receptor's binding affinity for Ang1 resulting in initiation of downstream signalling at lower Ang1 effector concentrations in situations where integrin α5β1 is present. The recently solved crystal structure of the Tie2-Ang2 complex suggests however that neither the oligomerization state nor a different binding mode causes the opposing cellular effects (Barton et al. Nat. Struc. Mol. Biol. 2006, advance online publication).

Ang1-Tie2 signalling plays also a role in the development of the lymphatic system and in lymphatic maintenance and sprouting (Tammela et al. Blood 2005, 105, 4642). An intimate cross-talk between Tie2 and VEGFR-3 signalling in lymphangiogenesis seems to equal the Tie2-KDR cross-talk in blood vessel angiogenesis.

A multitude of studies have underscored the functional significance of Tie2 signalling in the development and maintenance of the vasculature. Disruption of Tie2 function in Tie2^{-/-} transgenic mice leads to early embryonic lethality between days 9.5 and 12.5 as a consequence of vascular abnormalities. Tie2^{-/-} embryos fail to develop the normal vessel hierachy suggesting a failure of vascular branching and differentiation. The heart and vessels in Tie2^{-/-} embryos show a decreased lining of EC and a loosened interaction between EC and underlying pericyte/smooth muscle cell matrix. Mice lacking functional Ang1 expression and mice overexpressing Ang2 display a phenotype reminiscent of the phenotype of Tie2^{-/-} mice (Suri et al. Cell 1996, 87, 1171). Ang2^{-/-} mice have profound defects in the growth and patterning of lymphatic vasculature and fail to remodel and regress the hyaloid vasculature of the neonatal lens (Gale et al. Dev. Cell 2002, 3, 411). Ang1 rescued the lymphatic defects, but not the vascular remodeling defects. Therefore, Ang2 might function as a Tie2 antagonist in blood vasculature but as a Tie2 agonist in developing lymph vasculature suggesting redundant roles of Ang1 and Ang2 in lymphatic development.

Aberrant activation of the Tie2 pathway is involved in various pathological settings. Activating Tie2 mutations leading to increased ligand-dependent and ligand-independent Tie2 kinase activity cause inherited venous malformations (Vikkula et al. Cell 1996, 87, 1181). Increased Ang1 mRNA and protein levels as well as increased Tie2 activation have been reported in patients with pulmonary hypertension (PH). Increased pulmonary arterial pressure in PH patients results from increased coverage of pulmonary arterioles with smooth muscle cells (Sullivan et al. Proc. Natl. Acad. Sci. USA 2003, 100, 12331). In chronic inflammatory diseases, like in psoriasis, Tie2 and the ligands Ang1 and Ang2 are greatly upregulated in lesions, whereas a significant decrease in expression of Tie2 and ligands occur under anti-psoriatic treatment (Kuroda et al. J. Invest. Dermatol 2001, 116, 713). Direct association of pathogenesis of disease with Tie2 expression has been demonstrated recently in transgenic mice overexpressing Tie2 (Voskas et al. Am. J. Pathol. 2005, 166, 843). In these mice overexpression of Tie2 causes a psoriasis-like phenotype (such as epidermal thickening, rete ridges and lymphocyte infiltration).These skin abnormalities are resolved completely upon suppression of transgene expression, thereby illustrating a complete dependence on Tie2 signalling for disease maintenance and progression. A recent study underscored the connection of the Ang1/Ang2-Tie2 signalling axis to the induction of inflammation (Fiedler et al. Nat. Med. 2006, 12, 235). Inhibition of the Tie2 signalling pathway is therefore expected to be useful in the therapy of a broad range of inflammatory diseases.

Tie2 expression was investigated in human breast cancer specimens and Tie2 expression was found in the vascular endothelium both in normal breast tissue as well as in tumor tissue. The proportion of Tie2-positive microvessels was increased in tumors as compared to normal breast tissue (Peters et at. Br. J. Canc. 1998, 77, 51). However, significant heterogeneity in endothelial Tie2 expression was observed in clinical specimen from a variety of human cancers (Fathers et al. Am. J. Path. 2005, 167, 1753). In contrast, Tie2 and angiopoietins were found to be highly expressed in the cytoplasm of human colorectal adenocarcinoma cells indicating at the potential presence of an autocrine/paracrine growth loop in certain cancers (Nakayama et al. World J. Gastroenterol. 2005, 11, 964). A similar autocrine/paracrine Ang1-Ang2-Tie2 loop was postulated for certain human gastric cancer cell lines (Wang et al. Biochem. Biophys. Res. Comm. 2005, 337, 386). In addition, it was observed clinically that Ang2 is overexpressed in the bone marrow of AML (acute myelogenous leukemia) patients and Tie2 is additionally overexpressed in leukemic blasts (Schliemann *et al*. **2006,** *91*, 1203). Taking into account that Ang1-Tie2 signalling regulates hematopoietic stem cell quiescence in the bone marrow niche, Tie2 inhibition would therefore force promyeloid cells into differentiation resulting in purging the bone marrow from leukemic precursor cells (Arai et al. Cell 2004, 118, 149).

The relevance of the Ang1-Tie2 signalling axis was challenged with various biochemical techniques. Inhibition of Ang1 expression by an antisense RNA approach resulted in decreased xenograft tumor growth (Shim et al. Int. J. Canc. 2001, 94, 6 ; Shim et al. Exp. Cell Research 2002, 279, 299). However, other studies report that experimental overexpression of Ang1 in tumor models leads to decreased tumor growth (Hayes et al. Br. J. Canc. 2000, 83, 1154; Hawighorst et al. Am. J. Pathol. 2002, 160, 1381; Stoeltzing et al. Cancer Res. 2003, 63, 3370). The latter results can be rationalized by the ligand's ability to stabilize the endothelial lining of vessels rendering vessels less sensitive for angiogenic stimuli. Interference with the dynamics of Ang1-Tie2 signalling either by over-stimulation or by stimulus deprivation seemingly leads to similar phenotypes.

The pharmacological relevance of inhibiting Tie2 signalling was tested applying various non-small molecule approaches. A peptidic inhibitor of Ang1/2 binding to Tie2 was shown to inhibit Ang1-induced HUVEC migration and angiogenesis induction in an in vivo model (Tournaire et al. EMBO Rep. 2005, 5, 1). Corneal angiogenesis induced by tumor cell conditioned medium was inhibited by a recombinant soluble Tie2 receptor (sTie2) despite the presence of VEGF (Lin et al. J. Clin. Invest. 1997, 100, 2072; see also Singh et al. Biochem. Biophys. Res. Comm. 2005, 332, 194). Gene therapy by adenoviral vector delivered sTie2 was capable of reducing tumor growth rates of a murine mammary carcinoma and a murine melanoma and resulted in reduction of metastasis formation (Lin et al. Proc. Natl. Acad. Sci. USA 1998, 95, 8829). Similar effects were observed with related sTie2 constructs (Siemeister et al. Cancer Res. 1999, 59, 3185) and a Tek-Fc construct (Fathers et al. Am. J. Path. 2005, 167, 1753).

Adenovirus-delivered anti-Tie2 intrabodies were shown to inhibit growth of a human Kaposi's sarcoma and a human colon carcinoma upon peritumoral administration (Popkov et al. Cancer Res. 2005, 65, 972). Histopathological analysis revealed a marked decrease in vessel density in treated vs. control tumors. Phenotypic simultaneous knockout of KDR and Tie2 by an adenovirus delivered intradiabody resulted in significantly higher growth inhibition of a human melanoma xenograft model than KDR knockout alone (Jendreyko et al. Proc. Natl. Acad. Sci. USA 2005, 102, 8293). Similarly, the bispecific Tie2-KDR intradiabody was more active in an *in vitro* EC tube formation inhibition assay than the two monospecific intrabodies alone (Jendreyko et al. J. Biol. Chem. 2003, 278, 47812). Systematic treatment of tumor-bearing mice with Ang2-blocking antibodies and peptide-Fc fusion proteins led to tumor stasis and elimination of tumor burden in a subset of animals (Oliner et al. Cancer Cell 2004, 6, 507). For a recent report on an immunization approach, see Luo et al. Clin. Cancer Res. 2006, 12, 1813.

However, from the above studies using biochemical techniques to interfere with Tie2 signalling it is not clear, whether similar phenotypes will be observed with small molecule inhibitors of the Tie2 kinase activity. Small molecule inhibitors of kinases by definition block only those cellular effects which are mediated by the receptor's kinase activity and not those which might involve the kinase only as a co-receptor or scaffolding component in multi-enzyme complexes. So far, only a single study using a small molecule Tie2 inhibitor has been published (Scharpfenecker et al. J. Cell Sci. 2005, 118, 771). It remains to be shown that small molecule inhibitors of the Tie2 kinase will be as efficacious in inhibiting angiogenesis as e.g. ligand antibodies, soluble decoy receptors or receptor intrabodies.

### PRIOR ART

To date, a small number of therapeutic agents with antiangiogenic activity have been approved for cancer treatment. Avastin (Bevacizumab), a VEGF neutralizing antibody, blocks KDR and VEGFR1 signalling and has been approved for first-line treatment of metastatic colorectal cancer. The small molecule multi-targeted kinase inhibitor Nexavar® (Sorafenib) inhibits *inter alia* members of the VEGFR family and has been approved for the treatment of advanced renal cell carcinoma. Sutent (Sunitinib), another multi-targeted kinase inhibitor with activity vs. VEGFR family members, has been approved by the FDA for treatment of patients with gastrointestinal stromal tumors (GIST) or advanced kidney tumors. Several other small molecule inhibitors of angiogenesis-relevant targets are in clinical and preclinical development.

AMG-386, an angiopoietin-targeting recombinant Fc fusion protein, is in phase I clinical development in patients with advanced solid tumors. Several multi-targeted small molecule inhibitors with activity against Tie2 are (or have been) in preclinical evaluation for cancer therapy, including ABT-869, GW697465A and A-422885.88 (BSF466895). The first and most recent compound (Abt-869), however, was reported to be >10times more potent against at least 5 other kinase targets than against Tie2. In addition, Abt-869 has been reported to possess only modest activity as inhibitor of cellular Tie2 autophosphorylation (Albert et al. Mol. Cancer Ther. 2006, 5, 995).

Pyrazolopyridines have been disclosed as antimicrobiotic substances (*e*.*g*. Attaby et al., Phosphorus, Sulfur and Silicon and the related Elements 1999, 149, 49-64; Goda et al. Bioorg. Med. Chem. 2004, 12, 1845). US5478830 further discloses fused heterocycles for the treatment of atherosclerosis. Pyrazolopyridines have also been described as PDE4-Inhibitors (W02006004188, US20060004003).

A single 3-amino-1H-pyrazolo[3,4-b]pyridine with modest EGFR inhibitory activity has been published by Cavasotto et al. (Bioorg. Med. Chem. Lett. 2006, 16, 1969). 5-aryl-1H-3-aminopyrazolo[3,4-b]pyridines have been reported as GSK-3 inhibitors (Witherington et al. Bioorg. Med. Chem. Lett. 2003, 13, 1577). WO 2003068773 discloses 3-aminopyrazolopyridine derivatives as GSK-3 inhibitors.

WO 2004113304 (covering Abt-869, see above) discloses 3-amino-indazoles as inhibitors of protein tyrosine kinases, particularly as inhibitors as KDR kinase. WO 2006050109, WO2006077319 and W02006077168 disclose 3-aminopyrazolopyridines as tyrosine kinase inhibitors.

WO 2002024679 discloses tetrahydropyridine-substituted pyrazolopyridines as IKK inhibitors. WO 2004076450 further discloses 5-heteroaryl-pyrazolopyridines as p38 inhibitors. US20040192653 and US20040176325 inter alia disclose 4-H-pyrazolopyridines as p38 inhibitors. W02005044181 discloses pyrazolopyridines as Abl kinase inhibitors.

### TECHNICAL PROBLEM TO BE SOLVED

There is a great need for novel chemotypes for small molecule inhibitors of the Tie2 kinase, in particular inhibitors not only of the isolated kinase domain, but more importantly of cellular Tie-2 autophosphorylation. Fine tuning of additive anti-angiogenic activities as well as pharmacokinetic parameters such as e.g. solubility, membrane permeability, tissue distribution and metabolism will finally allow for choosing compounds of suitable profiles for various diseases caused by or associated with dysregulated vascular growth. Additional Inhibition of specific kinases, the activity of which is not relevant for angiogenesis but e.g. for tumor cell proliferation or invasion of surrounding tissues by tumor cells would be of special importance in treating certain diseases e.g. oncological diseases. However, compounds with purely antiangiogenic activity would be of special importance for e.g. the treatment of non-oncological diseases caused by or associated with dysregulated vascular growth.

It would be desirable to have compounds at one's disposal which display selectivity within the class of tyrosine kinases since inhibition of a broad spectrum of tyrosine kinases and side effects resulting thereof would limit pharmaceutical applications of those compounds. It would be especially desirable to have compounds at one's disposal which display potent inhibition of Tie2 while being significantly less active as inhibitors of specific other tyrosine kinases, particularly as inhibitors of the insulin receptor kinase (InsR).

Inhibition of InsR kinase may result in disadvantageous effects on the liver. The insulin/IGF-1 receptor inhibitor NVP-ADW742 for example at concentrations which inhibit both the insulin and IGF-1 receptors strongly potentiated desoxycholic acid-induced apoptotic cell death, which as a consequence predicts strong liver toxic effects in case of impaired bile flow (Dent et al. Biochem. Pharmacol. 2005, 70, 1685). Even worse, inhibition of the neuronal insulin receptor causes Alzheimer-like disturbances in oxidative/energy brain metabolism (Hoyer et al. Ann. N.Y. Acad. Sci. 1999, 893, 301).

It is known to the person skilled in the art that small structural changes to scaffolds previously used as kinase inhibitors will significantly impact selectivity profiles in an unpredictable manner. Inhibition of kinases by using ATP-competitive heteroaromatic compounds is well precedented in the patent and scientific literature (Parang, K.; Sun, G. Curr. Opin. Drug Disc. 2004, 7, 617: *Design Strategies for protein kinase inhibitors*.). It is known to the person skilled in the art that ATP-competitive compounds bind to the ATP-binding site in kinases by forming a hydrogen bonding network to a distinct region of the enzyme (the so called hinge region). 3-Aminopyrazoles were shown to form such a hydrogen bonding network to a kinase hinge region including the amino group of the 3-aminopyrazole moiety (Witherington et al.: "5-aryl-pyrazolo[3,4-b]pyridines: Potent inhibitors of glycogen synthase kinase-3" Bioorg. Med. Chem. Lett. 2003, 13, 1577). Prior art as cited above revealed 3-aminoindazoles and 3-aminopyrazolopyridines as inhibitors of various tyrosine kinases, e.g. of Tie2 kinase. In general, it would be desirable to have compounds at hand with a reduced number of H-bond donors - e.g. inhibitor compounds based on a scaffold lacking a primary amino group - in order to improve physicochemical or pharmacokinetic characteristics. However, the person skilled in the art would expect that removing the amino group in the 3-position of the pyrazole ring of the above cited prior art compounds should disrupt hydrogen bonding interactions of these inhibitors with e.g. the Tie2 kinase hinge region and therefore should lead to compounds with significantly reduced activity as kinase inhibitors.

### DESCRIPTION OF THE INVENTION

Surprisingly, it was now found that compounds of the present invention, which feature a pyrazolopyridine scaffold lacking the 3-amino group, not only display potent activity as inhibitors of Tie2 kinase activity and as inhibitors of cellular Tie2 autophosphorylation. Even more surprisingly, compounds of the present invention display an advantageous selectivity profile within in the class of tyrosine kinases, with more selective inhibition of Tie2 kinase relative to inhibition of other, not desired tyrosine kinases, particularly the insulin receptor kinase (InsR). Such a pharmacological profile is highly desirable not only for treating diseases of dysregulated vascular growth or diseases which are accompanied with dysregulated vascular growth, in particular solid tumors and metastases thereof, but also for treating non-oncological diseases of dysregulated vascular growth or non-oncological diseases which are accompanied with dysregulated vascular growth, such as, for example, retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel, diseases such as coronary and peripheral artery disease, wherein treatment of the said non-oncological diseases is preferably accomplished with less side-effects than in the treatment of oncological diseases. In addition, inhibition of one or more further specific tyrosine kinase(s) by compounds of the present invention, the activity of which is (are) of relevance for the pathogenesis of certain oncological diseases, allows for the use of prefered compounds of the present invention for the treatment of these oncological diseases.

The solution to the above-mentioned novel technical problem is achieved by providing compounds derived, in accordance with the present invention, from a class of 3-H-pyrazolopyridines and salts, *N*-oxides, solvates and prodrugs thereof, methods of preparing 3-H-pyrazolopyridines, a pharmaceutical composition containing said 3-H-pyrazolopyridines, use of said 3-H-pyrazolopyridines and a method for treating diseases with said 3-H-pyrazolopyridines, all in accordance with the description, as defined in the claims of the present application.

The compounds of Formula (I) below, salts, *N*-oxides, solvates and prodrugs thereof are collectively referred to as the "compounds of the present invention". The invention thus relates to compounds of general formula (I) : 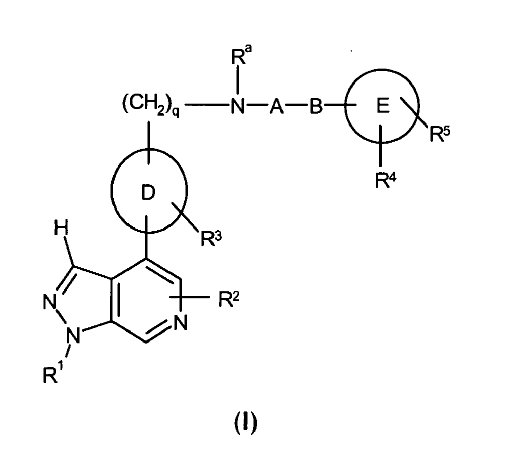 in which :
- R¹: represents H or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, wherein said residues are unsubstituted or substituted one or more times, independently from each other, with R⁶ ;
- R²: represents hydrogen, halogen, -NR^{d1}R^{d2}, -OR^{c}, -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, aryl, heteroaryl, wherein said residues are unsubstituted or one or more times substituted independently from each other with R⁷;
- R³: is selected from the group comprising, preferably consisting of, hydrogen, C₁-C₆-alkyl, C₁-C₆-alkoxy, C₁-C₆-haloalkyl, C₁-C₆-haloalkoxy, hydroxy, amino, halogen, and cyano ;
- R⁴, R⁵, R⁶, R⁷: independently from each other, are selected from the group comprising, preferably consisting of, hydrogen, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b},-S(O)₂R^{b}, -OR^{c}, -NR^{d1}R^{d2}, and -OP(O)(OR^{c})₂, wherein C₁-C₆-alkyl, aryl, heteroaryl, C₃-C₁₀-heterocycloalkyl and C₃-C₁₀-cycloalkyl are optionally substituted one or more times by R⁸ ;
- R⁸: is selected from the group comprising, preferably consisting of, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b}, -S(O)₂R^{b}, -OR^{c}, -NR^{d1}R^{d2}, and -OP(O)(OR^{c})₂;
- R^{a}: is selected from the group comprising, preferably consisting of, hydrogen and C₁-C₆-alkyl ;
- R^{b}: is selected from the group comprising, preferably consisting of, hydroxyl, -OR^{c}, -SR^{c}, -NR^{d1}R^{d2}, C₁-C₆-alkyl, and C₃-C₁₀-cycloalkyl, wherein C₁-C₆-alkyl, and C₃-C₁₀-cycloalkyl are optionally substituted one or more times with hydroxyl, halogen, or C₁-C₆-alkoxy;
- R^{c}: is selected from the group comprising, preferably consisting of, hydrogen, -C(O)R^{c}, C₁-C₆-alkyl, C₁-C₆-haloalkyl C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, aryl, -OR^{f}, -NR^{d1}R^{d2}, or -OP(O)(OR^{f})₂ ;
- R^{d1}, R^{d2}: independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl, or for a group -C(O)R^{e}, -S(O)₂R^{e}, or -C(O)NR^{g1}R^{g2} wherein C₁-C₆-alkyl, C₃-C₁₀-cycloalkyl, C₃-C₁₀-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}, or -OP(O)(OR^{f})₂ ; or
- R^{d1} and R^{d2}: together with the nitrogen atom to which they are attached, form a 3 to 10 membered heterocycloalkyl ring, which is optionally substituted one or more times, the same way or differently, with C₁-C₆-alkyl, halogen, -NR^{g1}R^{g2}, -OR^{f}, -C(O)R^{e}, -S(O)₂R^{e}, or -OP(O)(OR^{f})₂; whereby the carbon backbone of this heterocycloalkyl ring can optionally be interrupted one or more times, the same way or differently, by a member of the group comprising, preferably consisting of, NH, NR^{d3}, oxygen or sulphur, and can optionally be interrupted one or more times, the same way or differently, with a -C(O)-, -S(O)- , and/or-S(O)₂- group, and can optionally contain one or more double bonds
- R^{d3}: is selected from the group comprising, preferably consisting of hydrogen, C₁-C₆-alkyl, C₃-C₁₀-cycloalkyl, C₃-C₁₀-heterocycloalkyl, aryl, and heteroaryl, wherein C₁-C₆-alkyl, and C₃-C₁₀-cycloalkyl are optionally substituted one or more times with C₁-C₆-alkyl, C₃-C₁₀-cycloalkyl, hydroxyl, halogen, C₁-C₆-haloalkyl or C₁-C₆-alkoxy;
- R^{e}: is selected from the group comprising, preferably consisting of,-NR^{g1}R^{g2}, C₁-C₆-alkyl, C₃-C₆-cycloalkyl, C₁-C₆-alkoxy, aryl and heteroaryl;
- R^{f}: is selected from the group comprising, preferably consisting of, hydrogen, -C(O)R^{e}, C₁-C₆-alkyl, C₁-C₆-haloalkyl, C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, C₁-C₆-alkoxy, aryl, or -NR^{g1}R^{g2};
- R^{g1}, R^{g2}: independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl;
- R^{g1} and R^{g2}: together with the nitrogen atom to which they are attached, form a 3 to 10 membered heterocycloalkyl ring, which is optionally substituted one or more times, the same way or differently, with C₁-C₆-alkyl, -C₁-C₆-alkoxy, halogen or hydroxy; whereby the carbon backbone of this heterocycloalkyl ring can optionally be interrupted one or more times, the same way or differently, by a member of the group comprising, preferably consisting of, NH, NR^{a}, oxygen or sulphur, and can optionally be interrupted one or more times, the same way or differently, with a -C(O)-, -S(O)- , and/or -S(O)₂- group, and can optionally contain one or more double bonds ;
- A: is selected from the group comprising, preferably consisting of, -C(O)-, -C(S)-, -C(=NR^{a})-, -C(O)NR^{a}-, -C(=NR^{a})NR^{a}-, -S(O)₂-, -S(O)(=NR^{a})-, -S(=NR^{a})₂-, -C(S)NR^{a}-, -C(O)C(O)-, -C(O)C(O)NR^{a}-, -C(O)NR^{a}C(O)-, -C(S)NR^{a}C(O)-, and -C(O)NR^{a}C(S)- ;
- B: is a bond or selected from the group comprising, preferably consisting of C₁-C₆-alkylene, C₃-C₁₀-cycloalkylene, and C₃-C₁₀-heterocycloalkylene ;
- D, E: are, independently from each other, arylene or heteroarylene ;
and
- q: represents an integer of 0, 1, or 2 ;
or a salt, an *N*-oxide, a solvate or a prodrug thereof,
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a preferred embodiment, the present invention relates to compounds of formula I, *supra*, wherein :
- R¹: represents H or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, and C₃-C₁₀-heterocycloalkyl, wherein said residues are unsubstituted or substituted one or more times, independently from each other, with R⁶ ;
- R²: represents hydrogen, halogen, -NR^{d1}R^{d2}, -OR^{c}, or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, aryl, and heteroaryl, wherein said residues are unsubstituted or one or more times substituted independently from each other with R⁷;
- R³: is selected from the group comprising, preferably consisting of, hydrogen, C₁-C₆-alkyl, C₁-C₆-alkoxy, C₁-C₆-haloalkyl, C₁-C₆-haloalkoxy, hydroxy, amino, halogen, and cyano ;
- R⁴, R⁵, R⁶, R⁷: independently from each other, are selected from the group comprising, preferably consisting of, hydrogen, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b},-S(O)₂R^{b}, -OR^{c}, -NR^{d1}R^{d2}, and -OP(O)(OR^{c})₂, wherein C₁-C₆-alkyl, aryl, heteroaryl, C₃-C₁₀-heterocycloalkyl and C₃-C₁₀-cycloalkyl are optionally substituted one or more times by R⁸ ;
- R⁸: is selected from the group comprising, preferably consisting of, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b}, -S(O)₂R^{b}, -OR^{c}, -NR^{d1}R^{d2}, and -OP(O)(OR^{c})₂;
- R^{a}: is selected from the group comprising, preferably consisting of, hydrogen and C₁-C₆-alkyl ;
- R^{b}: is selected from the group comprising, preferably consisting of, hydroxyl, -OR^{c}, -SR^{c}, -NR^{d1}R^{d2}, C₁-C₆-alkyl, and C₃-C₁₀-cycloalkyl, wherein C₁-C₆-alkyl, and C₃-C₁₀-cycloalkyl are optionally substituted one or more times with hydroxyl, halogen, or C₁-C₆-alkoxy;
- R^{c}: is selected from the group comprising, preferably consisting of, hydrogen, -C(O)R^{e}, C₁-C₆-alkyl, C₁-C₆-haloalkyl C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, aryl, -OR^{f}, -NR^{d1}R^{d2}, or -OP(O)(OR^{f})₂ ;
- R^{d1}, R^{d2}: independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl, or for a group -C(O)R^{e}, -S(O)₂R^{e}, or -C(O)NR^{g1}R^{g2} wherein C₁-C₆-alkyl, C₃-C₁₀-cycloalkyl, C₃-C₁₀-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e},-OP(O)(OR^{f})₂; or
- R^{d1} and R^{d2}: together with the nitrogen atom to which they are attached, form a 3 to 10 membered heterocycloalkyl ring, which is optionally substituted one or more times, the same way or differently, with C₁-C₆-alkyl, halogen, -NR^{g1}R^{g2}, -OR^{f}, -C(O)R^{e}, -S(O)₂R^{e}, or -OP(O)(OR^{f})₂; whereby the carbon backbone of this heterocycloalkyl ring can optionally be interrupted one or more times, the same way or differently, by a member of the group comprising, preferably consisting of, NH, NR^{d3}, oxygen or sulphur, and can optionally be interrupted one or more times, the same way or differently, with a -C(O)-, -S(O)- , and/or-S(O)₂- group, and can optionally contain one or more double bonds
- R^{d3}: is selected from the group comprising, preferably consisting of hydrogen, C₁-C₆-alkyl, C₃-C₁₀-cycloalkyl, C₃-C₁₀-heterocycloalkyl, aryl, and heteroaryl, wherein C₁-C₆-alkyl, and C₃-C₁₀-cycloalkyl are optionally substituted one or more times with C₁-C₆-alkyl, C₃-C₁₀-cycloalkyl, hydroxyl, halogen, C₁-C₆-haloalkyl or C₁-C₆-alkoxy;
- R^{e}: is selected from the group comprising, preferably consisting of,-NR^{g1}R^{g2}, C₁-C₆-alkyl, C₃-C₆-cycloalkyl, C₁-C₆-alkoxy, aryl and heteroaryl;
- R^{f}: is selected from the group comprising, preferably consisting of, hydrogen, -C(O)R^{e}, C₁-C₆-alkyl, C₁-C₆-haloalkyl, C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, C₁-C₆-alkoxy, aryl, or -NR^{g1}R^{g2};
- R^{g1}, R^{g2}: independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl;
- R^{g1} and R^{g2}: together with the nitrogen atom to which they are attached, form a 3 to 10 membered heterocycloalkyl ring, which is optionally substituted one or more times, the same way or differently, with C₁-C₆-alkyl, -C₁-C₆-alkoxy, halogen or hydroxy; whereby the carbon backbone of this heterocycloalkyl ring can optionally be interrupted one or more times, the same way or differently, by a member of the group comprising, preferably consisting of, NH, NR^{a}, oxygen or sulphur, and can optionally be interrupted one or more times, the same way or differently, with a -C(O)-, -S(O)- , and/or -S(O)₂- group, and can optionally contain one or more double bonds ;
- A: represents -C(O)- or -C(O)NR^{a}-;
- B: is a bond or selected from the group comprising, preferably consisting of C₁-C₃-alkylene and C₃-C₅-cycloalkylene;
- D, E: are, independently from each other, arylene or heteroarylene ;
and
- q: represents an integer of 0, or 1;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a more preferred embodiment, the present invention relates to compounds of formula I, *supra*, wherein :
- R¹: represents H, C₁-C₆-alkyl, or C₂-C₆-alkenyl, wherein C₁-C₆-alkyl is unsubstituted or substituted one or more times, independently from each other, with R⁶ ;
- R²: represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
- R³: is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
- R⁴, R⁵, R⁶, R⁷: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸ ;
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
- R^{c}: is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
- R^{d1}, R^{d2}: independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, -OR^{f}, -C(O)R^{e}, or -S(O)₂R^{e}; whereby the carbon backbone of this heterocycloalkyl ring can optionally be interrupted one or more times, the same way or differently, by a member of the group comprising, preferably consisting of, NH, NR^{d3}, or oxygen, and can optionally be interrupted one or more times, the same way or differently, with a -C(O)- group;
- R^{d3}: is selected from the group comprising, preferably consisting of hydrogen and C₁-C₄-alkyl, wherein C₁-C₄-alkyl is optionally substituted one or more times with C₃-C₆-cycloalkyl, hydroxyl, halogen, C₁-C₄-haloalkyl or C₁-C₄-alkoxy;
- R^{e}: is selected from the group comprising, preferably consisting of,-NR^{g1}R^{g2}, C₁-C₃-alkyl, C₃-C₆-cycloalkyl, C₁-C₃-alkoxy, aryl and heteroaryl;
- R^{f}: is selected from the group comprising, preferably consisting of, hydrogen, -C(O)R^{e}, C₁-C₃-alkyl, C₁-C₃-haloalkyl, C₃-C₆-cycloalkyl, C₃- C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
- R^{g1}, R^{g2}: independently from each other are selected from the group comprising, preferably consisting of hydrogen, C₁-C₃-alkyl, C₃-C₆-cycloalkyl, C₃-C₆-heterocycloalkyl, aryl, and heteroaryl;
- R^{g1} and R^{g2}: together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted one or more times, the same way or differently, by a member of the group comprising, preferably consisting of, NH, NR^{a}, or oxygen;
- A: represents -C(O)NR^{a}-;
- B: is a bond;
- D, E: are, independently from each other, arylene or heteroarylene ;
and
- q: is 0;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a more particularly preferred embodiment, the present invention relates to compounds of formula I, *supra*, wherein :
- R¹: represents H, C₁-C₆-alkyl, or C₂-C₆-alkenyl, wherein C₁-C₆-alkyl is unsubstituted or substituted one or more times, independently from each other, with R⁶ ;
- R²: represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
- R³: is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
- R⁴, R⁵, R⁶, R⁷: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸ ;
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
- R^{c}: is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
- R^{d1}, R^{d2}: independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-C₆-cycloalkyl, hydroxyl, C₁-C₄-haloalkyl, C₁-C₄-alkoxy or halogen;
- R^{e}: is selected from the group comprising, preferably consisting of,-NR^{g1}R^{g2}, C₁-C₃-alkyl, C₃-C₆-cycloalkyl, C₁-C₃-alkoxy, aryl and heteroaryl;
- R^{f}: is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
- R^{g1}, R^{g2}: independently from each other are selected from the group comprising, preferably consisting of hydrogen, C₁-C₃-alkyl, C₃-C₆-cycloalkyl, C₃-C₆-heterocycloalkyl, aryl, and heteroaryl;
- R^{g1} and R^{g2}: together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted once by a member of the group comprising, preferably consisting of, NH, NR^{a}, or oxygen;
- A: represents -C(O)NR^{a}-;
- B: is a bond;
- D: is para-phenylene ;
- E: is phenylene or heteroarylene ;
and
- q: is 0;
wherein, when one or more of R^{a}, R^{b}, R^{c}, R^{d1}, R^{d2}, R^{d3}, R^{e}, R^{f}, R^{g1}, R^{g2}, or R⁸ is (are) present in one position in the molecule as well as in one or more further positions in the molecule, said R^{a}, R^{b}, R^{c}, R^{d1}, R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a more particularly preferred embodiment still, the present invention relates to compounds of formula I, *supra,* wherein :
- R¹: represents H, C₁-C₆-alkyl, or C₂-C₆-alkenyl, wherein C₁-C₆-alkyl is unsubstituted or substituted one or more times, independently from each other, with R⁶;
- R²: represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
- R³: is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
- R⁴, R⁵, R⁶, R⁷: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸;
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
- R^{c}: is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
- R^{d1}, R^{d2}: independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-C₆-cycloalkyl, hydroxyl, C₁-C₄-haloalkyl, C₁-C₄-alkoxy or halogen;
- R^{e}: is selected from the group comprising, preferably consisting of,-NR^{g1}R^{g2}, C₁-C₃-alkyl, C₃-C₆-cycloalkyl, C₁-C₃-alkoxy, aryl and heteroaryl;
- R^{f}: is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
- R^{g1}, R^{g2}: independently from each other are selected from the group comprising, preferably consisting of hydrogen, C₁-C₃-alkyl, C₃-C₆-cycloalkyl, C₃-C₆-heterocycloalkyl, aryl, and heteroaryl;
- R^{g1} and R^{g2}: together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted once by a member of the group comprising, preferably consisting of, NH, NR^{a}, or oxygen;
- A: represents -C(O)NR^{a}-;
- B: is a bond;
- D: is para-phenylene ;
- E: is heteroarylene ;
and
- q: is 0;
wherein, when one or more of R^{a}, R^{b}, R^{c} , R^{d1}, R^{d2}, R^{d3}, R^{e}, R^{f}, R^{g1}, R^{g2}, or R⁸ is (are) present in one position in the molecule as well as in one or more further positions in the molecule, said R^{a}, R^{b}, R^{c}, R^{d1}, R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with an even more particularly preferred embodiment, the present invention relates to compounds of formula I, *supra*, wherein :
- R¹: represents H or C₁-C₄-alkyl wherein C₁-C₄-alkyl is unsubstituted or substituted one or more times with R⁶;
- R²: represents hydrogen;
- R³: is selected from the group comprising, preferably consisting of, hydrogen, methyl, methoxy, fluorine and hydroxy;
- R⁴: is selected from the group comprising, preferably consisting of, hydrogen, C₁-C₄-alkyl, C₁-C₆-cycloalkyl, wherein C₁-C₄-alkyl is optionally substituted by R⁸ ;
- R⁵: is selected from the group comprising, preferably consisting of, hydrogen, C₁-C₄-alkyl, C₅-C₆-heterocycloalkyl, C₅-C₆-cycloalkyl, phenyl and pyridyl, wherein C₁-C₄-alkyl, C₅-C₆-heterocycloalkyl, C₅-C₆-cycloalkyl, phenyl and pyridyl are optionally substituted one or more times, independently from each other, with R⁸;
- R⁶,: represents hydroxy;
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c}, and -NR^{d1}R^{d2};
- R^{a}: represents hydrogen or methyl;
- R^{b}: is selected from the group comprising, preferably consisting of, hydroxy, -OR^{c}, -NR^{d1}R^{d2}, C₁-C₃-alkyl, and C₃-C₆-cycloalkyl,
- R^{c}: represents C₁-C₃-alkyl or C₆-C₇-heterocycloalkyl;
- R^{d1}, R^{d2}: independently from each other are selected from the group comprising, preferably consisting of hydrogen, and C₁-C₃-alkyl, or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-C₆-cycloalkyl, hydroxy, C₁-C₄-haloalkyl, C₁-C₄-alkoxy or halogen;
- R^{g1}, R^{g2}: independently from each other are selected from the group comprising, preferably consisting of hydrogen, C₁-C₃-alkyl and C₃-C₆-cycloalkyl;
- A: represents -C(O)NR^{a}- ;
- B: is a bond;
- D: is a para-phenylene
- E: is a pyrazole;
and
- q: is 0;
wherein, when one or more of R^{a}, R^{c}, or R^{d3} is (are) present in one position in the molecule as well as in one or more further positions in the molecule, said R^{a}, R^{c}, or R^{d3} has (have), independently from each other, the same meanings as defined above in said first position in the molecule and in said second or further positions in the molecule, it being possible for the two or more occurrences of R^{a}, R^{c}, or R^{d3} within a single molecule to be identical or different. For example, when R^{a} is present twice in the molecule, then the meaning of the first R^{a} may be H, for example, and the meaning of the second R^{a} may be methyl, for example.

In accordance with a variant, the present invention relates to compounds of formula I, *supra,* wherein :
- R¹: represents H, or C₁-C₃-alkyl, wherein C₁-C₃-alkyl is unsubstituted or substituted one or more times, independently from each other, with R⁶;
- R²: represents hydrogen, halogen, -NR^{d1}R^{d2}, -OR^{c}, -C(O)R^{b}, or C₁-C₃-alkyl, wherein C₁-C₃-alkyl is unsubstituted or one or more times substituted independently from each other with R⁷;
- R³: is selected from the group comprising, preferably consisting of, hydrogen, methyl, methoxy, hydroxy, halogen, and cyano ;
- R⁴, R⁵: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c}, and-NR^{d1}R^{d2}, wherein C₁-C₃-alkyl, C₃-C₆-heterocycloalkyl and C₃-C₆-cycloalkyl are optionally substituted one or more times by R⁸ ;
- R⁶: independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}R^{d2};
- R⁷: independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}R^{d2};
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c} and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
- R^{c}: is selected from the group comprising, preferably consisting of, hydrogen, -C(O)R^{e}, C₁-C₃-alkyl, C₁-C₃-haloalkyl C₃-C₆-cycloalkyl, and C₃-C₇-heterocycloalkyl, wherein C₁-C₃-alkyl, C₁-C₃-haloalkyl, C₃-C₆-cycloalkyl, C₃-C₆-heterocycloalkyl are optionally substituted one or more times with hydroxyl, halogen, aryl, -OR^{f}, or -NR^{d1}R^{d2};
- R^{d1}, R^{d2}: independently from each other are selected from the group comprising, preferably consisting of hydrogen, C₁-C₃-alkyl, C₃-C₆-cycloalkyl, and C₃-C₆-heterocycloalkyl, or for a group -C(O)R^{e},-S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
- R^{e}: is selected from the group comprising, preferably consisting of,-NR^{g1}R^{g2}, C₁-C₃-alkyl, C₃-C₆-cycloalkyl, C₁-C₃-alkoxy, aryl and heteroaryl;
- R^{f}: is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocyctoalkyl, aryl, and heteroaryl are optionally substituted one or more times with hydroxyl, halogen, C₁-C₃-alkoxy, aryl, or -NR^{g1}R^{g2};
- R^{g1}, R^{g2}: independently from each other are selected from the group comprising, preferably consisting of hydrogen, C₁-C₃-alkyl, C₃-C₆-cycloalkyl, C₃-C₆-heterocycloalkyl, aryl, and heteroaryl;
- R^{g1} and R^{g2}: together with the nitrogen atom to which they are attached, form a 3 to 6 membered heterocycloalkyl ring, whereby the carbon backbone of this heterocycloalkyl ring can optionally be interrupted once by a member of the group comprising, preferably consisting of, NH, NR^{d3}, or oxygen;
- A: represents -C(O)NR^{a}-;
- B: is a bond;
- D: is para-phenylene ;
- E: is phenylene ;
and
- q: is 0;
wherein, when one or more of R^{a}, R^{b}, R^{c}, R^{d1}, R^{d2}, R^{d3}, R^{e}, R^{f}, R^{g1}, R^{g2}, or R⁸ is (are) present in one position in the molecule as well as in one or more further positions in the molecule, said R^{a}, R^{b}, R^{c}, R^{d1}, R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3} R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a preferred embodiment of the above-mentioned variant, the present invention relates to compounds of formula I, *supra,* wherein :
- R¹: represents H, or C₁-C₃-alkyl, wherein C₁-C₃-alkyl is unsubstituted or substituted one or more times, independently from each other, with R⁶;
- R²: represents hydrogen;
- R³: is selected from the group comprising, preferably consisting of, hydrogen, methyl, methoxy, hydroxy, and halogen ;
- R⁴, R⁵: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c}, and - NR^{d1}R^{d2}, wherein C₁-C₃-alkyl, C₃-C₆-heterocycloalkyl and C₃-C₆-cycloalkyl are optionally substituted one or more times by R⁸ ;
- R⁶: independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}R^{d2};
- R⁸: is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
- R^{c}: is selected from the group comprising, preferably consisting of, hydrogen, -C(O)R^{e}, C₁-C₃-alkyl, C₁-C₃-haloalkyl C₃-C₆-cycloalkyl, and C₃-C₇-heterocycloalkyl, wherein C₁-C₃-alkyl, C₁-C₃-haloalkyl, C₃-C₆-cycloalkyl, and C₃-C₆-heterocycloalkyl are optionally substituted one or more times with hydroxyl, halogen, aryl, -OR^{f}, or -NR^{d1}R^{d2};
- R^{d1}, R^{d2}: independently from each other are selected from the group comprising, preferably consisting of hydrogen, C₁-C₃-alkyl, C₃-C₆-cycloalkyl, and C₃-C₆-heterocycloalkyl, or for a group -C(O)R^{e},-S(O)₂R^{e}, or -C(O)NR^{g1}R^{g2} wherein C₁-C₃-alkyl, C₃-C₆-cycloalkyl, and C₃-C₆-heterocycloalkyl are optionally substituted one or more times, the same way or differently with halogen, hydroxy or the group -NR^{g1}R^{g2},-OR^{f}, -C(O)R^{e}, -S(O)₂R^{e}; or
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
- R^{e}: is selected from the group comprising, preferably consisting of,-NR^{g1}R^{g2}, C₁-C₃-alkyl, C₃-C₆-cycloalkyl, C₁-C₃-alkoxy, aryl and heteroaryl;
- R^{f}: is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
- R^{g1}, R^{g2}: independently from each other are selected from the group comprising, preferably consisting of hydrogen, C₁-C₃-alkyl, C₃-C₆-cycloalkyl, C₃-C₆-heterocycloalkyl, aryl, and heteroaryl;
- R^{g1} and R^{g2}: together with the nitrogen atom to which they are attached, form a 3 to 6 membered heterocycloalkyl ring, whereby the carbon backbone of this heterocycloalkyl ring can optionally be interrupted once by a member of the group comprising, preferably consisting of, NH, NR^{d3}, or oxygen;
- A: represents C(O)NR^{a}-;
- B: is a bond;
- D: is para-phenylene ;
- E: is phenylene ;
and
- q: is 0;
wherein, when one or more of R^{a}, R^{b}, R^{c}, R^{d1}, R^{d2}, R^{d3}, R^{e}, R^{f}, R^{g1}, R^{g2}, or R⁸ is (are) present in one position in the molecule as well as in one or more further positions in the molecule, said R^{a}, R^{b}, R^{c}, R^{d1}, R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1} R^{d2}, R^{d3}, R^{e}, R^{f}, R^{g1}, R^{g2}, or R⁸ within a single molecule to be identical or different. For example, when R^{a} is present twice in the molecule, then the meaning of the first R^{a} may be H, for example, and the meaning of the second R^{a} may be methyl, for example.

In accordance with a more preferred embodiment of the above-mentioned variant, the present invention relates to compounds of formula I, *supra,* wherein :
- R¹: represents H, or C₁-C₃-alkyl, wherein C₁-C₃-alkyl is unsubstituted or substituted one or more times, independently from each other, with R⁶;
- R²: represents hydrogen;
- R³: is selected from the group comprising, preferably consisting of, hydrogen, methyl, methoxy, hydroxy, and halogen ;
- R⁴, R⁵: independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -OR^{c}, and -NR^{d1}R^{d2}, wherein C₁-C₃-alkyl and C₃-C₆-heterocycloalkyl are optionally substituted one or more times by R⁸ ;
- R⁶,: represents hydroxy;
- R⁸: is selected from the group comprising, preferably consisting of, C₃-C₆-cycloalkyl, C₃-C₇-heterocycloalkyl, C₁-C₃-haloalkyl, C₁-C₃-haLoalkoxy, hydroxy, halogen, -S(O)₂R^{b}, -OR^{c}, and -NR^{d1}R^{d2};
- R^{a}: is selected from the group comprising, preferably consisting of, hydrogen and methyl ;
- R^{b}: is selected from the group comprising, preferably consisting of, hydroxyl, -OR^{c}, -NR^{d1}R^{d2}, C₁-C₃-alkyl, and C₃-C₆-cycloalkyl;
- R^{c}: is selected from the group comprising, preferably consisting of, hydrogen, C₁-C₃-alkyl, C₁-C₃-haloalkyl C₃-C₆-cycloalkyl, and C₃-C₇-heterocycloalkyl;
- R^{d1}, R^{d2}: independently from each other are selected from the group comprising, preferably consisting of hydrogen, C₁-C₃-alkyl, C₃-C₆-cycloalkyl, and C₃-C₆-heterocycloalkyl, or for a group -C(O)R^{e}, or-S(O)₂R^{e} wherein C₁-C₃-alkyl, is optionally substituted one or more times, the same way or differently with halogen, hydroxy or C₁-C₃-alkoxy;
- R^{d1} and R^{d2}: together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
- R^{d3}: represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
- R^{e}: represents C₁-C₃-alkyl or C₃-C₆-cycloalkyl;
- A: represents C(O)NR^{a}-;
- B: is a bond;
- D: is para-phenylene ;
- E: is phenylene ;
and
- q: is 0;
wherein, when one or more of R^{a}, R^{b}, R^{e}, R^{d1}, R^{d2}, R^{d3}, or R⁸ is (are) present in one position in the molecule as well as in one or more further positions in the molecule, said R^{a}, R^{b} , R^{c} , R^{d1} , R^{d2}, R^{d3}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, or R⁸ within a single molecule to be identical or different. For example, when R^{a} is present twice in the molecule, then the meaning of the first R^{a} may be H, for example, and the meaning of the second R^{a} may be methyl, for example.

In accordance with an even more preferred embodiment of the above-mentioned variant, the present invention relates to compounds of formula I, *supra,* wherein :
- R¹: represents H or C₁-C₃-alkyl wherein said C₁-C₃-alkyl is unsubstituted or substituted one or more times with R⁶ ;
- R²: represents hydrogen;
- R³: is selected from the group comprising, preferably consisting of, hydrogen, methyl, fluorine, hydroxy and methoxy;
- R⁴: is selected from the group comprising, preferably consisting of, hydrogen, C₁-C₃-alkyl, C₁-C₃-haloalkyl, halogen, and -OR^{c}, wherein C₁-C₃-alkyl is optionally substituted by R⁸ ;
- R⁵: is selected from the group comprising, preferably consisting of, hydrogen, C₁-C₃-alkyl, C₁-C₃-haloalkyl, halogen, and -OR^{c};
- R⁶,: represents hydroxy;
- R⁸: is selected from the group comprising, preferably consisting of, C₆-heterocycloalkyl, -OR^{c}, and -NR^{d1}R^{d2};
- R^{a}: represents hydrogen or methyl;
- R^{c}: represents C₁-C₃-alkyl or C₆-heterocycloalkyl;
- R^{d1}, R^{d2}: independently from each other are selected from the group comprising, preferably consisting of hydrogen, and C₁-C₆-alkyl, or
- R^{d1} and R^{d2}: together with the nitrogen atom to which they are attached, form a 6 membered heterocycloalkyl ring, whereby the carbon backbone of this heterocycloalkyl ring can optionally be interrupted by a member of the group comprising, preferably consisting of, NH, NR^{d3}, or oxygen;
- R^{d3}: represents hydrogen or methyl;
- A: represents -C(O)NR^{a}- ;
- B: is a bond;
- D: is a para-phenylene
- E: is phenylene;
and
- q: is 0;
wherein, when one or more of R^{a} , R^{b}, or R^{d3} is (are) present in one position in the molecule as well as in one or more further positions in the molecule, said R^{a}, R^{c}, or R^{d3} has (have), independently from each other, the same meanings as defined above in said first position in the molecule and in said second or further positions in the molecule, it being possible for the two or more occurrences of R^{a}, R^{c}, or R^{d3} within a single molecule to be identical or different. For example, when R^{a} is present twice in the molecule, then the meaning of the first R^{a} may be H, for example, and the meaning of the second R^{a} may be methyl, for example.

### DEFINITIONS

Within the context of the present application, the terms as mentioned in this description and in the claims have preferably the following meanings :

The term "alkyl" is to be understood as preferably meaning branched and unbranched alkyl, meaning *e*.*g*. methyl, ethyl, *n*-propyl, *iso*-propyl, *n*-butyl, *iso*butyl, *tert*-butyl, *sec*-butyl, pentyl, *iso*-pentyl, hexyl, heptyl, octyl, nonyl and decyl and isomers thereof.

The term "haloalkyl" is to be understood as preferably meaning branched and unbranched alkyl, as defined *supra,* in which one or more of the hydrogen substituents is replaced in the same way or differently with halogen. Particularly preferably, said haloalkyl is, *e*.*g*. chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.

The term "alkoxy" is to be understood as preferably meaning branched and unbranched alkoxy, meaning *e*.*g*. methoxy, ethoxy, propyloxy, *iso*-propyloxy, butyloxy, *iso*-butyloxy, *tert*-butyloxy, *sec*-butyloxy, pentyloxy, *iso*-pentyloxy, hexyloxy, heptyloxy, octyloxy, nonyloxy, decyloxy, undecyloxy and dodecyloxy and isomers thereof.

The term "haloalkoxy" is to be understood as preferably meaning branched and unbranched alkoxy, as defined *supra,* in which one or more of the hydrogen substituents is replaced in the same way or differently with halogen, *e*.*g*. chloromethoxy, fluoromethoxy, pentafluoroethoxy, fluoropropyloxy, difluoromethyloxy, trichloromethoxy, 2,2,2-trifluoroethoxy, bromobutyloxy, trifluoromethoxy, iodoethoxy, and isomers thereof.

The term "cycloalkyl" is to be understood as preferably meaning a C₃-C₁₀ cycloalkyl group, more particularly a saturated cycloalkyl group of the indicated ring size, meaning *e*.*g*. a cyclopropyl, cyclobutyl, cyclopentyl, cyclohexyl, cycloheptyl, cyclooctyl, cyclononyl, or cyclodecyl group ; and also as meaning an unsaturated cycloalkyl group containing one or more double bonds in the C-backbone, *e*.*g*. a C₃-C₁₀ cycloalkenyl group, such as, for example, a cyclopropenyl, cyclobutenyl, cyclopentenyl, cyclohexenyl, cycloheptenyl, cyclooctenyl, cyclononenyl, or cyclodecenyl group, wherein the linkage of said cyclolalkyl group to the rest of the molecule can be provided to the double or single bond; and also as meaning such a saturated or unsaturated cycloalkyl group being optionally substituted one or more times, independently from each other, with a C₁-C₆ alkyl group and/or a halogen and/or an OR^{f} group and/or a NR^{g1}R^{g2} group; such as, for example, a 2-methylcyclopropyl group, a 2,2-dimethylcyclopropyl group, a 2,2-dimethylcyclobutyl group, a 3-hydroxycyclopentyl group, a 3-hydroxycyclohexylgroup, a 3-dimethylaminocyclobutyl group, a 3-dimethylaminocyclopentyl group or a 4-dimethylaminocyclohexyl group.

The term "heterocycloalkyl" is to be understood as preferably meaning a C₃-C₁₀ cycloalkyl group, as defined *supra,* featuring the indicated number of ring atoms, wherein one or more ring atom(s) is (are) (a) heteroatom(s) such as NH, NR^{d3}, O, S, or (a) group(s) such as a C(O), S(O), S(O)₂, or, otherwise stated, in a Cₙ-cycloalkyl group, (wherein ₙ is an integer of 3, 4, 5, 6, 7, 8, 9, or 10), one or more carbon atom(s) is (are) replaced by said heteroatom(s) or said group(s) to give such a Cₙ cycloheteroalkyl group; and also as meaning an unsaturated heterocycloalkyl group containing one or more double bonds in the C-backbone, wherein the linkage of said heterocyclolalkyl group to the rest of the molecule can be provided to the double or single bond; and also as meaning such a saturated or unsaturated heterocycloalkyl group being optionally substituted one or more times, independently from each other, with a C₁-C₆ alkyl group and/or a halogen and/or an OR^{f} group and/or a NR^{g1}R^{g2} group. Thus, said Cₙ cycloheteroalkyl group refers, for example, to a three-membered heterocycloalkyl, expressed as C₃-heterocycloalkyl, such as oxiranyl (C₃). Other examples of heterocycloalkyls are oxetanyl (C₄), aziridinyl (C₃), azetidinyl (C₄), tetrahydrofuranyl (C₅), pyrrolidinyl (C₅), morpholinyl (C₆), dithianyl (C₆), thiomorpholinyl (C₆), piperidinyl (C₆), tetrahydropyranyl (C₆), piperazinyl (C₆), trithianyl (C₆), homomorpholinyl (C₇), homopiperazinyl (C₇) and chinuclidinyl (C₈); said cycloheteroalkyl group refers also to, for example, 4-methylpiperazinyl, 3-methyl-4-methylpiperazine, 3-fluoro-4-methylpiperazine, 4-dimethylaminopiperidinyl, 4-methylaminopiperidinyl, 4-aminopiperidinyl, 3-dimethylaminopiperidinyl, 3-methylaminopiperidinyl, 3-aminopiperidinyl, 4-hydroxypiperidinyl, 3-hydroxypiperidinyl, 2-hydroxypiperidinyl, 4-methylpiperidinyl, 3-methylpiperidinyl, 3-dimethylaminopyrrolidinyl, 3-methylaminopyrrolidinyl, 3-aminopyrrolidinyl or methylmorpholinyl.

The term "halogen" or "Hal" is to be understood as preferably meaning fluorine, chlorine, bromine, or iodine.

The term "alkenyl" is to be understood as preferably meaning branched and unbranched alkenyl, *e*.*g*. a vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl, or 2-methyl-prop-1-en-1-yl group, and isomers thereof.

The term "alkynyl" is to be understood as preferably meaning branched and unbranched alkynyl, *e*.*g*. an ethynyl, prop-1-yn-1-yl, but-1-yn-1-yl, but-2-yn-1-yl,or but-3-yn-1-yl group, and isomers thereof.

As used herein, the term "aryl" is defined in each case as having 3-12 carbon atoms, preferably 6-12 carbon atoms, such as, for example, cyclopropenyl, phenyl, tropyl, indenyl, naphthyl, azulenyl, biphenyl, fluorenyl, anthracenyl etc, phenyl being preferred.

As used herein, the term "heteroaryl" is understood as meaning an aromatic ring system which comprises 3-16 ring atoms, preferably 5 or 6 or 9 or 10 atoms, and which contains at least one heteroatom which may be identical or different, said heteroatom being such as nitrogen, NH, NR^{d3}, oxygen, or sulphur, and can be monocyclic, bicyclic, or tricyclic, and in addition in each case can be benzocondensed. Preferably, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc*., and benzo derivatives thereof, such as, *e*.*g*., benzofuranyl, benzothienyl, benzoxazolyl, benzimidazolyl, benzotriazolyl, indazolyl, indolyl, isoindolyl, *etc*.; or pyridyl, pyridazinyl, pyrimidinyl, pyrazinyl, triazinyl, *etc*., and benzo derivatives thereof, such as, for example, quinolinyl, isoquinolinyl, *etc*.; or azocinyl, indolizinyl, purinyl, *etc*., and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, *etc.*

The term "alkylene", as used herein in the context of the compounds of general formula (I) is to be understood as meaning an optionally substituted alkyl chain or "tether", having 1, 2, 3, 4, 5, or 6 carbon atoms, *i*.*e*. an optionally substituted - CH₂- ("methylene" or "single membered tether" or e.g. -C(Me)₂-), -CH₂-CH₂-("ethylene", "dimethylene", or "two-membered tether"), -CH₂-CH₂-CH₂-("propylene", "trimethylene", or "three-membered tether"), -CH₂-CH₂-CH₂-CH₂-("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂- ("hexylene", "hexamethylene", or six-membered tether") group. Preferably, said alkylene tether is 1, 2, 3, 4, or 5 carbon atoms, more preferably 1 or 2 carbon atoms.

The term "cycloalkylene", as used herein in the context of the compounds of general formula (I) is to be understood as meaning an optionally substituted cycloalkyl ring, having 3, 4, 5, 6, 7, 8, 9 or 10, preferably 3, 4, 5, or 6, carbon atoms, *i*.*e*. an optionally substituted cyclopropyl, cyclobutyl, cyclopenyl, cyclohexyl, cycloheptyl, cyclooctyl, cyclononyl, or cyclodecyl ring, preferably a cyclopropyl, cyclobutyl, cyclopentyl or cyclohexyl ring.

The term "heterocycloalkylene", as used herein in the context of the compounds of general formula (I) is to be understood as meaning a cycloalkylene ring, as defined *supra,* but which contains at least one heteroatom which may be identical or different, said heteroatom being such as NH, NR^{d3}, oxygen or sulphur.

The term "arylene", as used herein in the context of the compounds of general formula (I) is to be understood as meaning an optionally substituted monocyclic or polycyclic arylene aromatic system *e*.*g*. arylene, naphthylene and biarylene, preferably an optionally substituted phenyl ring or "tether", having 6 or 10 carbon atoms. More preferably, said arylene tether is a ring having 6 carbon atoms, *i*.*e*. a "phenylene" ring. If the term "arylene" or e.g. "phenylene" is used it is to be understood that the linking residues can be arranged to each other in ortho-, para-and meta-position, eg. an optionally substituted moiety of structure : 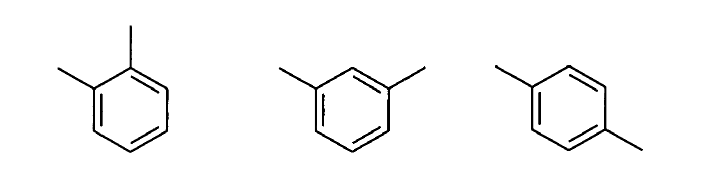 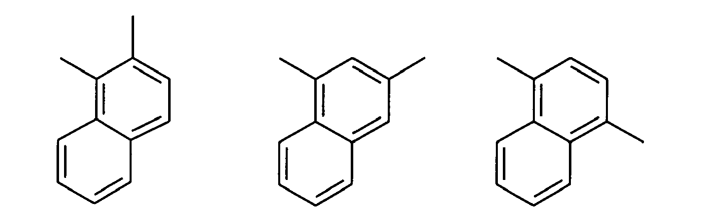 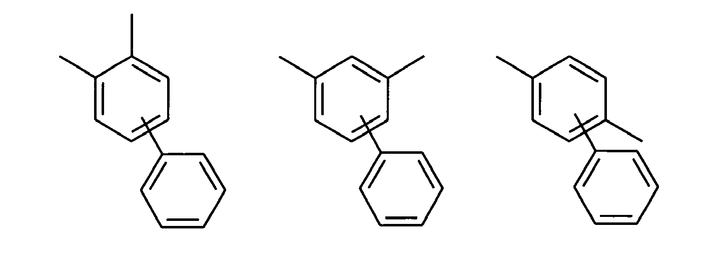 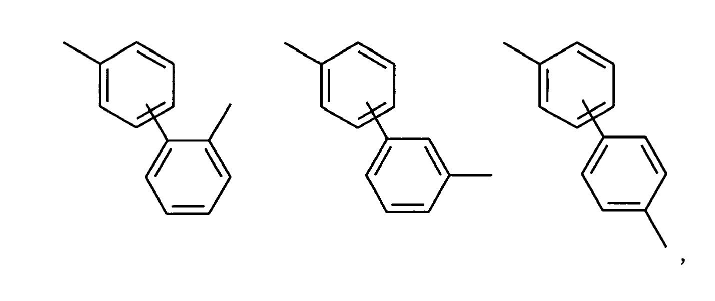 in which linking positions on the rings are shown as non-attached bonds.

The term "heteroarylene", as used herein in the context of the compounds of general formula (I) is to be understood as meaning an optionally substituted monocyclic or polycyclic heteroarylene aromatic system, e.g. heteroarylene, benzoheteroarylene, preferably an optionally substituted 5-membered heterocycle, such as, for example, furan, pyrrole, pyrazole, thiazole, oxazole, isoxazole, or thiophene or "tether", or a 6-membered heterocycle, such as, for example, pyridine, pyrimidine, pyrazine, pyridazine. More preferably, said heteroarylene tether is a ring having 6 carbon atoms, *e*.*g*. an optionally substituted structure as shown *supra* for the arylene moieties, but which contains at least one heteroatom which may be identical or different, said heteroatom being such as nitrogen, NH, NR^{d3}, oxygen, or sulphur. If the term "heteroarylene" is used it is to be understood that the linking residues can be arranged to each other in ortho-, para- and meta-position.

As used herein, the term "C₁-C₆", as used throughout this text, *e*.*g*. in the context of the definition of "C₁-C₆-alkyl", or "C₁-C₆-alkoxy", is to be understood as meaning an alkyl group having a finite number of carbon atoms of 1 to 6, *i*.*e*. 1, 2, 3, 4, 5, or 6 carbon atoms. It is to be understood further that said term "C₁-C₆" is to be interpreted as any sub-range comprised therein, *e*.*g*. C₁-C₆, C₂-C₅, C₃-C₄, C₁-C₂, C₁-C₃ , C₁-C₄, C₁-C₅ C₁-C_{6;} preferably C₁-C₂, C₁-C₃, C₁-C₄, C₁-C₅, C₁-C₆ ; more preferably C₁-C₃.

Similarly, as used herein, the term "C₂-C₆", as used throughout this text, *e*.*g*. in the context of the definitions of "C₂-C₆-alkenyl" and "C₂-C₆-alkynyl", is to be understood as meaning an alkenyl group or an alkynyl group having a finite number of carbon atoms of 2 to 6, *i*.*e*. 2, 3, 4, 5, or 6 carbon atoms. It is to be understood further that said term "C₂-C₆" is to be interpreted as any sub-range comprised therein, *e*.*g*. C₂-C₆, C₃-C₅, C₃-C₄, C₂-C₃, C₂-C₄, C₂-C₅; preferably C₂-C₃.

As used herein, the term "C₃-C₁₀", as used throughout this text, *e*.*g*. in the context of the definitions of "C₃-C₁₀-cycloalkyl" or "C₃-C₁₀-heterocycloalkyl", is to be understood as meaning a cycloalkyl group having a finite number of carbon atoms of 3 to 10, *i*.*e*. 3, 4, 5, 6, 7, 8, 9 or 10 carbon atoms, preferably 3, 4, 5 or 6 carbon atoms. It is to be understood further that said term "C₃-C₁₀" is to be interpreted as any sub-range comprised therein, *e*.*g*. C₃-C₁₀, C₄-C₉, C₅-C₈, C₆-C₇; preferably C₃-C₆.

As used herein, the term "C₃-C₆", as used throughout this text, e.g. in the context of the definitions of "C₃-C₆-cycloalkyl" or "C₃-C₆-heterocycloalkyl", is to be understood as meaning a cycloalkyl group having a finite number of carbon atoms of 3 to 6, *i.e.* 3, 4, 5, or 6 carbon atoms. It is to be understood further that said term "C₃-C₆" is to be interpreted as any sub-range comprised therein, *e*.*g*. C₃-C₄, C₄-C₆, C₅-C₆.

As used herein, the term "C₆-C₁₁", as used throughout this text, *e.g.* in the context of the definitions of "C₆-C₁₁-aryl", is to be understood as meaning an aryl group having a finite number of carbon atoms of 5 to 11, *i.e.* 5, 6, 7, 8, 9, 10 or 11 carbon atoms, preferably 5, 6, or 10 carbon atoms. It is to be understood further that said term "C₆-C₁₁" is to be interpreted as any sub-range comprised therein, *e*.*g*. C₅-C₁₀, C₆-C₉, C₇-C₈; preferably C₅-C₆.

As used herein, the term "C₅-C₁₀", as used throughout this text, *e.g*. in the context of the definitions of "C₅-C₁₀-heteroaryl", is to be understood as meaning a heteroaryl group having a finite number of carbon atoms of 5 to 10, in addition to the one or more heteroatoms present in the ring *i.e.* 5, 6, 7, 8, 9, or 10 carbon atoms, preferably 5, 6, or 10 carbon atoms. It is to be understood further that said term "C₅-C₁₀" is to be interpreted as any sub-range comprised therein, *e*.*g*. C₆-C₉, C₇-C₈, C₇-C₈; preferably C₅-C₆.

As used herein, the term "C₁-C₃", as used throughout this text, *e.g.* in the context of the definitions of "C₁-C₃-alkylene", is to be understood as meaning an alkylene group as defined *supra* having a finite number of carbon atoms of 1 to 3, *i.e.* 1, 2, or 3. It is to be understood further that said term "C₁-C₃" is to be interpreted as any sub-range comprised therein, *e*.*g*. C₁-C₂, or C₂-C₃.

As used herein, the term "one or more times", e.g. in the definition of the substituents of the compounds of the general formulae of the present invention, is understood as meaning "one, two, three, four or five times, particularly one, two, three or four times, more particularly one, two or three times, even more particularly one or two times".

The term "isomers" is to be understood as meaning chemical compounds with the same number and types of atoms as another chemical species. There are two main classes of isomers, constitutional isomers and stereoisomers.

The term "constitutional isomers" is to be understood as meaning chemical compounds with the same number and types of atoms, but they are connected in differing sequences. There are functional isomers, structural isomers, tautomers or valence isomers.

In "stereoisomers", the atoms are connected sequentially in the same way, such that condensed formulae for two isomeric molecules are identical. The isomers differ, however, in the way the atoms are arranged in space. There are two major sub-classes of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers.

In order to limit different types of isomers from each other reference is made to IUPAC Rules Section E (Pure Appl Chem 45, 11-30, 1976).

### FURTHER EMBODIMENTS

The compounds of the present invention according to Formula (I) can exist in free form or in a salt form. A suitable pharmaceutically acceptable salt of the 3-H-pyrazolopyridines of the present invention may be, for example, an acid-addition salt of a 3-H-pyrazolopyridine of the invention which is sufficiently basic, for example, an acid-addition salt with, for example, an inorganic or organic acid, for example hydrochloric, hydrobromic, sulphuric, phosphoric, trifluoroacetic, paratoluenesulphonic, methylsulphonic, citric, tartaric, succinic or maleic acid. In addition, another suitable pharmaceutically acceptable salt of a 3-H-pyrazolopyridine of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with *N*-methylglucamine, dimethyl-glucamine, ethyl-glucamine, lysine, 1,6-hexadiamine, ethanolamine, glucosamine, sarcosine, serinol, tris-hydroxy-methyl-aminomethane, aminopropandiol, sovak-base, 1-amino-2,3,4-butantriol.

The compounds of the present invention according to Formula (I) can exist as N-oxides which are defined in that at least one nitrogen of the compounds of the general Formula (I) may be oxidized.

The compounds of the present invention according to Formula (I) can exist as solvates, in particular as hydrates, wherein compounds of the present invention according to Formula (I) may contain polar solvents, in particular water, as structural element of the crystal lattice of the compounds. The amount of polar solvents, in particular water, may exist in a stoichiometric or unstoichiometric ratio. In case of stoichiometric solvates, *e*.*g*. hydrates, hemi-, (semi-), mono-, sesqui-, di-, tri-, tetra-, penta- etc. solvates or hydrates are possible.

The compounds of the present invention according to Formula (I) can exist as prodrugs, e.g. as *in vivo* hydrolysable esters. As used herein, the term "*in vivo* hydrolysable ester" is understood as meaning an *in vivo* hydrolysable ester of a compound of formula (I) containing a carboxy or hydroxyl group, for example, a pharmaceutically acceptable ester which is hydrolysed in the human or animal body to produce the parent acid or alcohol. Suitable pharmaceutically acceptable esters for carboxy groups include for example alkyl, cycloalkyl and optionally substituted phenylalkyl, in particular benzyl esters, C₁-C₆ alkoxymethyl esters, *e.g*. methoxymethyl, C₁-C₆ alkanoyloxymethyl esters, e.g. pivaloyloxymethyl, phthalidyl esters, C₃-C₁₀ cycloalkoxy-carbonyloxy-C₁-C₆ alkyl esters, *e.g*. 1-cyclohexylcarbonyloxyethyl; 1,3-dioxolen-2-onylmethyl esters, *e*.*g*. 5-methyl-1,3-dioxolen-2-onylmethyl; and C₁-C₆-alkoxycarbonyloxyethyl esters, *e*.*g*. 1-methoxycarbonyloxyethyl, and may be formed at any carboxy group in the compounds of this invention. An *in vivo* hydrolysable ester of a compound of formula (I) containing a hydroxyl group includes inorganic esters such as phosphate esters and α-acyloxyalkyl ethers and related compounds which as a result of the *in vivo* hydrolysis of the ester breakdown to give the parent hydroxyl group. Examples of α-acyloxyalkyl ethers include acetoxymethoxy and 2,2-dimethylpropionyloxymethoxy. A selection of *in vivo* hydrolysable ester forming groups for hydroxyl include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

The compounds of the present invention according to Formula (I) and salts, solvates, N-oxides and prodrugs thereof may contain one or more asymmetric centers. Asymmetric carbon atoms may be present in the (R) or (S) configuration or (R,S) configuration. Substituents on a ring may also be present in either cis or trans form. It is intended that all such configurations (including enantiomers and diastereomers), are included within the scope of the present invention. Preferred stereoisomers are those with the configuration which produces the more desirable biological activity. Separated, pure or partially purified configurational isomers or racemic mixtures of the compounds of this invention are also included within the scope of the present invention. The purification of said isomers and the separation of said isomeric mixtures can be accomplished by standard techniques known in the art.

Another embodiment of the present invention relates to the use of a compound of general formula (11) as mentioned *below* for the preparation of a compound of general formula (I) as defined *supra*.

Yet another embodiment of the present invention relates to the use of a compound of general formula (1) as mentioned *below* for the preparation of a compound of general formula (I) as defined *supra*, further to the use of a compound of general formula (1) as mentioned *below* for the preparation of a compound of general formula (Ia) as mentioned below.

Yet another embodiment of the present invention relates to the use of a compound of general formula (3) as mentioned *below* for the preparation of a compound of general formula (I) as defined *supra.*

Another embodiment of the present invention relates to the use of a compound of general formula (12) as mentioned *below* for the preparation of a compound of general formula (I) as defined *supra*, further to the use of a compound of general formula (12) as mentioned *below* for the preparation of a compound of general formula (Ia) as mentioned below.

Another embodiment of the present invention relates to the use of a compound of general formula (14) as mentioned *below* for the preparation of a compound of general formula (I) as defined *supra*, further to the use of a compound of general formula (14) as mentioned *below* for the preparation of a compound of general formula (Ia) as mentioned below.

The compounds of the present invention can be used in treating diseases of dysregulated vascular growth or diseases which are accompanied with dysregulated vascular growth. Especially, the compounds effectively interfere with cellular Tie2 signalling. The compounds of the present invention are selective inhibitors of Tie2 kinase activity vs. InsR kinase activity.

Therefore, another aspect of the present invention is a use of the compound of general formula (I) described *supra* for manufacturing a pharmaceutical composition for the treatment of diseases of dysregulated vascular growth or of diseases which are accompanied with dysregulated vascular growth.

In particular, said use is in the treatment of diseases, wherein the diseases are tumors and/or metastases thereof. The compounds of the present invention can be used in particular in therapy and prevention of tumor growth and metastases, especially in solid tumors of all indications and stages with or without pre-treatment if the tumor growth is accompanied with persistent angiogenesis, principally including all solid tumors, e.g. breast, colon, renal, ovarian, prostate, thyroid, lung and/or brain tumors, melanoma, or metastases thereof.

Additionally, said use is in the treatment of chronic myelogeneous leukaemia (or "CML"), acute myelogenous leukaemia (or "AML"), acute lymphatic leukaemia, acute lymphocytic leukaemia (or "ALL"), chronic lymphocytic leukaemia, chronic lymphatic leukaemia (or "CLL") as well as other myeloid precursor hyperplasias such as polycythemia vera and myelofibrosis.

Another use is in the treatment of diseases, wherein the diseases are retinopathy, other angiogenesis dependent diseases of the eye, in particular cornea transplant rejection or age-related macular degeneration.

Yet another use is in the treatment of rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and inflammatory diseases of the bowel, such as, for example, Crohn's disease.

A further use is in the suppression of the development of atherosclerotic plaque formation and for the treatment of coronary and peripheral artery disease.

Another use is in the treatment of diseases associated with stromal proliferation or characterized by pathological stromal reactions and for the treatment of diseases associated with deposition of fibrin or extracellular matrix, such as, for example, fibrosis, cirrhosis, carpal tunnel syndrome.

Yet another use is in the treatment of gynaecological diseases where inhibition of angiogenic, inflammatory and stromal processes with pathological character can be inhibited, such as, for example, endometriosis, pre-eclampsia, postmenopausal bleeding and ovarian hyperstimulation.

Another use is in the treatment of diseases, wherein the diseases are ascites, oedema such as brain tumor associated oedema, high altitude trauma, hypoxia induced cerebral oedema, pulmonary oedema and macular oedema or oedema following burns and trauma, chronic lung disease, adult respiratory distress syndrome, bone resorption and for the treatment of benign proliferating diseases such as myoma, benign prostate hyperplasia.

A further use is in wound healing for the reduction of scar formation, and for the reduction of scar formation during regeneration of damaged nerves.

Yet another aspect of the invention is a method of treating a disease of dysregulated vascular growth or diseases which are accompanied with dysregulated vascular growth, by administering an effective amount of a compound of general formula (I) described *supra*.

In particular, the diseases of said method are tumors and/or metastases thereof, in particular solid tumors of all indications and stages with or without pre-treatment if the tumor growth is accompanied with persistent angiogenesis, principally including all solid tumors, *e*.*g*. breast, colon, renal, ovarian, prostate, thyroid, lung and/or brain tumors, melanoma, or metastases thereof.

Additionally, diseases of said method are chronic myelogeneous leukaemia (or "CML"), acute myelogenous leukaemia (or "AML"), acute lymphatic leukaemia, acute lymphocytic leukaemia (or "ALL"), chronic lymphocytic leukaemia, chronic lymphatic leukaemia (or "CLL") as well as other myeloid precursor hyperplasias such as polycythemia vera and myelofibrosis.

Further diseases of said method are retinopathy, other angiogenesis dependent diseases of the eye, in particular cornea transplant rejection or age-related macular degeneration.

Further diseases of said method are rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and inflammatory diseases of the bowel, such as, for example, Crohn's disease.

Further diseases of said method are the development of atherosclerotic plaques and coronary and peripheral artery diseases.

Further diseases of said method are diseases associated with stromal proliferation or characterized by pathological stromal reactions and diseases associated with deposition of fibrin or extracellular matrix, such as, for example, fibrosis, cirrhosis, carpal tunnel syndrome.

Further diseases of said method are gynaecological diseases where inhibition of angiogenic, inflammatory and stromal processes with pathological character can be inhibited, such as, for example, endometriosis, pre-eclampsia, postmenopausal bleeding and ovarian hyperstimulation.

Further diseases of said method are ascites, oedema such as brain tumor associated oedema, high altitude trauma, hypoxia induced cerebral oedema, pulmonary oedema and macular oedema or oedema following burns and trauma, chronic lung disease, adult respiratory distress syndrome, bone resorption and benign proliferating diseases such as myoma, benign prostate hyperplasia.

Another aspect of the present invention is a pharmaceutical composition which comprises a compound of general formula (I) as defined above, or as obtainable by a method described in this invention, or a pharmaceutically acceptable salt or an N-oxide or a solvate or a prodrug of said compound, and a pharmaceutically acceptable diluent or carrier, the composition being particularly suited for the treatment of diseases of dysregulated vascular growth or of diseases which are accompanied with dysregulated vascular growth as explained above.

In order to use the compounds of the present invention as pharmaceutical products, the compounds or mixtures thereof may be provided in a pharmaceutical composition, which, as well as the compounds of the present invention for enteral, oral or parenteral application contains suitable pharmaceutically acceptable organic or inorganic inert base material, *e*.*g*. purified water, gelatine, gum Arabic, lactate, starch, magnesium stearate, talcum, vegetable oils, polyalkyleneglycol, *etc*.

The pharmaceutical compositions of the present invention may be provided in a solid form, *e*.*g*. as tablets, dragées, suppositories, capsules or in liquid form, *e.g.* as a solution, suspension or emulsion. The pharmaceutical composition may additionally contain auxiliary substances, e.g. preservatives, stabilisers, wetting agents or emulsifiers, salts for adjusting the osmotic pressure or buffers.

For parenteral applications, (including intravenous, subcutaneous, intramuscular, intravascular or infusion), sterile injection solutions or suspensions are preferred, especially aqueous solutions of the compounds in polyhydroxyethoxy containing castor oil.

The pharmaceutical compositions of the present invention may further contain surface active agents, e.g. salts of gallenic acid, phospholipids of animal or vegetable origin, mixtures thereof and liposomes and parts thereof.

For oral application tablets, dragées or capsules with talcum and/or hydrocarbon-containing carriers and binders, e.g. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. A dose can be administered as unit dose or in part thereof and distributed over the day. Accordingly the optimum dosage may be determined by the practitioner who is treating any particular patient.

It is possible for compounds of general formula (I) of the present invention to be used alone or, indeed in combination with one or more further drugs, particularly anti-cancer drugs or compositions thereof. Particularly, it is possible for said combination to be a single pharmaceutical composition entity, e.g. a single pharmaceutical formulation containing one or more compounds according to general formula (I) together with one or more further drugs, particularly anti-cancer drugs, or in a form, e.g. a "kit of parts", which comprises, for example, a first distinct part which contains one or more compounds according to general formula (I), and one or more further distinct parts each containing one or more further drugs, particularly anti-cancer drugs. More particularly, said first distinct part may be used concomitantly with said one or more further distinct parts, or sequentially. In addition, it is possible for compounds of general formula (I) of the present invention to be used in combination with other treatment paradigms, particularly other anti-cancer treatment paradigms, such as, for example, radiation therapy.

Another aspect of the present invention is a method which may be used for preparing the compounds according to the present invention.

### EXPERIMENTAL DETAILS AND GENERAL PROCESSES

The following table lists the abbreviations used in this paragraph and in the Examples section as far as they are not explained within the text body. NMR peak forms are stated as they appear in the spectra, possible higher order effects have not been considered. Assignments of substitution degrees (e.g. CH₃, CH₂, CH or Cq signals) of carbon atoms in ¹³C-NMR spectra are based on ¹³C-DEPT NMR analysis. Chemical names were generated using AutoNom2000 as implemented in MDL ISIS Draw. In some cases generally accepted names of commercially available reagents were used in place of AutoNom2000 generated names. The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, *e.g*. from Separtis such as Isolute® Flash silica gel or Isolute® Flash NH₂ silica gel in combination with a Flashmaster II autopurifier (Argonaut/Biotage) and eluents such as gradients of hexane/EtOAc or DCM/ethanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid or aqueous ammonia. In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the persion skilled in the art, or be used as salts in subsequent biological assays. It is to be understood that the specific form (e.g. salt, free base...) of a compound of the present invention as isolated as described herein is not necessarily the only form in which said compound can be applied to a biological assay in order to quantify the specific biological activity. Reactions employing microwave irradiation may be run with a Biotage Initator® microwave oven optionally equipped with a robotic unit. The reported reaction times employing microwave heating are intended to be understood as fixed reaction times after reaching the indicated reaction temperature.

| **Abbreviation** | **Meaning** |
|---|---|
| Ac | Acetyl |
| Boc | *tert*-butyloxycarbonyl |
| br | Broad |
| Cl | chemical ionisation |
| d | Doublet |
| dd | doublet of doublet |
| DCM | Dichloromethane |
| DIPEA | *N,N*-diisopropylethyl amine |
| DMF | *N,N*-dimethylformamide |
| DMSO | dimethyl sulfoxide |
| eq. | Equivalent |
| ESI | electrospray ionisation |
| GP | general procedure |
| HPLC | high performance liquid chromatography |
| LC-MS | Liquid chromatography mass spectrometry |
| m | Multiplet |
| mc | centred multiplet |
| MS | mass spectrometry |
| NMR | nuclear magnetic resonance spectroscopy : chemical shifts (δ) are given in ppm. |
| Pg | protecting group |
| q | Quartet |
| rf | at reflux |
| r.t. or rt | room temperature |
| s | Singlet |
| sept. | Septet |
| t | Triplet |
| TEA | Triethylamine |
| TFA | trifluoroacetic acid |
| THF | Tetrahydrofuran |

The following schemes and general procedures illustrate general synthetic routes to the compounds of general formula I of the invention and are not intended to be limiting. It is obvious to the person skilled in the art that the order of transformations as exemplified in Schemes 1 to 8 can be modified in various ways. The order of transformations exemplified in Schemes 1 to 8 is therefore not intended to be limiting. In addition, interconversion of substituents, for example of residues R¹, R², R³, R⁴, R⁵, R^{a}, R^{b}, R^{c} R^{d1}, R^{d2} and R^{d3} can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999).

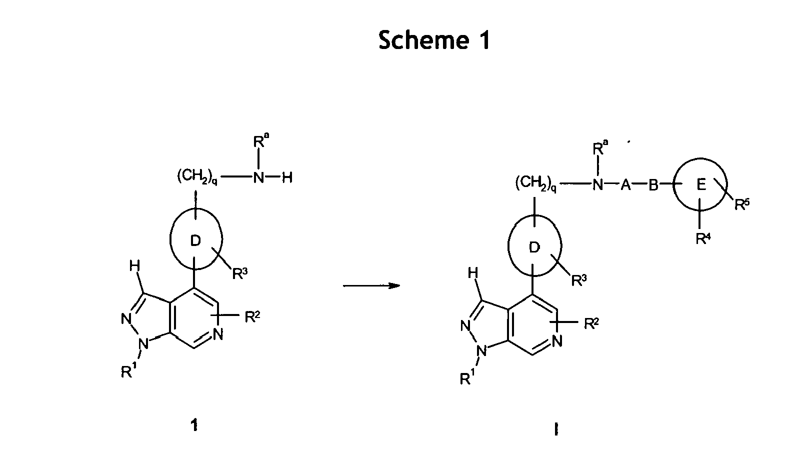

***Scheme 1** General procedure for the preparation of compounds of the general formula (I) by functionalization of amines of general formula **1**, wherein A, B, D, E, R^{a}, R¹, R², R³, R⁴, R⁵ and q are as defined in the description and claims of this invention*.

Compounds of general formula (I) can be synthesized according to the procedure depicted in **Scheme 1** from amines of general formula **1** by reaction with, for example, a suitably functionalized isocyanate (leading to ureas), a suitably functionalized sulfonyl chloride (leading to sulfonyl amides) or a suitably functionalized acid chloride (leading to carboxylic amides), in the presence of a suitable base as necessary, e.g. pyridine or triethylamine, which may also be used as solvent, optionally in the presence of an inert solvent, e.g. dichloromethane, acetonitrile, DMF or THF, at temperatures ranging from -20 °C to the boiling point of the solvent, whereby room temperature is preferred.

A variety of suitable isocyanates for the above described transformation is described in the literature or commercially available. The person skilled in the art is well aware of alternative methods of forming ureas, which may be of special importance in cases were the respective isocyanates are not readily available (see Schemes 2, 2b, 2c for exemplary, more specific urea-forming processes).

Processes for the preparation of functionalized (hetero)aryl sulfonyl chlorides are as well known to the person skilled in the art. Introduction of sulfonyl groups may be accomplished by sulfonylation or by oxidation of thiols. Sulfonyl chlorides may be accessible in turn from sulfonic acids by reaction with e.g. thionyl chloride, sulfuryl chloride, PCl₅, POCl₃ or oxalyl chloride.

In the case of the transformation of amines of general formula 1 into amides of general formula **I** [with A being -C(O)-], it is also possible to react amines of general formula 1 with an appropriate ester according to a method described in J. Org. Chem. 1995, 8414 in the presence of trimethylaluminium and in suitable solvents such as toluene, at temperatures of 0 °C to the boiling point of the solvent. For amide formations, however, all processes that are known from peptide chemistry to the person skilled in the art are also available. For example, the corresponding acid, which may be obtained from the corresponding ester by saponification, can be reacted with amines of general formula 1 in aprotic polar solvents, such as, for example, DMF, *via* an activated acid derivative, which is obtainable, for example, with hydroxybenzotriazole and a carbodiimide, such as, for example, diisopropylcarbodiimide (DIC), at temperatures of between 0 °C and the boiling point of the solvent, preferably at 80 °C, or else with preformed reagents, such as, for example, 0-(7-azabenzotriazol-1-yl)-1,1,3,3-tetramethyluronium hexafluorophosphate (HATU) (see for example Chem. Comm. 1994, 201), at temperatures of between 0 °C and the boiling point of the solvent, preferably at room temperature, or else with activating agents such as dicyclohexylcarbodiimide (DCC)/dimethylaminopyridine (DMAP) or *N*-ethyl-*N*'-dimethylaminopropylcarbodiimide (EDCI)/dimethylaminopyridine (DMAP) or T3P (1-propanephosphoric acid cyclic anhydride). The addition of a suitable base such as, for example, *N*-methylmorpholine, TEA, DIPEA may be necessary. Amide formation may also be accomplished via the acid halide (which can be formed from a carboxylic acid by reaction with e.g. oxalyl chloride, thionyl chloride or sulfuryl chloride), mixed acid anhydride (which can be formed from a carboxylic acid by reaction with e.g. isobutyrochloroformiate), imidazolide (which can be formed from a carboxylic acid by reaction with e.g. carbonyldiimidazolide) or azide (which can be formed from a carboxylic acid by reaction with e.g. diphenylphosphorylazide DPPA).

The carboxylic acids required for the above described amide coupling reactions are either commercially available or are accessible from commercially available carboxylic esters or nitriles. Alternatively, (hetero)aryls bearing a methylenenitrile substituent are easily accessible from the respective halides via a nucleophilic substitution reaction (e.g. KCN, cat. KI, EtOH/H₂O). Incorporation of additional functionality into commercially available starting materials can be accomplished by a multitude of aromatic transformation reactions known to the person skilled in the art, including, but not limited to, electrophilic halogenations, electrophilic nitrations, Friedel-Crafts acylations, nucleophilic displacement of fluorine by oxygen nucleophiles and transformation of (hetero)aryl carboxylic acids into amides and subsequent reduction into benzylic amines, whereby the latter two methods are of particular relevance for the introduction of ether and/or aminomethylene side chains.

Benzylic nitriles and esters (and heteroaryl analogs thereof) can be efficiently alkylated at the benzylic position under basic conditions and subsequently hydrolyzed to the corresponding alkylated acids. Conditions for α-alkylations of nitriles and esters include, but are not limited to, the use of alkyl bromides or alkyl iodides as electrophiles under basic conditions in the presence or absence of a phase-transfer catalyst in a mono- or biphasic solvent system. Particularly, by using excess alkyl iodides as electrophilic species α,α-dialkylated nitriles are accessible. More particularly, by using 1,ω-dihaloalkyls as electrophiles cycloalkyl moieties can be installed at the benzylic position of nitriles and esters (J. Med. Chem. 1975, 18, 144; W02003022852). Even more particularly, by using a 1,2-dihaloethane, such as, for example, 1,2-dibromoethane or 1-bromo-2-chloroethane, a cyclopropane ring can be installed at the benzylic position of a nitrile or ester. The hydrolysis of nitriles to yield carboxylic acids can be accomplished, as known to the person skilled in the art, under acid or base-mediated conditions.

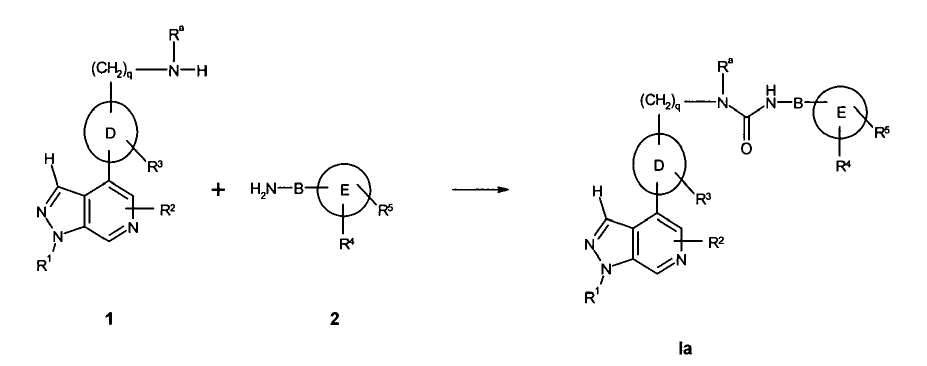

***Scheme 2** More specific procedure for the preparation of compounds of the general formula **(Ia)** by reacting amines of general formula **1** with (hetero)aryl amines of general formula **2** in the presence of triphosgene, for example, wherein B, D, E, R^{a}, R¹, R², R³, R⁴, R⁵ and q are as defined in the description and claims of this invention*.

An alternative, more specific process of generating ureas of general formula **Ia** is depicted in **Scheme 2**. In this case, urea formation starting from amines of general formula **1** may be achieved by coupling with a second functionalized amine of general formula **2** via *in situ* transformation of one of the reacting amines into the respective carbamoyl chloride, aryl- or alkenylcarbamate (see for example J. Org. Chem. 2005, 70, 6960 and references cited therein). This process may provide an alternative to the formation and isolation of the respective isocyanate derived from one of the starting amines (see for example Tetrahedron Lett. 2004, 45, 4769). More particularly, ureas of formula **Ia** may be formed from two suitably functionalized amines and a suitable phosgene equivalent, preferably triphosgene, in an inert solvent, preferably acetonitrile, at temperatures ranging from -20 °C to the boiling point of the solvent, whereby room temperature is preferred.

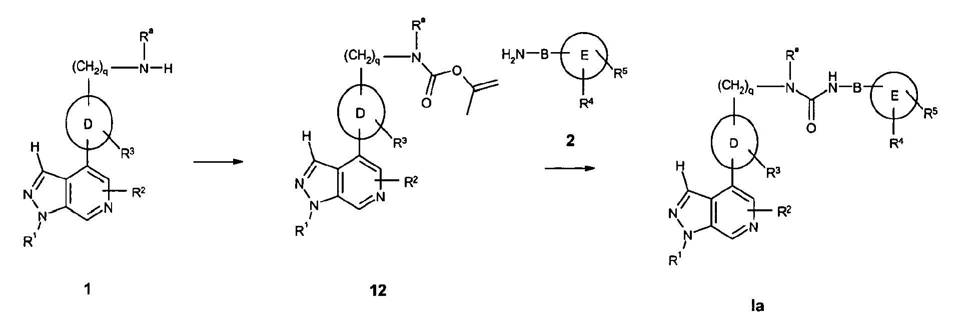

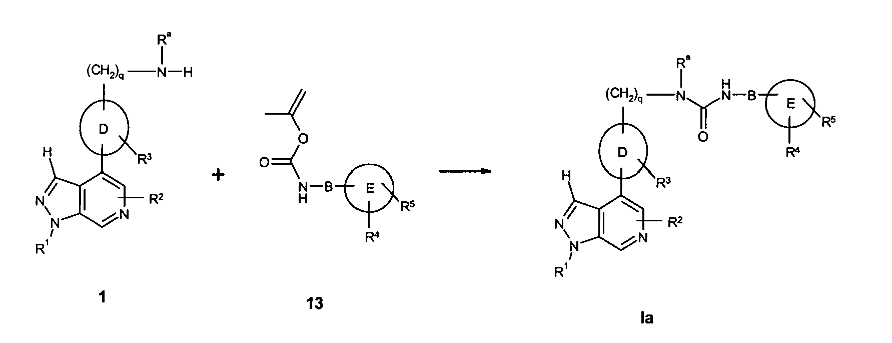

***Scheme 2b** Alternative more specific procedures for the preparation of compounds of the general formula **(Ia)** by either transforming amines of general formula **1** into their corresponding isopropenyl carbamates of general formula **12** and subsequent reaction with (hetero)aryl amines of general formula **2,** or reacting amines of general formula **1** with isopropenyl carbamates of general formula **13,** wherein B, D, E, R^{a}, R¹, R², R³, R⁴, R⁵ and q are as defined in the description and claims of this invention*.

The aforementioned alternative procedure for generating ureas of general formula **Ia** employing alkenylcarbamates, for example isopropenylcarbamates, is depicted in more detail in **Scheme 2b**. In analogy to the aforecited publication (J. Org. Chem. 2005, 70, 6960) transformation of amines of general formula **1** into their respective isopropenyl carbamates of general formula **12** can be accomplished by reaction with isopropenyl chloro formate in the presence of an appropriate base, such as, for example, N-methylmorpholine, in a suitable solvent, such as, for example, THF. Isopropenyl carbamates of general formula **12** can then be reacted, after isolation or *in situ,* with (hetero)aryl amines of general formula **2** in the presence of a suitable base, such as, for example, *N*-methyl pyrrolidine, in a suitable solvent, such as, for example THF, to yield ureas of general formula **Ia.** Alternatively, (hetero)aryl amines of general formula **2** can be transformed into their corresponding isopropenyl carbamates of general formula **13** employing condition as described above and subsequently reacted with amines of general formula **1** under conditions as described above to yield ureas of general formula **Ia.**

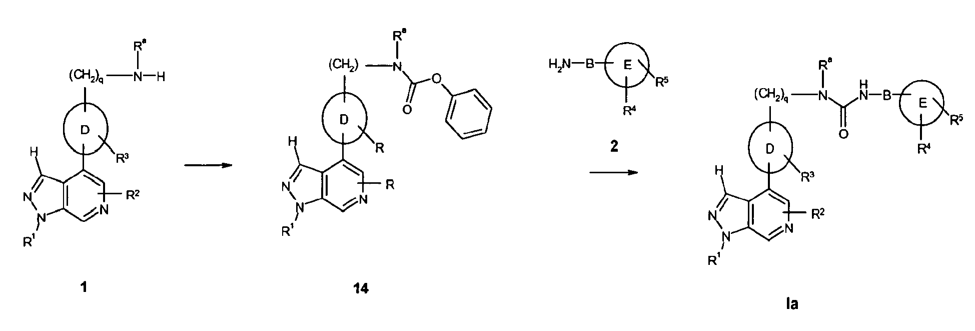

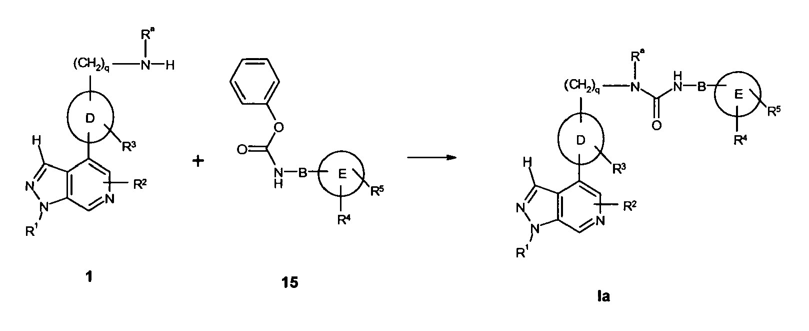

***Scheme 2c** Additional more specific procedures for the preparation of compounds of the general formula **(Ia)** by either transforming amines of general formula **1** into their corresponding phenyl carbamates of general formula **14** and subsequent reaction with (hetero)aryl amines of general formula **2,** or reacting amines of general formula **1** with isopropenyl carbamates of general formula **15,** wherein B, D, E, R^{a}, R¹, R², R³, R⁴, R⁵ and q are as defined in the description and claims of this invention*.

An additional method for generating ureas of general formula **Ia** employing aryl carbamates, for example phenyl carbamates or 4-NO₂-phenyl carbamates in analogy to procedures described for example in W02007064872 or W02005110994, is exemplified in **Scheme 2c**. Transformation of amines of general formula **1** into their respective phenyl carbamates of general formula **14** can be accomplished by reaction with phenyl chloro formate in the presence of an appropriate base, such as, for example, sodium carbonate, in a suitable solvent, such as, for example, THF. Phenyl carbamates of general formula **14** can then be reacted, after isolation or *in situ,* with (hetero)aryl amines of general formula **2** in the presence of a suitable base, such as, for example, pyridine, in a suitable solvent, such as, for example THF, to yield ureas of general formula **Ia.** Alternatively, (hetero)aryl amines of general formula **2** can be transformed into their corresponding phenyl carbamates of general formula **15** employing condition as described above and subsequently reacted with amines of general formula **1** under conditions as described above to yield ureas of general formula **Ia.**

Processes for the preparation of functionalized (hetero)aryl amines as coupling partners for the above described transformation are well known to the person skilled in the art. Starting from commercially available (hetero)aryl amines or nitro(hetero)arylenes well known transformations, including, but not limited to, alkylations, nucleophilic or electrophilic substitutions, acylations, halogenations, nitrations, sulfonylations, (transition) metal catalyzed couplings, metallations, rearrangements, reductions, and/or oxidations may be applied to prepare functionalized amines to be used in the urea formation step. In addition to specific procedures given in the following experimental section, detailed procedures may be found in the scientific and patent literature (see for example WO2005051366, W02005110410, W02005113494, W02006044823, and W02006124462; W02007064872 and W02005110994). 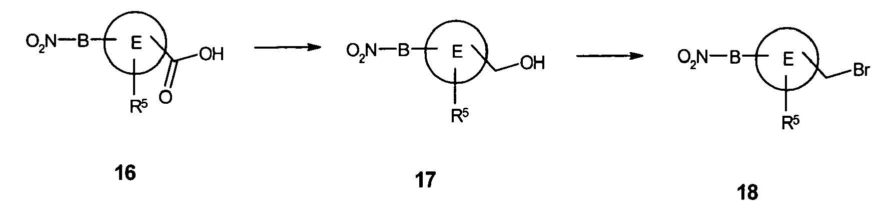 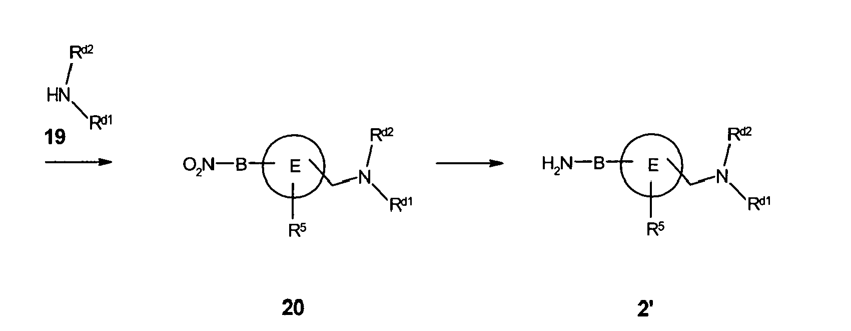 ***Scheme 2d** Reaction sequence for the preparation of especially suitable amines of general formula **2'** for urea formations according to Schemes 2, 2b and 2c, in which (hetero)aryl carboxylic acids of general formula **16** are transformed into benzylic alcohols of general formula **17,** then into benzylic bromides of general formula **18,** then reacted with amines of general formula **19** and finally transformed into amines of general formula **2',** wherein E, R⁵, R^{d1} and R^{d2} are as defined in the description and claims of this invention*.

A reaction sequence for the preparation of especially suitable (hetero)aryl amines for the above described urea formation processes is depicted in **Scheme 2d.** (Hetero)aryl carboxylic acids of general formula **16** can be reduced to benzylic alcohols of general formula **17** under standard conditions as known to the person skilled in the art, for example, by reaction with borane-THF complex or NaBH₄/I₂. Bromination of benzylic alcohols of general formula **17** leading to benzylic bromides of general formula **18** is feasible employing, for example, CBr₄ in the presence of triphenylphosphine. Reaction of benzylic bromides of general formula **18** with amines of general formula **19** gives rise to benzylic amines of general formula **20** which can subsequently be reduced under standard conditions as known to the person skilled in the art, for example, by Pd-catalyzed hydrogenation or by reaction with SnCl₂, into amines of general formula **2'.** 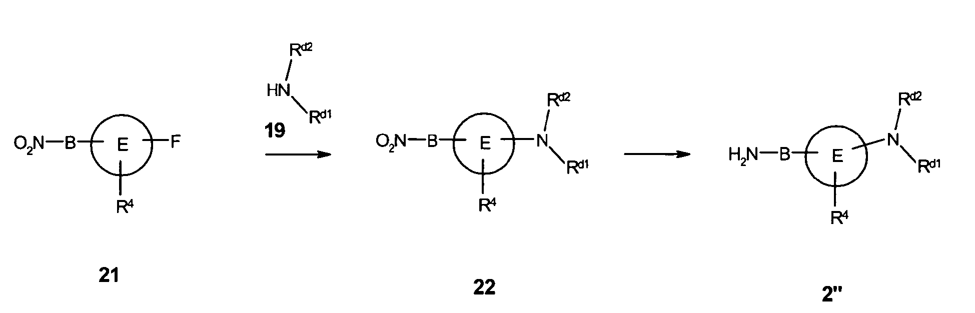 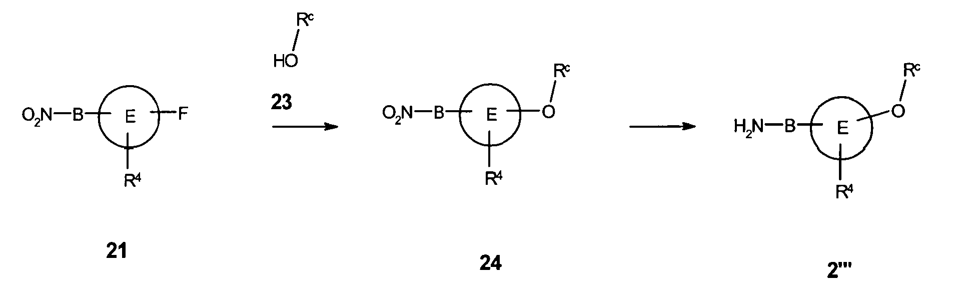 ***Scheme 2e** Further reaction sequence for the preparation of especially suitable amines of general formula **2''** and **2'''** for urea formations according to Schemes 2, 2b and 2c, in which (hetero)aryl fluorides of general formula **21** are reacted either with amines of general formula **19** or with alcohols of general formula **23** to yield after subsequent nitro reduction amines of general formula **2''** or general formula **2''',** respectively, wherein E, R⁴, R^{c}, R^{d1} and R^{d2} are as defined in the description and claims of this invention*.

Further reaction sequences for the preparation of especially suitable (hetero)aryl amines for the above described urea formation processes are depicted in **Scheme 2e.** (Hetero)aryl fluorides of general formula **21** are reacted with amines of general formula **19** in a nucleophilic aromatic substitution reaction in the presence of a suitable base, such as, for example, NaHCO₃, in a suitable solvent such as, for example, DMF, under heating, optionally by microwave irradiation, to form anilines of general formula **22.** Alternatively, reaction with alcohols of general formula **23** in the presence of a suitable base, such as, for example, cesium carbonate, optionally under heating, gives rise to nitro ethers of general formula **24.** Subsequent nitro reduction leads to amines of general formula **2''** or general formula **2''',** respectively.

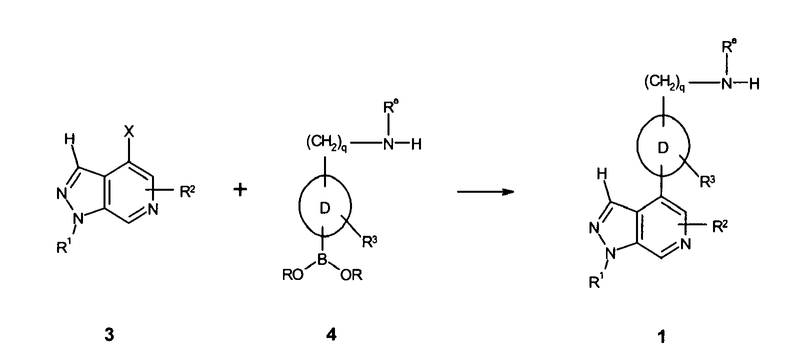

***Scheme 3** General procedure for the preparation of amines of the general formula **1** by transition metal-catalyzed coupling of 4-halopyrazolopyridines of general formula **3** with boronic acids or their respective esters of general formula **4**, wherein D, R^{a}, R¹, R², R³, and q are as defined in the description and claims of this invention and X represents Cl, Br or I and R represents H or alkyl or wherein the two OR groups form a pinacolate.*

Amines of general formula **1** are accessible, for example, by transition metal-catalyzed coupling of an appropriate 4-halopyrazolopyridine of general formula **3** with boronic acids or their respective esters of general formula **4.** More particularly, amines of formula **1** can be prepared starting from a halogenated pyrazolopyridine **3** by Pd-catalyzed Suzuki-type coupling reactions with (hetero)aryl boronic acids **4** or even more particularly with their respective boronate esters (e.g. a pinacolate ester). Transition metal-catalyzed couplings of heteroaryl halides with (hetero)aryl boronic acids or (hetero)aryl boronate esters are well known to the person skilled in the art. Various catalyst/ligand/base/solvent combinations have been published in the scientific literature (see for example Tetrahedron 2005, 61, 5131 and references cited therein; Eur. J. Org. Chem. 2006, 1917 and references cited therein; Eur. J. Org. Chem. 2006, 2063 and references cited therein), which allow a fine-tuning of the required reaction conditions in order to allow for a broad set of additional functional groups on both coupling partners. Alternatively, the boronic acids of general formula **4** may be replaced by, for example, a suitably substituted trifluoroboronate salt or a suitably substituted stannane. Conditions for Stille-type couplings of aryl- or heteroaryl stannanes to aryl or heteroaryl halides employing a Pd catalyst and optionally a mediator are well known to the person skilled in the art. In some cases introduction of an amine protecting group may facilitate the coupling reaction exemplified in Scheme 3. Appropiate protecting groups and their introduction and cleavage are well-known to the person skilled in the art (see for example T.W. Greene and P.G.M. Wuts in Protective Groups in Organic Synthesis, 3rd edition, Wiley 1999). Alternatively, the amino group in the coupling partner **4** can be replaced by a nitro group which is reduced to the corresponding amino group subsequent to the coupling reaction.

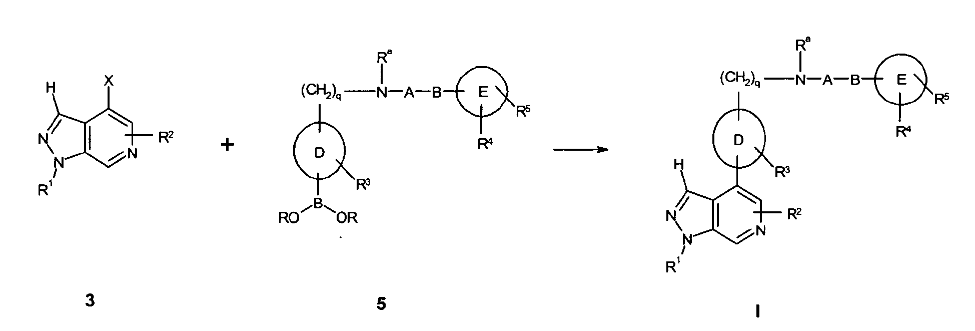

***Scheme 4** General procedure for the preparation of compounds of the general formula **(I)** by transition metal-catalyzed coupling of 4-halopyrazolopyridines of general formula **3** with boronic acids or their respective esters of general formula **5,** wherein A, B, D, E, R^{a}, R¹, R², R³, R⁴, R⁵ and q are as defined in the description and claims of this invention and X represents Cl, Br or I and R represents H or alkyl or wherein the two OR groups form a pinacolate.*

A more convergent alternative to the process exemplified before is depicted in Scheme 4, in which compounds of the present invention of general formula **I** are prepared by a transition metal catalyzed coupling of an appropriate halo precursor of general formula **3** and appropriately substituted boronic acids or boronate esters of general formula **5.** More particularly, compounds of the present invention can be prepared starting from a halogenated pyrazolopyridine **3** by Pd-catalyzed Suzuki-type coupling reactions with (hetero)aryl boronic acids **5** or even more particularly with their respective boronate esters (e.g. a pinacolate ester). Functionalized boronic acids and their respective pinacolate esters of general formula **5** can be prepared e.g. by urea formation or sulphonamide formation or amide coupling of accordingly substituted anilines (e.g. of general formula **4**). In addition, boronic acids or boronate esters can be introduced into aryl or heteroaryl compounds *inter alia* by substituting halogen atoms. This substitution can be accomplished by metallation followed by electrophilic borylation (Org. Biomol. Chem. 2004, 2, 852) or by direct Pd- or Cu-catalyzed borylation (Synlett 2003, 1204 and references cited therein; Org. Lett. 2006, 8, 261). Interconversion of boronic acids into the respective esters (e.g. their pinacolate esters) can be accomplished under standard conditions (for example by treatment with pinacol in EtOH at r.t.).

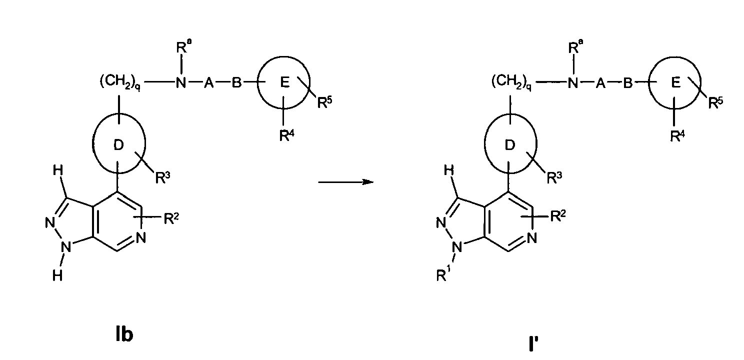

***Scheme 5** General procedure for the preparation of compounds of the general formula **(I')** with R¹* ≠ H *by e.g. alkylation or acylation of compounds of general formula **Ib,** wherein A, B, D, E, R^{a}, R¹, R², R³, R⁴, R⁵ and q are as defined in the description and claims of this invention*.

Compounds of general formula **I'** with R¹ ≠ H (Scheme 5) are accessible from precursors of general formula **1** or **3** (with R¹ ≠ H) by, for example, the aforementioned transformations. Alternatively, as depicted in Scheme 5, compounds of general formula **I'** with R¹ ≠ H are accessible from the respective 1 H-pyrazolopyridines of general formula **Ib** by, for example, alkylation or acylation reactions and subsequent transformations. This process is of particular importance if appropriately substituted hydrazines are not readily available (see below). Introduction of R¹-groups can be achieved employing various conditions for introducing substituents to nitrogen atoms as known to the person skilled in the art. These conditions include, but are not limited to, alkylations under basic conditions employing alkyl-, allyl-, benzylhalides or α-halocarbonyl compounds as electrophiles (e.g. W02005056532; Chem. Pharm. Bull. 1987, 35, 2292; J. Med. Chem. 2005, 48, 6843), alkylations under reductive conditions employing aldehydes as electrophiles and an appropriate reducing agent (e.g. BH₃•pyr, NaBH(OAc)₃, NaBH₃CN, NaBH₄), Mitsunobu-type alkylations employing primary or secondary alcohols as electrophiles (e.g. Tetrahedron 2006, 62, 1295; Bioorg. Med. Chem. Lett. 2002, 12, 1687), or *N*-acylations (see for example J. Med. Chem. 2005, 48, 6843) optionally followed by amide reduction.

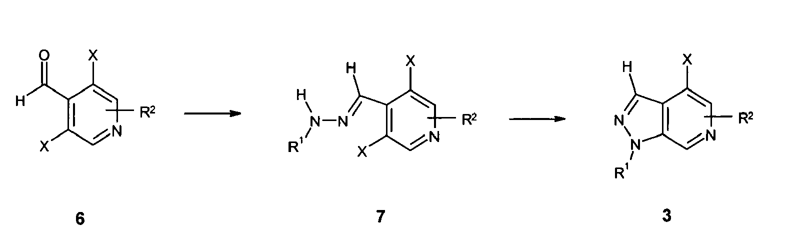

***Scheme 6** General procedure for the preparation of 4-halopyrazolopyridines of the general formula **3** by transformation of carbaldehydes of general formula **6** into their respective hydrazones of general formula **7** and cyclization, wherein R¹and R² are as defined in the description and claims of this invention and X represents Cl, Br or I.*

Halides of general formula **3** are accessible, for example, as depicted in Scheme **6,** from carbaldehydes of general formula **6** by transformation into hydrazones of formula **7** and subsequent cyclization. It is to be understood that hydrazone formation and cyclization can be accomplished in one preparative transformation or, alternatively, in two separate steps. More particularly, carbaldehydes of formula **6** can be reacted with hydrazine (e.g. hydrazine hydrate) or substituted hydrazines in an appropriate solvent, preferably in 1-PrOH, at an appropriate temperature, preferably at 100 to 120 °C, to yield hydrazones of formula **7** or halopyrazolopyridines of formula **3.** Isolated hydrazones of formula **7** can be cyclized to halopyrazolopyridines of formula **3** e.g. by applying basic conditions, preferably by reacting with sodium hydride, in an appropriate solvent, preferably DMF. A variety of substituted hydrazine building blocks required for the conversion of pyridines of formula **6** into intermediates of formula **7** and/or **3** is commercially available, either in form of their free base or as various types of salts (e.g. hydrochlorides, oxalates), which can be transformed into their respective free bases by alkaline treatment either before the cyclization or *in situ.* Additionally, substituted alkyl-, allyl-, and benzylhydrazines (or their respective hydrochloride salts) are accessible from the respective alkyl-, allyl- and benzylhalides, preferably the respective alkyl-, allyl- and benzylbromides, by nucleophilic substitution reaction with a protected hydrazine, such as BocNHNH₂, in an inert solvent, preferably MeOH, in the presence of an amine promoter, e.g. Et₃N, at temperatures ranging from room temperature up to the boiling point of the solvent, optionally followed by deprotection employing conditions known to the person skilled in the art, preferably, in the case of Boc deprotection, by treatment with HCl in a mixture of diethyl ether and methanol (for a representative procedure, see J. Med. Chem. 2006, 49, 2170). As an alternative to the use of hydrazine hydrate in the transformation exemplified in Scheme 6, protected analogues, e.g. Boc-hydrazine (also known as tert-butyl carbazate), benzyl hydrazine or para-methoxybenzyl hydrazine can be used instead. Removal of the respective protecting group is feasible by standard transformations as known to the person skilled in the art, e.g. by hydrogenation, acid treatment or base treatment. Carbaldeyhdes of general formula **6** are either commercially available or can be synthesized, for example, from the respective dihalopyridines by formylation reactions, more particularly, by metallation followed by formylation of the respective metallated species (see for example Tetrahedron Lett. 1996, 37, 2565, US6232320 or W02005110410).

As stated above, the order of transformations as exemplified in Scheme 4 and 6 is not intended to be limiting. For example, 3,5-dihalopyridine carbaldehydes of formula 6 can also be cross-coupled with an appropriately substituted boronic acid or boronic acid ester, for example of formula **4** or **5**, followed by pyrazolopyridine formation by reaction with, for example, hydrazine hydrate or a substituted hydrazine to yield compounds of formula **1** or **I.**

The respective R² substituent of compounds of the present invention of general formula **I** can be present in any of the afore- and below-mentioned synthetic intermediates or starting materials. Alternatively, a R² substituent can be introduced before or after any of the afore- or below-mentioned processes. One particular process for the introduction of R² substituents is exemplified in the following scheme and paragraph (Scheme 7). It is to be understood, that this process is not limited to the exemplified starting material, but can be applied to other commercially available pyridine starting materials or synthetic intermediates of processes exemplified in this application.

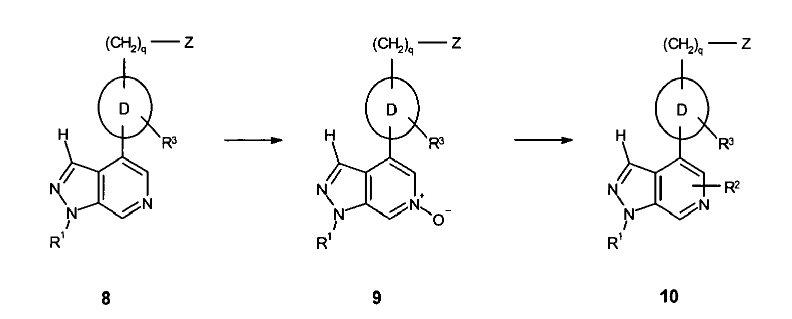

***Scheme 7** General exemplary procedure for the introduction of R² substituents by formation of N-oxides of general formula **9** and rearrangement optionally followed by subsequent transformations to provide compounds of general formula 10, wherein R¹, R², R³, D and q are as defined in the description and claims of this invention and Z represents an optionally functionalized amine or amine precursor.*

Functionalization of the position adjacent to the pyridine nitrogen, for example of the C5- or C7-position of pyrazolopyridines of general formula **8,** or alternatively of pyridine-containing synthetic intermediates exemplified in Schemes 1 to 6, is feasible, for example, via formation of the corresponding *N*-oxide of general formula **9.** Transformation of pyridines into pyridine *N*-oxides can be accomplished by various ways, preferably by reaction with an oxidizing reagent, such as, for example, meta-chloroperbenzoic acid. Pyridine *N*-oxides of general formula **9** can be rearranged to 5-chloro and/or 7-chloropyrazolopyridines (general formula **10** with R² = Cl) by reaction with, for example, phosphoroxychloride or methyl chloroformiate in the presence of hexamethyldisilazane (see for example WO2005058891). Reaction of pyridine *N*-oxides with, for example, phosphoroxychloride can lead to regioisomeric product mixtures, which can be separated by standard purification procedures. 5-Chloro- and/or 7-chloropyrazolopyridines allow for various subsequent transformations, for example nucleophilic displacements with amines or alcohols, transition metal-catalyzed cross coupling reactions or metallations followed by reactions with electrophiles. Alternatively, pyridine *N*-oxides of general formula **9** can be reacted with acetic anhydride to yield the respective 5- and/or 7-acetoxypyrazolopyridines resulting from the so-called Boekelheide rearrangement. Other processes are known to the person skilled in the art which allow for introducing a substituent into pyridine *N-*oxides (see for example Keith, J. M. J. Org. Chem. 2006, 71, 9540), *inter alia* transition metal catalyzed cross-coupling reactions (see for example Campeau, L.-C. et al. J. Am. Chem. Soc. 2005, 127, 18020).

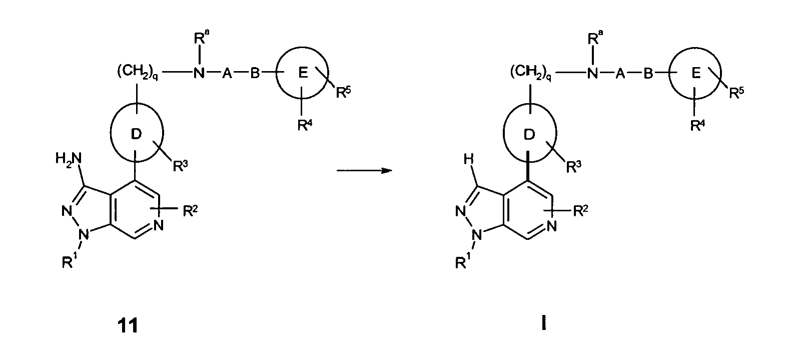

***Scheme 8** General procedure for the preparation of compounds of the general formula (I) by deamination of intermediates of general formula **11**, wherein A, B, D, E, R^{a}, R¹, R², R³, R⁴, R⁵ and q are* as *defined in the description and claims of this invention*.

In addition to the processes described in the preceding schemes and paragraphs, compounds of the present invention of general formula **I** can be prepared from the corresponding 3-aminopyrazolopyridines of general formula **11.** Desamination of 3-aminopyrazolopyridines of formula **11** is feasible, for example, by reaction with sodium nitrite followed by heating in the presence of an acid, such as, for example, sulphuric acid. In analogy to the process depicted in Scheme 8, compounds of e.g. formula **Ia, Ib, 1, 3, 8** and **10** are accessible from the corresponding 3-aminopyrazolopyridines by deamination as described above. The respective 3-aminopyrazolopyridines can be prepared in analogy to the described processes by substituting starting material **6** (Scheme 6) with the respective 3,5-dihalo-5-cyanopyridine, which in turn can be prepared as described in the literature.

### GENERAL PROCEDURES

In the subsequent paragraphs detailed general procedures for the synthesis of key intermediates and compounds of the present invention are described.

### General Procedure 1 (GP 1): Hydrazone formation

The respective heteroaryl carbaldehyde was dissolved in 1-PrOH (- 4-5 mL per mmol carbaldehyde), treated with the respective hydrazine (1.5-3.0 eq.) and subsequently heated to 100-120 °C in a microwave oven (Biotage Initiator®). The reaction mixture was concentrated, the residue partitioned between water and ethyl acetate, the aqueous layer reextracted with ethyl acetate, the combined organic layers dried and concentrated in vacuo to yield the desired product, which was typically used in the subsequent cyclization without further purification steps.

### General Procedure 2 (GP 2): Hydrazone cyclization

The respective hydrazone (prepared as described in GP 1) was dissolved in dry THF (- 9 mL per mmol hydrazone), treated with 50-60% NaH (1.2 to 2.2 eq.) and subsequently refluxed for 90 min. The reaction mixture was quenched with water, extracted with ethyl acetate, the combined organic layers dried and concentrated in vacuo. The precipitate was filtered and subsequently triturated with diisopropylether to yield the desired product. Flash column chromatography of the mother liquor provided a second batch of the analytically pure product. Alternatively, in most cases concentration of the crude reaction mixture to dryness provided the cyclized product in sufficient purity for subsequent transformations.

### General Procedure 3 (GP 3): Suzuki coupling (Conditions A)

The heteroaryl halide (1 eq), the respective aryl pinacolato boronate or aryl boronic acid (1.2 to 1.8 eq.) and Pd(PPh₃)₄ (6 mol%) were weighed into a Biotage microwave vial and capped. Toluene (6 mL per mmol halide), EtOH (6 mL per mmol halide) and 1M aq. Na₂CO₃ solution (2 eq.) were added by syringe. The resulting mixture was prestirred (10 sec) and subsequently heated to 100-120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo. The residue was optionally purified by flash column chromatography and/or trituration and/or preparative HPLC.

### General Procedure 4 (GP 4): Suzuki coupling (Conditions B)

The heteroaryl halide (1 eq), the respective aryl pinacolato boronate or aryl boronic acid (1.2 to 1.5 eq.) and FibreCat 1032 (Johnson-Matthey; 0.38 mmol/g loading; 6 mol%) were weighed into a Biotage microwave vial and capped. EtOH (-9 mL per mmol halide) and 1M aq. K₂CO₃ solution (1.5 eq.) were added by syringe. The resulting mixture was prestirred (10 sec) and subsequently heated to 100-130 °C for 20 min in a Biotage Initiator® microwave oven. After filtration and concentration in vacuo, the residue was taken up in ethyl acetate and water was added, the layers were separated and the aqueous layer was extracted with ethyl acetate. The combined organic layers were dried and concentrated in vacuo. The residue was optionally purified by flash column chromatography and/or trituration and/or preparative HPLC.

### General Procedure 5 (GP 5): Urea formation (Conditions A)

The respective (hetero)aryl amine (1 eq.) was dissolved in DCM (5-10 mL per mmol amine) and treated with the respective (commercially available) isocyanate (1-1.2 eq.). The reaction mixture was stirred at room temperature until TLC indicated complete consumption of the starting aniline (usually overnight). The reaction mixture was concentrated in vacuo, the residue was taken up in ethyl acetate and water was added, the layers were separated and the aqueous layer was extracted with ethyl acetate. The combined organic layers were dried and concentrated in vacuo. The residue was optionally purified by flash column chromatography and/or trituration and/or preparative HPLC.

### General Procedure 6 (GP 6): Urea formation (Conditions B)

1.2 Eq. of a (hetero)aryl amine (usually the less functionalized one of the two amines to be coupled) were dissolved in acetonitrile (- 8 mL per mmol amine), treated with triphosgene (0.4 eq.) and stirred at room temperature for 1 h upon which the second (hetero)aryl amine (usually the higher functionalized of the two amines to be coupled) was added and stirring was continued at r.t. until TLC indicated complete conversion. The reaction mixture was concentrated in vacuo, the residue was taken up in ethyl acetate and water was added, the layers were separated and the aqueous layer was extracted with ethyl acetate. The combined organic layers were dried and concentrated in vacuo. The residue was optionally purified by flash column chromatography and/or trituration and/or preparative HPLC.

### General Procedure 7 (GP 7): N1-Alkylation of 1H-pyrazolopyridines

The respective 1H-pyrazolopyridine was dissolved in dry DMF under an atmosphere of argon and treated with sodium hydride and subsequently stirred at 50 °C for 1 h. A solution of the respective alkyl halide in DMF was added dropwise and stirring was continued at 50 °C for 1 h. [In cases were the respective halide is only available as a salt (e.g. hydrochloride or hydrobromide salt), this salt was dissolved in DMF and treated with excess Et₃N, and the resulting slurry was added to the deprotonated 1H-pyrazolopyridine upon filtration through a Millipore filter.] The reaction mixture was diluted with EtOAc, quenched with water, the aqueous layer was extracted with EtOAc and the combined organic layers were dried and concentrated in vacuo. Flash column chromatography optionally followed by recrystallization or preparative HPLC purification yielded the desired alkylated pyrazolopyridines.

### General Procedure 8 (GP 8): Urea formation with phenylcarbamates

The respective (hetero)aryl amine (1 eq.) was dissolved in THF (∼ 10 mL per mmol amine) and treated with pyridine (40 eq.) and the respective (hetero)aryl carbamic acid phenyl ester (1 eq.; prepared from the respective (hetero)aryl amine precursor by treatment with phenyl chloroformate in analogy to procedures described in WO2007064872 or WO2005110994)). The reaction mixture was heated to 100 °C for 15 min in a Biotage Initiator microwave oven upon which LCMS analysis usually showed complete turnover (otherwise heating to 100 °C was continued until LCMS analysis showed completion of turnover). The reaction mixture was concentrated in vacuo and the residue was isolated either by trituration or by flash column chromatography or by preparative HPLC purification.

### General Procedure 9 (GP 9): Formation of isopropenyl carbamates

In analogy to J. Org. Chem. 2005, 70, 6960
The respective (hetero)aryl amine (1 eq.) was dissolved in THF (- 2.5 mL per mmol amine) and treated with N-methylmorpholine (1.2 eq.). The resulting solution was cooled to 4 °C and treated dropwise with chloro-isopropenyl formate (1.2 eq.). Stirring was continued at rt until TLC or LCMS analysis showed completion of turnover. The reaction mixture was quenched with water and usually extracted with ethyl acetate. The combined organic layers were dried and concentrated in vacuo. Trituration of the residue provided the target carbamate.

### General Procedure 10 (GP 10): Urea formation with isopropenyl carbamates

In analogy to J. Org. Chem. 2005, 70, 6960
The respective (hetero)aryl amine (1 eq.) was dissolved in THF (- 4 mL per mmol amine) and treated with *N*-methylpyrrolidine (0.2 eq.) and the respective (hetero)aryl carbamic acid isopropenyl ester (1 - 1.5 eq.). The mixture was stirred overnight at 55 °C. Extractive work-up followed by trituration and/or flash column chromatograpy and/or preparative HPLC purification provided the target urea.

### SYNTHESIS OF KEY INTERMEDIATES

### Intermediate 1.1

### Preparation of N-[1-(3,5-Dibromo-pyridin-4-yl)-meth-(E)-ylidene]-N'-methylhydrazine

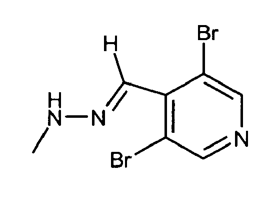

In analogy to GP 1, 2.15 g of 3,5-dibromo-pyridine-4-carbaldehyde (8.12 mmol, 1 eq; commercially available or prepared as described in US6232320 or WO2005110410) were dissolved in 36 mL 1-PrOH, treated with 0.65 mL *N*-methyl hydrazine (12.17 mmol, 1.5 eq.) and heated to 100 °C for 30 min (employing a Biotage Initiator@ microwave oven in batch mode). The reaction mixture was concentrated, the residue partitioned between water and ethyl acetate, the aqueous layer reextracted with ethyl acetate, the combined organic layers dried and concentrated in vacuo to yield 2.29 g of the desired product (7.82 mmol, 96% yield), which was used in the subsequent cyclization without further purification steps.
¹H-NMR (d₆-DMSO; 300 MHz): 8.57 - 8.63 (m, 3 H); 7.22 (s, 1 H); 2.86 (d, 3 H). ¹³C-NMR (d₆-DMSO; 150 MHz): 151.27 (CH); 141.44 (C_{q}); 124.39 (CH); 119.34 (Cq); 32.83 (CH₃).
MS (ESI): [M+H]⁺ = 294 (Br₂ isotope pattern)

### Intermediate 1.2

### Preparation of 2-{N'-[1-(3,5-Dibromo-pyridin-4-yl)-meth-(E)-ylidene]-hydrazino}-ethanol

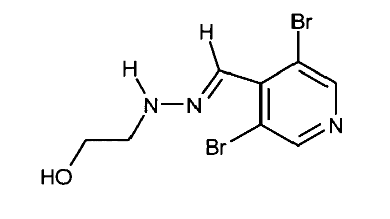

In analogy to GP1, 468 mg of 3,5-dibromo-pyridine-4-carbaldehyde (1.77 mmol, 1 eq; commercially available or prepared as described in US6232320 or WO2005110410) were dissolved in 8 mL 1-PrOH, treated with 0.36 mL 2-hydrazino-ethanol (5.3 mmol, 3 eq.) and heated to 120 °C for 30 min (employing a Biotage Initiator® microwave oven). The reaction mixture was concentrated, the residue partitioned between water and ethyl acetate, the aqueous layer reextracted with ethyl acetate, the combined organic layers dried and concentrated in vacuo to yield 530 mg of the desired product (1.64 mmol, 93% yield), which was used in the subsequent cyclization without further purification steps.
¹H-NMR (d₆-DMSO; 300 MHz): 8.59 (s, 2 H); 8.55 (t, 1 H); 7.51 (s, 1 H); 4.70 (t, 1 H); 3.58 (q, 2 H); 3.25 (q, 2 H).
MS (ESI): [M+H]⁺ = 324 (Br₂ isotope pattern)

### Intermediate 1.3

### Preparation of [1-(3,5-Dibromo-pyridin-4-yl)-meth-(E)-ylidene]-hydrazine

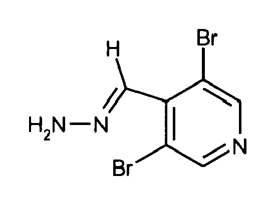

In analogy to GP 1, 54 mg of 3,5-dibromo-pyridine-4-carbaldehyde (0.2 mmol, 1 eq; commercially available or prepared as described in US6232320 or W02005110410) were dissolved in 1 mL 1-PrOH, treated with 30 µL 80% hydrazine hydrate (0.61 mmol, 3 eq.) and heated to 120 °C for 30 min (employing a Biotage Initiator@ microwave oven). The precipitate was filtered and washed with cold 1-PrOH to yield 27 mg of the hydrazone (0.1 mmol, 50% yield).
¹H-NMR (d₆-DMSO; 400 MHz): 8.61 (s, 2 H); 7.96 (s, 2 H); 7.72 (s, 1 H).
MS (LC-MS): >90% pure; [M+H]⁺ = 279 (Br₂ isotope pattern)

### Intermediate 1.4

### Preparation of N'-[1-(3,5-Dibromo-pyridin-4-yl)-meth-(E)-ylidene]-hydrazinecarboxylic acid tert-butyl ester

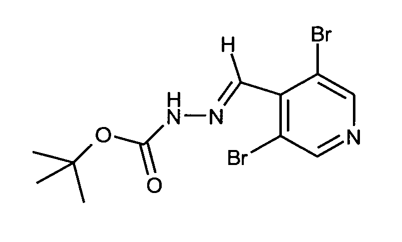

In analogy to GP 1, 1.37 g of 3,5-dibromo-pyridine-4-carbaldehyde (5.17 mmol, 1 eq; commercially available or prepared as described in US6232320 or W02005110410) were dissolved in 24 mL 1-PrOH, treated with 2.05 g *tert*-butyl carbazate (15.5 mmol, 3 eq.) and heated to 120 °C for 30 min (employing a Biotage Initiator® microwave oven in batch mode). The precipitate was filtered and washed with cold 1-PrOH to yield 1.66 g of the Boc-hydrazone (4.37 mmol, 85% yield).
¹H-NMR (d₆-DMSO; 400 MHz): 11.36 (br., 1 H); 8.74 (s, 2 H); 8.04 (s, 1 H); 1.44 (s, 9 H).

### Intermediate 1.5

### Preparation of N-[1-(3,5-Dibromo-pyridin-4-yl)-meth-(E)-ylidene]-N'-ethyl-hydrazine

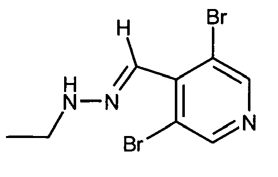

In analogy to GP 1, 2.65 g of 3,5-dibromo-pyridine-4-carbaldehyde (10 mmol, 1 eq; commercially available or prepared as described in US6232320 or W02005110410) were dissolved in 32 mL 1-PrOH, treated with 2.25 g *N*-ethyl hydrazine (oxalate salt; 15 mmol, 1.5 eq.) and heated to 100 °C for 30 min (employing a Biotage Initiator® microwave oven in batch mode). The reaction mixture was concentrated, the residue partitioned between conc. aq. NaHCO₃ solution and ethyl acetate, the aqueous layer reextracted with ethyl acetate, the combined organic layers dried and concentrated in vacuo to yield 3.08 g of the desired product (10 mmol, quantitative yield), which was used in the subsequent cyclization without further purification steps.
¹H-NMR (d₆-DMSO; 300 MHz): 8.60 (s, 2 H); 8.53 (t, 1 H); 7.40 (s, 1 H); 3.18 (dq, 2 H); 1.16 (t, 3 H).

### Intermediate 2.1

### Preparation of 4-Bromo-1-methyl-1H-pyrazolo[3,4-c]pyridine

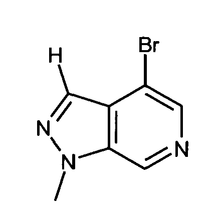

In analogy to GP 2, 5.34 g of N-[1-(3,5-Dibromo-pyridin-4-yl)-meth-(E)-ylidene]-N'-methyl-hydrazine (Intermediate 1.1, 18.23 mmol, 1 eq) were dissolved in 163 mL dry THF, treated at rt with 994 mg 50-60% NaH (22.78 mmol, 1.2 eq) and subsequently refluxed for 90 min. The reaction mixture was quenched with water, extracted with ethyl acetate, the combined organic layers dried and concentrated in vacuo. The precipitate was filtered and subsequently triturated with diisopropylether to yield 1.71 g of the desired product. Flash column chromatography of the mother liquor provided a second batch of the analytically pure product.
¹H-NMR (d₆-DMSO; 400 MHz): 9.16 (s, 1 H); 8.34 (s, 1 H); 8.16 (s, 1 H); 4.17 (s, 3 H).
MS (ESI): [M+H]⁺ = 212 (Br isotope pattern).

### Intermediate 2.2

### Preparation of 2-(4-Bromo-pyrazolo[3,4-c]pyridin-1-yl)-ethanol

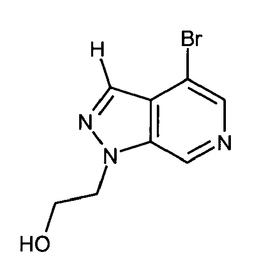

In analogy to GP2, 520 mg of 2-{N'-[1-(3,5-Dibromo-pyridin-4-yl)-meth-(E)-ylidene]-hydrazinol-ethanol (**Intermediate 1.2**, 1.61 mmol, 1 eq) were dissolved in 14 mL dry THF, treated at rt with 155 mg 50-60% NaH (3.54 mmol, 2.2 eq) and subsequently refluxed for 90 min. The reaction mixture was quenched with water, extracted with ethyl acetate, the combined organic layers dried and concentrated in vacuo to yield 424 mg of a crude product, which was optionally further purified by trituration or flash column chromatography.
LC-MS: [M+H]⁺ = 243 (Br isotope pattern)

### Intermediate 2.3

### Preparation of 4-Bromo-1H-pyrazolo[3,4-c]pyridine

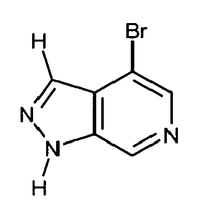

In analogy to GP 2, 578 mg of [1-(3,5-dibromo-pyridin-4-yl)-meth-(E)-ylidene]-hydrazine (**Intermediate 1.3,** 2.07 mmol, 1 eq) were dissolved in 18 mL dry THF, treated at rt with 200 mg 50-60% NaH (4.56 mmol, 2.2 eq) and subsequently refluxed for 90 min. The reaction mixture was quenched with water, extracted with ethyl acetate, the combined organic layers dried and concentrated in vacuo.
MS (LC-MS): [M+H]⁺ = 198 (Br₂ isotope pattern)

### Intermediate 2.4

### Preparation of 4-Bromo-1-ethyl-1 H-pyrazolo[3,4-c]pyridine

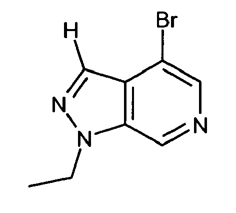

In analogy to GP 2, 2.1 g of **Intermediate 1.5** (6.83 mmol, 1 eq) were dissolved in 60 mL dry THF, treated at rt with 372 mg 50-60% NaH (8.53 mmol, 1.25 eq) and subsequently refluxed for 90 min. The reaction mixture was quenched with water, extracted with ethyl acetate, the combined organic layers dried and concentrated in vacuo. The precipitate was filtered and subsequently triturated with diisopropylether to yield 1.6 g of the desired product (quantitative yield).
¹H-NMR (d₆-DMSO; 400 MHz): 9.20 (s, 1 H); 8.34 (s, 1 H); 8.18 (s, 1 H); 4.56 (q, 2 H); 1.42 (t, 3 H).

### Intermediate 2.5

### Preparation of 4-Bromo-1-(2-methoxy-ethyl)-1H-pyrazolo[3,4-c]pyridine

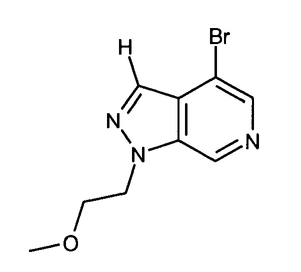

A solution of 675 mg of 2-(4-bromo-pyrazolo[3,4-c]pyridin-1-yl)-ethanol (Intermediate 2.2; 2.79 mmol, 1 eq.) in 33 mL THF was treated at Rt with 183 mg NaH (55-60% suspension; 4.18 mmol, 1.5 eq.) and stirred for 30 min upon which 0.194 mL methyl iodide (3.07 mmol, 1.1 eq.) were added and stirring was continued for 2h. The reaction mixture was quenched with water, extracted with ethyl acetate, the combined organic layers were dried and concentrated in vacuo. Flash column chromatography provided 500 mg of the corresponding methyl ether target compound (1.95 mmol, 70% yield).

### Intermediate 2.6

### Preparation of 4-Bromo-1-(2-bromo-ethyl)-1H-pyrazolo[3,4-c]pyridine

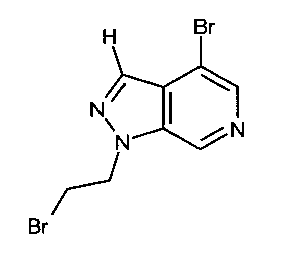

A solution of 709 mg of 2-(4-bromo-pyrazolo[3,4-c]pyridin-1-yl)-ethanol (Intermediate 2.2; 2.93 mmol, 1 eq.) in 3 mL DMF was treated at Rt with 1.93 g Ph₃P (7.32 mmol, 2.5 eq.) and 1.94 g CBr₄ (5.86 mmol, 2 eq.) and stirred for 90 min at rt. The reaction mixture was quenched with water, extracted with DCM, the combined organic layers were dried and concentrated in vacuo. Flash column chromatography provided 290 mg of the bromo compound (0.95 mmol, 33% yield).
¹H-NMR (d₆-DMSO; 300 MHz): 9.25 (s, 1 H); 8.37 (s, 1 H); 8.27 (d, 1 H); 4.98 (t, 2 H); 3.96 (t, 2 H).

### Intermediate 2.7

### Preparation of 4-Bromo-1-(2-methanesulfonyl-ethyl)-1H-pyrazolo[3,4-c]pyridine

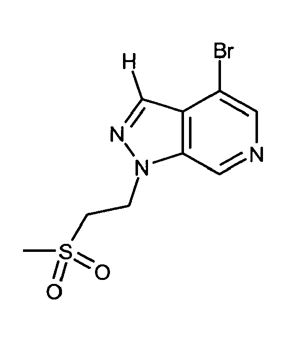

100 mg of 4-Bromo-1-(2-bromo-ethyl)-1H-pyrazolo[3,4-c]pyridine (Intermediate 2.6; 0.33 mmol, 1 eq.) were dissolved in 5 mL EtOH and treated with 150 mg sodium methyl sulfinate (1.5 mmol, 4.5 eq.) and heated to 120 °C for 4h in a Biotage Initiator microwave oven. The reaction mixture was quenched with water, extracted with DCM, the combined organic layers were dried and concentrated in vacuo to provide the crude Intermediate 2.7, which was used without further purification in the subsequent transformations.
MS (LC-MS): [M+H]⁺ = 304/306 (Br isotope pattern)

### Intermediate 3.1

### Preparation of 4-(1-Methyl-1H-pyrazolo[3,4-c]pyridin-4-yl)-phenylamine

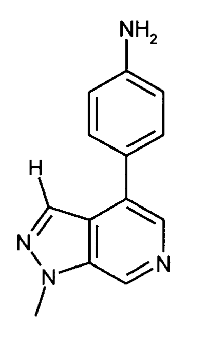

In analogy to GP 3, 1.06 g of Intermediate 2.1 (5 mmol, 1 eq.), 1.31 g of 4-(4,4,5,5-tetramethyl-[1,3,2]dioxaborolan-2-yl)-phenylamine (6 mmol, 1.2 eq.) and 347 mg Pd(PPh₃)₄ (6 mol%) were weighed into a Biotage microwave vial and capped. 30 mL toluene, 30 mL EtOH and 1M aq. Na₂CO₃ solution (9.65 mL, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after trituration 701 mg of the desired product (3.13 mmol, 63% yield), which was used for subsequent transformations without further purification.
¹H-NMR (d₆-DMSO; 400 MHz): 8.97 (s, 1 H); 8.24 - 8.25 (m, 2 H); 7.46 (d, 2 H); 6.69 (d, 2 H); 5.40 (br. s, 2 H); 4.15 (s, 3 H).

### Intermediate 3.2

### Preparation of 2-Fluoro-4-(1-methyl-1H-pyrazolo[3,4-c]pyridin-4-yl)-phenylamine

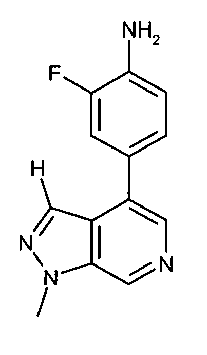

In analogy to GP 3, 390 mg of Intermediate 2.1 (1.84 mmol, 1 eq.), 523.24 mg of 2-fluoro-4-(4,4,5,5-tetramethyl-[1,3,2]dioxaborolan-2-yl)-phenylamine (2.21 mmol, 1.2 eq.) and 127.5 mg Pd(PPh₃)₄ (0.11 mmol, 6 mol%) were weighed into a Biotage microwave vial and capped. 7.5 mL toluene, 7.5 mL EtOH and 1M aq. Na₂CO₃ solution (3.55 mL, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with water and ethyl acetate, the layers were separated and the aqueous layer extracted with ethyl acetate. The combined organic layers were dried and concentrated in vacuo to yield after trituration 294 mg of the desired product (1.21 mmol, 66% yield), which was used for subsequent transformations without further purification.
¹H-NMR (d₆-DMSO; 400 MHz): 9.01 (s, 1 H); 8.28 - 8.30 (m, 2 H); 7.42 (dd, 1 H); 7.35 (dd, 1 H); 6.89 (dd, 1 H); 5.45 (br. s, 2 H); 4.16 (s, 3 H).
MS (ESI): [M+H]⁺ = 243.

### Intermediate 3.3

### Preparation of 2-Methyl-4-(1-methyl-1H-pyrazolo[3,4-c]pyridin-4-yl)-phenylamine

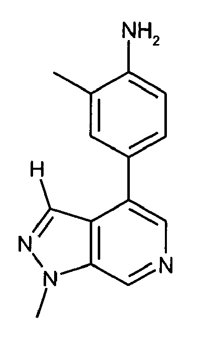

In analogy to GP 3, 228 mg of Intermediate 2.1 (1.08 mmol, 1 eq.), 455.7 mg of 2-methyl-4-(4,4,5,5-tetramethyl-[1,3,2]dioxaborolan-2-yl)-phenylamine (1.29 mmol, 1.2 eq.) and 74.6 mg Pd(PPh₃)₄ (0.065 mmol, 6 mol%) were weighed into a Biotage microwave vial and capped. 8 mL toluene, 8 mL EtOH and 1M aq. Na₂CO₃ solution (2.08 mL, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with water and ethyl acetate, the layers were separated and the aqueous layer extracted with ethyl acetate. The combined organic layers were dried and concentrated in vacuo to yield after flash column chromatography 89 mg of the desired product (0.37 mmol, 35% yield).
¹H-NMR (d₆-DMSO; 300 MHz): 8.96 (s, 1 H); 8.24 - 8.25 (m, 2 H); 7.32 - 7.37 (m, 2 H); 6.73 (d, 1 H); 5.14 (br. s, 2 H); 4.15 (s, 3 H); 2.12 (s, 3 H).
MS (ESI): [M+H]⁺ = 239.

### Intermediate 3.4

### Preparation of 2-Methoxy-4-(1-methyl-1H-pyrazolo[3,4-c]pyridin-4-yl)-phenylamine

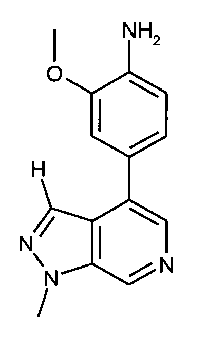

In analogy to GP 4, 355 mg of Intermediate 2.1 (1.67 mmol, 1 eq.), 630 mg of 2-methoxy-4-(4,4,5,5-tetramethyl-[1,3,2]dioxaborolan-2-yl)-phenylamine (2.51 mmol, 1.5 eq.) and 268 mg of FibreCat 1032 (0.1 mmol, 6 mol%) were weighed into a Biotage microwave vial and capped. 15 mL EtOH and 2.51 mL 1M aq. K₂CO₃ solution (2.51 mmol, 1.5 eq.) were added by syringe. The resulting mixture was prestirred (10 sec) and subsequently heated to 130 °C for 20 min in a Biotage Initiator® microwave oven. After filtration and concentration in vacuo, the residue was taken up in ethyl acetate and water was added, the layers were separated and the aqueous layer was extracted with ethyl acetate. The combined organic layers were dried and concentrated in vacuo. Flash column chromatography yielded 231 mg of the desired product (0.90 mmol, 54% yield) along with additional fractions of impure material.
¹H-NMR (d₆-DMSO; 400 MHz): 8.99 (s, 1 H); 8.30 - 8.32 (m, 2 H); 7.12 - 7.16 (m, 2 H); 6.76 (d, 1 H); 5.03 (br. s, 2 H); 4.16 (s, 3 H); 3.85 (s, 3 H).
MS (ESI): [M+H]⁺ = 255.

### Intermediate 3.5

### Preparation of 4-(1-Ethyl-1H-pyrazolo[3,4-c]pyridin-4-yl)-phenylamine

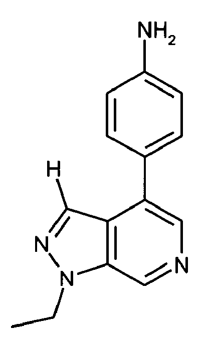

In analogy to GP 3, 1.65 g of Intermediate 2.4 (7.3 mmol, 1 eq.), 2.88 g of 4-(4,4,5,5-tetramethyl-[1,3,2]dioxaborolan-2-yl)-phenylamine (13.1 mmol, 1.8 eq.) and 506 mg Pd(PPh₃)₄ (6 mol%) were weighed into Biotage microwave vials and capped. 15 mL toluene, 15 mL EtOH and 1M aq. Na₂CO₃ solution (14 mL, 1.9 eq.) were subsequently added by syringe. The resulting mixture was prestirred (10 sec) and subsequently heated to 120 °C for 15 min (fixed hold time) in a Biotage Initiator@ microwave reactor (reaction was run in two batches). The combined reaction mixtures were diluted with water and ethyl acetate, the layers were separated and the aqueous layer extracted with ethyl acetate. The combined organic layers were dried and concentrated in vacuo to yield after flash column chromatography 1200 mg of the desired product (5.04 mmol, 69% yield).
¹H-NMR (d₆-DMSO; 300 MHz): 9.01 (s, 1 H); 8.25 (s, 1 H); 8.24 (s, 1 H); 7.46 (d, 2 H); 6.69 (d, 2 H); 5.39 (br. s, 2 H); 4.55 (q, 2 H); 1.42 (t, 3 H).
MS (ESI): [M+H]⁺ = 239.

### Intermediate 3.6

### Preparation of 4-(1-Ethyl-1H-pyrazolo[3,4-c]pyridin-4-yl)-2-fluoro-phenylamine

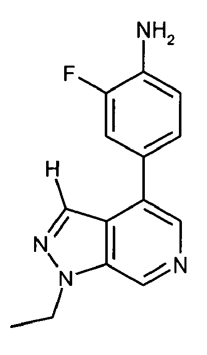

In analogy to GP 3, 825 mg of Intermediate 2.4 (3.65 mmol, 1 eq.), 1.0 g of 2-Fluoro-4-(4,4,5,5-tetramethyl-[1,3,2]dioxaborolan-2-yl)-phenylamine (4.2 mmol, 1.15 eq.) and 253 mg Pd(PPh₃)₄ (6 mol%) were weighed into a Biotage microwave vial and capped. 6.5 mL toluene, 6.5 mL EtOH and 1M aq. Na₂CO₃ solution (7 mL, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography 903 mg of the desired product.
¹H-NMR (d₆-DMSO; 300 MHz): 9.05 (s, 1 H); 8.29 (s, 1 H); 8.28 (s, 1 H); 7.42 (dd, 1 H); 7.34 (dd, 1 H); 6.89 (dd, 1 H); 5.44 (s, 2 H); 4.56 (q, 2 H); 1.42 (t, 3 H).
MS (ESI): [M+H]⁺ = 257.

### Intermediate 3.7

### Preparation of 4-[1-(2-Methoxy-ethyl)-1H-pyrazolo[3,4-c]pyridin-4-yl]-phenylamine

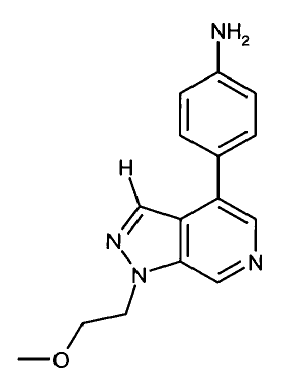

In analogy to GP 3, 300 mg of 4-bromo-1-(2-methoxy-ethyl)-1H-pyrazolo[3,4-c]pyridine (Intermediate 2.5; 1.17 mmol, 1 eq.), 462 mg of 4-(4,4,5,5-tetramethyl-[1,3,2]dioxaborolan-2-yl)-phenylamine (2.11 mmol, 1.8 eq.) and 81.22 mg Pd(PPh₃)₄ (6 mol%) were weighed into a Biotage microwave vial and capped. 2.1 mL toluene, 2.1 mL EtOH and 1M aq. Na₂CO₃ solution (2.25 mL, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography 148 mg of the desired product (0.55 mmol, 47% yield) along with a second slightly impure batch of 140 mg.
¹H-NMR (d₆-DMSO; 300 MHz): 8.98 (s, 1 H); 8.26 (s, 1 H); 8.22 (s, 1 H); 7.46 (d, 2 H); 6.70 (d, 2 H); 5.38 (br. s, 2 H); 4.69 (t, 2 H); 3.76 (t, 2 H); 3.17 (s, 3 H).

### Intermediate 3.8

### Preparation of 2-fluoro-4-[1-(2-methoxy-ethyl)-1H-pyrazolo[3,4-c]pyridin-4-yl]-phenylamine

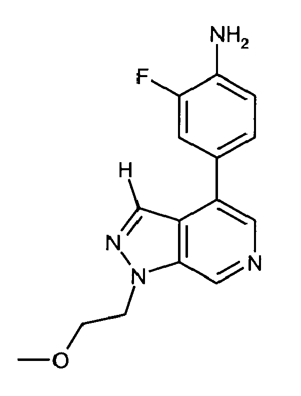

In analogy to GP 3, 200 mg of 4-bromo-1-(2-methoxy-ethyl)-1H-pyrazolo[3,4-c]pyridine (Intermediate 2.5; 0.78 mmol, 1 eq.), 333 mg of 2-fluoro-4-(4,4,5,5-tetramethyl-[1,3,2]dioxaborolan-2-yl)-phenylamine (1.41 mmol, 1.8 eq.) and 54 mg Pd(PPh₃)₄ (6 mol%) were weighed into a Biotage microwave vial and capped. 1.4 mL toluene, 1.4 mL EtOH and 1M aq. Na₂CO₃ solution (1.5 mL, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography the desired product (80% yield ).
¹H-NMR (d₆-DMSO; 300 MHz): 9.02 (s, 1 H); 8.31 (s, 1 H); 8.26 (s, 1 H); 7.42 (dd, 1 H); 7.34 (dd, 1 H); 6.89 (dd, 1 H); 5.44 (s, 2 H);4.69 (t, 2 H); 3.76 (t, 2 H); 3.17 (s, 3 H).

### Intermediate 4.1

### Preparation of [4-(1-Methyl-1H-pyrazolo[3,4-c]pyridin-4-yl)-phenyl]-carbamic acid isopropenyl ester

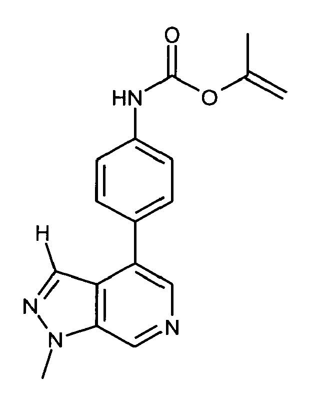

In analogy to J. Org. Chem. 2005, 70, 6960 and GP 9:
950 mg of 4-(1-methyl-1H-pyrazolo[3,4-c]pyridin-4-yl)-phenylamine (Intermediate 3.1, 4.24 mmol, 1 eq.) were dissolved in 10 mL THF and treated with 0.56 mL N-methylmorpholine (5.08 mmol, 1.2 eq.). The resulting solution was cooled to 4 °C and treated dropwise with 0.55 mL chloro-isopropenyl formate (5.08 mmol, 1.2 eq.). Stirring was continued at rt for 4h. The reaction mixture was quenched with water and extracted with ethyl acetate. The combined organic layers were dried and concentrated in vacuo. Trituration of the residue provided 674 mg of the target carbamate (2.2 mmol, 52 % yield).
¹H-NMR (d₆-DMSO; 300 MHz): 10.11 (br. s, 1 H); 9.09 (s, 1 H); 8.35 (s, 1 H); 8.31 (s, 1 H); 7.74 (d, 2 H); 7.63 (d, 2 H); 4.73 - 4.75 (m, 2 H); 4.18 (s, 3 H); 1.93 (s, 3 H).

### Intermediate 4.2

### Preparation of [2-Fluoro-4-(1-methyl-1H-pyrazolo[3,4-c]pyridin-4-yl)-phenyl]-carbamic acid isopropenyl ester

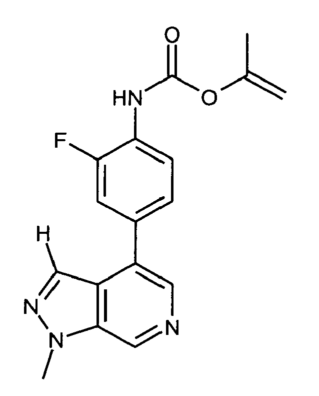

In analogy to J. Org. Chem. 2005, 70, 6960 and GP 9:
485 mg of 2-fluoro-4-(1-methyl-1H-pyrazolo[3,4-c]pyridin-4-yl)-phenylamine (Intermediate 3.2, 2 mmol, 1 eq.) were dissolved in 7 mL THF and treated with 0.26 mL N-methylmorpholine (2.4 mmol, 1.2 eq.). The resulting solution was cooled to 4 °C and treated dropwise with 0.26 mL chloro-isopropenyl formate (2.4 mmol, 1.2 eq.). Stirring was continued at rt for 4h. The reaction mixture was quenched with water and extracted with ethyl acetate. The combined organic layers were dried and concentrated in vacuo. Trituration of the residue provided 109 mg of the target carbamate (0.33 mmol, 17 % yield).
¹H-NMR (d₆-DMSO; 300 MHz): 9.81 (br. s, 1 H); 9.14 (s, 1 H); 8.40 (s, 1 H); 8.35 (s, 1 H); 7.82 (t, 1 H); 7.60 - 7.70 (m, 2 H); 4.72 - 4.75 (m, 2 H); 4.20 (s, 3 H); 1.93 (s, 3 H).

### Intermediate 5.1

### Preparation of [2-Fluoro-4-(1-methyl-1H-pyrazolo[3,4-c]pyridin-4-yl)-phenyl]-carbamic acid phenyl ester

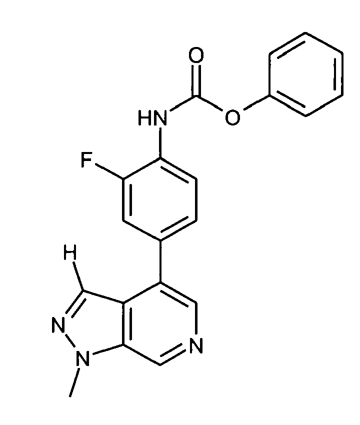

484 mg of 2-fluoro-4-(1-methyl-1H-pyrazolo[3,4-c]pyridin-4-yl)-phenylamine (Intermediate 3.2; 2 mmol, 1 eq.) were dissolved in 35 mL THF and 0.21 mg Na₂CO₃ (2 mmol, 1 eq.) were added. 0.76 mL of phenyl chloro formate (6 mmol, 3 eq.) were added dropwise and stirring was continued at rt overnight. The reaction mixture was quenched with water, extracted with ethyl acetate, the combined organic layers were dried and concentrated in vacuo. Flash column chromatography provided 415 mg (57% yield) of the target compound.
MS (LC-MS): [M+H]⁺ = 363.

### SYNTHESIS OF EXAMPLE COMPOUNDS

### Example Compound 1.1

### Preparation of 1-[4-(1-Methyl-1H-pyrazolo[3,4-c]pyridin-4-yl)-phenyl]-3-phenyl-urea

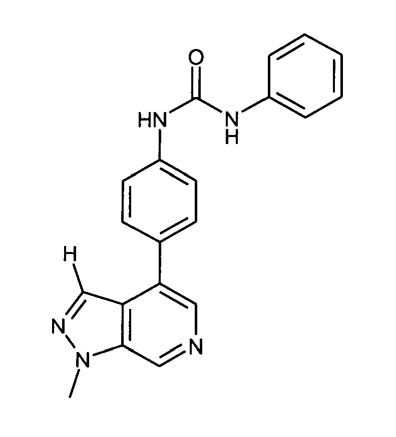

In analogy to GP 5, 112 mg of 4-(1-methyl-1H-pyrazolo[3,4-c]pyridin-4-yl)-phenylamine (Intermediate 3.1; 0.5 mmol, 1 eq.) were dissolved in 5.2 mL DCM and treated with 60 µL phenyl isocyanate (0.55 mmol, 1.1 eq.). Work-up as described in GP 5 and flash column chromatography followed by trituration provided 29 mg of the analytically pure product (0.084 mmol, 17% yield).
¹H-NMR (d₆-DMSO; 300 MHz): 9.08 (s, 1 H); 8.87 (s, 1 H); 8.71 (s, 1 H); 8.35 (s, 1 H); 8.31 (d, 1 H); 7.72 (d, 2 H); 7.62 (d, 2 H); 7.45 (dd, 2 H); 7.26 (t, 2 H); 6.95 (tt, 1 H); 4.18 (s, 3 H).
MS (ESI): [M+H]⁺ = 344.

### Example Compound 1.2

### Preparation of 1-(2-Fluoro-5-trifluoromethyl-phenyl)-3-[4-(1-methyl-1H-pyrazolo[3,4-c]pyridin-4-yl)-phenyl]-urea

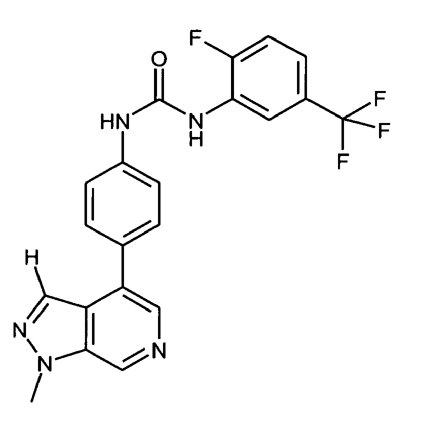

In analogy to GP 5, 112 mg of 4-(1-methyl-1H-pyrazolo[3,4-c]pyridin-4-yl)-phenylamine (Intermediate 3.1; 0.5 mmol, 1 eq.) were dissolved in 5.2 mL DCM and treated with 80 µL 1-fluoro-2-isocyanato-4-trifluoromethyl-benzene (0.55 mmol, 1.1 eq.). Work-up as described in GP 5 and flash column chromatography followed by trituration provided 27 mg of the analytically pure product (0.063 mmol, 13% yield).
¹H-NMR (d₆-DMSO; 400 MHz): 9.40 (s, 1 H); 9.10 (s, 1 H); 8.96 (s, 1 H); 8.61 (dd, 1 H); 8.36 (s, 1 H); 8.31 (s, 1 H); 7.75 (d, 2 H); 7.64 (d, 2 H); 7.48 (dd, 1 H); 7.35 - 7.39 (m, 1 H); 4.19 (s, 3 H).
MS (ESI): [M+H]⁺ = 430.

### Example Compound 1.3

### Preparation of 1-(2-Fluoro-5-methyl-phenyl)-3-[4-(1-methyl-1H-pyrazolo[3,4-c]pyridin-4-yl)-phenyl]-urea

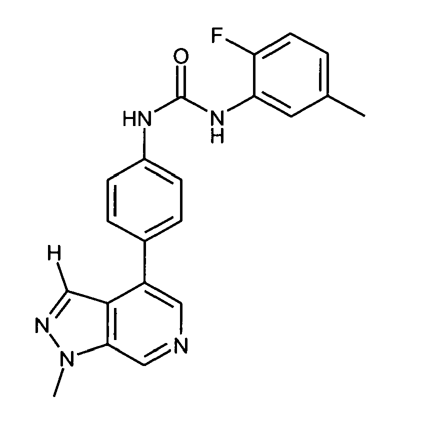

In analogy to GP 5, 112 mg of 4-(1-methyl-1H-pyrazolo[3,4-c]pyridin-4-yl)-phenylamine (Intermediate 3.1; 0.5 mmol, 1 eq.) were dissolved in 5.2 mL DCM and treated with 72 µL 1-fluoro-2-isocyanato-4-methyl-benzene (0.55 mmol, 1.1 eq.). Work-up as described in GP 5 and flash column chromatography followed by trituration provided 30 mg of the analytically pure product (0.080 mmol, 16% yield).
¹H-NMR (d₆-DMSO; 400 MHz): 9.25 (s, 1 H); 9.09 (s, 1 H); 8.51 (d, 1 H); 8.36 (s, 1 H); 8.31 (s, 1 H); 7.97 (dd, 1 H); 7.73 (d, 2 H); 7.62 (d, 2 H); 7.08 (dd, 2 H); 6.76 - 6.80 (m, 1 H); 4.19 (s, 3 H); 2.25 (s, 3 H).
MS (ESI): [M+H]⁺ = 376.

### Example Compound 1.4

### Preparation of 1-[4-(1-Methyl-1H-pyrazolo[3,4-c]pyridin-4-yl)-phenyl]-3-(3-trifluoromethyl-phenyl)-urea

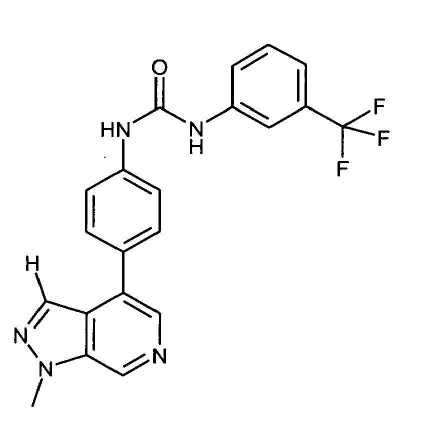

In analogy to GP 5, 112 mg of 4-(1-methyl-1H-pyrazolo[3,4-c]pyridin-4-yl)-phenylamine (Intermediate 3.1; 0.5 mmol, 1 eq.) were dissolved in 5.2 mL DCM and treated with 77 µL 1-isocyanato-3-trifluoromethyl-benzene (0.55 mmol, 1.1 eq.). Work-up as described in GP 5 followed by trituration provided 59 mg of the analytically pure product (0.143 mmol, 29% yield).
¹H-NMR (d₆-DMSO; 400 MHz): 9.10 (s, 1 H); 9.09 (s, 1 H); 9.02 (s, 1 H); 8.36 (s, 1 H); 8.31 (s, 1 H); 8.01 (s, 1 H); 7.74 (d, 2 H); 7.64 (d, 2 H); 7.58 (d, 1 H); 7.50 (t, 1 H); 7.29 (d, 1 H); 4.19 (s, 3 H).
MS (ESI): [M+H]⁺ = 412.

### Example Compound 1.5

### Preparation of 1-(3-Ethyl-phenyl)-3-[4-(1-methyl-1H-pyrazolo[3,4-c]pyridin-4-yl)-phenyl]-urea

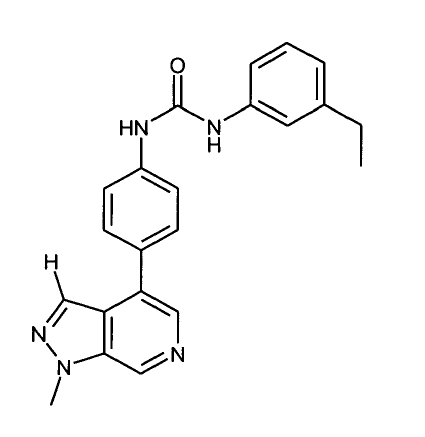

In analogy to GP 5, 224 mg of 4-(1-methyl-1H-pyrazolo[3,4-c]pyridin-4-yl)-phenylamine (Intermediate 3.1; 1 mmol, 1 eq.) were dissolved in 10 mL DCM and treated with 160 µL 1-ethyl-3-isocyanato-benzene (1.1 mmol, 1.1 eq.). Work-up as described in GP 5 followed by flash column chromatography and trituration provided 120 mg of the analytically pure product (0.323 mmol, 32% yield).
¹H-NMR (d₆-DMSO; 300 MHz): 9.08 (s, 1 H); 8.84 (s, 1 H); 8.64 (s, 1 H); 8.35 (s, 1 H); 8.31 (s, 1 H); 7.72 (d, 2 H); 7.62 (d, 2 H); 7.31 (s, 1 H); 7.25 (d, 1 H); 7.16 (t, 1 H); 6.80 (d, 1 H); 4.18 (s, 3 H); 2.55 (q, 2 H); 1.15 (t, 3 H).
MS (ESI): [M+H]⁺ = 372.

### Example Compound 1.6

### Preparation of 1-(3-Ethoxy-phenyl)-3-[4-(1-methyl-1H-pyrazolo[3,4-c]pyridin-4-yl)-phenyl]-urea

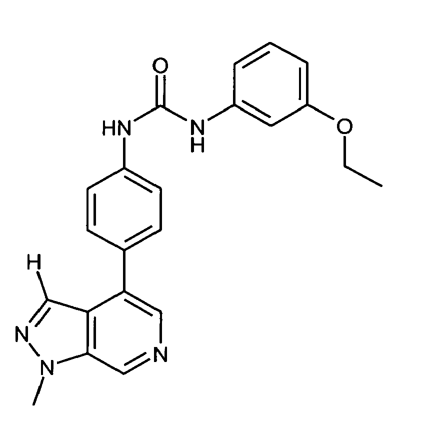

In analogy to GP 5, 224 mg of 4-(1-methyl-1H-pyrazolo[3,4-c]pyridin-4-yl)-phenylamine (Intermediate 3.1; 1 mmol, 1 eq.) were dissolved in 10 mL DCM and treated with 150 µL 1-ethoxy-3-isocyanato-benzene (1.1 mmol, 1.1 eq.). Work-up as described in GP 5 followed by flash column chromatography and trituration provided 93 mg of the analytically pure product (0.24 mmol, 24% yield).
¹H-NMR (d₆-DMSO; 400 MHz): 9.09 (s, 1 H); 8.87 (s, 1 H); 8.71 (s, 1 H); 8.35 (s, 1 H); 8.31 (s, 1 H); 7.72 (d, 2 H); 7.62 (d, 2 H); 7.12 - 7.17 (m, 2 H); 6.90 (dd, 1 H); 6.51 (dd, 1 H); 4.18 (s, 3 H); 3.97 (q, 2 H); 1.30 (t, 3 H).
MS (ESI): [M+H]⁺ = 388.

### Example Compound 1.7

### Preparation of 1-(4-Ethyl-pyridin-2-yl)-3-[4-(1-methyl-1H-pyrazolo[3,4-c]pyridin-4-yl)-phenyl]-urea

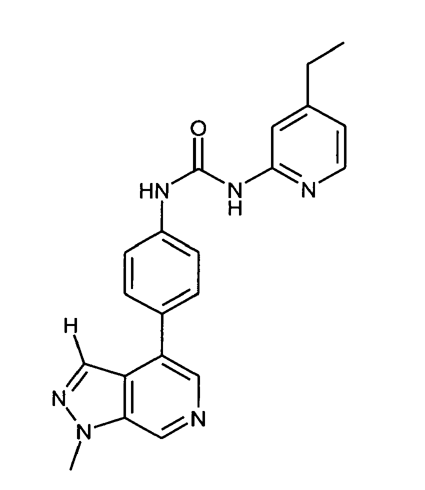

In analogy to GP 10, 112 mg of 4-(1-methyl-1H-pyrazolo[3,4-c]pyridin-4-yl)-phenylamine (Intermediate 3.1; 0.5 mmol, 1 eq.) were dissolved in 2 mL THF and treated with 10 µL *N*-methylpyrrolidine(0.1 mmol, 0.2 eq.) and 103 mg (4-ethyl-pyridin-2-yl)-carbamic acid isopropenyl ester (prepared in analogy to the above cited publication; 0.5 mmol, 1.0 eq.). The mixture was stirred overnight at 55 °C. Extractive work-up followed by trituration and preparative HPLC purification provided the target compound.
¹H-NMR (d₆-DMSO; 400 MHz): 10.83 (s., 1 H); 9.44 (s, 1 H); 9.10 (s, 1 H); 8.36 (s, 1 H); 8.31 (s, 1 H); 8.16 (d, 1 H); 7.75 (d, 2 H); 7.69 (d, 2 H); 7.31 (d, 1 H); 6.88 (dd, 1 H); 4.19 (s, 3 H); 2.57 (q, 2 H); 1.15 (q, 3 H).
MS (ESI): [M+H]⁺ = 373.

### Example Compound 1.8

### Preparation of 1-(4-Ethoxy-pyridin-2-yl)-3-[4-(1-methyl-1H-pyrazolo[3,4-c]pyridin-4-yl)-phenyl]-urea

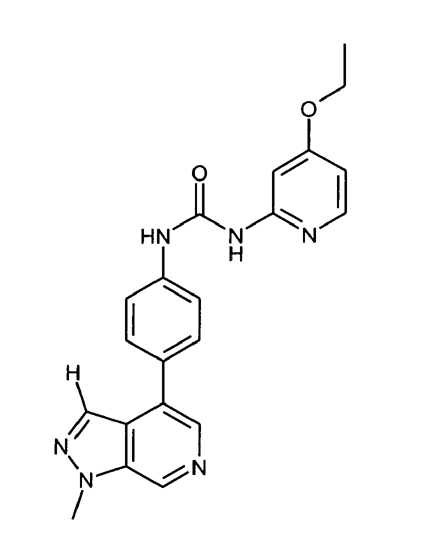

In analogy to GP 10, 224 mg of 4-(1-methyl-1H-pyrazolo[3,4-c]pyridin-4-yl)-phenylamine (Intermediate 3.1; 1 mmol, 1 eq.) were dissolved in 7 mL THF and treated with 21 µL *N*-methylpyrrolidine(0.2 mmol, 0.2 eq.) and 445 mg (4-ethoxy-pyridin-2-yl)-carbamic acid isopropenyl ester (prepared in analogy to the above cited publication; 2 mmol, 2.0 eq.). The mixture was stirred overnight at 55 °C. Extractive work-up followed by trituration provided the target compound.
¹H-NMR (d₆-DMSO; 400 MHz): 10.88 (br. s., 1 H); 9.41 (s, 1 H); 9.09 (s, 1 H); 8.36 (s, 1 H); 8.32 (s, 1 H); 8.08 (d, 1 H); 7.75 (d, 2 H); 7.69 (d, 2 H); 6.99 (d, 1 H); 6.61 (dd, 1 H); 4.19 (s, 3 H); 4.06 (q, 2 H); 1.32 (q, 3 H).
MS (ESI): [M+H]⁺ = 389.

### Example Compound 1.9

### Preparation of 1-[4-(1-Methyl-1H-pyrazolo[3,4-c]pyridin-4-yl)-phenyl]-3-(4-trifluoromethyl-pyridin-2-yl)-urea

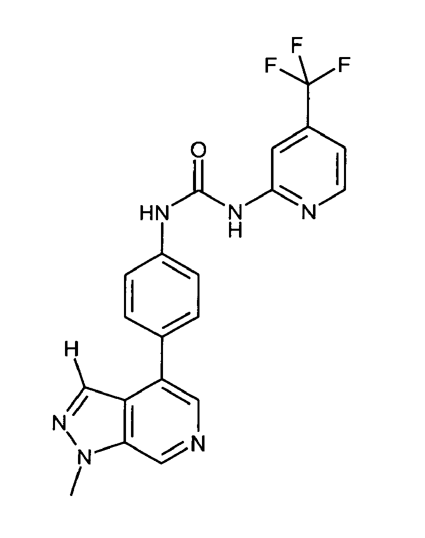

In analogy GP 10, 224 mg of 4-(1-methyl-1H-pyrazolo[3,4-c]pyridin-4-yl)-phenylamine (Intermediate 3.1; 1 mmol, 1 eq.) were dissolved in 7 mL THF and treated with 21 µL *N*-methylpyrrolidine(0.2 mmol, 0.2 eq.) and 492 mg (4-ethoxy-pyridin-2-yl)-carbamic acid isopropenyl ester (prepared in analogy to the above cited publication; 2 mmol, 2.0 eq.). The mixture was stirred overnight at 55 °C. Extractive work-up followed by trituration provided the target compound.
¹H-NMR (d₆-DMSO; 400 MHz): 9.83 (br. s., 2 H); 9.10 (s, 1 H); 8.53 (d, 1 H); 8.36 (s, 1 H); 8.31 (s, 1 H); 8.06 (s, 1 H); 7.76 (d, 2 H); 7.68 (d, 2 H); 7.34 (d, 1 H); 4.19 (s, 3 H).
MS (ESI): [M+H]⁺ = 413.

The following example compounds **1.10** to **1.13** were prepared from Intermediate 3.1 by reaction with the respective isocyanates according to GP 5 in analogy to the afore described procedures for compounds **1.1** to **1.6.**

| **Example** | **Structure** | **Name** | **Analytical data** |
|---|---|---|---|
| **1.10** | 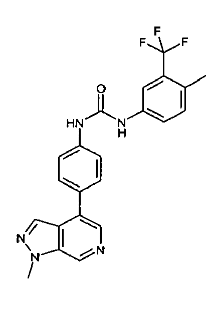 | 1-[4-(1-Methyl-1H-pyrazolo[3,4-c]pyridin-4-yl)-phenyl]-3-(4-methyl-3-trifluoromethyl-phenyl)-urea | ¹H-NMR: (DMSO, 300 MHz) 9.13 (s, 1 H); 9.02 (s, 1 H); 9.01 (s, 1 H); 8.40 (s, 1 H); 8.35 (s, 1 H); 7.97 (d, 1 H); 7.77 (d, 2 H); 7.68 (d, 2 H);7.54 (dd, 1 H); 7.36 (d, 1 H); 4.23 (s, 3 H); 2.39 (s, 3 H). MS (ESI): [M+H]⁺ = 426. |
| **1.11** | 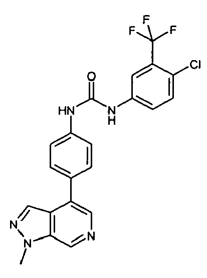 | 1-(4-Chloro-3- trifluoromethyl-phenyl)-3-[4-(1-methyl-1H-pyrazolo[3,4-c]pyridin-4-yl)-phenyl]-urea | ¹H-NMR: (DMSO, 300 MHz) 9.26 (s, 1 H); 9.14 (s, 1 H); 9.11 (s, 1 H); 8.40 (s, 1 H); 8.35 (s, 1 H); 8.14 (d, 1 H); 7.78 (d, 2 H); 7.62 - 7.70 (m, 4 H); 4.23 (s, 3 H). MS (ESI): [M+H]⁺ = 446. |
| **1.12** | 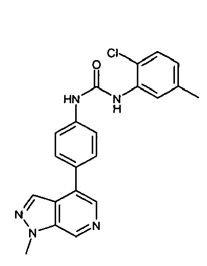 | 1-(2-Chloro-5-methyl-phenyl)-3-[4-(1-methyl-1H-pyrazolo[3,4-c]pyridin-4-yl)-phenyl]-urea | ¹H-NMR: (DMSO, 400 MHz) 9.59 (s, 1 H); 9.09 (s, 1 H); 8.36 (s, 1 H); 8.32 (s, 1 H); 8.28 (s, 1 H); 8.00 (s, 1 H); 7.74 (d, 2 H); 7.64 (d, 2 H); 7.30 (d, 1 H); 6.83 (d, 1 H); 4.19 (s, 3 H); 2.26 (s, 3 H). MS (ESI): [M+H]⁺ = 392. |
| **1.13** | 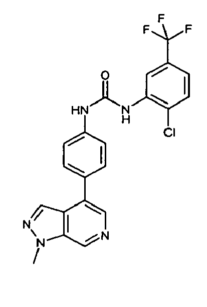 | 1-(2-Chloro-5-trifluoromethyl-phenyl)-3-[4-(1-methyl-1H-pyrazolo[3,4-c]pyridin-4-yl)-phenyl]-urea | ¹H-NMR: (DMSO, 400 MHz) 9.75 (s, 1 H); 9.10 (s, 1 H); 8.65 (s, 1 H); 8.63 (d, 1 H); 8.36 (s, 1 H); 8.32 (s, 1 H); 7.76 (d, 2 H); 7.70 (d, 1 H); 7.65 (d, 2 H); 7.36 (dd, 1 H); 4.19 (s, 3 H). MS (ESI): [M+H]⁺ = 446. |

### Example Compound 2.1

### Preparation of 1-(2-Methoxy-5-trifluoromethyl-phenyl)-3-[4-(1-methyl-1H-pyrazolo[3,4-c]pyridin-4-yl)-phenyl]-urea

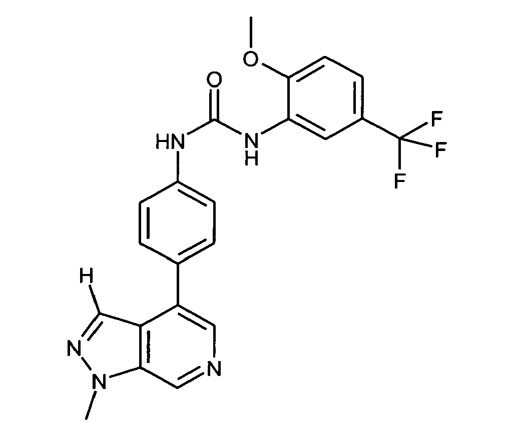

In analogy to GP 6, 115 mg of 2-methoxy-5-trifluoromethyl-phenylamine (0.6 mmol, 1.2 eq.) were dissolved in 10 mL acetonitrile, treated with 59.3 mg triphosgene (0.2 mmol, 0.4 eq.) and stirred at rt for 1 h upon which a precipitate was formed. After addition of 112 mg of 4-(1-methyl-1H-pyrazolo[3,4-c]pyridin-4-yl)-phenylamine (Intermediate 3.1; 0.5 mmol, 1 eq.) stirring at rt was continued for 48h. Work-up as described in GP 6 followed by trituration and flash column chromatography provided 19 mg of the analytically pure product (0.043 mmol, 9% yield).
¹H-NMR (d₆-DMSO; 400 MHz): 9.63 (s, 1 H); 9.09 (br. s, 1 H); 8.55 (br. s, 2 H); 8.36 (br. s, 1 H); 8.31 (s, 1 H); 7.74 (d, 2 H); 7.64 (d, 2 H); 7.30 (d, 1 H); 7.18 (d, 1 H); 4.19 (s, 3 H); 3.95 (s, 3 H).
MS (ESI): [M+H]⁺ = 442.

The following example compounds **2.2** to **X.3** were prepared from Intermediate **3.1** by triphosgene-mediated coupling with the respective anilines according to GP 6 in analogy to the afore described procedures for compounds **2.1.** For example compound **2.3,** 4-(4-amino-2-trifluoromethyl-benzyl)-piperazine-1-carboxylic acid tert-butyl ester was used as starting material for the triphosgene-mediated urea formation. Boc deprotection was achieved after urea formation by stirring with TFA in DCM.

| **Example** | **Structure** | **Name** | **Analytical data** |
|---|---|---|---|
| **2.2** | 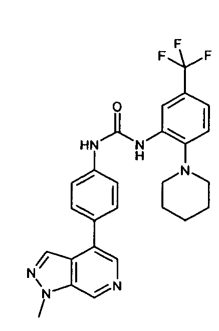 | 1-[4-(1-Methyl-1H-pyrazolo[3,4-c]pyridin-4-yl)-phenyl]-3-(2-piperidin-1-yl-5-trifluoromethyl-phenyl)-urea | ¹H-NMR: (DMSO, 300 MHz) 9.92 (br. s, 1 H); 9.14 (s, 1 H); 8.45 (s, 1 H); 8.41 (s, 1 H); 8.36 (s, 1 H); 8.24 (s, 1 H); 7.80 (d, 2 H); 7.72 (d, 2 H); 7.33 (s, 4 H); 4.23 (s, 3 H); 2.83 (t, 4 H); 1.78 - 1.85 (m, 4 H); 1.55 - 1.63 (m, 2 H). MS (ESI): [M+H]⁺ = 495. |
| **2.3** | 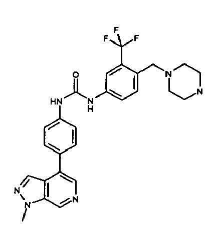 | 1-[4-(1-Methyl-1H-pyrazolo[3,4-c]pyridin-4-yl)-phenyl]-3-(4-piperazin-1-ylmethyl-3-trifluoromethyl-phenyl)-urea | ¹H-NMR: (DMSO, 300 MHz) 10.26 (s, 1 H); 10.13 (s, 1 H); 9.13 (s, 1 H); 8.45 (s, 1 H); 8.40 (s, 1 H); 8.36 (s, 1 H); 8.04 (d, 1 H); 7. 62 - 7.78 (m, 6 H); 4.24 (s, 3 H); 3.56 (s, 2 H); 2.88 - 2.93 (m, 4 H); 2.42 - 2.47 (m, 4 H). |

### Example Compound 3.1

### Preparation of 1-[2-Fluoro-4-(1-methyl-1H-pyrazolo[3,4-c]pyridin-4-yl)-phenyl]-3-(3-trifluoromethyl-phenyl)-urea

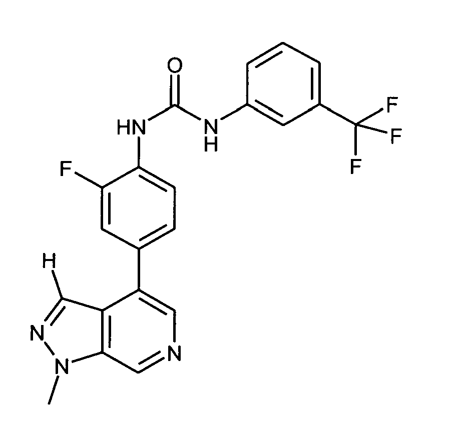

In analogy to GP 5, 121 mg of 2-fluoro-4-(1-methyl-1H-pyrazolo[3,4-c]pyridin-4-yl)-phenylamine (Intermediate 3.2; 0.5 mmol, 1 eq.) were dissolved in 5 mL DCM and treated with 77 µL 1-Isocyanato-3-trifluoromethyl-benzene (0.55 mmol, 1.1 eq.). The reaction mixture was concentrated in vacuo, the residue was taken up in ethyl acetate, water was added and the precipitate was filtered, washed with hexane and dried to provide 87 mg of the analytically pure product (0.20 mmol, 41% yield).
¹H-NMR (d₆-DMSO; 400 MHz): 9.45 (s, 1 H); 9.13 (s, 1 H); 8.82 (d, 1 H); 8.40 (s, 1 H); 8.36 (s, 1 H); 8.31 (t, 1 H); 8.03 (s, 1 H); 7.70 (dd, 1 H); 7.61 (dd, 1 H); 7.49 - 7.55 (m, 2 H); 7.32 (d, 1 H); 4.19 (s, 3 H).
MS (ESI): [M+H]⁺ = 430.

### Example Compound 4.1

### Preparation of 1-[2-Methyl-4-(1-methyl-1H-pyrazolo[3,4-c]pyridin-4-yl)-phenyl]-3-(3-trifluoromethyl-phenyl)-urea

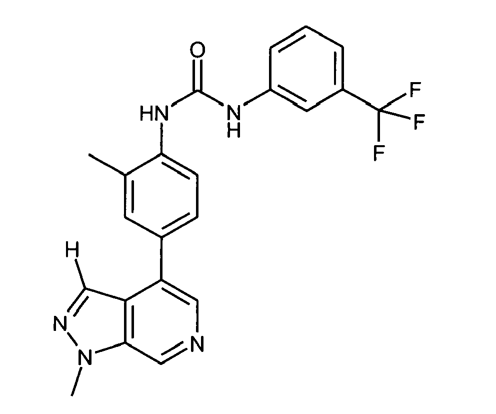

In analogy to GP 5, 80 mg of 2-methyl-4-(1-methyl-1H-pyrazolo[3,4-c]pyridin-4-yl)-phenylamine (Intermediate 3.3; 0.34 mmol, 1 eq.) were dissolved in 3.5 mL DCM and treated with 52 µL 1-isocyanato-3-trifluoromethyl-benzene (0.37 mmol, 1.1 eq.). The reaction mixture was concentrated in vacuo, the residue was taken up in ethyl acetate, water was added and the precipitate was filtered, washed with hexane and dried to provide 57 mg of the analytically pure product (0.134 mmol, 39% yield).
¹H-NMR (d₆-DMSO; 300 MHz): 9.45 (s, 1 H); 9.09 (s, 1 H); 8.36 (s, 1 H); 8.31 (s, 1 H); 8.17 (s, 1 H); 8.01 - 8.06 (m, 2 H); 7.46 - 7.64 (m, 4 H); 7.29 (d, 1 H); 4.19 (s, 3 H); 2.34 (s, 3 H).
MS (ESI): [M+H]⁺ = 426.

### Example Compound 4.2

### Preparation of 1-(2-Fluoro-5-trifluoromethyl-phenyl)-3-[2-methyl-4-(1-methyl-1H-pyrazolo[3,4-c]pyridin-4-yl)-phenyl]-urea

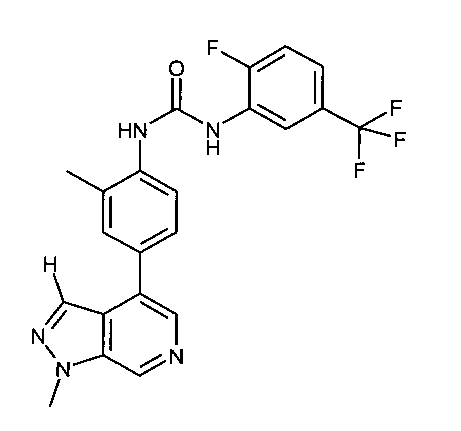

In analogy to GP 5, 160 mg of 2-methyl-4-(1-methyl-1H-pyrazolo[3,4-c]pyridin-4-yl)-phenylamine (Intermediate 3.3; 0.67 mmol, 1 eq.) were dissolved in 6 mL DCM and treated with 110 µL 1-fluoro-2-isocyanato-4-trifluoromethyl-benzene (0.74 mmol, 1.1 eq.). The reaction mixture was concentrated in vacuo, the residue was taken up in ethyl acetate, water was added and the precipitate was filtered, washed with hexane and dried to provide 75 mg of the analytically pure product (0.17 mmol, 25% yield).
¹H-NMR (d₆-DMSO; 300 MHz): 9.40 (s, 1 H); 9.10 (s, 1 H); 8.66 (dd, 1 H); 8.61 (s, 1 H); 8.36 (s, 1 H); 8.31 (s, 1 H); 8.08 (d, 1 H); 7.63 (s, 1 H); 7.60 (dd, 1 H); 7.48 (dd, 1 H); 7.34 - 7.38 (m, 1 H); 4.19 (s, 3 H); 2.35 (s, 3 H).
MS (ESI): [M+H]⁺ = 444.

### Example Compound 5.1

### Preparation of 1-[2-Methoxy-4-(1-methyl-1H-pyrazolo[3,4-c]pyridin-4-yl)-phenyl]-3-(3-trifluoromethyl-phenyl)-urea

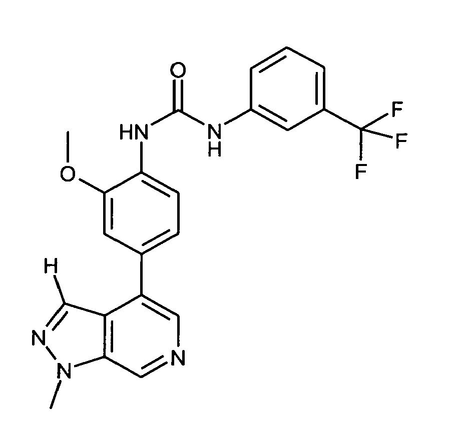

In analogy to GP 5, 127 mg of 2-methoxy-4-(1-methyl-1H-pyrazolo[3,4-c]pyridin-4-yl)-phenylamine (Intermediate 3.4; 0.5 mmol, 1 eq.) were dissolved in 5 mL DCM and treated with 77 µL 1-isocyanato-3-trifluoromethyl-benzene (0.55 mmol, 1.1 eq.). After work-up as described in GP 5, flash column chromatography followed by trituration provided 110 mg of the analytically pure product (0.25 mmol, 50% yield).
¹H-NMR (d₆-DMSO; 300 MHz): 9.77 (s, 1 H); 9.15 (s, 1 H); 8.49 (s, 1 H); 8.46 (s, 1 H); 8.42 (s, 1 H); 8.35 (d, 1 H); 8.07 (s, 1 H); 7.52 - 7.57 (m, 2 H); 7.32 - 7.43 (m, 3 H); 4.24 (s, 3 H); 4.05 (s, 3 H).
MS (ESI): [M+H]⁺ = 442.

### Example Compound 5.2

### Preparation of 1-(2-Fluoro-5-trifluoromethyl-phenyl)-3-[2-methoxy-4-(1-methyl-1 H-pyrazolo[3,4-c]pyridin-4-yl)-phenyl]-urea

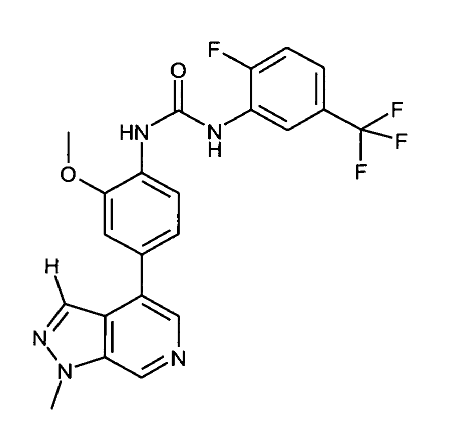

In analogy to GP 5, 127 mg of 2-methoxy-4-(1-methyl-1H-pyrazolo[3,4-c]pyridin-4-yl)-phenylamine (Intermediate 3.4; 0.5 mmol, 1 eq.) were dissolved in 5 mL DCM and treated with 80 µL 1-fluoro-2-isocyanato-4-trifluoromethyl-benzene (0.55 mmol, 1.1 eq.). The reaction mixture was concentrated in vacuo, the residue was taken up in ethyl acetate, water was added and the precipitate was filtered, washed with hexane and dried to provide 74 mg of the analytically pure product (0.16 mmol, 32% yield).
¹H-NMR (d₆-DMSO; 400 MHz): 9.63 (d, 1 H); 9.10 (s, 1 H); 9.06 (s, 1 H); 8.66 (dd, 1 H); 8.41 (s, 1 H); 8.37 (s, 1 H); 8.30 (d, 1 H); 7.47 (dd, 1 H); 7.33 - 7.38 (m, 3 H); 4.19 (s, 3 H); 4.00 (s, 3 H);
MS (ESI): [M+H]⁺ = 460.

### Example Compound 6.1

### Preparation of 1-Methyl-1-[4-(1-methyl-1H-pyrazolo[3,4-c]pyridin-4-yl)-phenyl]-3-(3-trifluoromethyl-phenyl)-urea

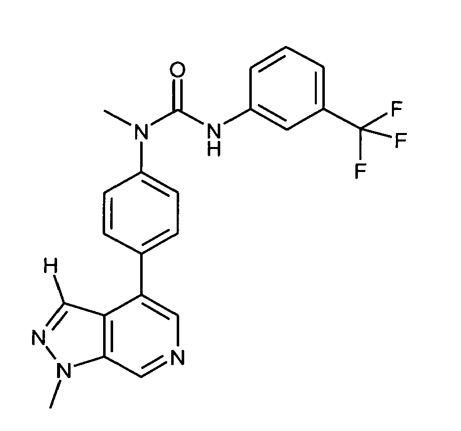

In analogy to GP 3, 170 mg of Intermediate 2.1 (0.8 mmol, 1 eq.), 403 mg of 1-methyl-1-[4-(4,4,5,5-tetramethyl-[1,3,2]dioxaborolan-2-yl)-phenyl]-3-(3-trifluoromethyl-phenyl)-urea (0.96 mmol, 1.2 eq.) and 55.5 mg Pd(PPh₃)₄ (0.048 mmol, 6 mol%) were weighed into a Biotage microwave vial and capped. 8 mL toluene, 8 mL EtOH and 1M aq. Na₂CO₃ solution (1.54 mL, 1.54 mmol, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with water and ethyl acetate, the layers were separated and the aqueous layer extracted with ethyl acetate. The combined organic layers were dried and concentrated in vacuo to yield after flash column chromatography and trituration 89 mg of the desired product (0.21 mmol, 26% yield).
¹H-NMR (d₆-DMSO; 300 MHz): 9.14 (s, 1 H); 8.74 (s, 1 H); 8.41 (s, 1 H); 8.33 (s, 1 H); 7.90 (s, 1 H); 7.84 (d, 2 H); 7.75 (d, 1 H); 7.50 (d, 2 H); 7.44 (t, 1 H); 7.25 (d, 1 H); 4.20 (s, 3 H); 3.33 (s, 3 H).
MS (ESI): [M+H]⁺ = 426.

### Example Compound 6.2

### Preparation of 1-Methyl-3-[4-(1-methyl-1H-pyrazolo[3,4-c]pyridin-4-yl)-phenyl]-1-(3-trifluoromethyl-phenyl)-urea

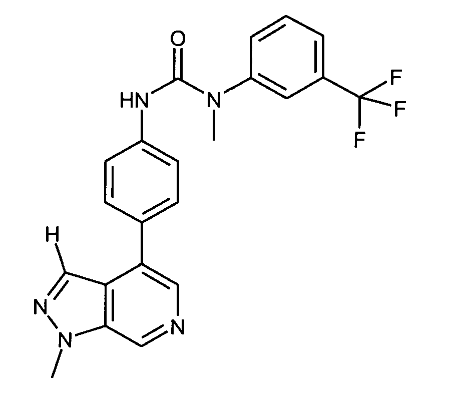

In analogy to GP 3, 170 mg of Intermediate 2.1 (0.8 mmol, 1 eq.), 403 mg of 1-methyl-3-[4-(4,4,5,5-tetramethyl-[1,3,2]dioxaborolan-2-yl)-phenyl]-1-(3-trifluoromethyl-phenyl)-urea (0.96 mmol, 1.2 eq.) and 55.5 mg Pd(PPh₃)₄ (0.048 mmol, 6 mol%) were weighed into a Biotage microwave vial and capped. 8 mL toluene, 8 mL EtOH and 1M aq. Na₂CO₃ solution (1.54 mL, 1.54 mmol, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography and trituration 96 mg of the desired product (0.23 mmol, 28% yield).
¹H-NMR (d₆-DMSO; 300 MHz): 9.09 (s, 1 H); 8.73 (s, 1 H); 8.34 (s, 1 H); 8.30 (s, 1 H); 7.51 - 7.71 (m, 8 H); 4.18 (s, 3 H); 3.35 (s, 3 H).
MS (ESI): [M+H]⁺ = 426.

### Example Compound 6.3

### Preparation of 1-[2-Fluoro-4-(1-methyl-1H-pyrazolo[3,4-c]pyridin-4-yl)-phenyl]-3-(2-fluoro-5-trifluoromethyl-phenyl)-urea

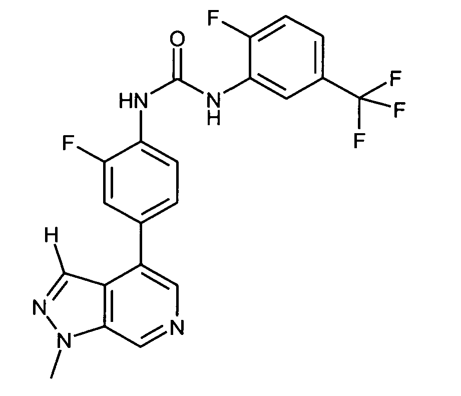

In analogy to GP 3, 106 mg of Intermediate 2.1 (0.5 mmol, 1 eq.), 265 mg of 1-[2-fluoro-4-(4,4,5,5-tetramethyl-[1,3,2]dioxaborolan-2-yl)-phenyl]-3-(2-fluoro-5-trifluoromethyl-phenyl)-urea (0.6 mmol, 1.2 eq.) and 35 mg Pd(PPh₃)₄ (0.03 mmol, 6 mol%) were weighed into a Biotage microwave vial and capped. 3 mL toluene, 3 mL EtOH and 1M aq. Na₂CO₃ solution (0.96 mL, 0.96 mmol, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with water and ethyl acetate, the layers were separated and the aqueous layer extracted with ethyl acetate. The combined organic layers were dried and concentrated in vacuo to yield after trituration 127 mg of the desired product (0.28 mmol, 57% yield).
¹H-NMR (d₆-DMSO; 300 MHz): 9.42 (s, 1 H); 9.33 (s, 1 H); 9.13 (s, 1 H); 8.63 (dd, 1 H); 8.40 (s, 1 H); 8.36 (d, 1 H); 8.35 (s, 1 H); 7.71 (dd, 1 H); 7.62 (dd, 1 H); 7.49 (dd, 1 H); 7.35 - 7.41 (m, 1 H); 4.19 (s, 1 H).
MS (ESI): [M+H]⁺ = 448.

### Example Compound 6.4

### Preparation of 1-[4-(4-Methyl-piperazin-1-ylmethyl)-3-trifluoromethyl-phenyl]-3-[4-(1-methyl-1H-pyrazolo[3,4-c]pyridin-4-yl)-phenyl]-urea

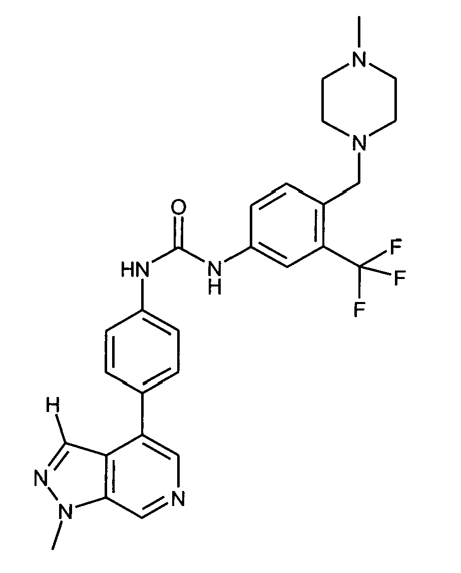

In analogy to GP 3, 84.8 mg of Intermediate 2.1 (0.4 mmol, 1 eq.), 249 mg of 1-[4-(4-methyl-piperazin-1-ylmethyl)-3-trifluoromethyl-phenyl]-3-[4-(4,4,5,5-tetramethyl-[1,3,2]dioxaborolan-2-yl)-phenyl]-urea (0.48 mmol, 1.2 eq.) and 28 mg Pd(PPh₃)₄ (0.024 mmol, 6 mol%) were weighed into a Biotage microwave vial and capped. 2 mL toluene, 2 mL EtOH and 1M aq. Na₂CO₃ solution (0.77 mL, 0.77 mmol, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after preparative HPLC purification 66.3 mg of the desired product (0.127 mmol, 32% yield).
¹H-NMR (d₆-DMSO; 300 MHz): 9.09 (s, 1 H); 9.03 (s, 1 H); 8.96 (s, 1 H); 8.35 (s, 1 H); 8.31 (s, 1 H); 7.95 (d, 1 H); 7.73 (d, 2 H); 7.63 (d, 2 H); 7.60 (d, 1 H); 7.54 - 7.57 (m, 1 H); 4.19 (s, 3 H); 3.50 (s, 2 H); 2.21 - 2.42 (m, 8 H); 2.12 (s, 3 H).
MS (ESI): [M+H]⁺ = 524.

### Example Compound 7.1

### Preparation of 1-[2-Hydroxy-4-(1-methyl-1H-pyrazolo[3,4-c]pyridin-4-yl)-phenyl]-3-(3-trifluoromethyl-phenyl)-urea

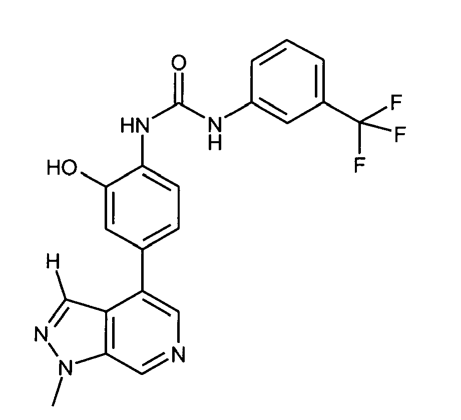

157 mg of 1-[2-methoxy-4-(1-methyl-1H-pyrazolo[3,4-c]pyridin-4-yl)-phenyl]-3-(3-trifluoromethyl-phenyl)-urea (Example Compound 5.1; 0.36 mmol, 1 eq.) were dissolved in 10 mL DCM/DMF (30:1), treated with 0.53 mL BBr₃ solution (1.0 M in DCM; 0.53 mmol, 1.5 eq) at 0 °C and subsequently stirred for 1h at rt. TLC indicated incomplete conversion. 1.24 mL BBr₃ solution (1.24 mmol, 3.5 eq.) were added at 0 °C and stirring was continued at rt for 16h. The reaction mixture was quenched with aq. NaHCO₃ solution and diluted with ethyl acetate. The precipitate was filtered off and the organic layer was dried and concentrated. The residue was combined with the solid (see above) and triturated from ethyl acetate to yield 70 mg of the target compound (0.164 mmol, 46% yield).
¹H-NMR (d₆-DMSO; 300 MHz): 10.37 (br., 1 H); 9.73 (s, 1 H); 9.08 (s, 1 H); 8.39 (s, 1 H); 8.31 (s, 1 H); 8.26 (s, 1 H); 8.23 (d, 1 H); 8.02 (s, 1 H); 7.51 (s, 1 H); 7.49 (t, 1 H); 7.26 - 7.29 (m, 2 H); 7.21 (dd, 1 H); 4.18 (s, 3 H).
MS (ESI): [M+H]⁺ = 428.

### Example Compound 8.1

### Preparation of 1-{2-Fluoro-4-[1-(2-hydroxy-ethyl)-1H-pyrazolo[3,4-c]pyridin-4-yl]-phenyl}-3-(2-fluoro-5-trifluoromethyl-phenyl)-urea

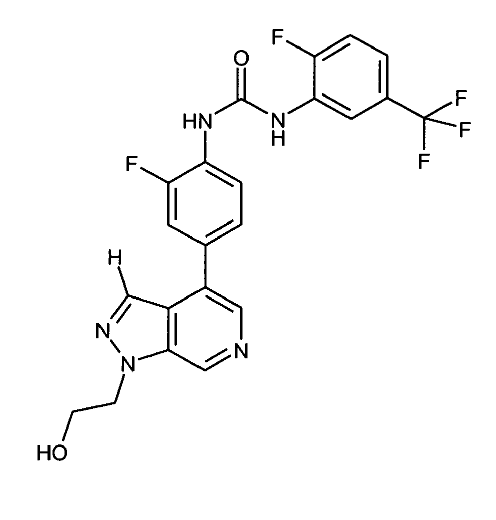

In analogy to GP 3, 80 mg of Intermediate 2.2 (0.33 mmol, 1 eq.), 175 mg of 1-[2-fluoro-4-(4,4,5,5-tetramethyl-[1,3,2]dioxaborolan-2-yl)-phenyl]-3-(2-fluoro-5-trifluoromethyl-phenyl)-urea (0.4 mmol, 1.2 eq.) and 23 mg Pd(PPh₃)₄ (0.02 mmol, 6 mol%) were weighed into a Biotage microwave vial and capped. 1.5 mL toluene, 1.5 mL EtOH and 1M aq. Na₂CO₃ solution (0.64 mL, 0.64 mmol, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after trituration 64 mg of the desired product (0.134 mmol, 41% yield).
¹H-NMR (d₆-DMSO; 400 MHz): 9.43 (s, 1 H); 9.33 (s, 1 H); 9.13 (s, 1 H); 8.64 (dd, 1 H); 8.37 (s, 1 H); 8.34 - 8.38 (m, 2 H); 7.70 (dd, 1 H); 7.62 (dd, 1 H); 7.49 (dd, 1 H); 7.36 - 7.41 (m, 1 H); 4.90 (t, 1 H); 4.60 (t, 2 H); 3.82 (q, 2 H).
MS (ESI): [M+H]⁺ = 478.

### Example Compound 8.2

### Preparation of 1-(2-Fluoro-5-trifluoromethyl-phenyl)-3-{4-[1-(2-hydroxy-ethyl)-1 H-pyrazolo[3,4-c]pyridin-4-yl]-phenyl}-urea

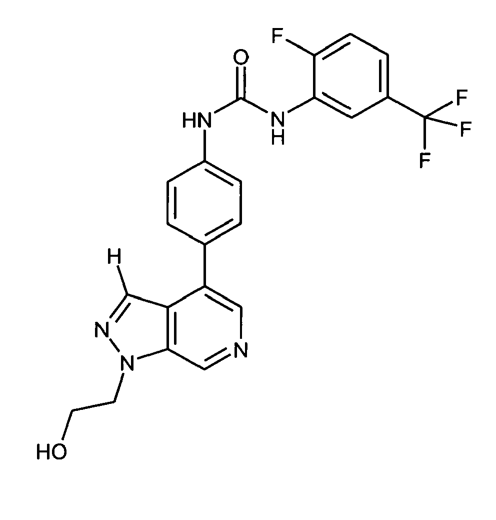

In analogy to GP 3, 230 mg of Intermediate 2.2 (used without prior purification; 0.95 mmol, 1 eq.), 484 mg of 1-(2-fluoro-5-trifluoromethyl-phenyl)-3-[4-(4,4,5,5-tetramethyl-[1,3,2]dioxaborolan-2-yl)-phenyl]-urea (1.14 mmol, 1.2 eq.) and 66 mg Pd(PPh₃)₄ (0.057 mmol, 6 mol%) were weighed into a Biotage microwave vial and capped. 4.3 mL toluene, 4.3 mL EtOH and 1M aq. Na₂CO₃ solution (1.83 mL, 1.83 mmol, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography and trituration 33 mg of the desired product (0.072 mmol, 6% yield).
¹H-NMR (d₆-DMSO; 400 MHz): 9.39 (s, 1 H); 9.14 (s, 1 H); 8.97 (d, 1 H); 8.66 (dd, 1 H); 8.38 (s, 1 H); 8.37 (s, 1 H); 7.79 (d, 2 H); 7.69 (d, 2 H); 7.53 (dd, 1 H); 7.39 - 7.46 (m, 1 H); 4.94 (t, 1 H); 4.64 (t, 2 H); 3.87 (q, 2 H).
MS (ESI): [M+H]⁺ = 460.

### Example Compound 8.3

### Preparation of 1-{4-[1-(2-Hydroxy-ethyl)-1H-pyrazolo[3,4-c]pyridin-4-yl]-phenyl}-3-(3-trifluoromethyl-phenyl)-urea

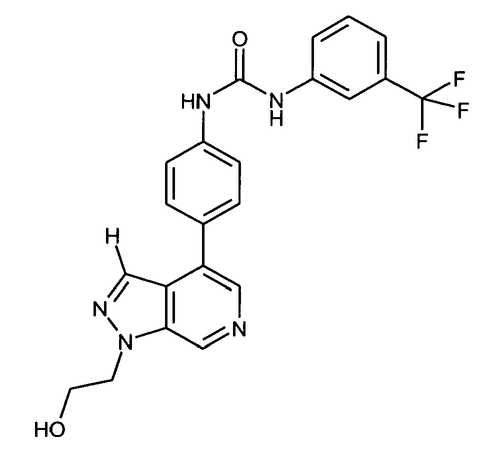

In analogy to GP 3, 100 mg of Intermediate 2.2 (0.41 mmol, 1 eq.), 201 mg of 1-[4-(4,4,5,5-tetramethyl-[1,3,2]dioxaborolan-2-yl)-phenyl]-3-(3-trifluoromethylphenyl)-urea (0.5 mmol, 1.2 eq.) and 29 mg Pd(PPh₃)₄ (0.025 mmol, 6 mol%) were weighed into a Biotage microwave vial and capped. 1.9 mL toluene, 1.9 mL EtOH and 1M aq. Na₂CO₃ solution (0.8 mL, 0.8 mmol, 1.9 eq.) were subsequently added by syringe. The resulting mixture was prestirred (10 sec) and subsequently heated to 120 °C for 15 min (fixed hold time) in a Biotage Initiator@ microwave reactor. The reaction mixture was diluted with water and ethyl acetate, the layers were separated and the aqueous layer extracted with ethyl acetate. The combined organic layers were dried and concentrated in vacuo to yield after flash column chromatography 87 mg of the desired product (0.197 mmol, 48% yield).
¹H-NMR (d₆-DMSO; 400 MHz): 9.12 (s, 1 H); 9.09 (s, 1 H); 9.03 (s, 1 H); 8.33 (s, 2 H); 8.01 (s, 1 H); 7.73 (d, 2 H); 7.64 (d, 2 H); 7.58 (d, 1 H); 7.50 (t, 1 H); 7.29 (d, 1 H); 4.91 (t, 1 H); 4.59 (t, 2 H); 3.82 (q, 2 H).
MS (ESI): [M+H]⁺ = 442.

### Example Compound 8.4

### Preparation of 1-(3-Ethyl-phenyl)-3-{2-fluoro-4-[1-(2-hydroxy-ethyl)-1H-pyrazolo[3,4-c]pyridin-4-yl]-phenyl}-urea

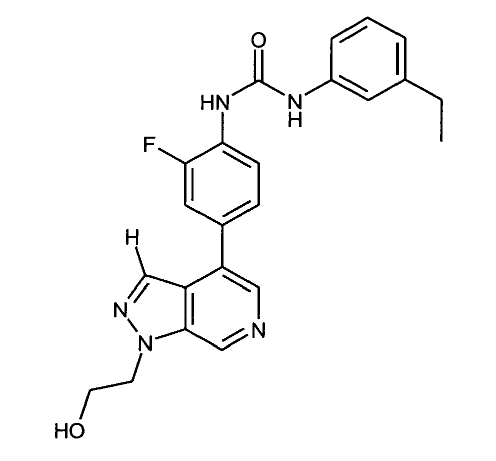

In analogy to GP 3, 100 mg of Intermediate 2.2 (0.41 mmol, 1 eq.), 363 mg of 1-(3-ethyl-phenyl)-3-[2-fluoro-4-(4,4,5,5-tetramethyl-[1,3,2]dioxaborolan-2-yl)-phenyl]-urea (0.95 mmol, 2.3 eq.) and 29 mg Pd(PPh₃)₄ (0.025 mmol, 6 mol%) were weighed into a Biotage microwave vial and capped. 1.9 mL toluene, 1.9 mL EtOH and 1M aq. Na₂CO₃ solution (0.8 mL, 0.8 mmol, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with water and ethyl acetate, the layers were separated and the aqueous layer extracted with ethyl acetate. The combined organic layers were dried and concentrated in vacuo to yield after flash column chromatography the desired product.
MS (ESI): [M+H]⁺ = 420.

The following example compounds **8.5** to **8.6** were prepared from Intermediate 2.2 by Suzuki coupling according to GP 3 with the respective boronic acid pinacolate esters in analogy to the afore described procedures for compounds **8.1** to **8.4.**

| **Example** | **Structure** | **Name** | **Analytical data** |
|---|---|---|---|
| **8.5** | 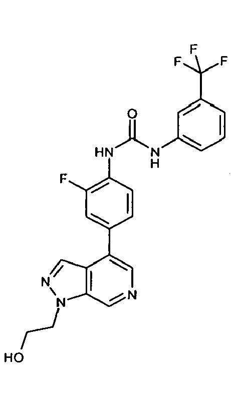 | 1-{2-Fluoro-4-[1-(2-hydroxy-ethyl)-1H-pyrazolo[3,4-c]pyridin-4-yl]-phenyl}-3-(3-trifluoromethyl-phenyl)-urea | ¹H-NMR: (DMSO, 300 MHz) 9.45 (s, 1 H); 9.12 (s, 1 H); 8.80 (s, 1 H); 8.37 (s, 1 H); 8.36 (s, 1 H); 8.31 (t, 1 H); 8.03 (s, 1 H); 7.69 (dd, 1 H); 7.61 (dd, 1 H); 7.53 (s, 1 H); 7.51 (t, 1 H); 7.32 (d, 1 H); 4.91 (t, 1 H); 4.60 (t, 2 H); 3.82 (q, 2 H). MS (ESI): [M+H]⁺ = 460. |
| **8.6** | 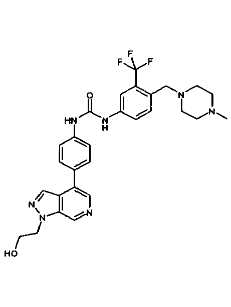 | 1-{4-[1-(2-Hydroxy-ethyl)-1 H-pyrazolo[3,4-c]pyridin-4-yl]-phenyl}-3-[4-(4-methyl-piperazin-1-ylmethyl)-3-trifluoromethyl-phenyl]-urea | ¹H-NMR: (DMSO, 400 MHz) 9.08 (s, 2 H); 9.00 (s, 1 H); 8.32 (s, 2 H); 7.96 (s, 1 H); 7.72 (d, 2 H); 7.64 (d, 2 H); 7.60 (d, 1 H); 7.56 (dd, 1 H); 4.91 (t, 1 H); 4.59 (t, 2 H); 3.82 (q, 2 H); 3.50 (s, 2 H); 2.23 - 2.41 (m, 8 H); 2.12 (s, 3 H). |

### Example Compound 9.1

### Preparation of 1,3-Bis-[4-(1-methyl-1H-pyrazolo[3,4-c]pyridin-4-yl)-phenyl]-urea

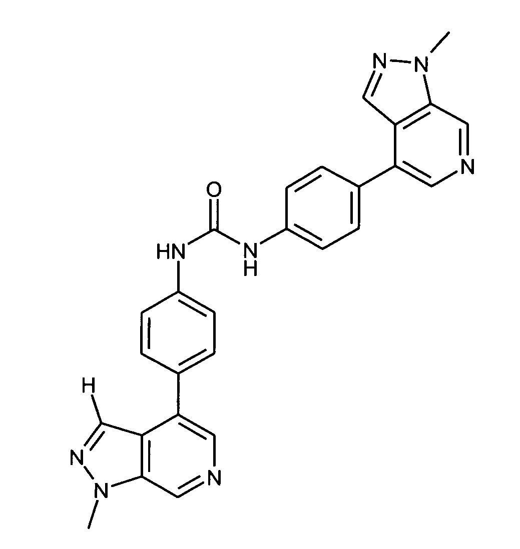

Example Compound 9.1 is accessible from Intermediate 3.1 by triphosgene coupling according to GP 6.
¹H-NMR (d₆-DMSO; 300 MHz): 9.09 (s, 2 H); 8.98 (s, 2 H); 8.36 (s, 2 H); 8.33 (s, 2 H); 7.64 - 7.77 (m, 8 H); 4.19 (s, 6 H).
MS (ESI): [M+H]⁺ = 475.

### Example Compound 10.1

### Preparation of 1-[4-(1-Ethyl-1H-pyrazolo[3,4-c]pyridin-4-yl)-phenyl]-3-(3-trifluoromethyl-phenyl)-urea

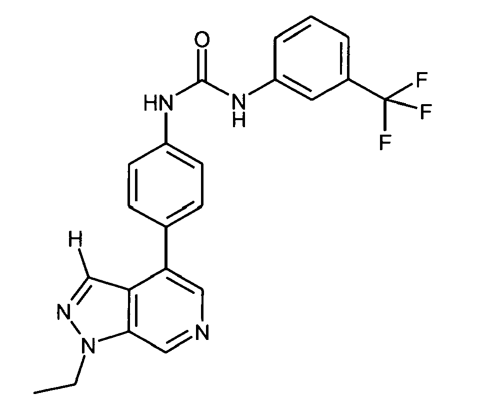

In analogy to GP 5, 119 mg of 4-(1-ethyl-1H-pyrazolo[3,4-c]pyridin-4-yl)-phenylamine (Intermediate 3.5; 0.5 mmol, 1 eq.) were dissolved in 5 mL DCM and treated with 70 µL 1-isocyanato-3-trifluoromethyl-benzene (0.5 mmol, 1 eq.). The reaction mixture was stirred at rt overnight upon which it was concentrated in vacuo, the residue was partitioned between ethyl acetate and water and the aqueous phase was re-extracted with ethyl acetate several times. The combined organic layers were dried and concentrated in vacuo. Trituration of the residue provided 56 mg of the analytically pure target compound.
¹H-NMR (d₆-DMSO; 300 MHz): 9.13 (s, 1 H); 9.09 (s, 1 H); 9.00 (s, 1 H); 8.35 (s, 1 H); 8.32 (s, 1 H); 8.01 (s, 1 H); 7.73 (d, 2 H); 7.64 (d, 2 H); 7.58 (d, 1 H); 7.49 (t, 1 H); 7.29 (d, 1 H); 4.59 (q, 2 H); 1.44 (t, 3 H).
MS (ESI): [M+H]⁺ = 426.

The following example compounds **10.2** to **10.8** were prepared from Intermediate 3.5 by reaction with the respective isocyanates according to GP 5 in analogy to example compound 10.1.

| **Example** | **Structure** | **Name** | **Analytical data** |
|---|---|---|---|
| **10.2** | 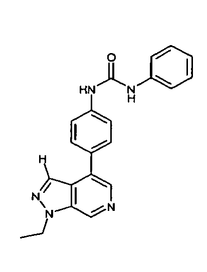 | 1-[4-(1-Ethyl-1H-pyrazolo[3,4-c]pyridin-4-yl)-phenyl]-3-phenyl-urea | ¹H-NMR: (DMSO, 300 MHz) 9.12 (s, 1 H); 8.86 (s, 1 H); 8.71 (s, 1 H); 8.35 (s, 1 H); 8.32 (s, 1 H); 7.72 (d, 2 H); 7.62 (d, 2 H); 7.45 (d, 2 H); 7.26 (t, 2 H); 6.95 (t, 1 H); 4.58 (q, 2 H); 1.44 (t, 3 H). MS (ESI): [M+H]⁺ = 358. |
| **10.3** | 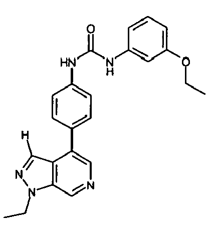 | 1-(3-Ethoxy-phenyl)-3-[4-(1-ethyl-1H-pyrazolo[3,4-c]pyridin-4-yl)-phenyl]-urea | ¹H-NMR: (DMSO, 300 MHz) 9.13 (s, 1 H); 8.85 (s, 1 H); 8.70 (s, 1 H); 8.34 (s, 1 H); 8.32 (s, 1 H); 7.72 (d, 2 H); 7.62 (d, 2 H); 7.11 7.18 (m, 2 H); 6.90 (dd, 1 H); 6.51 (dd, 1 H); 4.58 (q, 2 H); 3.97 (q, 2 H); 1.44 (t, 3 H); 1.30 (t, 3 H). MS (ESI): [M+H]+ = 402. |
| **10.4** | 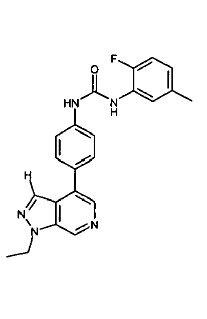 | 1-[4-(1-Ethyl-1 H-pyrazolo[3,4-c]pyridin-4-yl)-phenyl]-3-(2-fluoro-5-methyl-phenyl)-urea | ¹H-NMR: (DMSO, 300 MHz) 9.24 (s, 1 H); 9.13 (s, 1 H); 8.51 (s, 1 H); 8.35 (s, 1 H); 8.32 (s, 1 H); 7.98 (dd, 1 H); 7.74 (d, 2 H); 7.63 (d, 2 H); 7.08 (dd, 1 H); 6.75 - 6.80 (m, 1 H); 4.58 (q, 2 H); 2.25 (s, 3 H); 1.44 (t, 3 H). MS (ESI): [M+H]+ = 390. |
| **10.5** | 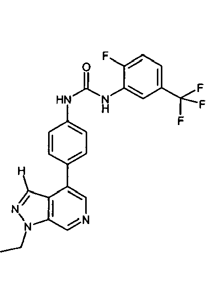 | 1-[4-(1-Ethyl-1 H-pyrazolo[3,4-c]pyridin-4-yl)-phenyl]-3-(2-fluoro-5-trifluoromethyl-phenyl)-urea | ¹H-NMR: (DMSO, 300 MHz) 9.36 (s, 1 H); 9.14 (s, 1 H); 8.94 (s, 1 H); 8.61 (dd, 1 H); 8.35 (s, 1 H); 8.32 (s, 1 H); 7.75 (d, 2 H); 7.64 (d, 2 H); 7.49 (dd, 1 H); 7.34 - 7.40 (m, 1 H); 4.58 (q, 2 H); 1.44 (t, 3 H). MS (ESI): [M+H]+ = 444. |
| **10.6** | 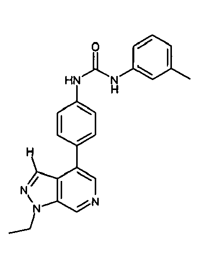 | 1-[4-(1-Ethyl-1H-pyrazolo[3,4-c]pyridin-4-yl)-phenyl]-3-m-tolyl-urea | ¹H-NMR: (DMSO, 400 MHz) 9.13 (s, 1 H); 8.84 (s, 1 H); 8.62 (s, 1 H); 8.34 (s, 1 H); 8.32 (s, 1 H); 7.72 (d, 2 H); 7.62 (d, 2 H); 7.28 (s, 1 H); 7.23 (d, 1 H); 7.14 (t, 1 H);6.77 (d, 1 H); 4.58 (q, 2 H); 2.26 (s, 3 H); 1.44 (t, 3 H). MS (ESI): [M+H]+ = 372. |
| **10.7** | 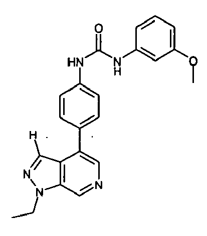 | 1-[4-(1-Ethyl-1H-pyrazolo[3,4-c]pyridin-4-yl)-phenyl]-3-(3-methoxy-phenyl)-urea | ¹H-NMR: (DMSO, 400 MHz) 9.13 (s, 1 H); 8.86 (s, 1 H); 8.73 (s, 1 H); 8.34 (s, 1 H); 8.32 (s, 1 H); 7.72 (d, 2 H); 7.62 (d, 2 H); 7.18 (s, 1 H); 7.16 (t, 1 H); 6.92 (d, 1 H); 6.53 (d, 1 H); 4.60 (q, 2 H); 3.70 (s, 3 H); 1.44 (t, 3 H). MS (ESI): [M+H]+ = 388. |
| **10.8** | 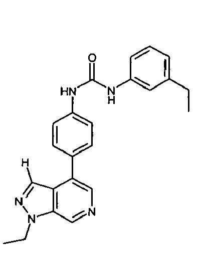 | 1-(3-Ethyl-phenyl)-3-[4-(1-ethyl-1H-pyrazolo[3,4-c]pyridin-4-yl)-phenyl]-urea | ¹H-NMR: (DMSO, 400 MHz) 9.13 (s, 1 H); 8.86 (s, 1 H); 8.67 (s, 1 H); 8.35 (s, 1 H); 8.32 (s, 1 H); 7.72 (d, 2 H); 7.62 (d, 2 H); 7.31 (s, 1 H); 7.25 (d, 1 H); 7.16 (t, 1 H); 6.80 (d, 1 H); 4.59 (q, 2 H); 2.55 (q, 2 H); 1.44 (t, 3 H); 1.15 (t, 3 H). MS (ESI): [M+H]+ = 386. |

### Example Compound 11.1

### Preparation of 1-[4-(1-Ethyl-1H-pyrazolo[3,4-c]pyridin-4-yl)-2-fluoro-phenyl]-3-(3-trifluoromethyl-phenyl)-urea

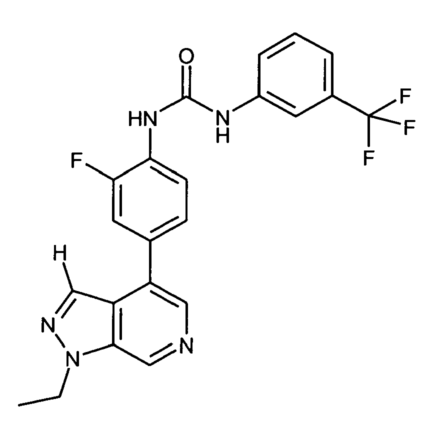

In analogy to GP 5, 128 mg of 4-(1-ethyl-1H-pyrazolo[3,4-c]pyridin-4-yl)-2-fluoro-phenylamine (Intermediate 3.6; 0.5 mmol, 1 eq.) were dissolved in 5 mL DCM and treated with 70 µL 1-isocyanato-3-trifluoromethyl-benzene (0.5 mmol, 1 eq.). The reaction mixture was stirred at rt overnight upon which it was concentrated in vacuo, the residue was partitioned between ethyl acetate and water and the aqueous phase was re-extracted with ethyl acetate several times. The combined organic layers were dried and concentrated in vacuo. Trituration of the residue provided 71 mg of the analytically pure target compound.
¹H-NMR (d₆-DMSO; 300 MHz): 9.50 (s, 1 H); 9.22 (s, 1 H); 8.86 (s, 1H); 8.44 (s, 1 H); 8.41 (s, 1 H); 8.36 (t, 1 H); 8.07 (s, 1 H); 7.74 (dd, 1 H); 7.66 (dd, 1 H); 7.53 - 7.60 (m, 2 H); 7.34 - 7.38 (m, 1 H); 4.64 (q, 2 H); 1.49 (t, 3 H).
MS (ESI): [M+H]⁺ = 444.

The following example compounds 11.2 to 11.4 were prepared from Intermediate 3.6 by reaction with the respective isocyanates according to GP 5 in analogy to example compound 11.1.

| **Example** | **Structure** | **Name** | **Analytical data** |
|---|---|---|---|
| **11.2** | 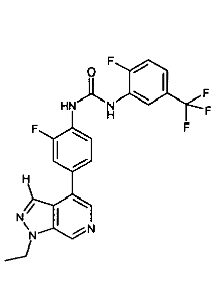 | 1-[4-(1-Ethyl-1H-pyrazolo[3,4-c]pyridin-4-yl)-2-fluoro-phenyl]-3-(2-fluoro-5-trifluoromethyl-phenyl)-urea | ¹H-NMR: (DMSO, 300 MHz) 9.47 (s, 1 H); 9.37 (s, 1 H); 9.22 (s, 1 H); 8.68 (dd, 1 H); 8.44 (s, 1 H); 8.41 (s, 1 H); 8.40 (t, 1 H); 7.75 (dd, 1 H); 7.66 (dd, 1 H); 7.54 (dd, 1 H); 7.40 - 7.46 (m, 1 H); 4.64 (q, 2 H); 1.48 (t, 3 H). MS (ESI): [M+H]⁺ = 462. |
| **11.3** | 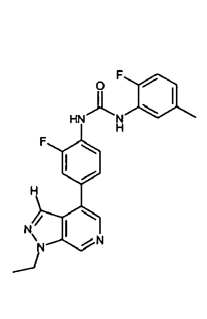 | 1-[4-(1-Ethyl-1H-pyrazolo[3,4-c]pyridin-4-yl)-2-fluoro-phenyl]-3-(2-fluoro-5-methyl-phenyl)-urea | ¹H-NMR: (DMSO, 300 MHz) 9.25 (s, 1 H); 9.21 (s, 1 H); 9.08 (s, 1 H); 8.43 (s, 1 H); 8.42 (s, 1 H); 8.40 (d, 1 H); 8.04 (dd, 1 H); 7.74 (dd, 1 H); 7.65 (dd, 1 H); 7.14 (dd, 1 H); 6.81 - 6.86 (m, 1 H); 4.64 (q, 2 H); 2.30 (s, 3 H); 1.49 (t, 3 H). MS (ESI): [M+H]⁺ = 408. |
| **11.4** | 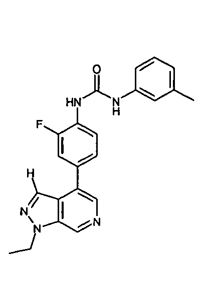 | 1-[4-(1-Ethyl-1H-pyrazolo[3,4-c]pyridin-4-yl)-2-fluoro-phenyl]-3-m-tolyl-urea | ¹H-NMR: (DMSO, 400 MHz) 9.16 (s, 1 H); 9.04 (s, 1 H); 8.70 (s, 1 H); 8.39 (s, 1 H); 8.37 (s, 1 H); 8.33 (d, 1 H); 7.68 (dd, 1 H); 7.60 (dd, 1 H); 7.28 (s, 1 H); 7.23 (d, 1 H); 7.15 (t, 1 H); 6.79 (d, 1 H); 4.59 (q, 2 H); 2.26 (s, 3 H); 1.44 (t, 3 H). MS (ESI): [M+H]⁺ = 390. |

### Example Compound 12.1

### Preparation of 1-{4-[1-(2-Methoxy-ethyl)-1H-pyrazolo[3,4-c]pyridin-4-yl]-phenyl}-3-(3-trifluoromethyl-phenyl)-urea

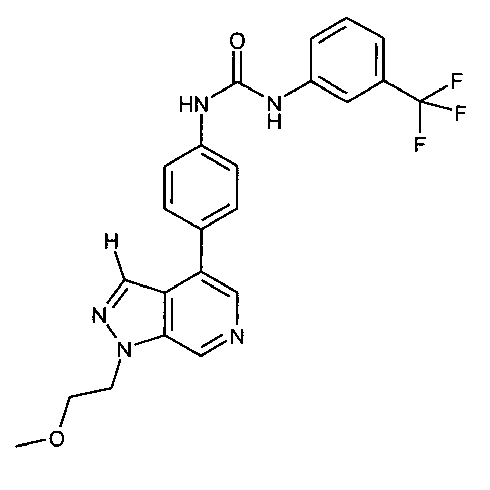

In analogy to GP 5, 85 mg of 4-[1-(2-methoxy-ethyl)-1H-pyrazolo[3,4-c]pyridin-4-yl]-phenylamine (Intermediate 3.7; 0.32 mmol, 1 eq.) were dissolved in 3 mL DCM and treated with 49 µL 1-isocyanato-3-trifluoromethyl-benzene (0.35 mmol, 1.1 eq.). The reaction mixture was stirred at rt overnight upon which it was concentrated in vacuo, the residue was partitioned between ethyl acetate and water and the aqueous phase was re-extracted with ethyl acetate several times. The combined organic layers were dried and concentrated in vacuo. Flash column chromatography followed by trituration provided 64 mg of the analytically pure target compound (0.140 mmol, 44 % yield).
¹H-NMR (d₆-DMSO; 00 MHz): 9.10 (br. s, 2 H); 9.00 (br. s, 1 H); 8.34 (s, 1 H); 8.33 (s, 1 H); 8.01 (s, 1 H); 7.73 (d, 2 H); 7.64 (d, 2 H); 7.58 (d, 1 H); 7.49 (t, 1 H); 7.29 (d, 1 H); 4.72 (t, 2 H); 3.77 (t, 2 H); 3.17 (s, 3 H);
MS (ESI): [M+H]⁺ = 456.

The following example compounds **12.2** to **12.5** were prepared from Intermediates 3.7 or 3.8, respectively, by reaction with the respective isocyanates according to GP 5 in analogy to example compound 12.1.

| | | | |
|---|---|---|---|
| **Example** | **Structure** | **Name** | **Analytical data** |
| **12.2** | 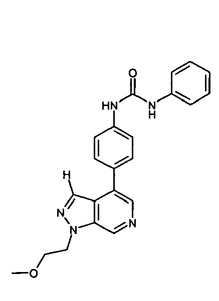 | 1-{4-[1-(2-Methoxy-ethyl)-1H-pyrazolo[3,4-c]pyridin-4-yl]-phenyl}-3-phenyl-urea | ¹H-NMR: (DMSO, 300 MHz) 9.09 (s, 1 H); 8.87 (s, 1 H); 8.71 (s, 1 H); 8.33 (s, 2 H); 7.72 (d, 2 H); 7.62 (d, 2 H); 7.45 (d, 2 H); 7.26 (t, 2 H); 6.95 (t, 1 H); 4.72 (t, 2 H); 6.95 (t, 1 H); 4.72 (t, 2 H); 3.77 (t, 2 H); 3.17 (s, 3 H). |
| **12.3** | 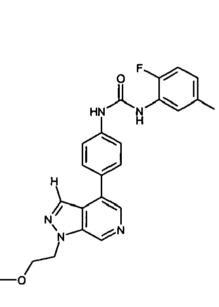 | 1-(2-Fluoro-5-methyl-phenyl)-3-{4-[1-(2-methoxy-ethyl)-1H-pyrazolo[3,4-c]pyridin-4-yl]-phenyl}-urea | ¹H-NMR: (DMSO, 300 MHz) 9.23 (s, 1 H); 9.10 (s, 1 H); 8.51 (d, 1 H); 8.33 (s, 2 H); 7.97 (dd, 1 H); 7.73 (d, 2 H); 7.63 (d, 2 H); 7.08 (dd, 1 H); 6.75 - 6.81 (m, 1 H); 4.72 (t, 2 H); 3.77 (t, 2 H); 3.17 (s, 3 H). MS (ESI): [M+H]⁺ = 420. |
| **12.4** | 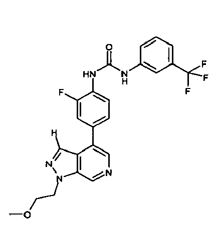 | 1-{2-Fluoro-4-[1-(2-methoxy-ethyl)-1 H-pyrazolo[3,4-c]pyridin-4-yl]-phenyl}-3-(3-trifluoromethyl-phenyl)-urea | ¹H-NMR: (DMSO, 300 MHz) 9.50 (s, 1 H); 9.18 (s, 1 H); 8.86 (d, 1 H); 8.42 (s, 2 H); 8.36 (t, 1 H); 8.01 (s, 1 H); 7.75 (dd, 1 H); 7.66 (dd, 1 H); 7.53 - 7.61 (m, 2 H); 7.37 (d, 1 H); 4.78 (t, 2 H); 3.82 (t, 2 H); 3.22 (s, 3 H). |
| **12.5** | 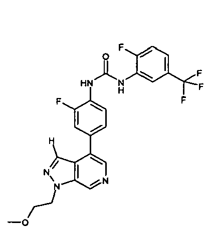 | 1-{2-Fluoro-4-[1-(2-methoxy-ethyl)-1H-pyrazolo[3,4-c]pyridin-4-yl]-phenyl}-3-(2-fluoro-5-trifluoromethyl-phenyl)-urea | ¹H-NMR: (DMSO, 300 MHz) 9.42 (d, 1 H); 9.32 (d, 1 H); 9.14 (s, 1 H); 8.64 (dd, 1 H); 8.38 (s, 2 H); 8.36 (t, 1 H); 7.71 (dd, 1 H); 7.62 (dd, 1 H); 7.49 (dd, 1 H); 7.35 - 7.42 (m, 1 H); 4.73 (t, 2 H); 3.77 (t, 2 H); 3.17 (s, 3 H). |

### Example Compound 13.1

### Preparation of 1-[4-(1-Methyl-1H-pyrazolo[3,4-c]pyridin-4-yl)-phenyl]-3-(2-methyl-pyridin-4-yl)-urea

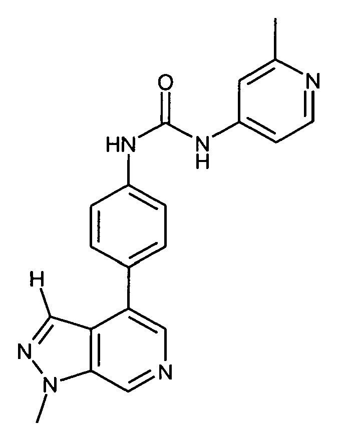

In analogy to GP 10, 101 mg of [4-(1-Methyl-1H-pyrazolo[3,4-c]pyridin-4-yl)-phenyl]-carbamic acid isopropenyl ester (Intermediate 4.1; 0.33 mmol, 1 eq.) were dissolved in 2.2 mL THF and treated with 7 µL N-methyl pyrrolidine (0.065 mmol, 0.2 eq.) and 35 mg 2-methyl-pyridin-4-ylamine (0.33 mmol, 1 eq.). The resultung reaction mixture was refluxed for 7h, concentrated in vacuo and purified by preparative HPLC yielding 50 mg of the target compound (0.14 mmol, 43 % yield).
¹H-NMR (d₆-DMSO; 300 MHz): 9.09 (s, 1 H); 9.06 (s, 1 H); 8.36 (s, 1 H); 8.31 (s, 1 H); 8.20 (d, 1 H); 7.74 (d, 2 H); 7.63 (d, 2 H); 7.29 (s, 1 ); 7.23 (d, 1 H); 4.18 (s, 3 H); 2.37 (s, 3 H).
MS (LC-MS): [M+H]⁺ = 359.

The following example compounds **13.2** to **13.22** were prepared from Intermediate 4.1 or Intermediate 4.2, respectively, by reaction with the respective aniline in analogy to example compound 13.1 and GP 10.

| **Example** | **Structure** | **Name** | **Analytical data** |
|---|---|---|---|
| **13.2** | 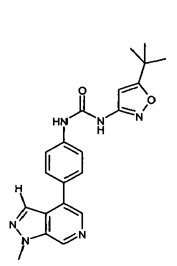 | 1-(5-tert-Butyl- isoxazol-3-yl)-3-[4-(1-methyl-1 H-pyrazolo[3,4-c]pyridin-4-yl)-phenyl]-urea | ¹H-NMR: (DMSO, 300 MHz) 9.53 (s, 1 H); 9.09 (s, 1 H); 8.98 (s, 1 H); 8.35 (s, 1 H); 8.31 (s, 1 H); 7.74 (d, 2 H); 7.61 (d, 2 H); 6.49 (s, 1 H); 4.18 (s, 3 H); 1.27 (s, 9 H). MS (LC-MS): [M+H]⁺ = 391. |
| **13.3** | 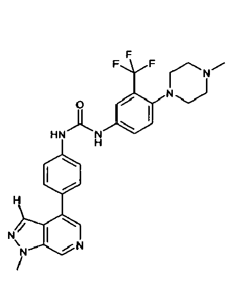 | 1-[4-(4-Methyl-piperazin-1-yl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.09 (s, 1 H); 8.98 (s, 1 H); 8.94 (s, 1 H); 8.35 (s, 1 H); 8.30 (s, 1 H); 7.89 (d, 1 H); 7.72 (d, 2 H); 7.62 (d, 2 H); 7.58 (dd, 1 H); 7.48 (d, 1 H); 4.18 (s, 3 H); 2.75 - 2.82 (m, 4 H); 2.35 - 2.44 (m, 4 H); 2.20 (s, 3 H). MS (LC-MS): [M+H]⁺ = 510. |
| **13.4** | 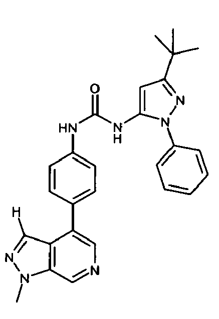 | 1-(5-tert-Butyl-2-phenyl-2H-pyrazol-3-yl)-3-[4-(1-methyl-1H-pyrazolo[3,4-c] pyridin-4-yl)-phenyl]-urea | ¹H-NMR: (DMSO, 300 MHz) 9.22 (s, 1 H); 9.08 (s, 1 H); 8.44 (s, 1 H); 8.34 (s, 1 H); 8.29 (s, 1 H); 7.70 (d, 2 H); 7.57 (d, 2 H); 7.49 - 7.53 (m, 4 H); 7.35 - 7.43 (m, 1 H); 6.37 (s, 1 H); 4.18 (s, 3 H); 1.26 (s, 9 H). MS (LC-MS): [M+H]⁺ = 466. |
| **13.5** | 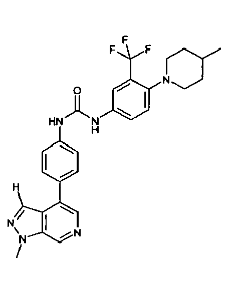 | 1-[4-(4-Methyl-piperidin-1-yl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.09 (s, 1 H); 8.95 (s, 1 H); 8.94 (s, 1 H); 8.35 (s, 1 H); 8.31 (s, 1 H); 7.86 (d, 1 H); 7.72 (d, 2 H); 7.62 (d, 2 H); 7.57 (dd, 1 H); 7.44 (d, 1 H); 4.18 (s, 3 H); 2.78 - 2.85 (m, 2 H); 2.62 - 2.70 (m, 2 H); 1.59 - 1.63 (m, 2 H); 1.35 -1.50 (m, 1 H); 1.15 - 1.29 (m, 2 H); 0.92 (d, 3 H). MS (LC-MS): [M+H]⁺ = 509. |
| **13.6** | 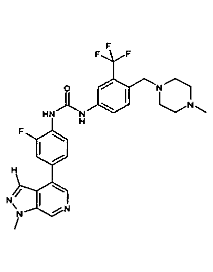 | 1-[2-Fluoro-4-(1-methyl-1H-pyrazolo[3,4-c]pyridin-4-yl)-phenyl]-3-[4-(4-methyl-piperazin-1-ylmethyl)-3-trifluoromethyl-phenyl]-urea | ¹H-NMR: (DMSO, 400 MHz) 9.47 (s, 1 H); 9.13 (s, 1 H); 8.82 (s, 1 H); 8.39 (s, 1 H); 8.35 (s, 1 H); 8.31 (t, 1 H); 7.97 (d, 1 H); 7.69 (dd, 1 H); 7.59 - 7.63 (m, 2 H); 7.53 (dd, 1 H); 4.19 (s, 3 H); 3.50 (s, 2 H); 2.20 - 2.42 (m, 8 H); 2.12 (s, 3 H). MS (LC-MS): [M+H]⁺ = 542. |
| **13.7** | 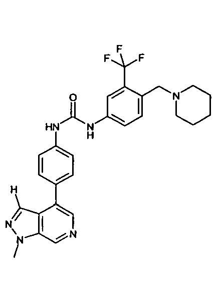 | 1-[4-(1-Methyl-1H-pyrazolo[3,4-c]pyridin-4-yl)-phenyl]-3-(4-piperidin-1-ylmethyl-3-trifluoromethyl-phenyl)-urea | ¹H-NMR: (DMSO, 300 MHz) 9.13 (s, 1 H); 9.12 (s, 1 H); 9.05 (s, 1 H); 8.40 (s, 1 H); 8.35 (s, 1 H); 8.00 (d, 1 H); 7.78 (d, 2 H); 7.59 - 7.69 (m, 4 H); 4.23 (s, 3 H); 3.51 (s, 2 H); 2.31 - 2.39 (m, 4 H); 1.47 - 1.57 (m, 4 H); 1.37 - 1.46 (m, 2 H). MS (LC-MS): [M+H]⁺ = 509. |
| **13.8** | 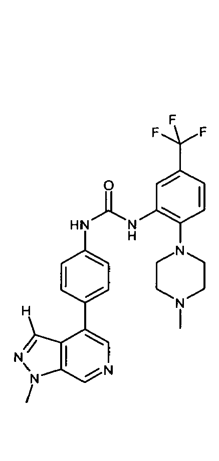 | 1-[2-(4-Methyl-piperazin-1-yl)-5-trifluoromethyl-phenyl]-3-[4-(1-methyl-1H-pyrazolo[3,4-c]pyridin-4-yl)-phenyl]-urea | ¹H-NMR: (DMSO, 300 MHz) 9.90 (s, 1 H); 9.14 (s, 1 H); 8.45 (s, 1 H); 8.41 (s, 1 H); 8.36 (s, 1 H); 8.20 (s, 1 H); 7.80 (d, 2 H); 7.72 (d, 2 H); 7.31 - 7.38 (m, 2 H); 4.24 (s, 3 H); 2.89 - 2.92 (m, 4 H); 2.62 - 2.67 (m, 4 H); 2.31 (s, 3 H). MS (LC-MS): [M+H]⁺ = 510. |
| **13.9** | 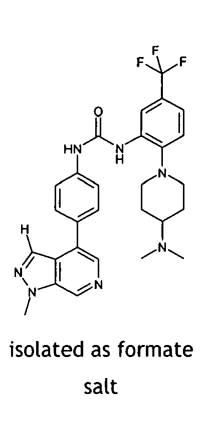 | 1-[2-(4-Dimethylamino-piperidin-1-yl)-5-trifluoromethyl-phenyl]-3-[4-(1-methyl-1H-pyrazolo[3,4-c]pyridin-4-yl)-phenyl]-urea | ¹H-NMR: (DMSO, 300 MHz) 10.04 (s, 1 H); 9.14 (s, 1 H); 8.47 (s, 1 H); 8.41 (s, 1 H); 8.36 (s, 1 H); 8.29 (s, 1 H); 7.71 - 7.81 (m, 4 H); 7.30 - 7.37 (m, 2 H); 4.24 (s, 3 H); 3.10 - 3.14 (m, 2 H); 2.64 - 2.72 (m, 2 H); 2.33 - 2.45 (m, 1 H); 2.37 (s, 6 H); 1.82 - 2.00 (m, 4 H). MS (LC-MS): [M+H]⁺ = 538. |
| **13.10** | 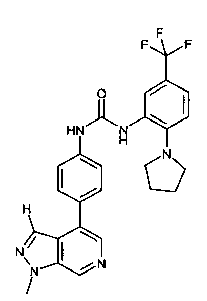 | 1-[4-(1-Methyl-1H-pyrazolo[3,4-c]pyridin-4-yl)-phenyl]-3-(2-pyrrolidin-1-yl-5-trifluoromethyl-phenyl)-urea | ¹H-NMR: (DMSO, 300 MHz) 9.34 (s, 1 H); 9.09 (s, 1 H); 8.35 (s, 1 H); 8.31 (s, 1 H); 8.03 (s, 1 H); 7.93 (d, 1 H); 7.72 (d, 2 H); 7.64 (d, 2 H); 7.28 (dd, 1 H); 7.05 (d, 1 H); 4.19 (s, 3 H); 3.19 - 3.23 (m, 4 H); 1.88 - 1.93 (m, 4 H). |
| **13.11** | 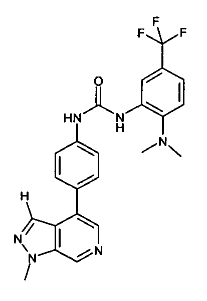 | 1-(2-Dimethylamino-5-trifluoromethyl-phenyl)-3-[4-(1-methyl-1H-pyrazolo[3,4-c]pyridin-4-yl)-phenyl]-urea | ¹H-NMR: (DMSO, 300 MHz) 9.74 (s, 1 H); 9.09 (s, 1 H); 8.53 (s, 1 H); 8.50 (d, 1 H); 8.36 (s, 1 H); 8.31 (s, 1 H); 7.74 (d, 2 H); 7.66 (d, 2 H); 7.32 (d, 1 H); 7.27 (dd, 1 H); 4.19 (s, 3 H); 2.66 (s, 6 H). |
| **13.12** | 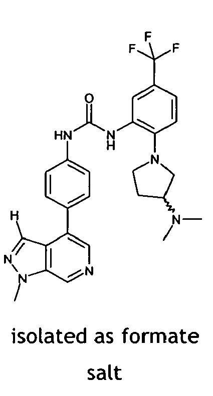 | 1-[2-(3-Dimethylamino-pyrrolidin-1-yl)-5-trifluoromethyl-phenyl]-3-[4-(1-methyl-1H-pyrazolo[3,4-c]pyridin-4-yl)-phenyl]-urea | ¹H-NMR: (DMSO, 400 MHz) 9.40 (s, 1 H); 9.12 (s, 1 H); 8.39 (s, 1 H); 8.34 (s, 1 H); 8.16 (HCO₂H signal); 8.06 (s, 1 H); 7.92 (d, 1 H); 7.75 (d, 2 H); 7.68 (d, 2 H); 7.32 (dd, 1 H); 7.07 (d, 1 H); 4.22 (s, 3 H); 3.18 - 3.47 (m, 4 H); 2.76 - 2.84 (m, 1 H); 2.10 - 2.19 (m, 1 H); 1.76 - 1.85 (m, 1 H). MS (ESI-MS): [M+H]⁺ = 524. |
| **13.13** | 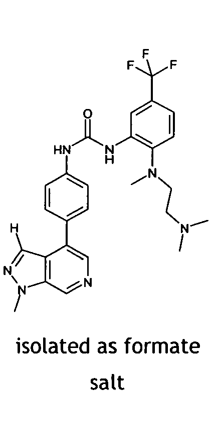 | 1-{2-[(2-Dimethylamino-ethyl)-methyl-amino]-5-trifluoromethyl-phenyl}-3-[4-(1-methyl-1H-pyrazolo[3,4-c]pyridin-4-yl)-phenyl]-urea | ¹H-NMR: (DMSO, 400 MHz) 10.07 (s, 1 H); 9.12 (s, 1 H); 9.03 (s, 1 H); 8.53 (d, 1 H); 8.40 (s, 1 H); 8.35 (HCO₂H signal); 8.31 (s, 1 H); 7.73 - 7.78 (m, 4 H); 7.41 (d, 1 H); 7.31 (dd, 1 H); 4.23 (s, 3 H); 3.11 (t,2H);2.70(t,2H); 2.68 (s, 3 H); 2.35 (s, 6 H). MS (ESI-MS): [M+H]⁺ = 512. |
| **13.14** | 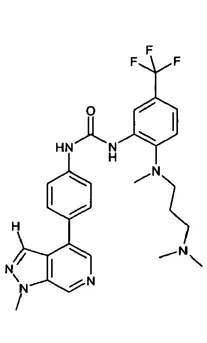 | 1-{2-[(3-Dimethylamino-propyl)-methyl-amino]-5-trifluoromethyl-phenyl}-3-[4-(1-methyl-1H-pyrazolo[3,4-c]pyridin-4-yl)-phenyl]-urea | ¹H-NMR: (DMSO, 400 MHz) 9.82 (s, 1 H); 9.10 (s, 1 H); 8.53 (s, 1 H); 8.50 (d, 1 H); 8.36 (s, 1 H); 8.33 (s, 1 H); 7.75 (d, 2 H); 7.66 (d, 2 H); 7.35 (d, 1 H); 7.27 (dd, 1 H); 4.19 (s, 3 H); 2.91 (t, 2 H); 2.64 (s, 3 H); 2.16 (t, 2 H); 2.02 (s, 6 H); 1.53 (quint., 2 H). MS (LC-MS): [M+H]⁺ = 526. |
| **13.15** | 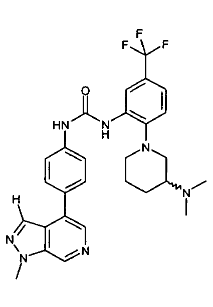 | 1-[2-(3-Dimethylamino-piperidin-1-yl)-5-trifluoromethyl-phenyl]-3-[4-(1-methyl-1H-pyrazolo[3,4-c] pyridin-4-yl)-phenyl]-urea | MS (ESI-MS): [M+H]⁺ = 538. |
| **13.16** | 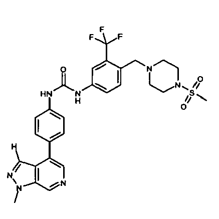 | 1-[4-(4-Methanesulfonyl-piperazin-1-ylmethyl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.12 (s, 1 H); 9.10 (s, 1 H); 8.95 (s, 1 H); 8.36 (s, 1 H); 8.31 (s, 1 H); 7.95 (s, 1 H); 7.73 (d, 2 H); 7.55 - 7.65 (m, 4 H); 4.19 (s, 3 H); 3.55 (s, 2 H); 3.05 - 3.15 (m, 4 H); 2.85 (s, 3 H); 2.40 - 2.50 (overlap with DMSO signal; 4 H). MS (LC-MS): [M+H]⁺ = 588. |
| **13.17** | 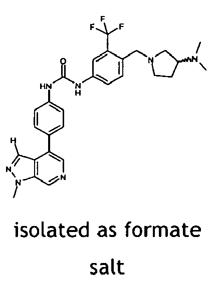 | 1-[4-(3-Dimethylamino-pyrrolidin-1-ylmethyl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.58 (br. s, 1 H); 9.50 (br. s, 1 H); 9.09 (s, 1 H); 8.36 (s, 1 H); 8.31 (s, 1 H); 8.25 (br. s, HCO₂H signal); 7.96 (d, 1 H); 7.72 (d, 2 H); 7.64 (d, 2 H); 7.61 (dd, 1 H); 7.57 (d, 1 H); 4.19 (s, 3 H); 3.61 (higher order q, 2 H); 2.87 - 2.93 (m, 1 H); 2.61 (dd, 1 H); 2.45 - 2.55 (overlap with DMSO signal; 2 H);2.38 (dd, 1 H); 2.17 (s, 6 H); 1.83 - 1.91 (m, 1 H); 1.60 - 1.69 (m, 1 H). MS (LC-MS): [M+H]⁺ = 538. |
| **13.18** | 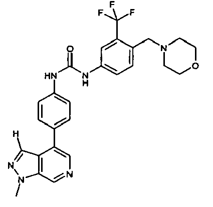 | 1-[4-(1-Methyl-1H-pyrazolo[3,4-c]pyridin-4-yl)-phenyl]-3-(4-morpholin-4-ylmethyl-3-trifluoromethyl-phenyl)-urea | ¹H-NMR: (DMSO, 400 MHz) 9.09 (br. s, 2 H); 9.01 (s, 1 H); 8.36 (s, 1 H); 8.31 (s, 1 H); 7.97 (d, 1 H); 7.73 (d, 2 H); 7.64 (d, 2 H); 7.62 (d, 1 H); 7.58 (dd, 1 H); 4.19 (s, 3 H); 3.55 (t, 4 H); 3.51 (s, 2 H); 2.32 - 2.36 (m, 4 H). MS (LC-MS): [M+H]⁺ = 511. |
| **13.19** | 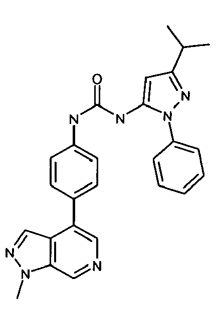 | 1-(5-Isopropyl-2-phenyl-2H-pyrazol-3-yl)-3-[4-(1-methyl-1H-pyrazolo[3,4-c]pyridin-4-yl)-phenyl]-urea | ¹H-NMR: (DMSO, 300 MHz) 9.21 (s, 1 H); 9.08 (s, 1 H); 8.46 (s, 1 H); 8.33 (s, 1 H); 8.28 (s, 1 H); 7.70 (d, 2 H); 7.57 (d, 2 H); 7.49 - 7.53 (m, 4 H); 7.33 - 7.43 (m, 1 H); 6.30 (s, 1 H); 4.18 (s, 3 H); 2.88 (sept, 1 H); 1.18 (d, 6 h). MS (LC-MS): [M+H]⁺ = 452. |
| **13.20** | 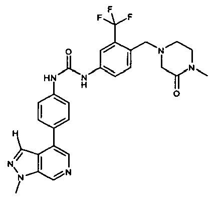 | 1-[4-(4-Methyl-3-oxo-piperazin-1-ylmethyl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.12 (s, 1 H); 9.08 (br. s, 1 H); 8.37 (br. s, 1 H); 8.31 (s, 1 H); 7.97 (s, 1 H); 7.73 (d, 2 H); 7.64 (d, 2 H); 7.57 - 7.62 (m, 2 H); 4.19 (s, 3 H); 3.58 (m, 2 H); 4.19 (s, 3 H); 3.58 2 H); 2.79 (s, 3 H); 2.60 (t, 2 H). MS (LC-MS): [M+H]⁺ = 438. |
| **13.21** | 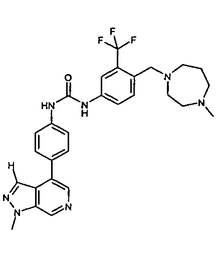 | 1-[4-(4-Methyl-[1,4]diazepan-1-ylmethyl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.79 (br. s, 3 H); 9.70 (br. s, 1 H); 9.08 (s, 1 H); 8.35 (s, 1 H); 8.31 (s, 1 H); 7.97 (d, 1 H); 7.60 - 7.73 (m, 5 H); 4.18 (s, 3 H); 3.65 (s, 2 H); 3.37 - 3.43 (m, 2 H); 2.66 - 2.70 (m, 2 H); 2.58 - 2.64 (m, 4 H); 2.32 (s, 3 H); 1.72 (quint., 2 H). MS (LC-MS): [M+H]⁺ = 538. |
| **13.22** | 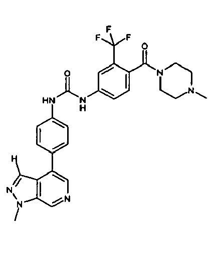 | 1-[4-(4-Methyl-piperazine-1-carbonyl)-3-trifluoromethyl-phenyl]-3-[4-(1-methyl-1H-pyrazolo[3,4-c]pyridin-4-yl)-phenyl]-urea | ¹H-NMR: (DMSO, 300 MHz) 9.34 (br. s, 1 H); 9.18 (br. s, 1 H); 9.14 (s, 1 H); 8.41 (s, 1 H); 8.36 (s, 1 H); 8.08 (d, 1 H); 7.79 (d, 2 H);7.67 - 7.71 (m, 3 H);7.37 (d, 1 H); 4.23 (s, 3 H); 3.55 - 3.71 (m, 2 H); 3.05 - 3.20 (m, 2 H); 2.13 - 2.46 (m, 4 H); 2.20 (s, 3 H). MS (LC-MS): [M+H]⁺ = 538. |

### Example Compound 14.1

### Preparation of 1-(4-Cyano-3-trifluoromethyl-phenyl)-3-[4-(1-methyl-1H-pyrazolo[3,4-c]pyridin-4-yl)-phenyl]-urea

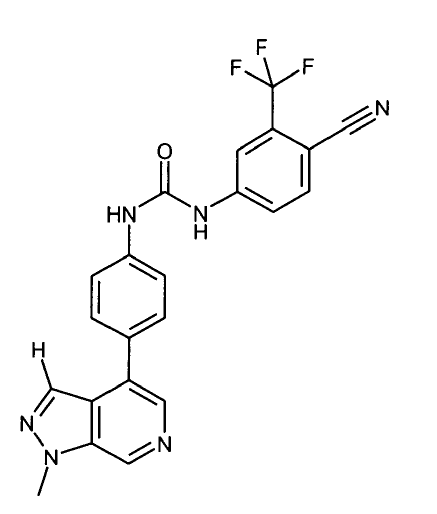

### Step 1

In analogy to GP 9, 500 mg 4-amino-2-trifluoromethyl-benzonitrile (1 eq.) were dissolved in 4.6 mL THF and treated with 0.35 mL N-methyl morpholine (1.2 eq.). The resulting mixture was cooled to 4 °C and treated dropwise with 0.35 mL chloro isopropenyl formate (1.2 eq.) and stirring was continued at rt for 5h. The reaction mixture was quenched with water, extracted with ethyl acetate, the combined organic layers were dried and concentrated in vacuo. Flash column chromatography of the residue yielded 787 mg of a 1:1 mixture of the mono- and bis-isopropenyl carbamate of the starting aniline, which was used in the subsequent transformation without further purification.

### Step 2

In analogy to GP 10, 100 mg of 4-(1-methyl-1H-pyrazolo[3,4-c]pyridin-4-yl)-phenylamine (Intermediate 3.1; 0.45 mmol, 1 eq.) were dissolved in 3 mL THF and treated with 9 µL *N*-methylpyrrolidine (0.09 mmol, 0.2 eq.) and 362 mg of the product mixture of step 1. The reaction mixture was stirred at 55 °C for 5h upon which the reaction mixture was concentrated in vacuo and the residue was purified by preparative HPLC to yield 103 mg of the target compound (0.24 mmol, 53 % yield).
¹H-NMR (d₆-DMSO; 400 MHz): 9.65 (s, 1 H); 9.26 (s, 1 H); 9.10 (s, 1 H); 8.36 (s, 1 H); 8.31 (s, 1 H); 8.20 (d, 1 H); 8.02 (d, 1 H); 7.73 - 7.79 (m, 3 H); 7.65 (d, 2 H); 4.19 (s, 3 H).
MS (ESI): [M+H]⁺ = 437.

The following example compounds **14.2** to **14.6** were prepared from the respective aniline precursors by transformation into their respective isopropenyl carbamates in analogy to GP 9 and subsequent reaction with Intermediate 3.1 or Intermediate 3.2, respectively, in analogy to example compound 14.1 and GP 10.

| **Example** | **Structure** | **Name** | **Analytical data** |
|---|---|---|---|
| **14.2** | 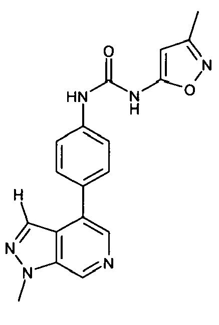 | 1-(3-Methyl-isoxazol-5-yl)-3-[4-(1-methyl-1 H-pyrazolo[3,4- c]pyridin-4-yl)-phenyl]-urea | ¹H-NMR: (DMSO, 300 MHz) 10.12 (br. s, 1 H); 9.10 (s, 1 H); 9.05 (br. s, 1 H); 8.36 (s, 1 H); 8.31 (s, 1 H); 7.75 (d, 2 H); 7.63 (d, 2 H); 5.96 (s, 1 H); 4.18 (s, 3 H); 2.14 (s, 3 H). MS (LC-MS): [M+H]⁺ = 349. |
| **14.3** | 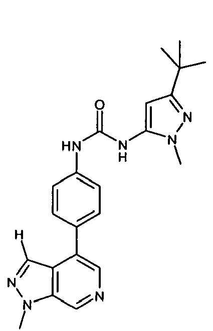 | 1-(5-tert-Butyl-2-methyl-2H-pyrazol-3-yl)-3-[4-(1-methyl-1H-pyrazolo[3,4-c]pyridin-4-yl)-phenyl]-urea | ¹H-NMR: (DMSO, 400 MHz) 9.09 (s, 1 H); 9.07 (s, 1 H); 8.50 (s, 1 H); 8.35 (s, 1 H); 8.31 (s, 1 H); 7.72 (d, 2 H); 7.62 (d, 2 H); 6.05 (s, 1 H); 4.18 (s, 3 H); 3.58 (s, 3 H); 1.19 (s, 9 H). MS (LC-MS): [M+H]⁺ = 404. |
| **14.4** | 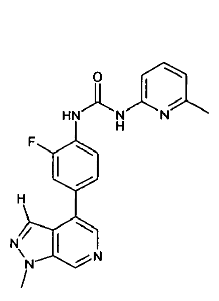 | 1-[2-Fluoro-4-(1-methyl-1H-pyrazolo[3,4-c]pyridin-4-yl)-phenyl]-3-(6-methyl-pyridin-2-yl)-urea | ¹H-NMR: (DMSO, 400 MHz) 12.1 (br. s, 1 H); 9.93 (s, 1 H); 9.12 (s, 1 H); 8.47 (t, 1 H); 8.41 (s, 1 H); 8.37 (s, 1 H); 7.73 (dd, 1 H); 7.62 - 7.67 (m, 2 H); 6.99 (br. d, 1 H); 6.89 (d, 1 H); 4.19 (s, 3 H); 2.45 (s, 3 H). MS (LC-MS): [M+H]⁺ = 377. |
| **14.5** | 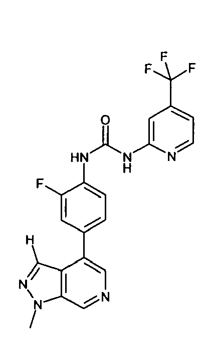 | 1-[2-Fluoro-4-(1-methyl-1H-pyrazolo[3,4-c]pyridin-4-yl)-phenyl]-3-(4-trifluoromethyl-pyridin-2-yl)-urea | ¹H-NMR: (DMSO, 300 MHz) 10.0 - 10.15 (br., 2 H); 9.13 (s, 1 H); 8.53 (dd, 1 H); 8.41 (s, 1 H); 8.36 (s, 1 H); 7.99 (s, 1 H); 7.73 (dd, 1 H); 7.64 (dd, 1 H); 7.36 (d, 1 H); 4.19 (s, 3 H). MS (LC-MS): [M+H]⁺ = 431. |
| **14.6** | 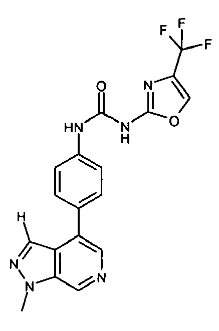 | 1-[4-(1-Methyl-1H-pyrazolo[3,4-c]pyridin-4-yl)-phenyl]-3-(4-trifluoromethyl-oxazol-2-yl)-urea | ¹H-NMR: (DMSO, 400 MHz) 9.71 (s, 1 H); 9.09 (s, 1 H); 8.46 (s, 1 H); 8.36 (s, 1 H); 8.32 (s, 1 H); 7.75 (d, 2 H); 7.66 (d, 2 H); 4.18 (s, 3 H). MS (LC-MS): [M+H]⁺ = 403. |

### Example Compound 15.1

### Preparation of 1-[2-(3-Fluoro-phenyl)-5-isopropyl-2H-pyrazol-3-yl]-3-[4-(1-methyl-1H-pyrazolo[3,4-c]pyridin-4-yl)-phenyl]-urea

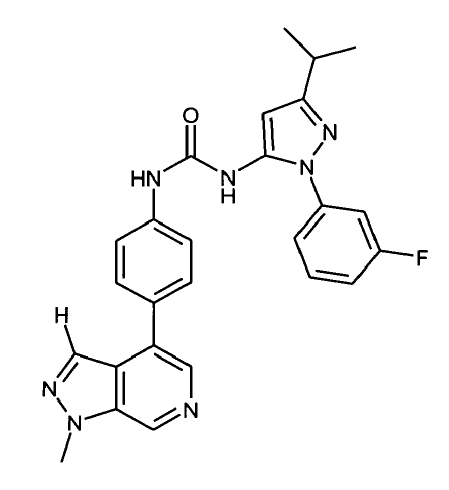

In analogy to GP 8, 70 mg of 4-(1-methyl-1H-pyrazolo[3,4-c]pyridin-4-yl)-phenylamine (Intermediate 3.1; 0.31 mmol, 1 eq.) were dissolved in 3.8 mL THF and treated with 1 mL pyridine (12.49 mmol, 40 eq.) and 106 mg of [2-(3-fluorophenyl)-5-isopropyl-2H-pyrazol-3-yl]-carbamic acid phenyl ester (0.31 mmol, 1 eq.; prepared from the respective amino pyrazole precursor by treatment with phenyl chloroformate in analogy to procedures described in WO2007064872 or WO2005110994). The reaction mixture was heated to 100 °C for 15 min in a Biotage Initiator microwave oven upon which the reaction mixture was concentrated in vacuo and the residue was isolated by trituration to yield 79 mg of the target compound (0.17 mmol, 54 % yield).
¹H-NMR (d₆-DMSO; 300 MHz): 9.28 (br. s, 1 H); 9.13 (s, 1 H); 8.60 (br. s, 1 H); 8.38 (s, 1 H); 8.34 (s, 1 H); 7.75 (d, 2 H); 7.62 (d, 2 H); 7.55 - 7.62 (m, 1 H); 7.43 - 7.48 (m, 2 H); 7.23 - 7.30 (m, 1 H); 6.39 (s, 1 H); 4.23 (s, 3 H); 2.92 (sept., 1 H); 1.26 (d, 6 H).
MS (ESI): [M+H]⁺ = 470.

### Example Compound 15.2

### Preparation of 1-[2-Fluoro-4-(1-methyl-1H-pyrazolo[3,4-c]pyridin-4-yl)-phenyl]-3-[2-(3-fluoro-phenyl)-5-isopropyl-2H-pyrazol-3-yl]-urea

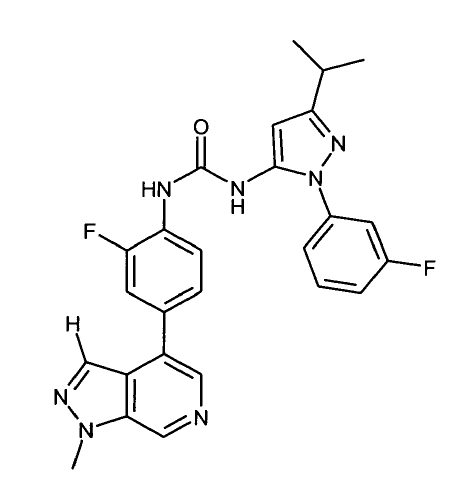

In analogy to GP 8, 76 mg of 2-fluoro-4-(1-methyl-1H-pyrazolo[3,4-c]pyridin-4-yl)-phenylamine (Intermediate 3.2; 0.31 mmol, 1 eq.) were dissolved in 3.8 mL THF and treated with 1 mL pyridine (12.49 mmol, 40 eq.) and 106 mg of [2-(3-fluorophenyl)-5-isopropyl-2H-pyrazol-3-yl]-carbamic acid phenyl ester (0.31 mmol, 1 eq.; prepared from the respective amino pyrazole precursor by treatment with phenyl chloroformate in analogy to procedures described in W02007064872 or W02005110994). The reaction mixture was heated to 100 °C for 15 min in a Biotage Initiator microwave oven upon which the reaction mixture was concentrated in vacuo and the residue was isolated by trituration to yield 77 mg of the target compound (0.16 mmol, 52 % yield).
¹H-NMR (d₆-DMSO; 300 MHz): 9.14 - 9.17 (m, 2 H); 8.99 (m, 1 H); 8.43 (s, 1 H); 8.40 (s, 1 H); 8.31 (t, 1 H); 7.72 (dd, 1 H); 7.57 - 7.65 (m, 2 H); 7.42 - 7.48 (m, 2 H); 7.29 (dt, 1 H); 6.42 (s, 1 H); 4.24 (s, 3 H); 2.92 (sept., 1 H); 1.25 (d, 6 H).
MS (ESI): [M+H]⁺ = 488.

The following example compounds **15.3** to **15.27** were prepared from the respective Intermediates 3.1 or 3.2 by treatment with the respective (hetero)aryl carbamic acid phenyl esters in analogy to example compounds 15.1 and 15.2 and in analogy to GP 8.

| **Example** | **Structure** | **Name** | **Analytical data** |
|---|---|---|---|
| **15.3** | 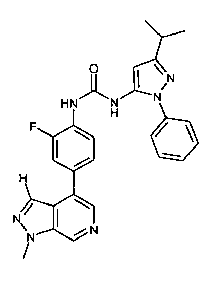 | 1-[2-Fluoro-4-(1-methyl-1H-pyrazolo[3,4-c]pyridin-4-yl)-phenyl]-3-(5-isopropyl-2-phenyl-2H-pyrazol-3-yl)-urea | 1 H-NMR: (DMSO, 300 MHz) 9.12 (s, 1 H); 9.11 (br. s, 1 H); 8.99 (br. s, 1 H); 8.38 (s, 1 H); 8.34 (s, 1 H); 8.29 (t, 1 H); 7.67 (dd, 1 H); 7.59 (dd, 1 H); 7.48 - 7.56 (m, 4 H); 7.37 - 7.45 (m, 1 H); 6.36 (s, 1 H); 4.19 (s, 3 H); 2.86 (sept., 1 H); 1.21 (d, 6 H). MS (LC-MS): [M+H]⁺ = 470. |
| **15.4** | 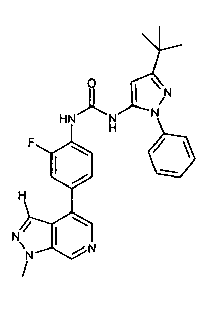 | 1-(5-tert-Butyl-2-phenyl-2H-pyrazol-3-yl)-3-[2-fluoro-4-(1-methyl-1H-pyrazolo[3,4-c]pyridin-4-yl)-phenyl]-urea | 1H-NMR: (DMSO, 300 MHz) 9.12 (s, 1 H); 9.11 (br. s, 1 H); 8.92 (br. s, 1 H); 8.38 (s, 1 H); 8.34 (s, 1 H); 8.30 (t, 1 H); 7.67 (dd, 1 H); 7.59 (dd, 1 H); 7.49 - 7.55 (m, 4 H); 7.38 - 7.43 (m, 1 H); 6.41 (s, 1 H); 4.18 (s, 3 H); 1.26 (s, 9 H). MS (LC-MS): [M+H]⁺ = 484. |
| **15.5** | 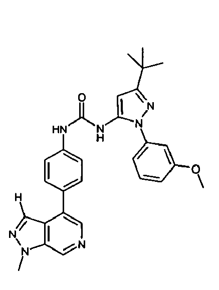 | 1-[5-tert-Butyl-2-(3-methoxy-phenyl)-2H-pyrazol-3-yl]-3-[4-(1-methyl-1H-pyrazolo[3,4-c]pyridin-4-yl)-phenyl]-urea | 1 H-NMR: (DMSO, 300 MHz) 9.33 (br. s, 1 H); 9.13 (s, 1 H); 8.50 (br. s, 1 H); 8.39 (s, 1 H); 8.34 (s, 1 H); 7.75 (d, 2 H); 7.63 (d, 2 H); 7.45 (t, 1 H); 7.10 - 7.15 (m 2 H); 7.00 (dd, 1 H); 6.42 (s, 1 H); 4.23 (s, 3 H); 3.83 (s, 3 H); 1.30 (s, 9 H). MS (MS-ESI): [M+H]⁺ = 496. |
| **15.6** | 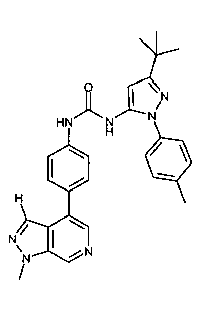 | 1-(5-tert-Butyl-2-p-tolyl-2H-pyrazol-3-yl)-3-[4-(1-methyl-1H-pyrazolo[3,4-c]pyridin-4-yl)-phenyl]-urea | 1 H-NMR: (DMSO, 300 MHz) 9.28 (br. s, 1 H); 9.13 (s, 1 H); 8.45 (br. s, 1 H); 8.38 (s, 1 H); 8.34 (s, 1 H); 7.75 (d, 2 H); 7.62 (d, 2 H); 7.43 (d, 2 H); 7.35 (d, 2 H); 6.39 (s, 1 H); 4.23 (s, 3 H); 2.39 (s, 3 H); 1.29 (s, 9 H). MS (MS-ESI): [M+H]⁺ = 480. |
| **15.7** | 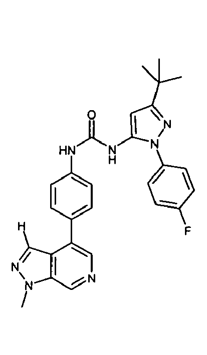 | 1-[5-tert-Butyl-2-(4-fluoro-phenyl)-2H-pyrazol-3-yl]-3-[4-(1-methyl-1 H-pyrazolo[3,4-c]pyridin-4-yl)-phenyl]-urea | 1 H-NMR: (DMSO, 300 MHz) 9.22 (br. s, 1 H); 9.13 (s, 1 H); 8.47 (br. s, 1 H); 8.38 (s, 1 H); 8.34 (s, 1 H); 7.75 (d, 2 H); 7.62 (d, 2 H); 7.57 - 7.62 (m, 2 H); 7.39 (t, 2 H); 6.41 (s, 1 H); 4.23 (s, 3 H); 1.30 (s, 9 H). MS (MS-ESI): [M+H]⁺ = 484. |
| **15.8** | 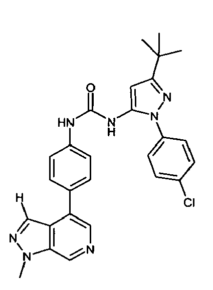 | 1-[5-tert-Butyl-2-(4-chloro-phenyl)-2H-pyrazol-3-yl]-3-[4-(1-methyl-1H-pyrazolo[3,4-c]pyridin-4-yl)-phenyl]-urea | 1 H-NMR: (DMSO, 300 MHz) 9.24 (br. s, 1 H); 9.13 (s, 1 H); 8.52 (br. s, 1 H); 8.39 (s, 1 H); 8.34 (s, 1 H); 7.75 (d, 2 H); 7.62 (d, 2 H); 7.60 (s, 4 H); 6.42 (s, 1 H); 4.23 (s, 3 H); 1.30 (s, 9 H). MS (MS-ESI): [M+H]⁺ = 500. |
| **15.9** | 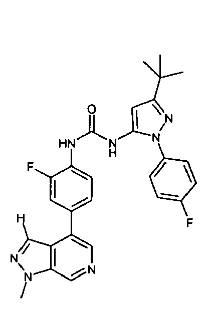 | 1-[5-tert-Butyl-2-(4-fluoro-phenyl)-2H-pyrazol-3-yl]-3-[2-fluoro-4-(1-methyl-1 H-pyrazolo[3,4-c]pyridin-4-yl)-phenyl]-urea | 1 H-NMR: (DMSO, 300 MHz) 9.17 (s, 1 H); 9.10 (br., 1 H); 8.94 (br., 1 H); 8.43 (s, 1 H); 8.39 (s, 1 H); 8.33 (t, 1 H); 7.72 (dd, 1 H); 7.55 - 7.65 (m, 3 H); 7.41 (t, 2 H); 6.44 (s, 1 H); 4.23 (s, 3 H); 1.30 (s, 9 H). MS (MS-ESI): [M+H]⁺ = 502. |
| **15.10** | 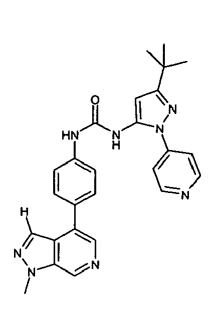 | 1-(5-tert-Butyl-2-pyridin-4-yl-2H-pyrazol-3-yl)-3-[4-(1-methyl-1H-pyrazolo[3,4-c]pyridin-4-yl)-phenyl]-urea | 1 H-NMR: (DMSO, 400 MHz) 9.31 (s, 1 H); 9.09 (s, 1 H); 8.69 (s, 1 H); 8.63 (d, 2 H); 8.34 (s, 1 H); 8.30 (s, 1 H); 7.71 (d, 2 H); 7.64 (d, 2 H); 7.59 (d, 2 H); 6.43 (s, 1 H); 4.18 (s, 3 H); 1.27 (s, 9 H). MS (LC-MS): [M+H]⁺ = 467. |
| **15.11** | 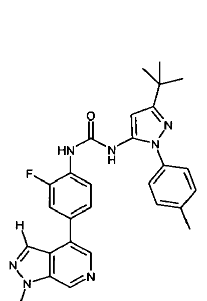 | 1-(5-tert-Butyl-2-p-tolyl-2H-pyrazol-3-yl)-3-[2-fluoro-4-(1-methyl-1H-pyrazolo[3,4-c]pyridin-4-yl)-phenyl]-urea | 1 H-NMR: (DMSO, 300 MHz) 9.16 (s, 1H); 9.15 (br. s, 1 H); 8.90 (br. s, 1 H); 8.43 (s, 1 H); 8.39 (s, 1 H); 8.35 (t, 1 H); 7.71 (dd, 1 H); 7.64 (dd, 1 H); 7.35 - 7.43 (m, 4 H); 6.43 (s, 1 H); 4.23 (s, 3 H); 2.40 (s, 3 H); 1.29(s, 9 H). MS (ESI-MS): [M+H]⁺ = 498. |
| **15.12** | 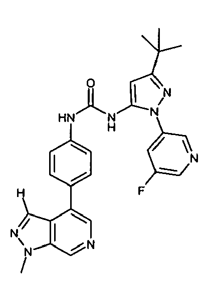 | 1-[5-tert-Butyl-2-(5-fluoro-pyridin-3-yl)-2H-pyrazol-3-yl]-3-[4-(1-methyl-1H-pyrazolo[3,4-c]pyridin-4-yl)-phenyl]-urea | 1 H-NMR: (DMSO, 400 MHz) 9.26 (s, 1 H); 9.09 (s, 1 H); 8.71 (s, 1 H); 8.65 (s, 1 H); 8.59 (d, 1 H); 8.34 (s, 1 H); 8.29 (s, 1 H); 7.98 (dt, 1 H); 7.71 (d, 2 H); 7.57 (d, 2 H); 6.43 (s, 1 H); 4.18 (s, 3 H); 1.27 (s, 9 H). MS (LC-MS): [M+H]⁺ = 485. |
| **15.13** | 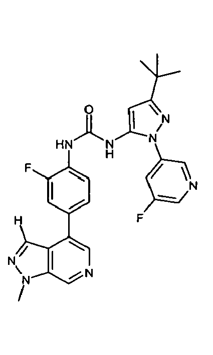 | 1-[5-tert-Butyl-2-(5-fluoro-pyridin-3-yl)-2H-pyrazol-3-yl]-3-[2-fluoro-4-(1-methyl-1H-pyrazolo[3,4-c]pyridin-4-yl)-phenyl]-urea | 1 H-NMR: (DMSO, 400 MHz) 9.13 (s, 1 H); 9.11 (br. s, 1 H); 9.02 (br. s, 1 H); 8.70 (s, 1 H); 8.62 (d, 1 H); 8.38 (s, 1 H); 8.35 (s, 1 H); 8.22 (t, 1 H); 8.00 (dt, 1 H); 7.68 (dd, 1 H); 7.59 (dd, 1 H); 6.46 (s, 1 H); 4.19 (s, 1 H); 1.27 (s, 9 H). MS (LC-MS): [M+H]⁺ = 503. |
| **15.14** | 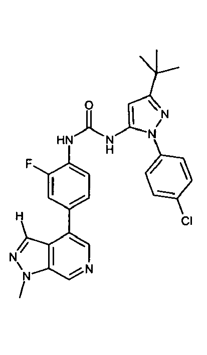 | 1-[5-tert-Butyl-2-(4-chloro-phenyl)-2H-pyrazol-3-yl]-3-[2-fluoro-4-(1-methyl-1 H-pyrazolo[3,4-c]pyridin-4-yl)-phenyl]-urea | 1 H-NMR: (DMSO, 300 MHz) 9.12 (s, 1 H); 9.08 (d, 1 H); 8.91 (s, 1 H); 8.38 (s, 1 H); 8.34 (s, 1 H); 8.27 (t, 1 H); 7.67 (dd, 1 H); 7.52 - 7.61 (m, 5 H); 6.40 (s, 1 H); 4.19 (s, 3 H); 1.25 (s, 9 H). MS (LC-MS): [M+H]⁺ = 518/520 (Cl isotope pattern). |
| **15.15** | 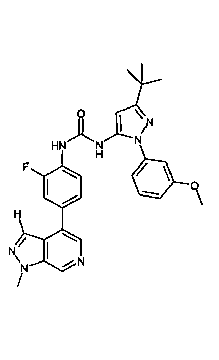 | 1-[5-tert-Butyl-2-(3-methoxy-phenyl)-2H-pyrazol-3-yl]-3-[2-fluoro-4-(1-methyl-1H-pyrazolo[3,4-c]pyridin-4-yl)-phenyl]-urea | 1 H-NMR: (DMSO, 400 MHz) 9.14 (br. s, 1 H); 9.12 (s, 1 H); 8.90 (br. s, 1 H); 8.38 (s, 1 H); 8.35 (s, 1 H); 8.19 (t, 1 H); 7.67 (dd, 1 H); 7.59 (dd, 1 H); 7.43 (t, 1 H); 7.05 - 7.09 (m, 2 H); 6.98 (ddd, 1 H); 6.40 (s, 1 H); 4.19 (s, 3 H); 3.79 (s, 3 H); 1.25 (s, 9 H). MS (MS-ESI): [M+H]⁺ = 514. |
| **15.16** | 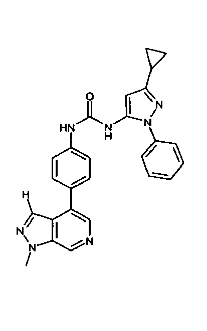 | 1-(5-Cyclopropyl-2-phenyl-2H-pyrazol-3-yl)-3-[4-(1-methyl-1H-pyrazolo[3,4-c]pyridin-4-yl)-phenyl]-urea | 1 H-NMR: (DMSO, 300 MHz) 9.18 (s, 1 H); 9.08 (s, 1 H); 8.44 (s, 1 H); 8.33 (s, 1 H); 8.29 (s, 1 H); 7.77 (d, 2 H); 7.57 (d, 2 H); 7.47 - 7.51 (m, 4 H); 7.35 - 7.42 (m, 1 H);6.17 (s, 1 H); 4.18 (s, 3 H); 1.82 - 1.90 (m, 1 H); 0.83 - 0.89 (m, 2 H); 0.64 - 0.70 (m, 2 H). MS (LC-MS): [M+H]⁺ = 450. |
| **15.17** | 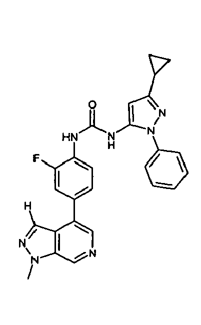 | 1-(5-Cyclopropyl-2-phenyl-2H-pyrazol-3-yl)-3-[2-fluoro-4-(1-methyl-1H-pyrazolo[3,4-c]pyridin-4-yl)-phenyl]-urea | 1 H-NMR: (DMSO, 300 MHz) 9.12 (s, 1 H); 9.10 (br. s, 1 H); 8.92 (br. s, 1 H); 8.38 (s, 1 H); 8.34 (s, 1 H); 8.28 (t, 1 H); 7.66 (dd, 1 H); 7.59 (dd, 1 H); 7.47 - 7.55 (m, 4 H); 7.37 - 7.43 (m, 1 H); 6.20 (s, 1 H); 4.19 (s, 3 H); 1.81 - 1.90 (m, 1 H); 0.83 - 0.89 (m, 2 H); 0.64 - 0.69 (m, 2 H). MS (LC-MS): [M+H]⁺ = 468. |
| **15.18** | 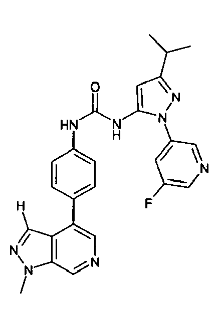 | 1-[2-(5-Fluoro-pyridin-3-yl)-5-isopropyl-2H-pyrazol-3-yl]-3-[4-(1-methyl-1H-pyrazolo[3,4-c]pyridin-4-yl)-phenyl]-urea | MS (LC-MS): [M+H]+ = 471. |
| **15.19** | 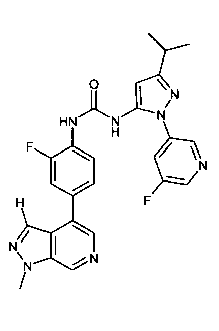 | 1-[2-Fluoro-4-(1-methyl-1 H-pyrazolo[3,4-c]pyridin-4-yl)-phenyl]-3-[2-(5-fluoro-pyridin-3-yl)-5-isopropyl-2H-pyrazol-3-yl]-urea | MS (LC-MS): [M+H]+ = 489. |
| **15.20** | 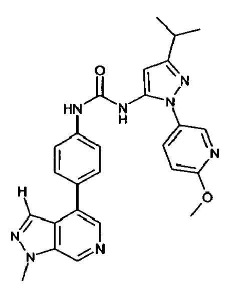 | 1-[5-Isopropyl-2-(6-methoxy-pyridin-3-yl)-2H-pyrazol-3-yl]-3-[4-(1-methyl-1H-pyrazolo[3,4-c]pyridin-4-yl)-phenyl]-urea | MS (LC-MS): [M+H]+ = 483. |
| **15.21** | 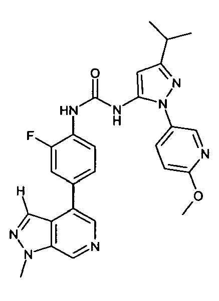 | 1-[2-Fluoro-4-(1-methyl-1H-pyrazolo[3,4-c]pyridin-4-yl)-phenyl]-3-[5-isopropyl-2-(6-methoxy-pyridin-3-yl)-2H-pyrazol-3-yl]-urea | MS (LC-MS): [M+H]+ = 501. |
| **15.22** | 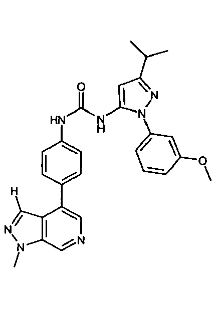 | 1-[5-Isopropyl-2-(3-methoxy-phenyl)-2H-pyrazol-3-yl]-3-[4-(1-methyl-1H-pyrazolo[3,4-c]pyridin-4-yl)-phenyl]-urea | 1 H-NMR: (DMSO, 300 MHz) 9.32 (s, 1 H); 9.13 (s, 1 H); 8.52 (s, 1 H); 8.39 (s, 1 H); 8.34 (s, 1 H); 7.75 (d, 2 H); 7.62 (d, 2 H); 7.45 (t, 1 H); 7.10 - 7.15 (m, 2 H); 7.00 (dd, 1 H); 6.38 (s, 1 H); 4.23 (s, 3 H); 3.82 (s, 3 H); 2.91 (sept, 1 H); 1.25 (d, 6 H). MS (MS-ESI): [M+H]⁺ = 482. |
| **15.23** | 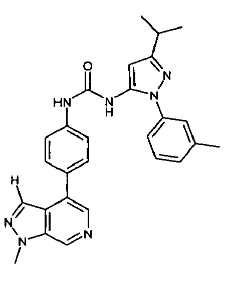 | 1-(5-Isopropyl-2-m-tolyl-2H-pyrazol-3-yl)-3-[4-(1-methyl-1H-pyrazolo[3,4-c]pyridin-4-yl)-phenyl]-urea | 1H-NMR: (DMSO, 300 MHz) 9.29 (s, 1 H); 9.13 (s, 1 H); 8.52 (s, 1 H); 8.38 (s, 1 H); 8.34 (s, 1 H); 7.75 (d, 2 H); 7.62 (d, 2 H); 7.44 (t, 1 H); 7.37 (s, 1 H); 7.34 (d, 1 H); 7.25 (d, 1 H); 6.37 (s, 1 H); 4.23 (s, 3 H); 2.90 (sept, 1 H); 2.40 (s, 3 H); 1.25 (d, 6 H). MS (MS-ESI): [M+H]+ = 466. |
| **15.24** | 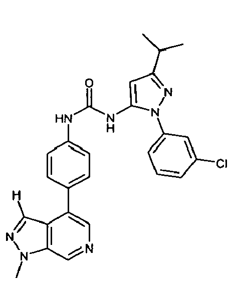 | 1-[2-(3-Chloro-phenyl)-5-isopropyl-2H-pyrazol-3-yl]-3-[4-(1-methyl-1H-pyrazolo[3,4-c]pyridin-4-yl)-phenyl]-urea | 1 H-NMR: (DMSO, 300 MHz) 9.28 (s, 1 H); 9.13 (s, 1 H); 8.60 (s, 1 H); 8.39 (s, 1 H); 8.34 (s, 1 H); 7.75 (d, 2 H); 7.65 (s, 1 H); 7.62 (d, 2 H); 7.56 - 7.58 (m, 2 H); 7.45 - 7.51 (m, 1 H); 6.38 (s, 1 H); 4.23 (s, 3 H); 2.92 (sept, 1 H); 1.26 (d, 6 H). MS (MS-ESI): [M+H]+ = 486. |
| **15.25** | 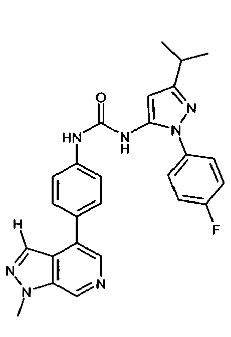 | 1-[2-(4-Fluoro-phenyl)-5-isopropyl-2H-pyrazol-3-yl]-3-[4-(1-methyl-1H-pyrazolo[3,4-c]pyridin-4-yl)-phenyl]-urea | 1 H-NMR: (DMSO, 300 MHz) 9.23 (s, 1 H); 9.13 (s, 1 H); 8.51 (s, 1 H); 8.38 (s, 1 H); 8.34 (s, 1 H); 7.75 (d, 2 H); 7.57 - 7.64 (m, 4 H); 7.39 (t, 2 H); 6.36 (s, 1 H); 4.23 (s, 3 H); 2.91 (sept, 1 H); 1.25 (d, 6 H). MS (MS-ESI): [M+H]+ = 470. |
| **15.26** | 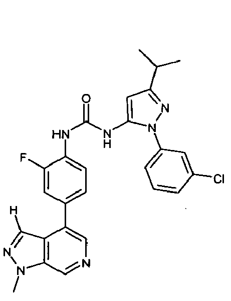 | 1-[2-(3-Chloro-phenyl)-5-isopropyl-2H-pyrazol-3-yl]-3-[2-fluoro-4-(1-methyl-1H-pyrazolo[3,4-c] pyridin-4-yl)-phenyl]-urea | 1 H-NMR: (DMSO, 400 MHz) 9.12 (s, 1 H); 9.10 (d, 1 H); 8.97 (s, 1 H); 8.38 (s, 1 H); 8.35 (s, 1 H); 8.25 (t, 1 H); 7.68 (dd, 1 H); 7.45 - 7.60 (m, 4 H); 6.37 (s, 1 H); 4.19 (s, 3 H); 2.87 (sept, 1 H); 1.21 (d, 6 H). MS (MS-ESI): [M+H]+ = 504/506 (Cl isotope pattern). |
| **15.27** | 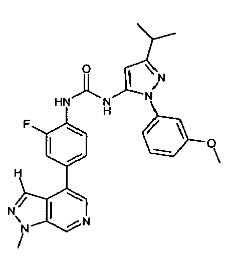 | 1-[2-Fluoro-4-(1-methyl-1H-pyrazolo[3,4-c]pyridin-4-yl)-phenyl]-3-[5-isopropyl-2-(3-methoxy-phenyl)-2H-pyrazol-3-yl]-urea | 1 H-NMR: (DMSO, 400 MHz) 9.15 (d, 1 H); 9.12 (s, 1 H); 8.93 (s, 1 H); 8.38 (s, 1 H); 8.35 (s, 1 H); 8.29 (t, 1 H); 7.67 (dd, 1 H); 7.60 (dd, 1 H); 7.42 (t, 1 H); 7.05 - 7.08 (m, 2 H); 6.97 (m, 2 H); 6.36 (s, 1 H); 4.19 (s, 3 H); 3.79 (s, 3 H); 2.86 (sept, 1 H); 1.21 (d, 6 H). MS (MS-ESI): [M+H]+ = 504. |

### Example Compound 16.1

### Preparation of 1-{4-[1-(2-Methanesulfonyl-ethyl)-1H-pyrazolo[3,4-c]pyridin-4-yl]-phenyl}-3-(3-trifluoromethyl-phenyl)-urea

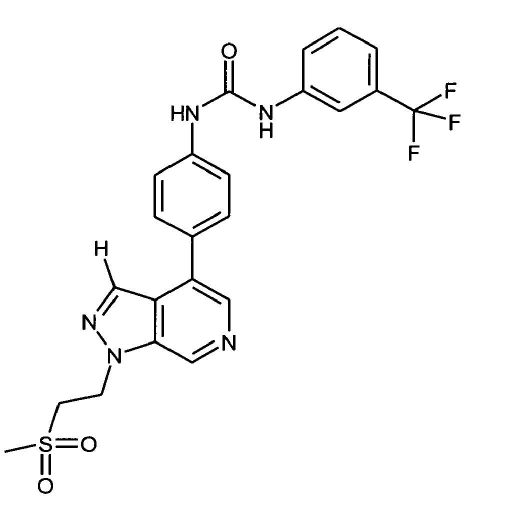

In analogy to GP 3, 66 mg of crude 4-bromo-1-(2-methanesulfonyl-ethyl)-1H-pyrazolo[3,4-c]pyridine (Intermediate 2.7, 0.22 mmol, 1 eq.), 132 mg of 1-[4-(4,4,5,5-tetramethyl-[1,3,2]dioxaborolan-2-yl)-phenyl]-3-(3-trifluoromethylphenyl)-urea (0.33 mmol, 1.5 eq.) and 15 mg Pd(PPh₃)₄ (0.013 mmol, 6 mol%) were weighed into a Biotage microwave vial and capped. 1.0 mL toluene, 1.0 mL EtOH and 1M aq. Na₂CO₃ solution (0.42 mL, 0.42 mmol, 1.9 eq.) were subsequently added by syringe. The resulting mixture was prestirred (10 sec) and subsequently heated to 120 °C for 15 min (fixed hold time) in a Biotage Initiator® microwave reactor. The reaction mixture was diluted with DCM, dried and concentrated in vacuo. Preparative HPLC purification provided 24 mg of the pure target compound along with additional impure fractions.
1 H-NMR (DMSO, 300 MHz): 9.16 (s, 1 H); 9.13 (s, 1 H); 9.05 (s, 1 H); 8.40 (s, 1 H); 8.38 (s, 1 H); 8.00 (s, 1 H); 7.73 (d, 2 H); 7.65 (d, 2 H); 7.58 (d, 1 H); 7.49 (t, 1 H); 7.29 (d, 1 H); 4.98 (t, 2 H); 3.81 (t, 2 H); 2.95 (s, 3 H).
MS (LC-MS): [M+H]⁺ = 504.

### Example Compound 16.2

### Preparation of 1-[2-Fluoro-4-(1-methyl-1H-pyrazolo[3,4-c]pyridin-4-yl)-phenyl]-3-[5-isopropyl-2-(4-methoxy-phenyl)-2H-pyrazol-3-yl]-urea

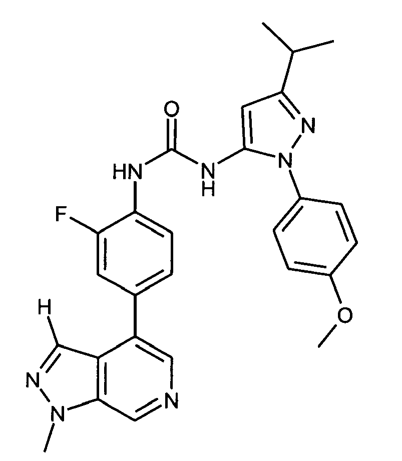

In an adaption of GP 8, 130 mg of [2-fluoro-4-(1-methyl-1H-pyrazolo[3,4-c]pyridin-4-yl)-phenyl]-carbamic acid phenyl ester (Intermediate 5.1; 0.36 mmol, 1.1 eq.) and 69 mg of 5-isopropyl-2-(4-methoxy-phenyl)-2H-pyrazol-3-ylamine (0.3 mmol, 1 eq.) were dissolved in 4 mL THF and treated with 0.97 mL pyridine (12 mmol, 40 eq.). The reaction mixture was heated to 120 °C for 45 min in a Biotage Initiator microwave oven upon which the reaction mixture was concentrated in vacuo and the residue was isolated by trituration to yield 77 mg of the target compound (0.16 mmol, 52 % yield). The reaction mixture was concentrated in vacuo and the target compound was isolated by preparative HPLC purification.
¹H-NMR (d₆-DMSO; 400 MHz): 9.12 (s, 1 H); 9.11 (br. s, 1 H); 8.84 (s, 1 H); 8.38 (s, 1 H); 8.35 (s, 1 H); 8.31 (t, 1 H); 7.67 (dd, 1 H); 7.59 (dd, 1 H); 7.39 (d, 2 H); 7.07 (d, 2 H); 6.32 (s, 1 H); 4.19 (s, 3 H); 3.80 (s, 3 H); 2.84 (sept, 1 H); 1.20 (d, 6 H).
MS (ESI): [M+H]⁺ = 500.

The following example compounds **16.3** to **16.4** were prepared from Intermediate 5.1 by treatment with the respective (hetero)aryl amines in analogy to example compounds 16.2 and in analogy to GP 8.

| **Example** | **Structure** | **Name** | **Analytical data** |
|---|---|---|---|
| **16.3** | 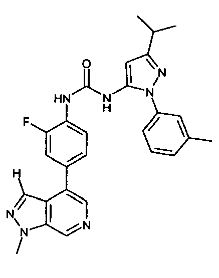 | 1-[2-Fluoro-4-(1-methyl-1H-pyrazolo[3,4-c]pyridin-4-yl)-phenyl]-3-(5-isopropyl-2-m-tolyl-2H-pyrazol-3-yl)-urea | 1 H-NMR: (DMSO, 400 MHz) 9.12 (br. s, 2 H); 8.91 (s, 1 H); 8.38 (s, 1 H); 8.35 (s, 1 H); 8.29 (t, 1 H); 7.67 (dd, 1 H); 7.59 (dd, 2 H); 7.40 (t, 1 H); 7.32 (s, 1 H); 7.30 (d, 1 H); 7.22 (d, 1 H); 6.35 (s, 1 H); 4.19 (s, 3 H); 2.86 (sept., 1 H); 2.36 (s, 3 H); 1.20 (d, 6 H). MS (LC-MS): [M+H]⁺ = 484. |
| **16.4** | 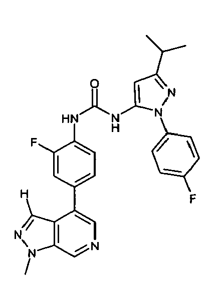 | 1-[2-Fluoro-4-(1-methyl-1H-pyrazolo[3,4-c]pyridin-4-yl)-phenyl]-3-[2-(4-fluoro-phenyl)-5-isopropyl-2H-pyrazol-3-yl]-urea | ¹H-NMR: (d₆-DMSO; 400 MHz) 9.12 (s, 1 H); 9.08 (br. s, 1 H); 8.90 (br. s, 1 H); 8.38 (s, 1 H); 8.35 (s, 1 H); 8.28 (t, 1 H); 7.67 (dd, 1 H); 7.59 (dd, 1 H); 7.51 - 7.57 (m, 2 H); 7.36 (t, 2 H); 6.35 (s, 1 H); 4.19 (s, 3 H); 2.86 (sept, 1 H); 1.20 (d, 6 H). MS (ESI): [M+H]⁺ = 500. |

The following example compounds are accessible in analogy to the general descriptions of this invention and/or the exemplified procedures given above or from example compounds or intermediates by standard transformations known to the person skilled in the art.

| | | |
|---|---|---|
| 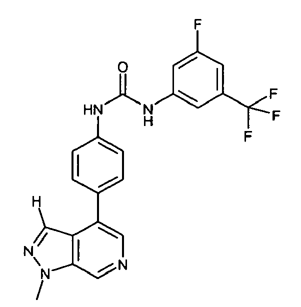 | 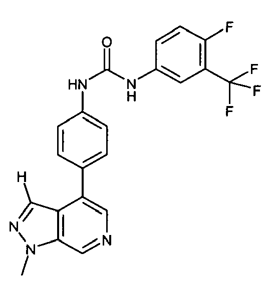 | 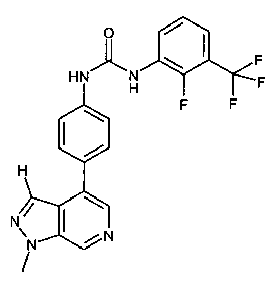 |
| **Example 17.1** | **Example 17.2** | **Example 17.3** |
| 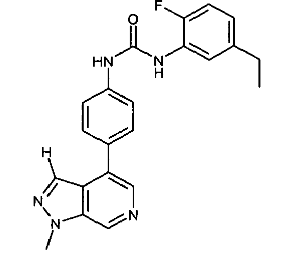 | 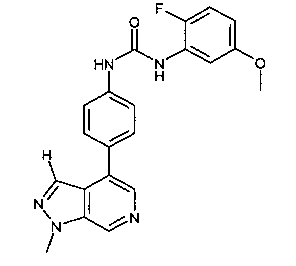 | 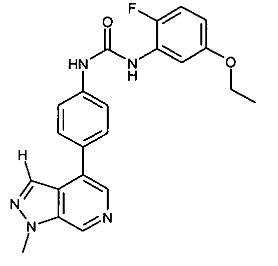 |
| **Example 17.4** | **Example 17.5** | **Example 17.6** |
| 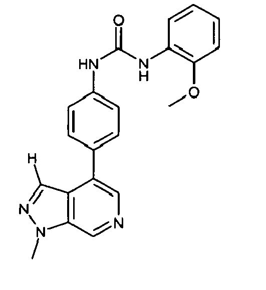 | 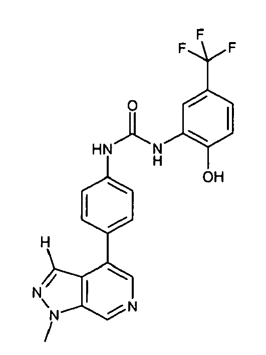 | 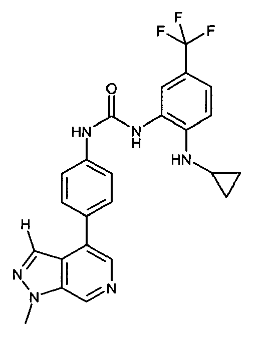 |
| **Example 17.7** | **Example 17.8** | **Example 17.9** |
| 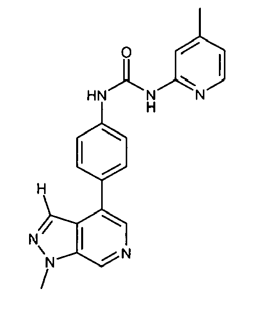 | 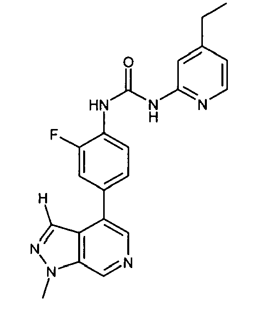 | 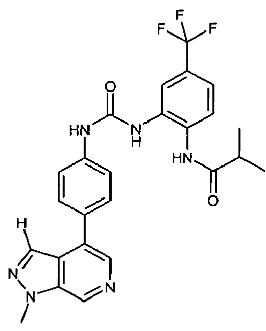 |
| **Example 17.10** | **Example 17.11** | **Example 17.12** |
| 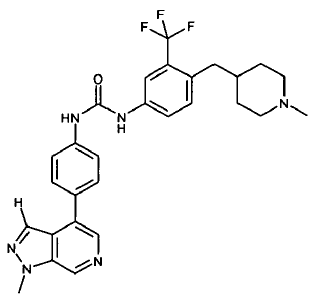 | 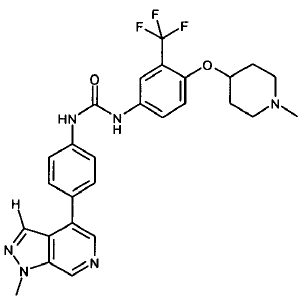 | 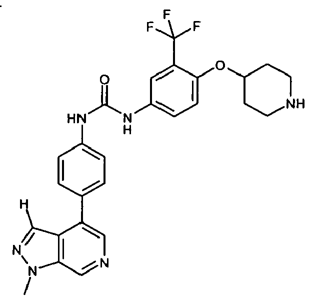 |
| **Example 17.13** | **Example 17.14** | **Example 17.15** |
| 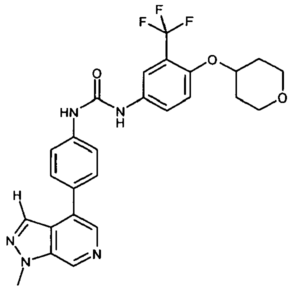 | 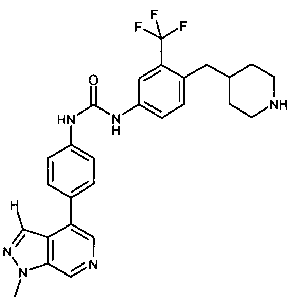 | 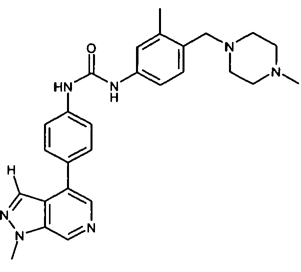 |
| **Example 17.16** | **Example 17.17** | **Example 17.18** |
| 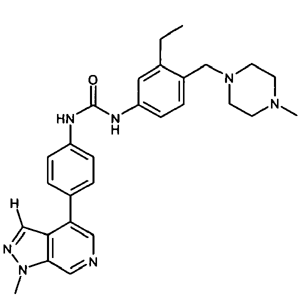 | 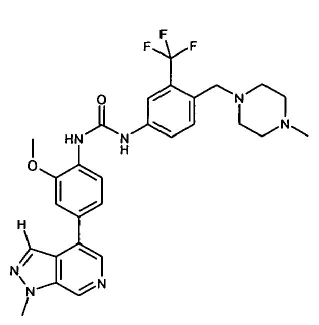 | 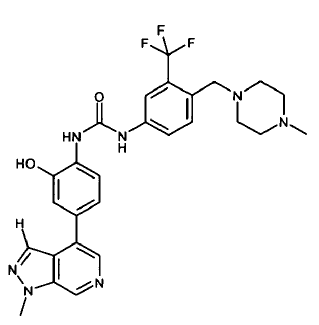 |
| **Example 17.19** | **Example 17.20** | **Example 17.21** |
| 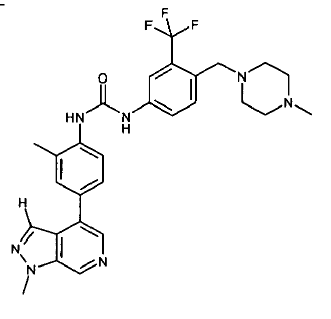 | 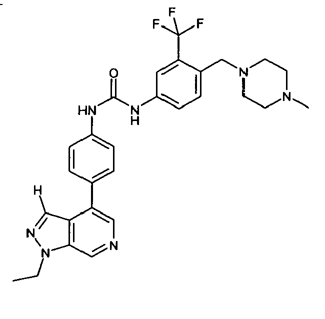 | 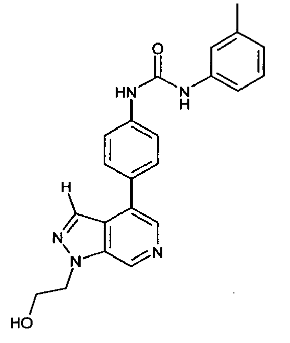 |
| **Example 17.22** | **Example 17.23** | **Example 17.24** |
| 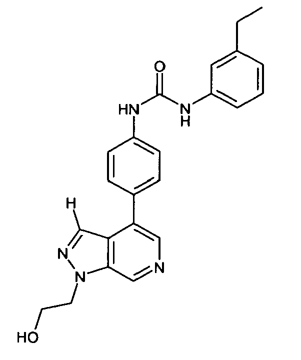 | 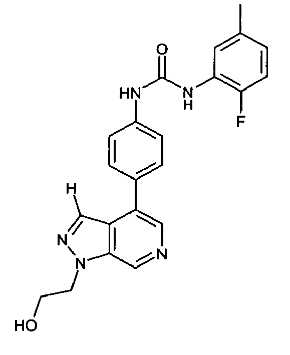 | 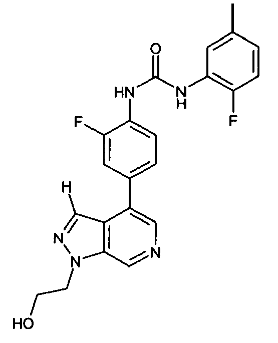 |
| **Example 17.25** | **Example 17.26** | **Example 17.27** |
| 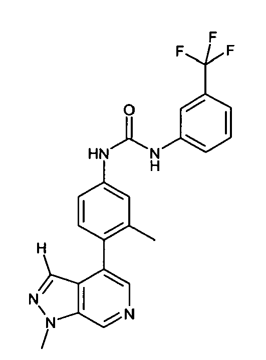 | 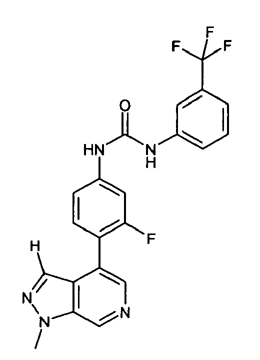 | 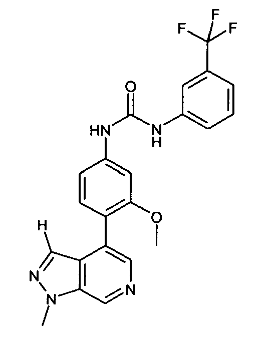 |
| **Example 17.28** | **Example .17.29** | **Example 17.30** |
| 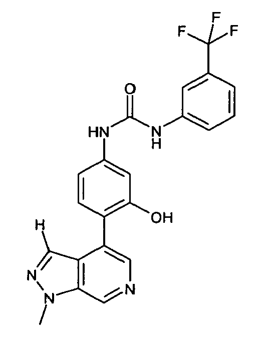 | 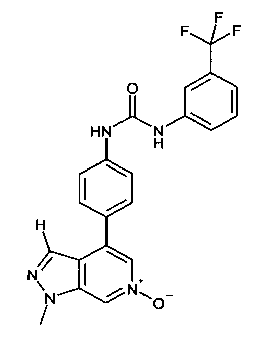 | 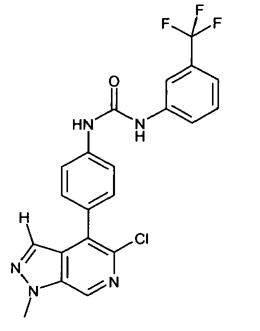 |
| **Example 17.31** | **Example 17.32** | **Example 17.33** |
| 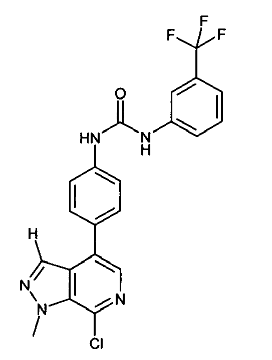 | 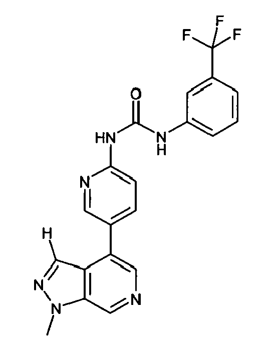 | 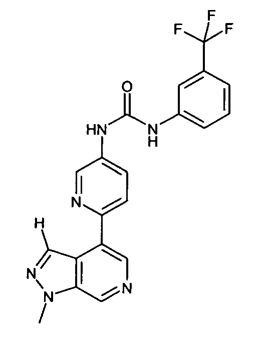 |
| **Example 17.34** | **Example 17.35** | **Example 17.36** |
| 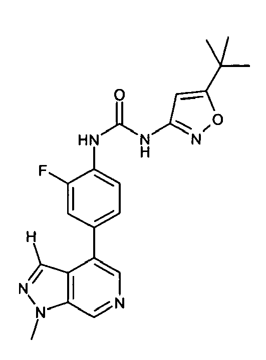 | 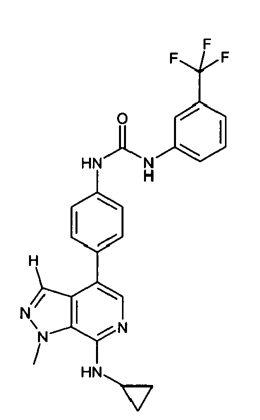 | 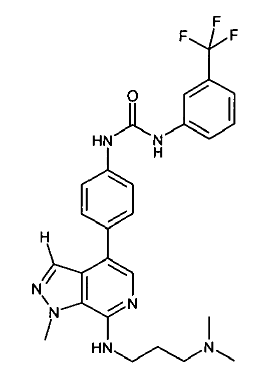 |
| **Example 17.37** | **Example 17.38** | **Example 17.39** |
| 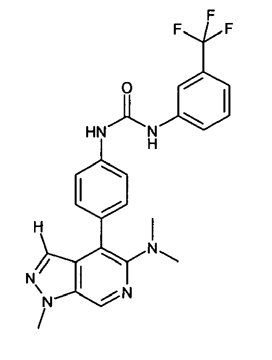 | 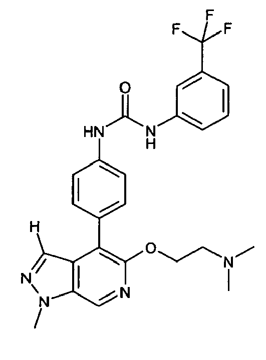 | 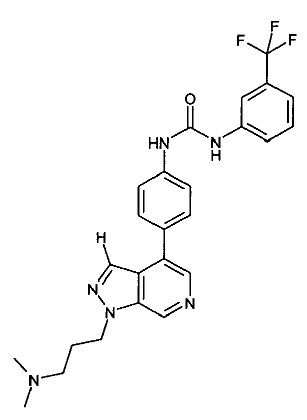 |
| **Example 17.40** | **Example 17.41** | **Example 17.42** |
| 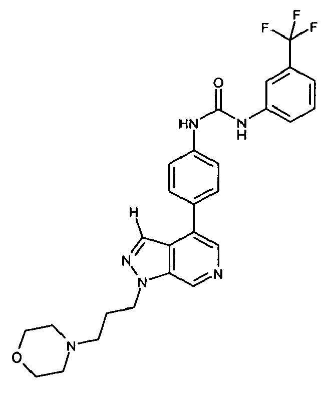 | 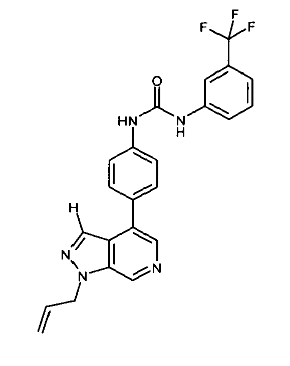 | 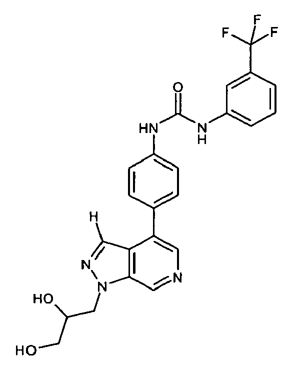 |
| **Example 17.43** | **Example 17.44** | **Example 17.45** |
| 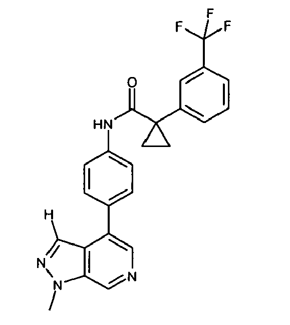 | 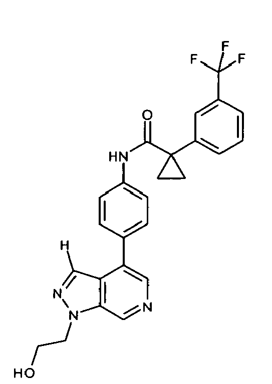 | 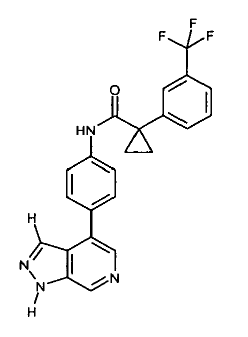 |
| **Example 17.46** | **Example 17.47** | **Example 17.48** |
| 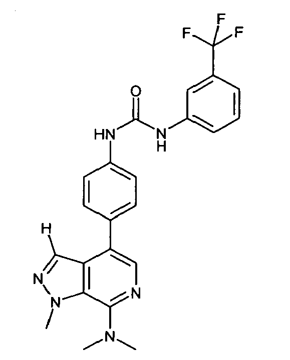 | 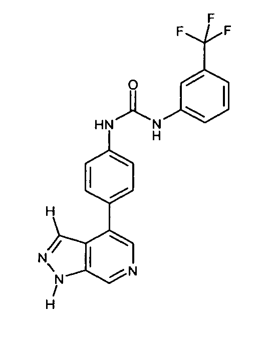 | 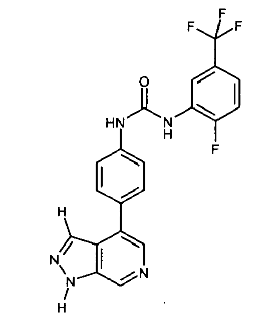 |
| **Example 17.49** | **Example 17.50** | **Example 17.51** |
| 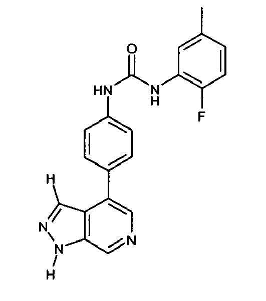 | 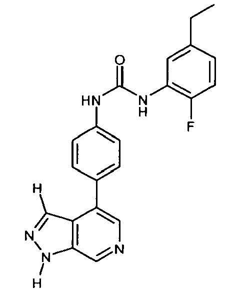 | 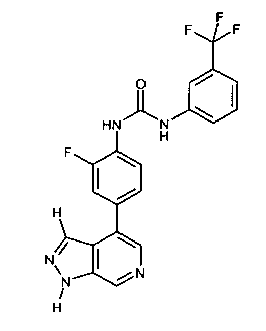 |
| **Example 17.52** | **Example 17.53** | **Example 17.54** |
| 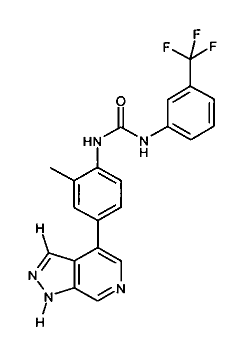 | 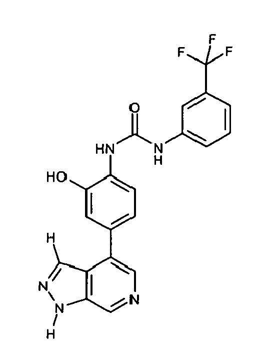 | 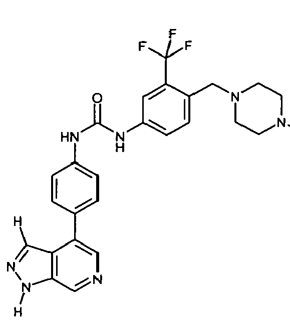 |
| **Example 17.55** | **Example 17.56** | **Example 17.57** |
| 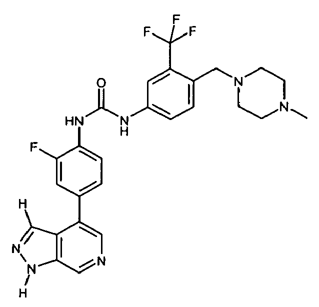 | 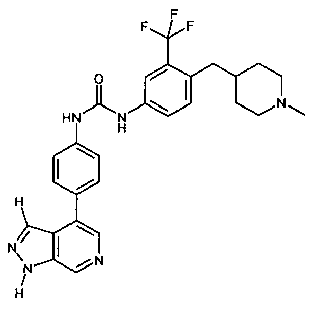 | 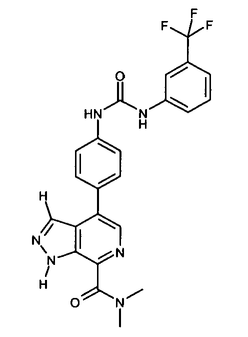 |
| **Example 17.58** | **Example 17.59** | **Example 17.60** |
| 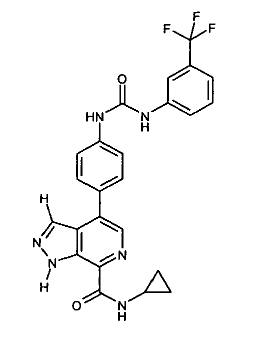 | 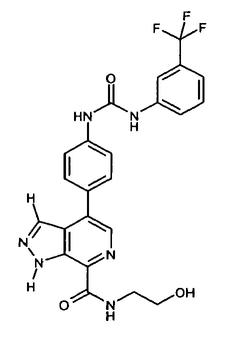 | 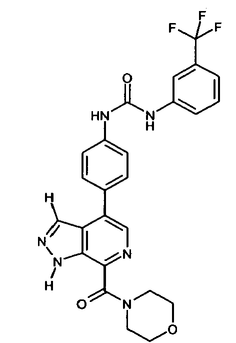 |
| **Example 17.61** | **Example 17.62** | **Example 17.63** |
| 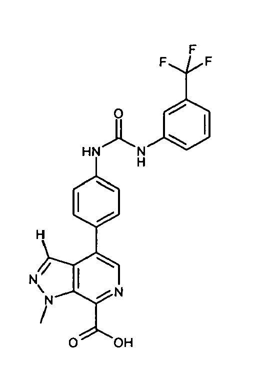 | 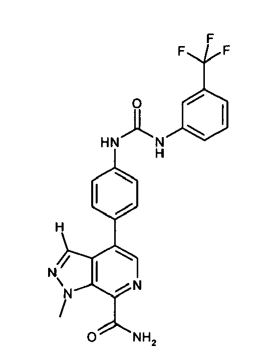 | 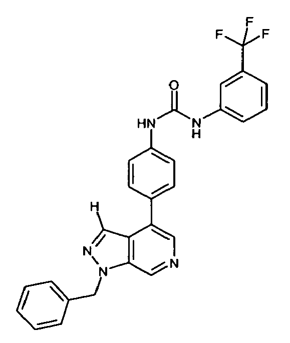 |
| **Example 17.64** | **Example 17.65** | **Example 17.66** |
| 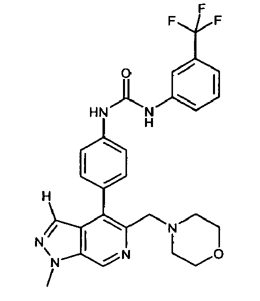 | 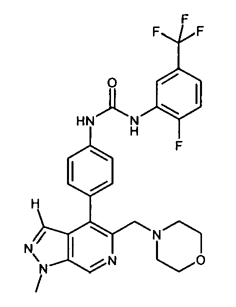 | 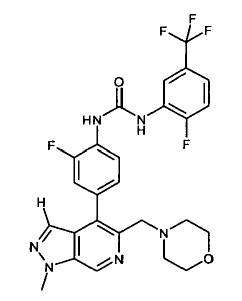 |
| **Example 17.67** | **Example 17.68** | **Example 17.69** |
| 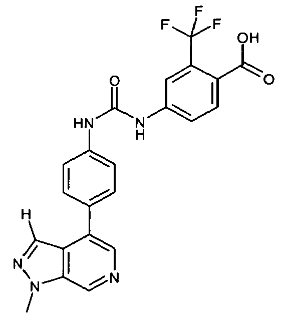 | 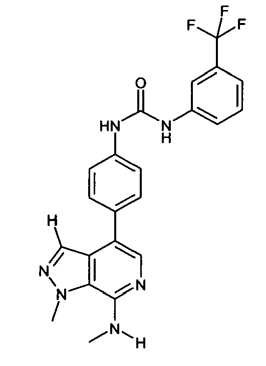 | 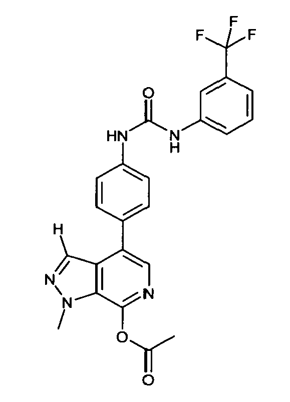 |
| **Example 17.70** | **Example 17.71** | **Example 17.72** |
| 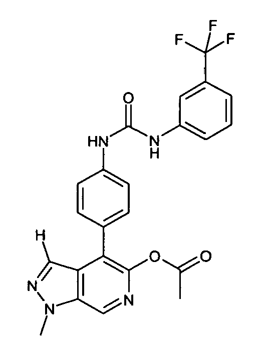 | 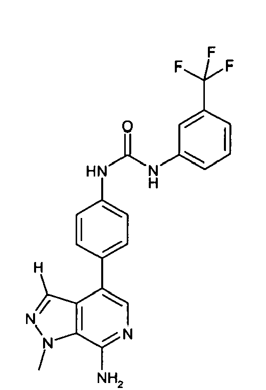 | 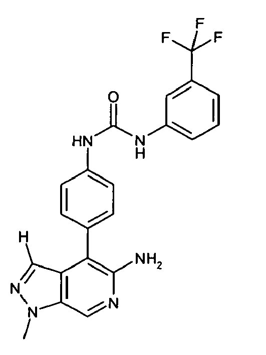 |
| **Example 17.73** | **Example 17.74** | **Example 17.75** |
| 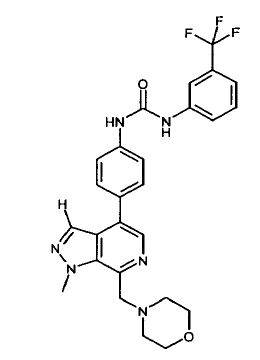 | 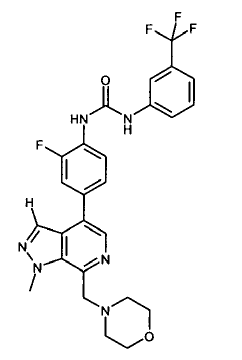 | 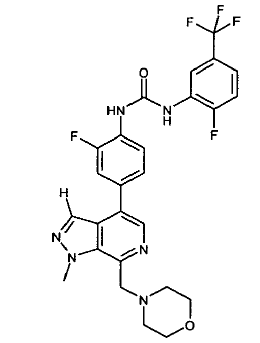 |
| **Example 17.76** | **Example 17.77** | **Example 17.78** |
| 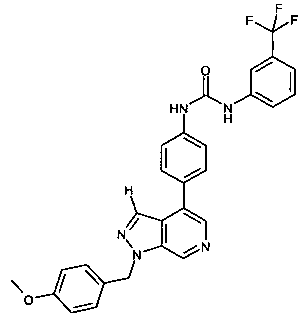 | 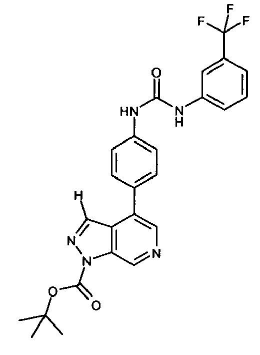 | 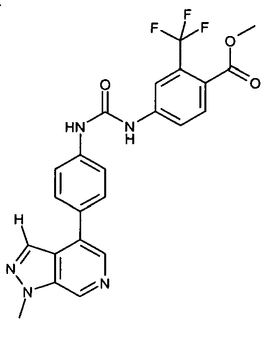 |
| **Example 17.79** | **Example 17.80** | **Example 17.81** |

### DESCRIPTION OF BIOLOGICAL ASSAYS

A selection of assays to profile compounds of the present invention is described in the following paragraphs.

### Assay 1: Tie2 ELISA Assay

Cellular activity of compounds of the present invention as inhibitors of Tie2 kinase activity was measured employing a Tie2 ELISA assay as described in the following paragraphs. Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP for detection.

### Materials:

96well tissue culture plate, sterile, Greiner
96well FluoroNunc plate MaxiSorp Surface C, Nunc
96well plate polypropylene for compound dilution in DMSO
CHO Tie2/DHFR (transfected cells)
PBS-; PBS++, DMSO
MEM alpha Medium with Glutamax-I without Ribonucleosides and
   Deoxyribonucleosides (Gibco #32561-029)
   with 10% FCS after dialysis! and 1% PenStrep
Lysis buffer: 1 Tablet "Complete" protease inhibitor
   1 cap Vanadate (1 mL > 40 mg/mL; working solution 2 mM)
   ad 50 mL with Duschl-Puffer
   pH 7.6
Anti-Tie2-antibody 1 : 425 in Coating Buffer pH 9.6
   Stock solution: 1.275 mg/mL > working.: 3 µg/mL
PBST: 2 bottles PBS(10x) + 10ml Tween, fill up with VE-water
RotiBlock 1 : 10 in VE-water
Anti-Phosphotyrosine HRP-Conjugated 1 : 10000 in 3% TopBlock
   3% TopBlock in PBST
BM Chemiluminescence ELISA Substrate (POD)
   solution B 1 : 100 solution A
SF9 cell culture medium
Ang2-Fc in SF9 cell culture medium

### Cell experiment:

Dispense 5 x 10⁴ cells / well / 98 µL in 96well tissue culture plate
Incubate at 37 °C / 5% CO₂
After 24 h add compounds according to desired concentrations
Add also to control and stimulated values without compounds 2 µL DMSO
And mix for a few min at room temperature
Add 100 µL Ang2-Fc to all wells except control, which receives insect medium
Incubate 20 min at 37 °C.
Wash 3x with PBS++
Add 100 µl Lysis buffer / well and shake a couple of min at room temperature
Store lysates at 20 °C before utilizing for the ELISA

### Performance of sandwich-ELISA

Coat 96well FluoroNunc Plate MaxiSorp Surface C with anti-Tie2 mAb
1 : 425 in Coating buffer pH 9.6; 100 µL / well overnight at 4 °C
Wash 2x with PBST
Block plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C
Wash 2x with PBST
Add 100 µL / well anti-Phosphotyrosine HRP-Conjugated 1 : 10000 in 3%
TopBlock (3% TopBlock in PBST) and incubate overnight under shaking
Wash 6x with PBST
Add 100 µL / well BM Chemiluminescence ELISA Substrate (POD) solutions 1 und 2 (1 : 100)
Determine luminescence with the LumiCount.

### Assay 2: Tie-2-Kinase HTRF-Assay without kinase preactivation

Tie2-inhibitory activity of compounds of the present invention was quantified employing two Tie2 HTRF assay as described in the following paragraphs.

A recombinant fusion protein of GST and the intracellular domains of Tie-2, expressed in insect cells (Hi-5) and purified by Glutathion-Sepharose affinity chromatography was used as kinase. Alternatively, commercially available GST-Tie2-fusion protein (Upstate Biotechnology, Dundee, Scotland) can be used As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine.

Tie-2 (3.5 ng/measurement point) was incubated for 60 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer ( 25 mM Hepes/NaOH pH 7.5, 0.28 % (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 3: Tie-2-Kinase HTRF-Assay with kinase preactivation

A recombinant fusion protein of GST and the intracellular domains of Tie-2, expressed in insect cells (Hi-5) and purified by Glutathion-Sepharose affinity chromatography was used as kinase. As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany).

For activation, Tie-2 was incubated at a conc. 12.5 ng/µl of for 20 min at 22°C in the presence of 250 µM adenosine-tri-phosphate (ATP) in assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml)].

For the subsequent kinase reaction, the preactivated Tie-2 (0.5 ng/measurement point) was incubated for 20 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 0.1 mM sodium ortho-vanadate, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer ( 25 mM Hepes/NaOH pH 7.5, 0.28% (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 4: InsR HTRF Assay

Inhibitory activity of compounds against the kinase activity of the insulin receptor was quantified employing the Ins-R HTRF assay as described in the following paragraphs.

GST-tagged recombinant kinase domain of the human insuline receptor (Ins-R, purchase from ProQinase, Freiburg, Germany) expressed in SF-9 cells was used as kinase. As substrate for the kinase reaction biotinylated poly-(Glu,Tyr) (Cis biointernational, France) was used.
Ins-R was incubated for 20 min at 22°C in the presence of different concentrations of test compounds in 5 µl assay buffer [50 mM Hepes/NaOH pH 7, 15 mM MnCl₂, 1 mM dithiothreitol, 0.1 µM sodium ortho-vanadate, 0.015 % (v/v) PEG20000, 10 µM adenosine-tri-phosphate (ATP), 0.3 µg/ml substrate, 1 % (v/v) dimethylsulfoxide]. The concentration of Ins-R was adjusted depending of the activity of the enzyme lot and was chosen appropriate to have the assay in the linear range, typical concentrations were in the range of 10 pg/µl. The reaction was stopped by the addition of 5 µl of a solution of HTRF detection reagents (0.1 µM streptavidine-XLent and 1 nM PT66-Eu-Chelate, an europium-chelate labelled anti-phospho-tyrosine antibody from Perkin Elmer) in an aqueous EDTA-solution (80 mM EDTA, 0.2 % (w/v) bovine serum albumin in 50 mM HEPES/NaOH pH 7.0).
The resulting mixture was incubated 1 h at 22°C to allow the binding of the biotinylated phosphorylated peptide to the streptavidine-XLent and the PT66-Eu-Chelate. Subsequently the amount of phosphorylated substrate was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition) and IC50 values were calculated by a 4 parameter fit using an inhouse software.

### Additional Assays: Upstate KinaseProfiler® - Radio-enzymatic filter binding assay :

### Upstate KinaseProfiler^{™} - Radio-enzymatic filter binding Assay

Compounds of the present invention are assessed for their ability to inhibit individual members of the kinase panel. The compounds are tested in duplicates at a final concentration of 10 µM following this generic protocol. Note that the kinase buffer composition and the substrates vary for the different kinases included in the "Upstate KinaseProfiler^{™}" panel. Kinase buffer (2.5 µL, 10x - containing MnCl₂ when required), active kinase (0.001 - 0.01 Units ; 2.5 µL), specific or Poly(Glu4-Tyr) peptide (5 - 500 µM or 0.01 mg/ml) in kinase buffer and kinase buffer (50 µM ; 5 µL) are mixed in an eppendorf on ice. A Mg/ATP mix (10 µL ; 67.5 (or 33.75) mM MgCl₂ , 450 (or 225) µM ATP and 1 µCi/µl [γ-³²P]-ATP (3000 Ci/mmol)) is added and the reaction is incubated at about 30°C for about 10 minutes. The reaction mixture is spotted (20 µL) onto a 2 cm x 2 cm P81 (phosphocellulose, for positively charged peptide substrates) or Whatman No. 1 (for Poly(Glu4-Tyr) peptide substrate) paper square. The assay squares are washed 4 times, for 5 minutes each, with 0.75% phosphoric acid and washed once with acetone for 5 minutes. The assay squares are transferred to a scintillation vial, 5 ml scintillation cocktail are added and ³²P incorporation (cpm) to the peptide substrate is quantified with a Beckman scintillation counter. Percentage inhibition is calculated for each reaction.

Further kinase assay protocols which may be used are given in the document "KinaseProfiler^{™} Assay Protocols", Protocol Guide, Fall 2004, and "KinaseProfiler^{™} Protocol Guide - Addendum I", published by the company Upstate Ltd under http://www.upstate.com/features/kp_protocols.asp, which is hereby incorporated by reference in its entirety.

### BIOLOGICAL DATA

Compounds of the present invention were found to possess enzymatic and cellular activity as inhibitors of Tie2 kinase. Prefered compounds of the present invention inhibit Tie2 kinase activity and cellular Tie2 autophosphorylation with IC₅₀ values below 1 µM, more preferred compounds inhibit Tie2 autophosphorylation with IC₅₀ values below 0.5 µM. Compounds of the present invention possess inhibitory selectivity for Tie2 kinase vs. insulin receptor kinase. Prefered compounds of the present invention are capable of inhibiting additional specific tyrosine kinases, the inhibition of which is of therapeutic use for the treatment of certain oncological diseases, for example.

Selected data are given in the following table. The IC₅₀ values were converted to pIC₅₀ values, i.e. -log IC₅₀ in molar concentration.

| Example No. | Tie 2 activity (assay 1) | Tie 2 activity (assay 2) | Selectivity vs. InsR |
|---|---|---|---|
| 1.2 | +++ | +++ | > 50fold |
| 1.3 | +++ | +++ | > 50fold |
| 1.4 | +++ | +++ | > 50fold |
| 1.5 | +++ | +++ | > 50fold |
| 1.9 | +++ | +++ | > 50fold |
| 1.13 | +++ | +++ | > 50fold |
| 3.1 | +++ | +++ | > 50fold |
| 4.1 | +++ | +++ | > 50fold |
| 6.1 | ++ | ++ | > 25fold |
| 6.3 | +++ | +++ | > 50fold |
| 6.4 | +++ | +++ | > 50fold |
| 8.1 | +++ | +++ | > 50fold |
| 8.2 | +++ | +++ | > 50fold |
| 8.5 | +++ | +++ | > 50fold |
| 8.6 | +++ | +++ | > 50fold |
| 10.5 | +++ | +++ | > 50fold |
| 11.1 | +++ | +++ | > 50fold |
| 11.2 | +++ | +++ | > 50fold |
| 11.3 | +++ | +++ | > 50fold |
| 12.1 | +++ | +++ | > 50fold |
| 12.3 | +++ | +++ | > 50fold |
| 12.4 | +++ | +++ | > 50fold |
| 12.5 | +++ | +++ | > 50fold |
| 13.2 | +++ | +++ | > 50fold |
| 13.4 | +++ | +++ | > 50fold |
| 13.6 | +++ | +++ | > 50fold |
| 13.11 | +++ | +++ | > 50fold |
| 13.12 | +++ | +++ | > 50fold |
| 13.13 | +++ | +++ | > 50fold |
| 14.3 | +++ | +++ | > 50fold |
| 14.5 | +++ | +++ | > 50fold |

| | | | |
|---|---|---|---|
| + represents pIC₅₀ 5.0 - 6.0 ++ represents pIC₅₀ 6.0 - 6.3 +++ represents pIC₅₀ > 6.3 Selectivity vs. InsR: IC₅₀ assay 4 / IC₅₀ assay 2 | | | |

### GENERAL REMARKS

It is believed that one skilled in the art, using the preceding information and information available in the art, can utilize the present invention to its fullest extent. It should be apparent to one of ordinary skill in the art that changes and modifications can be made to this invention without departing from the spirit or scope of the invention as it is set forth herein. All publications, applications and patents cited above are incorporated herein by reference.

The topic headings set forth above and below are meant as guidance where certain information can be found in the application, but are not intended to be the only source in the application where information on such topic can be found.

## Claims

1. A compound of general formula (I) : 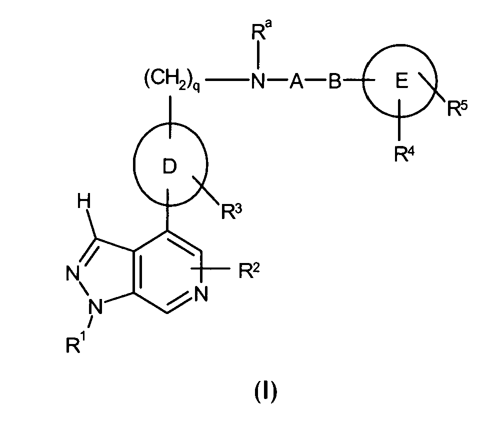 in which :
R¹ represents H or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, wherein said residues are unsubstituted or substituted one or more times, independently from each other, with R⁶ ;
R² represents hydrogen, halogen, -NR^{d1}R^{d2}, -OR^{c}, -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, aryl, heteroaryl, wherein said residues are unsubstituted or one or more times substituted independently from each other with R⁷;
R³ is selected from the group comprising, preferably consisting of, hydrogen, C₁-C₆-alkyl, C₁-C₆-alkoxy, C₁-C₆-haloalkyl, C₁-C₆-haloalkoxy, hydroxy, amino, halogen, and cyano ;
R⁴, R⁵, R⁶, R⁷ independently from each other, are selected from the group comprising, preferably consisting of, hydrogen, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b},-S(O)₂R^{b}, -OR^{c}, -NR^{d1}R^{d2}, and -OP(O)(OR^{c})₂, wherein C₁-C₆-alkyl, aryl, heteroaryl, C₃-C₁₀-heterocycloalkyl and C₃-C₁₀-cycloalkyl are optionally substituted one or more times by R⁸ ;
R⁸ is selected from the group comprising, preferably consisting of, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b}, -S(O)₂R^{b}, -OR^{c}, -NR^{d1}R^{d2}, and -OP(O)(OR^{c})₂;
R^{a} is selected from the group comprising, preferably consisting of, hydrogen and C₁-C₆-alkyl ;
R^{b} is selected from the group comprising, preferably consisting of, hydroxyl, -OR^{c}, -SR^{c}, -NR^{d1}R^{d2}, C₁-C₆-alkyl, and C₃-C₁₀-cycloalkyl, wherein C₁-C₆-alkyl, and C₃-C₁₀-cycloalkyl are optionally substituted one or more times with hydroxyl, halogen, or C₁-C₆-alkoxy;
R^{c} is selected from the group comprising, preferably consisting of, hydrogen, -C(O)R^{e}, C₁-C₆-alkyl, C₁-C₆-haloalkyl C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, aryl, -OR^{f}, -NR^{d1}R^{d2}, or -OP(O)(OR^{f})₂ ;
R^{d1}, R^{d2} independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl, or for a group -C(O)R^{e}, -S(O)₂R^{e}, or -C(O)NR^{g1}R^{g2} wherein C₁-C₆-alkyl, C₃-C₁₀-cycloalkyl, C₃-C₁₀-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}, or -OP(O)(OR^{f})₂ ; or
R^{d1} and R^{d2} together with the nitrogen atom to which they are attached, form a 3 to 10 membered heterocycloalkyl ring, which is optionally substituted one or more times, the same way or differently, with C₁-C₆-alkyl, halogen, -NR^{g1}R^{g2}, -OR^{f}, -C(O)R^{e}, -S(O)₂R^{e}, or -OP(O)(OR^{f})₂; whereby the carbon backbone of this heterocycloalkyl ring can optionally be interrupted one or more times, the same way or differently, by a member of the group comprising, preferably consisting of, NH, NR^{d3}, oxygen or sulphur, and can optionally be interrupted one or more times, the same way or differently, with a -C(O)-, -S(O)- , and/or -S(O)₂- group, and can optionally contain one or more double bonds
R^{d3} is selected from the group comprising, preferably consisting of hydrogen, C₁-C₆-alkyl, C₃-C₁₀-cycloalkyl, C₃-C₁₀-heterocycloalkyl, aryl, and heteroaryl, wherein C₁-C₆-alkyl, and C₃-C₁₀-cycloalkyl are optionally substituted one or more times with C₁-C₆-alkyl, C₃-C₁₀-cycloalkyl, hydroxyl, halogen, C₁-C₆-haloalkyl or C₁-C₆-alkoxy;
R^{e} is selected from the group comprising, preferably consisting of, -NR^{g1}R^{g2}, C₁-C₆-alkyl, C₃-C₆-cycloalkyl, C₁-C₆-alkoxy, aryl and heteroaryl;
R^{f} is selected from the group comprising, preferably consisting of, hydrogen, -C(O)R^{e}, C₁-C₆-alkyl, C₁-C₆-haloalkyl, C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, C₁-C₆-alkoxy, aryl, or -NR^{g1}R^{g2};
R^{g1}, R^{g2} independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl;
R^{g1} and R^{g2} together with the nitrogen atom to which they are attached, form a 3 to 10 membered heterocycloalkyl ring, which is optionally substituted one or more times, the same way or differently, with C₁-C₆-alkyl, -C₁-C₆-alkoxy, halogen or hydroxy; whereby the carbon backbone of this heterocycloalkyl ring can optionally be interrupted one or more times, the same way or differently, by a member of the group comprising, preferably consisting of, NH, NR^{a}, oxygen or sulphur, and can optionally be interrupted one or more times, the same way or differently, with a -C(O)-, -S(O)- , and/or -S(O)₂- group, and can optionally contain one or more double bonds ;
A is selected from the group comprising, preferably consisting of, -C(O)-, -C(S)-, -C(=NR^{a})-, -C(O)NR^{a}-, -C(=NR^{a})NR^{a}-, -S(O)₂-, -S(O)(=NR^{a})-, -S(=NR^{a})₂-, -C(S)NR^{a}-, -C(O)C(O)-, -C(O)C(O)NR^{a}-, -C(O)NR^{a}C(O)-, -C(S)NR^{a}C(O)-, and -C(O)NR^{a}C(S)- ;
B is a bond or selected from the group comprising, preferably consisting of C₁-C₆-alkylene, C₃-C₁₀-cycloalkylene, and C₃-C₁₀-heterocycloalkylene ;
D, E are, independently from each other, arylene or heteroarylene ;
and
q represents an integer of 0, 1, or 2 ;
or a salt, an N-oxide, a solvate or a prodrug thereof,
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

2. The compound according to claim 1, wherein :
R¹ represents H or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, and C₃-C₁₀-heterocycloalkyl, wherein said residues are unsubstituted or substituted one or more times, independently from each other, with R⁶ ;
R² represents hydrogen, halogen, -NR^{d1}R^{d2}, -OR^{c}, or -C(O)R^{b}, or is selected from the group comprising, preferably consisting of, C₁-C₆-alkyl, C₂-C₆-alkenyl, C₂-C₆-alkynyl, C₃-C₁₀-cycloalkyl, C₃-C₁₀-heterocycloalkyl, aryl, and heteroaryl, wherein said residues are unsubstituted or one or more times substituted independently from each other with R⁷;
R³ is selected from the group comprising, preferably consisting of, hydrogen, C₁-C₆-alkyl, C₁-C₆-alkoxy, C₁-C₆-haloalkyl, C₁-C₆-haloalkoxy, hydroxy, amino, halogen, and cyano ;
R⁴, R⁵, R⁶, R⁷ independently from each other, are selected from the group comprising, preferably consisting of, hydrogen, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b},-S(O)₂R^{b}, -OR^{c}, -NR^{d1}R^{d2}, and -OP(O)(OR^{c})₂, wherein C₁-C₆-alkyl, aryl, heteroaryl, C₃-C₁₀-heterocycloalkyl and C₃-C₁₀-cycloalkyl are optionally substituted one or more times by R⁸ ;
R⁸ is selected from the group comprising, preferably consisting of, C₁-C₆-alkyl, C₃-C₁₀-cycloalkyl, C₃-C₁₀-heterocycloalkyl, C₁-C₆-haloalkyl, C₁-C₆-haloalkoxy, aryl, heteroaryl, hydroxy, amino, halogen, cyano, nitro, -C(O)R^{b}, -S(O)₂R^{b}, -OR^{c}, -NR^{d1}R^{d2}, and -OP(O)(OR^{c})₂;
R^{a} is selected from the group comprising, preferably consisting of, hydrogen and C₁-C₆-alkyl ;
R^{b} is selected from the group comprising, preferably consisting of, hydroxyl, -OR^{c}, -SR^{c}, -NR^{d1}R^{d2}, C₁-C₆-alkyl, and C₃-C₁₀-cycloalkyl, wherein C₁-C₆-alkyl, and C₃-C₁₀-cycloalkyl are optionally substituted one or more times with hydroxyl, halogen, or C₁-C₆-alkoxy;
R^{c} is selected from the group comprising, preferably consisting of, hydrogen, -C(O)R^{e}, C₁-C₆-alkyl, C₁-C₆-haloalkyl C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, aryl, -OR^{f}, -NR^{d1}R^{d2}, or -OP(O)(OR^{f})₂ ;
R^{d1}, R^{d2} independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl, or for a group -C(O)R^{e}, -S(O)₂R^{e}, or -C(O)NR^{g1}R^{g2} wherein C₁-C₆-alkyl, C₃-C₁₀-cycloalkyl, C₃-C₁₀-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e},-OP(O)(OR^{f})₂; or
R^{d1} and R^{d2} together with the nitrogen atom to which they are attached, form a 3 to 10 membered heterocycloalkyl ring, which is optionally substituted one or more times, the same way or differently, with C₁-C₆-alkyl, halogen, -NR^{g1}R^{g2}, -OR^{f}, -C(O)R^{e}, -S(O)₂R^{e}, or -OP(O)(OR^{f})₂; whereby the carbon backbone of this heterocycloalkyl ring can optionally be interrupted one or more times, the same way or differently, by a member of the group comprising, preferably consisting of, NH, NR^{d3}, oxygen or sulphur, and can optionally be interrupted one or more times, the same way or differently, with a -C(O)-, -S(O)- , and/or-S(O)₂- group, and can optionally contain one or more double bonds
R^{d3} is selected from the group comprising, preferably consisting of hydrogen, C₁-C₆-alkyl, C₃-C₁₀-cycloalkyl, C₃-C₁₀-heterocycloalkyl, aryl, and heteroaryl, wherein C₁-C₆-alkyl, and C₃-C₁₀-cycloalkyl are optionally substituted one or more times with C₁-C₆-alkyl, C₃-C₁₀-cycloalkyl, hydroxyl, halogen, C₁-C₆-haloalkyl or C₁-C₆-alkoxy;
R^{e} is selected from the group comprising, preferably consisting of,-NR^{g1}R^{g2}, C₁-C₆-alkyl, C₃-C₆-cycloalkyl, C₁-C₆-alkoxy, aryl and heteroaryl;
R^{f} is selected from the group comprising, preferably consisting of, hydrogen, -C(O)R^{e}, C₁-C₆-alkyl, C₁-C₆-haloalkyl, C₃-C₁₀-cycloalkyl, C₃-C₁₀-heterocycloalkyl, aryl, and heteroaryl, wherein C₁-C₆-alkyl, C₁-C₆-haloalkyl, C₃-C₁₀-cycloalkyl, C₃-C₁₀-heterocycloalkyl, aryl, and heteroaryl are optionally substituted one or more times with hydroxyl, halogen, C₁-C₆-alkoxy, aryl, or -NR^{g1}R^{g2};
R^{g1}, R^{g2} independently from each other are selected from the group comprising, preferably consisting of hydrogen, C₁-C₆-alkyl, C₃-C₁₀-cycloalkyl, C₃-C₁₀-heterocycloalkyl, aryl, and heteroaryl;
R^{g1} and R^{g2} together with the nitrogen atom to which they are attached, form a 3 to 10 membered heterocycloalkyl ring, which is optionally substituted one or more times, the same way or differently, with C₁-C₆-alkyl, -C₁-C₆-alkoxy, halogen or hydroxy; whereby the carbon backbone of this heterocycloalkyl ring can optionally be interrupted one or more times, the same way or differently, by a member of the group comprising, preferably consisting of, NH, NR^{a}, oxygen or sulphur, and can optionally be interrupted one or more times, the same way or differently, with a -C(O)-, -S(O)- , and/or -S(O)₂- group, and can optionally contain one or more double bonds ;
A represents -C(O)- or -C(O)NR^{a}-;
B is a bond or selected from the group comprising, preferably consisting of C₁-C₃-alkylene and C₃-C₅-cycloalkylene;
D, E are, independently from each other, arylene or heteroarylene ;
and
q represents an integer of 0, or 1;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

3. The compound according to claim 1 or 2, wherein :
R¹ represents H, C₁-C₆-alkyl, or C₂-C₆-alkenyl, wherein C₁-C₆-alkyl is unsubstituted or substituted one or more times, independently from each other, with R⁶ ;
R² represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
R³ is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
R⁴, R⁵, R⁶, R⁷ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸ ;
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
R^{c} is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
R^{d1}, R^{d2} independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, -OR^{f}, -C(O)R^{e}, or -S(O)₂R^{e}; whereby the carbon backbone of this heterocycloalkyl ring can optionally be interrupted one or more times, the same way or differently, by a member of the group comprising, preferably consisting of, NH, NR^{d3}, or oxygen, and can optionally be interrupted one or more times, the same way or differently, with a -C(O)- group;
R^{d3} is selected from the group comprising, preferably consisting of hydrogen and C₁-C₄-alkyl, wherein C₁-C₄-alkyl is optionally substituted one or more times with C₃-C₆-cycloalkyl, hydroxyl, halogen, C₁-C₄-haloalkyl or C₁-C₄-alkoxy;
R^{e} is selected from the group comprising, preferably consisting of,-NR^{g1}R^{g2}, C₁-C₃-alkyl, C₃-C₆-cycloalkyl, C₁-C₃-alkoxy, aryl and heteroaryl;
R^{f} is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
R^{g1}, R^{g2} independently from each other are selected from the group comprising, preferably consisting of hydrogen, C₁-C₃-alkyl, C₃-C₆-cycloalkyl, C₃-C₆-heterocycloalkyl, aryl, and heteroaryl;
R^{g1} and R^{g2} together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted one or more times, the same way or differently, by a member of the group comprising, preferably consisting of, NH, NR^{a}, or oxygen;
A represents -C(O)NR^{a}-;
B is a bond;
D, E are, independently from each other, arylene or heteroarylene ;
and
q is 0;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

4. The compound according to any one of claims 1 to 3, wherein :
R¹ represents H, C₁-C₆-alkyl, or C₂-C₆-alkenyl, wherein C₁-C₆-alkyl is unsubstituted or substituted one or more times, independently from each other, with R⁶ ;
R² represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
R³ is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
R⁴, R⁵, R⁶, R⁷ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸ ;
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
R^{c} is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
R^{d1}, R^{d2} independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-C₆-cycloalkyl, hydroxyl, C₁-C₄-haloalkyl, C₁-C₄-atkoxy or halogen;
R^{e} is selected from the group comprising, preferably consisting of,-NR^{g1}R^{g2}, C₁-C₃-alkyl, C₃-C₆-cycloalkyl, C₁-C₃-alkoxy, aryl and heteroaryl;
R^{f} is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
R^{g1}, R^{g2} independently from each other are selected from the group comprising, preferably consisting of hydrogen, C₁-C₃-alkyl, C₃-C₆-cycloalkyl, C₃-C₆-heterocycloalkyl, aryl, and heteroaryl;
R^{g1} and R^{g2} together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted once by a member of the group comprising, preferably consisting of, NH, NR^{a}, or oxygen;
A represents -C(O)NR^{a}-;
B is a bond;
D is para-phenylene ;
E is phenylene or heteroarylene ;
and
q is 0;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R³, R^{f}, R^{g1}, R^{g2}, or R⁸ within a single molecule to be identical or different.

5. The compound according to any one of claims 1 to 4, wherein :
R¹ represents H, C₁-C₆-alkyl, or C₂-C₆-alkenyl, wherein C₁-C₆-alkyl is unsubstituted or substituted one or more times, independently from each other, with R⁶ ;
R² represents hydrogen, halogen, -NR^{d1}R^{d2}, -OR^{c}, -C(O)R^{b}, or C₁-C₆-alkyl, wherein C₁-C₆-alkyl is unsubstituted or one or more times substituted independently from each other with R⁷;
R³ is selected from the group comprising, preferably consisting of, hydrogen, C₁-C₃-alkyl, C₁-C₃-alkoxy, C₁-C₃-haloalkyl, C₁-C₃-haloalkoxy, hydroxy, amino, halogen, and cyano ;
R⁴, R⁵, R⁶, R⁷ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, aryl, heteroaryl, hydroxy, amino, halogen, cyano, nitro, -C(O)R^{b},-S(O)₂R^{b}, -OR^{c}, and -NR^{d1}R^{d2}, wherein C₁-C₃-alkyl, aryl, heteroaryl, C₃-C₆-heterocycloalkyl and C₃-C₆-cycloalkyl are optionally substituted one or more times by R⁸ ;
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(O)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -SR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
R^{c} is selected from the group comprising, preferably consisting of, hydrogen, -C(O)R^{e}, C₁-C₃-alkyl, C₁-C₃-haloalkyl C₃-C₆-cycloalkyl, C₃-C₇-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, aryl, -OR^{f}, or -NR^{d1}R^{d2};
R^{d1}, R^{d2} independently from each other are selected from the group comprising, preferably consisting of hydrogen, C₁-C₃-alkyl, C₃-C₆-cycloalkyl, C₃-C₆-heterocycloalkyl, aryl, and heteroaryl, or for a group -C(O)R^{e}, -S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-C₆-cycloalkyl, hydroxyl, C₁-C₄-haloalkyl, C₁-C₄-alkoxy or halogen;
R^{e} is selected from the group comprising, preferably consisting of,-NR^{g1}R^{g2}, C₁-C₃-alkyl, C₃-C₆-cycloalkyl, C₁-C₃-alkoxy, aryl and heteroaryl;
R^{f} is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
R^{g1}, R^{g2} independently from each other are selected from the group comprising, preferably consisting of hydrogen, C₁-C₃-alkyl, C₃-C₆-cycloalkyl, C₃-C₆-heterocycloalkyl, aryl, and heteroaryl;
R^{g1} and R^{g2} together with the nitrogen atom to which they are attached, form a 3 to 6 membered heterocycloalkyl ring, which is optionally substituted one or more times, the same way or differently, with C₁-C₄-alkyl, -C₁-C₄-alkoxy, halogen or hydroxy; whereby the carbon backbone of this heterocycloalkyl ring can optionally be interrupted once by a member of the group comprising, preferably consisting of, NH, NR^{a}, or oxygen;
A represents -C(O)NR^{a}-;
B is a bond;
D is para-phenylene ;
E is heteroarylene ;
and
q is 0;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

6. The compound according to any one of claims 1 to 5, wherein :
R¹ represents H or C₁-C₄-alkyl wherein C₁-C₄-alkyl is unsubstituted or substituted one or more times with R⁶ ;
R² represents hydrogen;
R³ is selected from the group comprising, preferably consisting of, hydrogen, methyl, methoxy, fluorine and hydroxy;
R⁴ is selected from the group comprising, preferably consisting of, hydrogen, C₁-C₄-alkyl, C₁-C₆-cycloalkyl, wherein C₁-C₄-alkyl is optionally substituted by R⁸ ;
R⁵ is selected from the group comprising, preferably consisting of, hydrogen, C₁-C₄-alkyl, C₅-C₆-heterocycloalkyl, C₅-C₆-cycloalkyl, phenyl and pyridyl, wherein C₁-C₄-alkyl, C₅-C₆-heterocycloalkyl, C₅-C₆-cycloalkyl, phenyl and pyridyl are optionally substituted one or more times, independently from each other, with R⁸;
R⁶, represents hydroxy;
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c} and -NR^{d1}R^{d2};
R^{a} represents hydrogen or methyl;
R^{b} is selected from the group comprising, preferably consisting of, hydroxy, -OR^{c}, -NR^{d1}R^{d2}, C₁-C₃-alkyl, and C₃-C₆-cycloalkyl,
R^{c} represents C₁-C₃-alkyl or C₆-C₇-heterocycloalkyl;
R^{d1}, R^{d2} independently from each other are selected from the group comprising, preferably consisting of hydrogen, and C₁-C₃-alkyl, or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-C₆-cycloalkyl, hydroxy, C₁-C₄-haloalkyl, C₁-C₄-alkoxy or halogen;
R^{g1}, R^{g2} independently from each other are selected from the group comprising, preferably consisting of hydrogen, C₁-C₃-alkyl and C₃-C₆-cycloalkyl;
A represents -C(O)NR^{a}- ;
B is a bond;
D is a para-phenylene
E is a pyrazole;
and
q is 0;
wherein, when one or more of R^{a} , R^{c}, or R^{d3} is (are) present in one position in the molecule as well as in one or more further positions in the molecule, said R^{a}, R^{c}, or R^{d3} has (have), independently from each other, the same meanings as defined above in said first position in the molecule and in said second or further positions in the molecule, it being possible for the two or more occurrences of R^{a}, R^{c}, or R^{d3} within a single molecule to be identical or different.

7. The compound according to any one of claims 1 to 4, wherein:
R¹ represents H, or C₁-C₃-alkyl, wherein C₁-C₃-alkyl is unsubstituted or substituted one or more times, independently from each other, with R⁶ ;
R² represents hydrogen, halogen, -NR^{d1}R^{d2}, -OR^{c}, -C(O)R^{b}, or C₁-C₃-alkyl, wherein C₁-C₃-alkyl is unsubstituted or one or more times substituted independently from each other with R⁷;
R³ is selected from the group comprising, preferably consisting of, hydrogen, methyl, methoxy, hydroxy, halogen, and cyano ;
R⁴, R⁵ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c}, and -NR^{d1}R^{d2}, wherein C₁-C₃-alkyl, C₃-C₆-heterocycloalkyl and C₃-C₆-cycloalkyl are optionally substituted one or more times by R⁸ ;
R⁶ independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}Rd²;
R⁷ independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}R^{d2};
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}, -S(P)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
R^{c} is selected from the group comprising, preferably consisting of, hydrogen, -C(O)R^{e}, C₁-C₃-alkyl, C₁-C₃-haloalkyl C₃-C₆-cycloalkyl, and C₃-C₇-heterocycloalkyl, wherein C₁-C₃-alkyl, C₁-C₃-haloatkyl, C₃-C₆-cycloalkyl, C₃-C₆-heterocycloalkyl are optionally substituted one or more times with hydroxyl, halogen, aryl, -OR^{f}, or -NR^{d1}R^{d2};
R^{d1}, R^{d2} independently from each other are selected from the group comprising, preferably consisting of hydrogen, C₁-C₃-alkyl, C₃-C₆-cycloalkyl, and C₃-C₆-heterocycloalkyl, or for a group -C(O)R^{e},-S(O)₂R^{e}, or -C(O)NR^{g1}R^{g2} wherein C₁-C₃-alkyl, C₃-C₆-cycloalkyl, C₃-C₆-heterocycloalkyl, aryl, and heteroaryl are optionally substituted one or more times, the same way or differently with halogen, hydroxy or the group aryl, -NR^{g1}R^{g2}, -OR^{f}, -C(O)R^{e}, -S(O)₂R^{e}; or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
R^{e} is selected from the group comprising, preferably consisting of,-NR^{g1}R^{g2}, C₁-C₃-alkyl, C₃-C₆-cycloalkyl, C₁-C₃-alkoxy, aryl and heteroaryl;
R^{f} is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃-haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
R^{g1}, R^{g2} independently from each other are selected from the group comprising, preferably consisting of hydrogen, C₁-C₃-alkyl, C₃-C₆-cycloalkyl, C₃-C₆-heterocycloalkyl, aryl, and heteroaryl;
R^{g1} and R^{g2} together with the nitrogen atom to which they are attached, form a 3 to 6 membered heterocycloalkyl ring, whereby the carbon backbone of this heterocycloalkyl ring can optionally be interrupted once by a member of the group comprising, preferably consisting of, NH, NR^{d3}, or oxygen;
A represents -C(O)NR^{a}-;
B is a bond;
D is para-phenylene ;
E is phenylene ;
and
q is 0;
wherein, when one or more of R^{a} , R^{b} , R^{c} , R^{d1} , R^{d2}, R^{d3}, R^{e}, R^{f}, R^{g1} R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

8. The compound according to claim 1, 2, 3, 4 or 7, wherein :
R¹ represents H, or C₁-C₃-alkyl, wherein C₁-C₃-alkyl is unsubstituted or substituted one or more times, independently from each other, with R⁶ ;
R² represents hydrogen;
R³ is selected from the group comprising, preferably consisting of, hydrogen, methyl, methoxy, hydroxy, and halogen ;
R⁴, R⁵ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -S(O)₂R^{b}, -OR^{c}, and-NR^{d1}R^{d2}, wherein C₁-C₃-alkyl, C₃-C₆-heterocycloalkyl and C₃-C₆-cycloalkyl are optionally substituted one or more times by R⁸ ;
R⁶ independently from each other, are selected from the group comprising, preferably consisting of, C₃-C₆-cycloalkyl, C₃-C₆-heterocycloalkyl, hydroxy, halogen, cyano, -C(O)R^{b}, -S(O)₂R^{b}, -OR^{c}, and -NR^{d1}R^{d2};
R⁸ is selected from the group comprising, preferably consisting of, C₁-C₃-alkyl, C₃-C₆-cycloalkyl, C₃-C₇-heterocycloalkyl, C₁-C₃-haloalkyl, C₁-C₃-haloalkoxy, aryl, heteroaryl, hydroxy, amino, halogen, cyano, nitro,-C(O)R^{b}_{,} -S(O)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -NR^{d1}R^{d2}, C₁-C₃-alkyl, and C₃-C₆-cycloalkyl, wherein C₁-C₃-alkyl, and C₃-C₆-cycloalkyl are optionally substituted one or more times with hydroxyl, halogen, or C₁-C₃-alkoxy;
R^{c} is selected from the group comprising, preferably consisting of, hydrogen, -C(O)R^{e}, C₁-C₃-alkyl, C₁-C₃-haloalkyl C₃-C₆-cycloalkyl, and C₃-C₇-heterocycloalkyl, wherein C₁-C₃-alkyl, C₁-C₃-haloalkyl, C₃-C₆-cycloalkyl, and C₃-C₆-heterocycloalkyl are optionally substituted one or more times with hydroxyl, halogen, aryl, -OR^{f}, or -NR^{d1}R^{d2};
R^{d1}, R^{d2} independently from each other are selected from the group comprising, preferably consisting of hydrogen, C₁-C₃-alkyl, C₃-C₆-cycloalkyl, and C₃-C₆-heterocycloalkyl, or for a group -C(O)R^{e},-S(O)₂R^{e}, or -C(O)NR^{g1}R^{g2} wherein C₁-C₃-alkyl, C₃-C₆-cycloalkyl, and C₃-C₆-heterocycloalkyl are optionally substituted one or more times, the same way or differently with halogen, hydroxy or the group -NR^{g1}R^{g2},-OR^{f}, -C(O)R^{e}, -S(O)₂R^{e}; or
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
R^{e} is selected from the group comprising, preferably consisting of,-NR^{g1}R^{g2}, C₁-C₃-alkyl, C₃-C₆-cycloalkyl, C₁-C₃-alkoxy, aryl and heteroaryl;
R^{f} is selected from the group comprising, preferably consisting of, hydrogen, -C(O)R^{e}, C₁-C₃-alkyl, C₁-C₃-haloalkyl, C₃-C₆-cycloalkyl, C₃-C₆-heterocycloalkyl, aryl, and heteroaryl, wherein C₁-C₃-alkyl, C₁-C₃- haloalkyl, C₃-C₆-cycloalkyl, C₃-C₆-heterocycloalkyl, aryl, and heteroaryl are optionally substituted one or more times with hydroxyl, halogen, C₁-C₃-alkoxy, aryl, or -NR^{g1}R^{g2};
R^{g1}, R^{g2} independently from each other are selected from the group comprising, preferably consisting of hydrogen, C₁-C₃-alkyl, C₃-C₆-cycloalkyl, C₃-C₆-heterocycloalkyl, aryl, and heteroaryl;
R^{g1} and R^{g2} together with the nitrogen atom to which they are attached, form a 3 to 6 membered heterocycloalkyl ring, whereby the carbon backbone of this heterocycloalkyl ring can optionally be interrupted once by a member of the group comprising, preferably consisting of, NH, NR^{d3}, or oxygen;
A represents C(O)NR^{a}-;
B is a bond;
D is para-phenylene ;
E is phenylene ;
and
q is 0;
wherein, when one or more of R^{a} , R^{b} , R^{c} , R^{d1} , R^{d2}, R^{d3}, R^{e}, R^{f}, R^{g1}, R^{g2}, or R⁸ is (are) present in one position in the molecule as well as in one or more further positions in the molecule, said R^{a}, R^{b} , R^{c} , R^{d1} , R^{d2}, R^{d3}, R^{e}, R^{f}, R^{g1}, R^{g2}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, R^{e}, R^{f}, R^{g1}, R^{g2}, or R⁸ within a single molecule to be identical or different.

9. The compound according to claim 1, 2, 3, 4, 7 or 8, wherein :
R¹ represents H, or C₁-C₃-alkyl, wherein C₁-C₃-alkyl is unsubstituted or substituted one or more times, independently from each other, with R⁶ ;
R² represents hydrogen;
R³ is selected from the group comprising, preferably consisting of, hydrogen, methyl, methoxy, hydroxy, and halogen ;
R⁴, R⁵ independently from each other, are selected from the group comprising, preferably consisting of, hydrogen, C₁-C₃-alkyl, C₃-C₆-cycloalkyl, C₃-C₆-heterocycloalkyl, C₁-C₃-haloalkyl, C₁-C₃-haloalkoxy, hydroxy, amino, halogen, cyano, nitro, -C(O)R^{b}, -OR^{c}, and -NR^{d1}R^{d2}, wherein C₁-C₃-alkyl and C₃-C₆-heterocycloalkyl are optionally substituted one or more times by R⁸ ;
R⁶, represents hydroxy;
R⁸ is selected from the group comprising, preferably consisting of, C₃-C₆-cycloalkyl, C₃-C₇-heterocycloalkyl, C₁-C₃-haloalkyl, C₁-C₃-haloalkoxy, hydroxy, halogen, -S(O)₂R^{b}, -OR^{c}, and -NR^{d1}R^{d2};
R^{a} is selected from the group comprising, preferably consisting of, hydrogen and methyl ;
R^{b} is selected from the group comprising, preferably consisting of, hydroxyl, -OR^{c}, -NR^{d1}R^{d2}, C₁-C₃-alkyl, and C₃-C₆-cycloalkyl;
R^{c} is selected from the group comprising, preferably consisting of, hydrogen, C₁-C₃-alkyl, C₁-C₃-haloalkyl C₃-C₆-cycloalkyl, and C₃-C₇-heterocycloalkyl;
R^{d1}, R^{d2} independently from each other are selected from the group comprising, preferably consisting of hydrogen, C₁-C₃-alkyl, C₃-C₆-cycloalkyl, and C₃-C₆-heterocycloalkyl, or for a group -C(O)R^{e}, or-S(O)₂R^{e} wherein C₁-C₃-alkyl, is optionally substituted one or more times, the same way or differently with halogen, hydroxy or C₁-C₃-alkoxy;
R^{d1} and R^{d2} together with the nitrogen atom to which they are attached, form a 3 to 7 membered heterocycloalkyl ring, which is optionally substituted one or more times, the same way or differently, with C₁-C₄-alkyl, halogen, -NR^{g1}R^{g2}, or -OR^{f}; whereby the carbon backbone of this heterocycloalkyl ring can optionally be interrupted once by a member of the group comprising, preferably consisting of, NH, NR^{d3}, or oxygen;
R^{d3} represents hydrogen or C₁-C₄-alkyl, wherein C₁-C₄-alkyl is optionally substituted once by a C₃-cycloalkyl, hydroxyl, C₁-C₄-haloalkyl, C₁-C₄-alkoxy or halogen;
R^{e} represents C₁-C₃-alkyl or C₃-C₆-cycloalkyl;
A represents C(O)NR^{a}-;
B is a bond;
D is para-phenylene ;
E is phenylene ;
and
q is 0;
wherein, when one or more of R^{a} , R^{b} , R^{e} , R^{d1} , R^{d2}, R^{d3}, or R⁸ is (are) present in one position in the molecule as well as in one or more further positions in the molecule, said R^{a}, R^{b} , R^{c} , R^{d1} , R^{d2}, R^{d3}, or R⁸ has (have), independently from each other, the same meanings as defined above in said first position in the molecule and in said second or further positions in the molecule, it being possible for the two or more occurrences of R^{a}, R^{b}, R^{c}, R^{d1}, R^{d2}, R^{d3}, or R⁸ within a single molecule to be identical or different.

10. The compound according to claim 1, 2, 3, 4, 7, 8 or 9, wherein:
R¹ represents H or C₁-C₃-alkyl wherein said C₁-C₃-alkyl is unsubstituted or substituted one or more times with R⁶ ;
R² represents hydrogen;
R³ is selected from the group comprising, preferably consisting of, hydrogen, methyl, fluorine, hydroxy and methoxy;
R⁴ is selected from the group comprising, preferably consisting of, hydrogen, C₁-C₃-alkyl, C₁-C₃-haloalkyl, halogen, and -OR^{c}, wherein C₁-C₃-alkyl is optionally substituted by R⁸ ;
R⁵ is selected from the group comprising, preferably consisting of, hydrogen, C₁-C₃-alkyl, C₁-C₃-haloalkyl, halogen, and -OR^{c};
R⁶, represents hydroxy;
R⁸ is selected from the group comprising, preferably consisting of, C₆-heterocycloalkyl, -OR^{c}, and -NR^{d1}R^{d2};
R^{a} represents hydrogen or methyl;
R^{c} represents C₁-C₃-alkyl or C₆-heterocycloalkyl;
R^{d1}, R^{d2} independently from each other are selected from the group comprising, preferably consisting of hydrogen, and C₁-C₆-alkyl, or
R^{d1} and R^{d2} together with the nitrogen atom to which they are attached, form a 6 membered heterocycloalkyl ring, whereby the carbon backbone of this heterocycloalkyl ring can optionally be interrupted by a member of the group comprising, preferably consisting of, NH, NR^{d3}, or oxygen;
R^{d3} represents hydrogen or methyl;
A represents -C(O)NR^{a}- ;
B is a bond;
D is a para-phenylene
E is phenylene;
and
q is 0;
wherein, when one or more of R^{a} , R^{c}, or R^{d3} is (are) present in one position in the molecule as well as in one or more further positions in the molecule, said R^{a}, R^{c}, or R^{d3} has (have), independently from each other, the same meanings as defined above in said first position in the molecule and in said second or further positions in the molecule, it being possible for the two or more occurrences of R^{a}, R^{c}, or R^{d3} within a single molecule to be identical or different.

11. The compound according to any one of claims 1 to 10 selected from the group consisting of :
1-[4-(1-Methyl-1H-pyrazolo[3,4-c]pyridin-4-yl)-phenyl]-3-phenyl-urea;
1-(2-Fluoro-5-trifluoromethyl-phenyl)-3-[4-(1-methyl-1H-pyrazolo[3,4-c]pyridin-4-yl)-phenyl]-urea;
1-(2-Fluoro-5-methyl-phenyl)-3-[4-(1-methyl-1H-pyrazolo[3,4-c]pyridin-4-yl)-phenyl]-urea;
1-[4-(1-Methyl-1H-pyrazolo[3,4-c]pyridin-4-yl)-phenyl]-3-(3-trifluoromethylphenyl)-urea;
1-(3-Ethyl-phenyl)-3-[4-(1-methyl-1H-pyrazolo[3,4-c]pyridin-4-yl)-phenyl]-urea;
1-(3-Ethoxy-phenyl)-3-[4-(1-methyl-1H-pyrazolo[3,4-c]pyridin-4-yl)-phenyl]-urea;
1-(4-Ethyl-pyridin-2 -yl)-3-[4-(1-methyl-1H-pyrazolo[3,4-c]pyridin-4-yl)-phenyl]-urea;
1-(2-Methoxy-5-trifluoromethyl-phenyl)-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-(3-trifluoromethyl-phenyl)-urea;
1-[2-Methyl-4-(1-methyl-1H-pyrazolo[3,4-c]pyridin-4-yl)-phenyl]-3-(3-trifluoromethyl-phenyl)-urea;
1-(2-Fluoro-5-trifluoromethyl-phenyl)-3-[2-methyl-4-(1-methyl-1H-pyrazolo[3,4-c]pyridin-4-yl)-phenyl]-urea;
1-[2-Methoxy-4-(1-methyl-1H-pyrazolo[3,4-c]pyridin-4-yl)-phenyl]-3-(3-trifluoromethyl-phenyl)-urea;
1-(2-Fluoro-5-trifluoromethyl-phenyl)-3-[2-methoxy-4-(1-methyl-1H-pyrazolo[3,4-c]pyridin-4-yl)-phenyl]-urea;
1-Methyl-1-[4-(1-methyl-1H-pyrazolo[3,4-c]pyridin-4-yl)-phenyl]-3-(3-trifluoromethyl-phenyl)-urea;
1-Methyl-3-[4-(1-methyl-1H-pyrazolo[3,4-c]pyridin-4-yl)-phenyl]-1-(3-trifluoromethyl-phenyl)-urea;
1-[2-Fluoro-4-(1-methyl-1H-pyrazolo[3,4-c]pyridin-4-yl)-phenyl]-3-(2-fluoro-5-trifluoromethyl-phenyl)-urea;
1-[4-(4-Methyl-piperazin-1-ylmethyl)-3-trifluoromethyl-phenyl]-3-[4-(1-methyl-1H-pyrazolo[3,4-c]pyridin-4-yl)-phenyl]-urea;
1-[2-Hydroxy-4-(1-methyl-1 H-pyrazolo[3,4-c]pyridin-4-yl)-phenyl]-3-(3-trifluoromethyl-phenyl)-urea;
1-{2-Fluoro-4-[1-(2-hydroxy-ethyl)-1H-pyrazolo[3,4-c]pyridin-4-yl]-phenyl}-3-(2-fluoro-5-trifluoromethyl-phenyl)-urea;
1-(2-Fluoro-5-trifluoromethyl-phenyl)-3-{4-[1-(2-hydroxy-ethyl)-1H-pyrazolo[3,4-c]pyridin-4-yl]-phenyl}-urea;
1-{4-[1-(2-Hydroxy-ethyl)-1H-pyrazolo[3,4-c]pyridin-4-yl]-phenyl}-3-(3-trifluoromethyl-phenyl)-urea;
1-(3-Ethyl-phenyl)-3-{2-fluoro-4-[1-(2-hydroxy-ethyl)-1H-pyrazolo[3,4-c]pyridin-4-yl]-phenyl}-urea;
1-(4-Ethoxy-pyridin-2-yl)-3-[4-(1-methyl-1H-pyrazolo[3,4-c]pyridin-4-yl)-phenyl]-urea;
1-[4-(1-Methyl-1H-pyrazolo[3,4-c]pyridin-4-yl)-phenyl]-3-(4-trifluoromethyl-pyridin-2-yl)-urea;
1-[4-(1-Methyl-1H-pyrazolo[3,4-c]pyridin-4-yl)-phenyl]-3-(4-methyl-3-trifluoromethyl-phenyl)-urea;
1-(4-Chloro-3-trifluoromethyl-phenyl)-3-[4-(1-methyl-1H-pyrazolo[3,4-c]pyridin-4-yl)-phenyl]-urea;
1-(2-Chloro-5-methyl-phenyl)-3-[4-(1-methyl-1H-pyrazolo[3,4-c]pyridin-4-yl)-phenyl]-urea;
1-(2-Chloro-5-trifluoromethyl-phenyl)-3-[4-(1-methyl-1H-pyrazolo[3,4-c]pyridin-4-yl)-phenyl]-urea;
1-[4-(1-Methyl-1H-pyrazolo[3,4-c]pyridin-4-yl)-phenyl]-3-(2-piperidin-1-yl-5-trifluoromethyl-phenyl)-urea;
1-[4-(1-Methyl-1H-pyrazolo[3,4-c]pyridin-4-yl)-phenyl]-3-(4-piperazin-1-ylmethyl-3-trifluoromethyl-phenyl)-urea;
1-{2-Fluoro-4-[1-(2-hydroxy-ethyl)-1H-pyrazolo[3,4-c]pyridin-4-yl]-phenyl}-3-(3-trifluoromethyl-phenyl)-urea;
1-{4-[1-(2-Hydroxy-ethyl)-1 H-pyrazolo[3,4-c]pyridin-4-yl]-phenyl}-3-[4-(4-methyl-piperazin-1-ylmethyl)-3-trifluoromethyl-phenyl]-urea;
1,3-Bis-[4-(1-methyl-1H-pyrazolo[3,4-c]pyridin-4-yl)-phenyl]-urea;
1-[4-(1-Ethyl-1H-pyrazolo[3,4-c]pyridin-4-yl)-phenyl]-3-(3-trifluoromethyl-phenyl)-urea;
1-[4-(1-Ethyl-1H-pyrazolo[3,4-c]pyridin-4-yl)-phenyl]-3-phenyl-urea;
1-(3-Ethoxy-phenyl)-3-[4-(1-ethyl-1H-pyrazolo[3,4-c]pyridin-4-yl)-phenyl]-urea;
1-[4-(1-Ethyl-1H-pyrazolo[3,4-c]pyridin-4-yl)-phenyl]-3-(2-fluoro-5-methyl-phenyl)-urea;
1-[4-(1-Ethyl-1H-pyrazolo[3,4-c]pyridin-4-yl)-phenyl]-3-(2-fluoro-5-trifluoromethylphenyl)-urea;
1-[4-(1-Ethyl-1H-pyrazolo[3,4-c]pyridin-4-yl)-phenyl]-3-m-tolyl-urea;
1-[4-(1-Ethyl-1H-pyrazolo[3,4-c]pyridin-4-yl)-phenyl]-3-(3-methoxy-phenyl)-urea;
1-(3-Ethyl-phenyl)-3-[4-(1-ethyl-1H-pyrazolo[3,4-c]pyridin-4-yl)-phenyl]-urea;
1-[4-(1-Ethyl-1H-pyrazolo[3,4-c]pyridin-4-yl)-2-fluoro-phenyl]-3-(3-trifluoromethylphenyl)-urea;
1-[4-(1-Ethyl-1H-pyrazolo[3,4-c]pyridin-4-yl)-2-fluoro-phenyl]-3-(2-fluoro-5-trifluoromethyl-phenyl)-urea;
1-[4-(1-Ethyl-1H-pyrazolo[3,4-c]pyridin-4-yl)-2-fluoro-phenyl]-3-(2-fluoro-5-methylphenyl)-urea;
1-[4-(1-Ethyl-1H-pyrazolo[3,4-c]pyridin-4-yl)-2-fluoro-phenyl]-3-m-tolyl-urea;
1-{4-[1-(2-Methoxy-ethyl)-1H-pyrazolo[3,4-c]pyridin-4-yl]-phenyl}-3-(3-trifluoromethyl-phenyl)-urea;
1-{4-[1-(2-Methoxy-ethyl)-1H-pyrazolo[3,4-c]pyridin-4-yl]-phenyl}-3-phenyl-urea;
1-(2-Fluoro-5-methyl-phenyl)-3-{4-[1-(2-methoxy-ethyl)-1H-pyrazolo[3,4-c]pyridin-4-yl]-phenyl}-urea;
1-{2-Fluoro-4-[1-(2-methoxy-ethyl)-1H-pyrazolo[3,4-c]pyridin-4-yl]-phenyl}-3-(3-trifluoromethyl-phenyl)-urea;
1-{2-Fluoro-4-[1-(2-methoxy-ethyl)-1H-pyrazolo[3,4-c]pyridin-4-yl]-phenyl}-3-(2-fluoro-5-trifluoromethyl-phenyl)-urea;
1-[4-(1-Methyl-1H-pyrazolo[3,4-c]pyridin-4-yl)-phenyl]-3-(2-methyl-pyridin-4-yl)-urea;
1-(5-tert-Butyl-isoxazol-3-yl)-3-[4-(1-methyl-1H-pyrazolo[3,4-c]pyridin-4-yl)-phenyl]-urea;
1-[4-(4-Methyl-piperazin-1-yl)-3-trifluoromethyl-phenyl]-3-[4-(1-methyl-1H-pyrazolo[3,4-c]pyridin-4-yl)-phenyl]-urea;
1-(5-tert-Butyl-2-phenyl-2H-pyrazol-3-yl)-3-[4-(1-methyl-1H-pyrazolo[3,4-c]pyridin-4-yl)-phenyl]-urea;
1-[4-(4-Methyl-piperidin-1-yl)-3-trifluoromethyl-phenyl]-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-[4-(4-methyl-piperazin-1-ylmethyl)-3-trifluoromethyl-phenyl]-urea;
1-[4-(1-Methyl-1H-pyrazolo[3,4-c]pyridin-4-yl)-phenyl]-3-(4-piperidin-1-ylmethyl-3-trifluoromethyl-phenyl)-urea;
1-[2-(4-Methyl-piperazin-1-yl)-5-trifluoromethyl-phenyl]-3-[4-(1-methyl-1H-pyrazolo[3,4-c]pyridin-4-yl)-phenyl]-urea;
1-[2-(4-Dimethylamino-piperidin-1-yl)-5-trifluoromethyl-phenyl]-3-[4-(1-methyl-1H-pyrazolo[3,4-c]pyridin-4-yl)-phenyl]-urea;
1-[4-(1-Methyl-1H-pyrazolo[3,4-c]pyridin-4-yl)-phenyl]-3-(2-pyrrolidin-1-yl-5-trifluoromethyl-phenyl)-urea;
1-(2-Dimethylamino-5-trifluoromethyl-phenyl)-3-[4-(1-methyl-1H-pyrazolo[3,4-c]pyridin-4-yl)-phenyl]-urea;
1-[2-(3-Dimethylamino-pyrrolidin-1-yl)-5-trifluoromethyl-phenyl]-3-[4-(1-methyl-1H-pyrazolo[3,4-c]pyridin-4-yl)-phenyl]-urea;
1-{2-[(2-Dimethylamino-ethyl)-methyl-amino]-5-trifluoromethyl-phenyl}-3-[4-(1-methyl-1H-pyrazolo[3,4-c]pyridin-4-yl)-phenyl]-urea;
1-{2-[(3-Dimethylamino-propyl)-methyl-amino]-5-trifluoromethyl-phenyl}-3-[4-(1-methyl-1H-pyrazolo[3,4-c]pyridin-4-yl)-phenyl]-urea;
1-[2-(3-Dimethylamino-piperidin-1-yl)-5-trifluoromethyl-phenyl]-3-[4-(1-methyl-1H-pyrazolo[3,4-c]pyridin-4-yl)-phenyl]-urea;
1-[4-(4-Methanesulfonyl-piperazin-1-ylmethyl)-3-trifluoromethyl-phenyl]-3-[4-(1-methyl-1H-pyrazolo[3,4-c]pyridin-4-yl)-phenyl]-urea;
1-[4-(3-Dimethylamino-pyrrolidin-1-ylmethyl)-3-trifluoromethyl-phenyl]-3-[4-(1-methyl-1H-pyrazolo[3,4-c]pyridin-4-yl)-phenyl]-urea;
1-[4-(1-Methyl-1H-pyrazolo[3,4-c]pyridin-4-yl)-phenyl]-3-(4-morpholin-4-ylmethyl-3-trifluoromethyl-phenyl)-urea;
1-(5-lsopropyl-2-phenyl-2H-pyrazol-3-yl)-3-[4-(1-methyl-1H-pyrazolo[3,4-c]pyridin-4-yl)-phenyl]-urea;
1-[4-(4-Methyl-3-oxo-piperazin-1-ylmethyl)-3-trifluoromethyl-phenyl]-3-[4-(1-methyl-1H-pyrazolo[3,4-c]pyridin-4-yl)-phenyl]-urea;
1-[4-(4-Methyl-[1,4]diazepan-1-ylmethyl)-3-trifluoromethyl-phenyl]-3-[4-(1-methyl-1H-pyrazolo[3,4-c]pyridin-4-yl)-phenyl]-urea;
1-(4-Cyano-3-trifluoromethyl-phenyl)-3-[4-(1-methyl-1H-pyrazolo[3,4-c]pyridin-4-yl)-phenyl]-urea;
1-(3-Methyl-isoxazol-5-yl)-3-[4-(1-methyl-1H-pyrazolo[3,4-c]pyridin-4-yl)-phenyl]-urea;
1-(5-tert-Butyl-2-methyl-2H-pyrazol-3-yl)-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-(6-methyl-pyridin-2-yl)-urea;
1-[2-Fluoro-4-(1-methyl-1H-pyrazolo[3,4-c]pyridin-4-yl)-phenyl]-3-(4-trifluoromethyl-pyridin-2-yl)-urea;
1-[4-(1-Methyl-1H-pyrazolo[3,4-c]pyridin-4-yl)-phenyl]-3-(4-trifluoromethyl-oxazol-2-yl)-urea;
1-[2-(3-Fluoro-phenyl)-5-isopropyl-2H-pyrazol-3-yl]-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-[2-(3-fluorophenyl)-5-isopropyl-2H-pyrazol-3-yl]-urea;
1-[2-Fluoro-4-(1-methyl-1H-pyrazolo[3,4-c]pyridin-4-yl)-phenyl]-3-(5-isopropyl-2-phenyl-2H-pyrazol-3-yl)-urea;
1-(5-tert-Butyl-2-phenyl-2H-pyrazol-3-yl)-3-[2-fluoro-4-(1-methyl-1H-pyrazolo[3,4-c]pyridin-4-yl)-phenyl]-urea;
1-[5-tert-Butyl-2-(3-methoxy-phenyl)-2H-pyrazol-3-yl]-3-[4-(1-methyl-1H-pyrazolo[3,4-c]pyridin-4-yl)-phenyl]-urea;
1-(5-tert-Butyl-2-p-tolyl-2H-pyrazol-3-yl)-3-[4-(1-methyl-1H-pyrazolo[3,4-c]pyridin-4-yl)-phenyl]-urea;
1-[5-tert-Butyl-2-(4-fluoro-phenyl)-2H-pyrazol-3-yl]-3-[4-(1-methyl-1H-pyrazolo[3,4-c]pyridin-4-yl)-phenyl]-urea;
1-[5-tert-Butyl-2-(4-chloro-phenyl)-2H-pyrazol-3-yl]-3-[4-(1-methyl-1H-pyrazolo[3,4-c]pyridin-4-yl)-phenyl]-urea;
1-[5-tert-Butyl-2-(4-fluoro-phenyl)-2H-pyrazol-3-yl]-3-[2-fluoro-4-(1-methyl-1H-pyrazolo[3,4-c]pyridin-4-yl)-phenyl]-urea;
1-(5-tert-Butyl-2-pyridin-4-yl-2H-pyrazol-3-yl)-3-[4-(1-methyl-1H-pyrazolo[3,4-c]pyridin-4-yl)-phenyl]-urea;
1-(5-tert-Butyl-2-p-tolyl-2H-pyrazol-3-yl)-3-[2-fluoro-4-(1-methyl-1H-pyrazolo[3,4-c]pyridin-4-yl)-phenyl]-urea;
1-[5-tert-Butyl-2-(5-fluoro-pyridin-3-yl)-2H-pyrazol-3-yl]-3-[4-(1-methyl-1H-pyrazolo[3,4-c]pyridin-4-yl)-phenyl]-urea;
1-[5-tert-Butyl-2-(5-fluoro-pyridin-3-yl)-2H-pyrazol-3-yl]-3-[2-fluoro-4-(1-methyl-1H-pyrazolo[3,4-c]pyridin-4-yl)-phenyl]-urea;
1-[5-tert-Butyl-2-(4-chloro-phenyl)-2H-pyrazol-3-yl]-3-[2-fluoro-4-(1-methyl-1H-pyrazolo[3,4-c]pyridin-4-yl)-phenyl]-urea;
1-[5-tert-Butyl-2-(3-methoxy-phenyl)-2H-pyrazol-3-yl]-3-[2-fluoro-4-(1-methyl-1H-pyrazolo[3,4-c]pyridin-4-yl)-phenyl]-urea;
1-(5-Cyclopropyl-2-phenyl-2H-pyrazol-3-yl)-3-[4-(1-methyl-1H-pyrazolo[3,4-c]pyridin-4-yl)-phenyl]-urea;
1-[4-(4-Methyl-piperazine-1-carbonyl)-3-trifluoromethyl-phenyl]-3-[4-(1-methyl-1H-pyrazolo[3,4-c]pyridin-4-yl)-phenyl]-urea;
1-[2-(5-Fluoro-pyridin-3-yl)-5-isopropyl-2H-pyrazol-3-yl]-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-[2-(5-fluoro-pyridin-3-yl)-5-isopropyl-2H-pyrazol-3-yl]-urea;
1-[5-Isopropyl-2-(6-methoxy-pyridin-3-yl)-2H-pyrazol-3-yl]-3-[4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-[5-isopropyl-2-(6-methoxy-pyridin-3-yl)-2H-pyrazol-3-yl]-urea;
1-[5-Isopropyl-2-(3-methoxy-phenyl)-2H-pyrazol-3-yl]-3-[4-(1-methyl-1H-pyrazolo[3,4-c]pyridin-4-yl)-phenyl]-urea;
1-(5-Isopropyl-2-m-tolyl-2H-pyrazol-3-yl)-3-[4-(1-methyl-1H-pyrazolo[3,4-c]pyridin-4-yl)-phenyl]-urea;
1-[2-(3-Chloro-phenyl)-5-isopropyl-2H-pyrazol-3-yl]-3-[4-(1-methyl-1H-pyrazolo[3,4-c] pyridin-4-yl)-phenyl]-urea;
1-[2-(4-Fluoro-phenyl)-5-isopropyl-2H-pyrazol-3-yl]-3-[4-(1-methyl-1H-pyrazolo[3,4-c]pyridin-4-yl)-phenyl]-urea;
1-[2-(3-Chloro-phenyl)-5-isopropyl-2H-pyrazol-3-yl]-3-[2-fluoro-4-(1-methyl-1H-pyrazolo[3,4-c]pyridin-4-yl)-phenyl]-urea;
1-[2-Fluoro-4-(1-methyl-1H-pyrazolo[3,4-c]pyridin-4-yl)-phenyl]-3-[5-isopropyl-2-(3-methoxy-phenyl)-2H-pyrazol-3-yl]-urea;
1-[2-Fluoro-4-(1-methyl-1H-pyrazolo[3,4-c]pyridin-4-yl)-phenyl]-3-[5-isopropyl-2-(4-methoxy-phenyl)-2H-pyrazol-3-yl]-urea;
1-[2-Fluoro-4-(1-methyl-1H-pyrazolo[3,4-c]pyridin-4-yl)-phenyl]-3-(5-isopropyl-2-m-tolyl-2H-pyrazol-3-yl)-urea;
1-[2-Fluoro-4-(1-methyl-1H-pyrazolo[3,4-c]pyridin-4-yl)-phenyl]-3-[2-(4-fluorophenyl)-5-isopropyl-2H-pyrazol-3-yl]-urea;
1-(5-Cyclopropyl-2-phenyl-2H-pyrazol-3-yl)-3-[2-fluoro-4-(1-methyl-1H-pyrazolo[3,4-c]pyridin-4-yl)-phenyl]-urea; and
1-{4-[1-(2-Methanesulfonyl-ethyl)-1H-pyrazolo[3,4-c]pyridin-4-yl]-phenyl}-3-(3-trifluoromethyl-phenyl)-urea.

12. A method of preparing a compound of general formula (I) according to any one of claims 1 to 11, said method comprising the step of allowing an intermediate compound of general formula 11 : 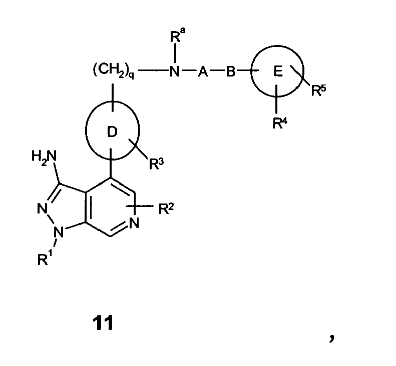 in which A, B, D, E, R^{a}, R¹, R², R³, R⁴, R⁵ and q are as defined in any one of claims 1 to 11,
to undergo a deamination, via a diazotization with an appropriate diazotizing agent, such as NaNO₂, and a subsequent de-diazotization, with an acid, such as sulphuric or hydrochloric acid, thus providing a compound of general formula (I) : 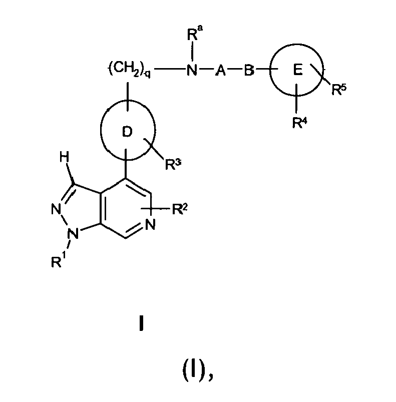 in which A, B, D, E, R^{a}, R¹, R², R³, R⁴, R⁵ and q are as defined in any one of claims 1 to 11.

13. A method of preparing a compound of general formula (I) according to any one of claims 1 to 11, said method comprising the step of allowing an intermediate compound of general formula 1 : 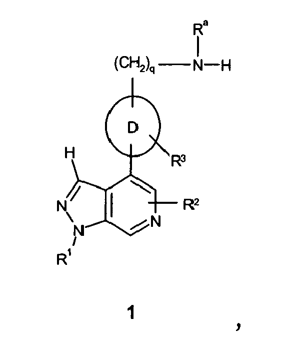 in which D, R^{a}, R¹, R², R³, and q are as defined in any one of claims 1 to 11, to undergo a reaction with an isocyanate of formula (la') : 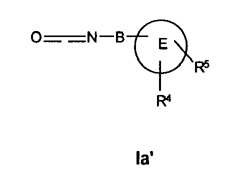 in which B, E, R⁴, and R⁵ are as defined in any one of claims 1 to 11, thus providing a compound of general formula (Ia) : 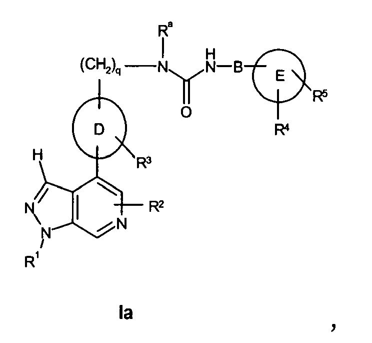 in which B, D, E, R^{a}, R¹, R², R³, R⁴, R⁵ and q are as defined in any one of claims 1 to 11.

14. A method of preparing a compound of general formula (I) according to any one of claims 1 to 11, said method comprising the step of allowing an intermediate compound of general formula 1: 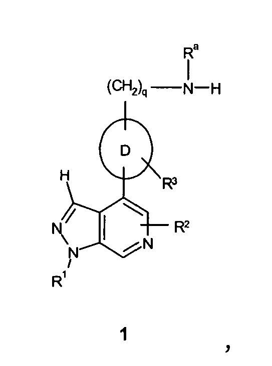 in which D, R^{a}, R¹, R², R³, and q are as defined in any one of claims 1 to 11, to react with a compound of general formula 2 : 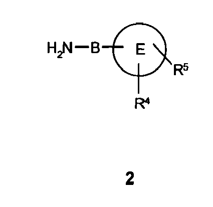 in which B, E, R⁴, and R⁵ are defined as in any one of claims 1 to 11 ;
in the presence of a phosgene equivalent, such as triphosgene, thus providing a compound of general formula (Ia) : 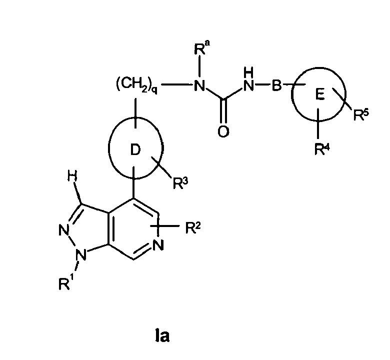 in which B, D, E, R^{a}, R¹, R², R³, R⁴, R⁵ and q are as defined in any one of claims 1 to 11.

15. A method of preparing a compound of general formula (I) according to any one of claims 1 to 11, said method comprising the step of allowing an intermediate compound of general formula 12: 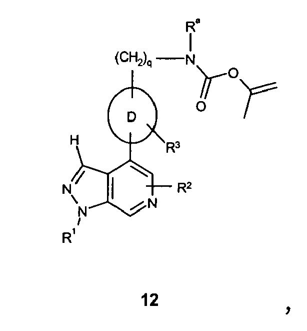 in which D, R^{a}, R¹, R², R³, and q are as defined in any one of claims 1 to 11, to react with a compound of general formula 2 : 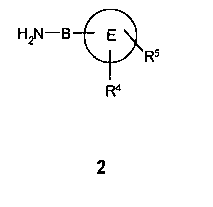 in which B, E, R⁴, and R⁵ are defined as in any one of claims 1 to 11 ;
preferably in the presence of a base, such as N-methyl pyrrolidine, thus providing a compound of general formula (Ia) : 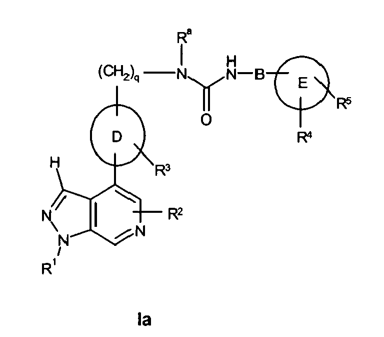 in which B, D, E, R^{a}, R¹, R², R³, R⁴, R⁵ and q are as defined in any one of claims 1 to 11.

16. A method of preparing a compound of general formula (I) according to any one of claims 1 to 11, said method comprising the step of allowing an intermediate compound of general formula 1: 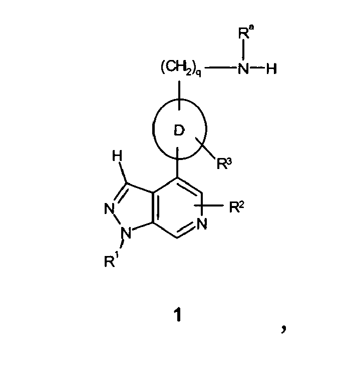 in which D, R^{a}, R¹, R², R³, and q are as defined in any one of claims 1 to 11, to react with a compound of general formula 13 : 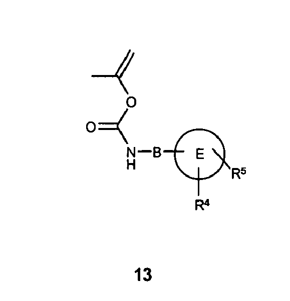 in which B, E, R⁴, and R⁵ are defined as in any one of claims 1 to 11 ;
preferably in the presence of a base, such as N-methyl pyrrolidine, thus providing a compound of general formula (Ia) : 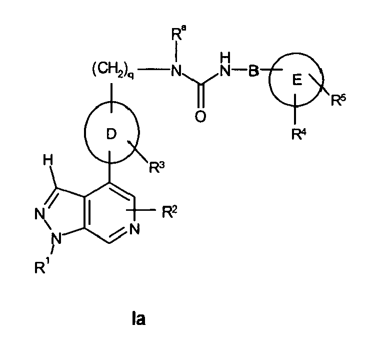 in which B, D, E, R^{a}, R¹, R², R³, R⁴, R⁵ and q are as defined in any one of claims 1 to 11.

17. A method of preparing a compound of general formula (I) according to any one of claims 1 to 11, said method comprising the step of allowing an intermediate compound of general formula 14: 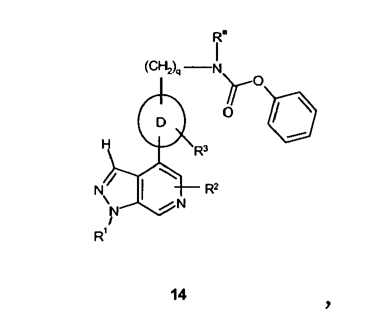 in which D, R^{a}, R¹, R², R³, and q are as defined in any one of claims 1 to 11,
to react with a compound of general formula 2 : 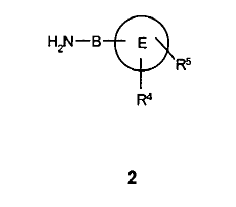 in which B, E, R⁴, and R⁵ are defined as in any one of claims 1 to 11 ;
preferably in the presence of a base, such as pyridine, thus providing a compound of general formula (Ia) : 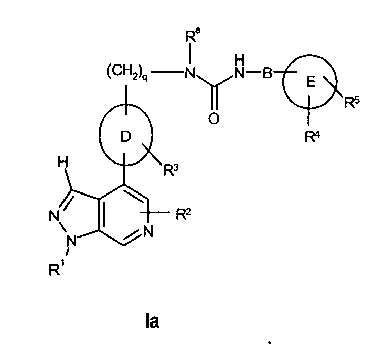 in which B, D, E, R^{a}, R¹, R², R³, R⁴, R⁵ and q are as defined in any one of claims 1 to 11.

18. A method of preparing a compound of general formula (I) according to any one of claims 1 to 11, said method comprising the step of allowing an intermediate compound of general formula 1: 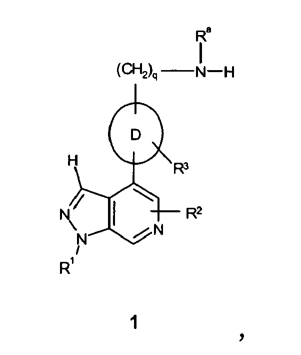 in which D, R^{a}, R¹, R², R³, and q are as defined in any one of claims 1 to 11,
to react with a compound of general formula 15 : 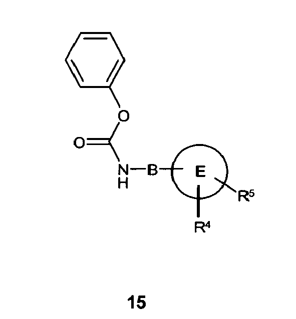 in which B, E, R⁴, and R⁵ are defined as in any one of claims 1 to 11 ;
preferably in the presence of a base, such as pyridine, thus providing a compound of general formula (Ia) : 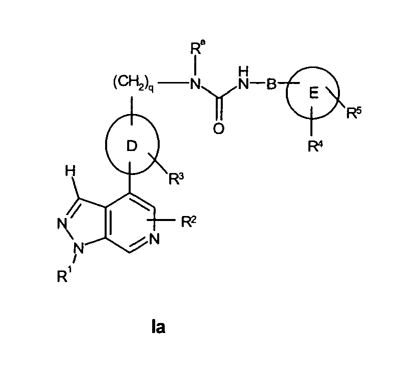 in which B, D, E, R^{a}, R¹, R², R³, R⁴, R⁵ and q are as defined in any one of claims 1 to 11.

19. A method of preparing a compound of general formula (I) according to any one of claims 1 to 11, said method comprising the step of allowing an intermediate compound of general formula 3 : 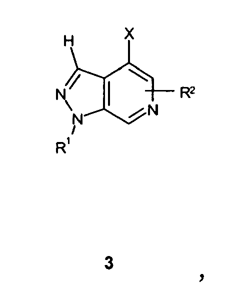 in which R¹ and R² are as defined in any one of claims 1 to 11, and X is Cl, Br, or I,
to react in a coupling reaction with a compound of general formula 5 : 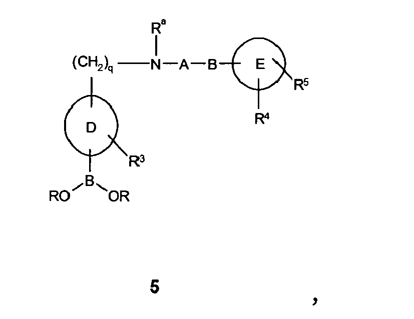 in which A, B, D, E, R^{a}, R³, R⁴, R⁵ and q are as defined in any one of claims 1 to 11, and R is H or alkyl ;
thus providing a compound of general formula (I) : 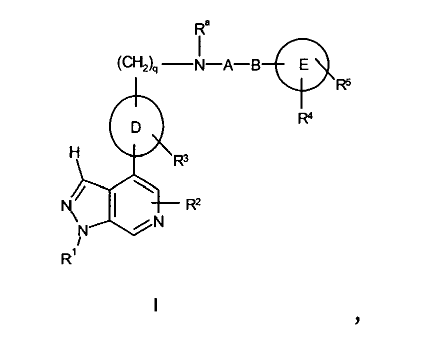 in which A, B, D, E, R^{a}, R¹, R², R³, R⁴, R⁵ and q are as defined in any one of claims 1 to 11.

20. A method of preparing a compound of general formula (I) according to any one of claims 1 to 11, said method comprising the step of allowing an intermediate compound of general formula Ib : 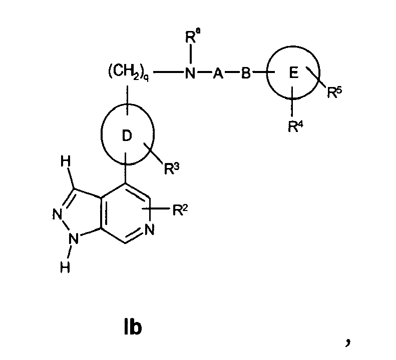 in which A, B, D, E, R^{a}, R², R³, R⁴, R⁵ and q are as defined in any one of claims 1 to 11;
to undergo a reaction with a reagent of formula 12,
R¹-X 12,
in which R¹ is as defined in any one of claims 1 to 11 and X represents a leaving group such as Cl, Br or I;
thus providing a compound of general formula (I') : 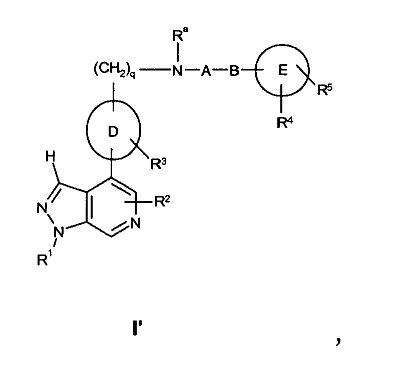 in which A, B, D, E, R^{a}, R¹, R², R³, R⁴, R⁵ and q are as defined in any one of claims 1 to 11, with the proviso that R¹ is not H.

21. A pharmaceutical composition which comprises a compound of general formula (I) according to any one of claims 1 to 11, or as obtainable by a method according to any one of claims 12 to 20, or a pharmaceutically acceptable salt or an N-oxide or a solvate or a prodrug of said compound, and a pharmaceutically acceptable diluent or carrier.

22. Use of a compound of any one of claims 1 to 11 for manufacturing a pharmaceutical composition for the treatment of diseases of dysregulated vascular growth or of diseases which are accompanied with dysregulated vascular growth.

23. Use according to claim 22, wherein said diseases are tumors and/or metastases thereof.

24. Use according to claim 22, wherein said diseases are selected from the group chronic myelogeneous leukaemia, acute myelogenous leukaemia, acute lymphatic leukaemia, acute lymphocytic leukaemia, chronic lymphocytic leukaemia, chronic lymphatic leukaemia as well as other myeloid precursor hyperplasias such as polycythemia vera and myelofibrosis.

25. Use according to claim 22, wherein said diseases are retinopathy, other angiogenesis dependent diseases of the eye, in particular cornea transplant rejection or age-related macular degeneration.

26. Use according to claim 22, wherein said diseases are rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and inflammatory diseases of the bowel, such as, Crohn's disease.

27. Use according to claim 22, wherein said diseases are coronary and peripheral artery disease and for the suppression of atherosclerotic plaque formation.

28. Use according to claim 22, wherein said diseases are diseases associated with stromal proliferation or **characterized by** pathological stromal reactions diseases associated with deposition of fibrin or extracellular matrix, such as, fibrosis, cirrhosis, carpal tunnel syndrome.

29. Use according to claim 22, wherein said diseases are gynaecological diseases where inhibition of angiogenic, inflammatory and stromal processes with pathological character can be inhibited, such as, endometriosis, pre-eclampsia, postmenopausal bleeding and ovarian hyperstimulation.

30. Use according to claim 22, wherein said diseases are ascites, oedema such as brain tumor associated oedema, high altitude trauma, hypoxia induced cerebral oedema, pulmonary oedema and macular oedema or oedema following burns and trauma, chronic lung disease, adult respiratory distress syndrome, bone resorption and benign proliferating diseases such as myoma, benign prostate hyperplasia.

31. A use according to claim 22 is in supporting wound healing, particularly for the reduction of scar formation, and for the reduction of scar formation during regeneration of damaged nerves.

32. A method of treating a disease of dysregulated vascular growth or diseases which are accompanied with dysregulated vascular growth by administering an effective amount of a compound of general formula (I) according to any one of claims 1 to 11.

33. The method according to claim 32, wherein said disease are tumors and/or metastases thereof.

34. The method according to claim 32, wherein said diseases are selected from chronic myelogeneous leukaemia, acute myelogenous leukaemia, acute lymphatic leukaemia, acute lymphocytic leukaemia, chronic lymphocytic leukaemia, chronic lymphatic leukaemia as well as other myeloid precursor hyperplasias such as polycythemia vera and myelofibrosis.

35. The method according to claim 32, wherein said disease are retinopathy, other angiogenesis dependent diseases of the eye, in particular cornea transplant rejection or age-related macular degeneration.

36. The method according to claim 32, wherein said disease are rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and inflammatory diseases of the bowel, such as, Crohn's disease.

37. The method according to claim 32, wherein said disease are coronary and peripheral artery disease and for the suppression of atherosclerotic plaque formation.

38. The method according to claim 32, wherein said disease are diseases associated with stromal proliferation or **characterized by** pathological stromal reactions diseases associated with deposition of fibrin or extracellular matrix, such as, fibrosis, cirrhosis, carpal tunnel syndrome.

39. The method according to claim 32, wherein said disease are gynaecological diseases where inhibition of angiogenic, inflammatory and stromal processes with pathological character can be inhibited, such as, endometriosis, pre-eclampsia, postmenopausal bleeding and ovarian hyperstimulation.

40. The method according to claim 32, wherein said disease are ascites, oedema such as brain tumor associated oedema, high altitude trauma, hypoxia induced cerebral oedema, pulmonary oedema and macular oedema or oedema following burns and trauma, chronic lung disease, adult respiratory distress syndrome, bone resorption and benign proliferating diseases such as myoma, benign prostate hyperplasia.

41. The method according to claim 32 for supporting wound healing, particularly for the reduction of scar formation, and for the reduction of scar formation during regeneration of damaged nerves.

42. A compound of general formula 1 : 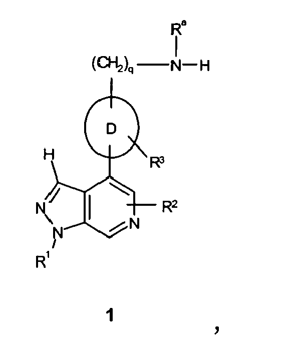 in which D, R^{a}, R¹, R², R³, and q are as defined in any one of claims 1 to 11.

43. A compound of general formula 12 : 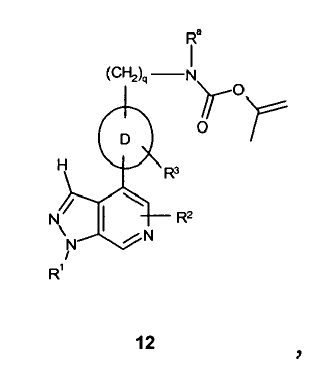 in which D, R^{a}, R¹, R², R³, and q are as defined in any one of claims 1 to 11.

44. A compound of general formula 14 : 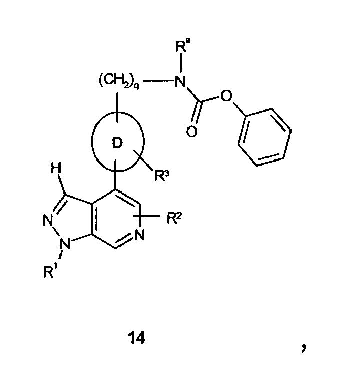 in which D, R^{a}, R¹, R², R³, and q are as defined in any one of claims 1 to 11.

45. Use of a compound of general formula 1 according to claims 13, 14, 16, 18 and 42 for the preparation of a compound of general formula (I) according to any one of claims 1 to 11.

46. Use of a compound of general formula 3 according to claim 19 for the preparation of a compound of general formula (I) according to any one of claims 1 to 11.

47. Use of a compound of general formula 11 according to claim 12 for the preparation of a compound of general formula (I) according to any one of claims 1 to 11.

48. Use of a compound of general formula 12 according to claims 15 and 43 for the preparation of a compound of general formula (I) according to any one of claims 1 to 11.

49. Use of a compound of general formula 14 according to claims 17 and 44 for the preparation of a compound of general formula (I) according to any one of claims 1 to 11.
